# EUROPEAN PATENT APPLICATION

(11) **EP 3 967 682 A1**
(43) Date of publication of application: **16.03.2022**
(21) Application number: 20801656.8
(22) Date of filing: 09.05.2020
(51) Int. Cl.: C07D 231/06, A61K 31/4155, A61P 35/00, A61P 37/00

(54) **BISHETEROCYCLIC CARBONYL SUBSTITUTED DIHYDROPYRAZOLE COMPOUND, PREPARATION METHOD THEREFOR AND PHARMACEUTICAL USE THEREOF**

(30) Priority: 09.05.2019 CN 201910383195
(71) Applicant: Genfleet Therapeutics (Shanghai) Inc., Shanghai 201203 (CN); Zhejiang Genfleet Therapeutics Co., Ltd., Shaoxing Binhai New City, Zhejiang 312000 (CN)
(72) Inventor: ZHOU, Fusheng, Shanghai 201203 (CN); XU, Xiaoming, Shanghai 201203 (CN); ZHANG, Leitao, Shanghai 201203 (CN); JIANG, Tao, Shanghai 201203 (CN); LIU, Yingtao, Shanghai 201203 (CN); LAN, Jiong, Shanghai 201203 (CN)
(74) Representative: Abel & Imray LLP
(86) International application number: PCT/CN2020/089328
(87) International publication number: WO 2020/224656

(57) **Abstract**

Provided are a substituted dihydropyrazole compound as shown in formula I, which compound has a selective inhibitory effect on RIPK1, and a pharmaceutically acceptable salt, a stereoisomer, a solvate or a prodrug thereof, wherein the definition of each group in the formula is detailed in the description. In addition, also disclosed are a pharmaceutical composition containing the compound, and the use thereof in the preparation of a drug for treating RIPK1-related diseases or conditions.

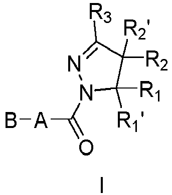

## Description

### Technical Field

The present invention relates to the technical field of medicine, in particular, to a substituted dihydropyrazole compound, and its use as a RIPK1 inhibitor as well as a pharmaceutical composition prepared therefrom.

### Background of the Invention

Receptor interacting protein 1 (RIP1) kinase is a serine/threonine protein kinase of the TKL family involved in innate immune signaling. RIP1 kinase is a protein containing RHIM domain with an N-terminal kinase domain and a C-terminal death domain. The RIP1 death domain mediates an intereaction with another protein containing a death domain, including Fas and TNFR-1, TRAIL-R1 and TRAIL-R2 and TRADD, while the RHIM domain is critical for binding to another protein containing a RHIM domain, such as, TRIF, DAI and RIP3. A variety of effects are achieved through these intereactions.

The effect of RIP1 on cell signaling has been evaluated under virious conditions [including TLR3, TLR4, TRAIL, FAS], but is best interpreted in a signaling mediated downstream the death receptor TNFR1. TNFR adaption is achieved through TNF, resulting in oligomerization, which recruits various proteins, including linear K63 linked polyubiquitinated RIP1, TRAF2/5, TRADD and cIAPs, to the cytoplasmic tail of the receptor. A RIP1-dependent complex acting as a scaffold protein (i.e., non-kinase-dependent) is called complex I, which provides a platform for pro-survival signaling pathway by activating the NFκB and MAP kinase pathways. Moreover, in a condition promoting the RIP1 deubiquitination, TNF binding to its receptor (inhibited by, such as, A20 and CYLD proteins or cIAP) will result in internalization of the receptor and formation of a complex II or DISC (death-inducing signaling complex). Formation of DISC (including RIP1, TRADD, FADD and Caspase 8) results in activation of Caspase 8, and starts a pogrammed apoptosis cell death in a non-RIP1 kinase-dependent manner. Apoptosis is a static form of cell death to a great extent, which is involved in routine processes such as development and cell homeostasis.

In a condition under which DISC is formed and RIP3 is expressed, but apoptosis is inhibited (such as, deleting FADD/Caspase 8, inhibiting Caspase or infected by virus), it is possible that there is a third RIP1 kinase dependency. At present, RIP3 can enter such a complex, and is phosphorylated through RIP1, and starts a Caspase-independent programmed necrosis apoptosis through activation of MLKL and PGAM5. In contrast to apoptosis, programmed necrosis (not to be confused with non-programmed passive necrosis) results in release of a risk-associated molecular pattern (DAMP) from the cell. These DAMP is capable of provding "risk signal" to surrounding cells and tissues, triggering a pro-inflammatory response including inflammasome activation, cytokine production, and cell recruitment.

Abnormal regulation of programmed cell death mediated by RIP1 kinase has been demonstrated to be associated with various types of inflammation by using RIP3 gene knockout mice (in which the RIP1-mediated programmed necrosis is completely blocked) and Necrostin-1 (a tool inhibitor of RIP1 kinase activity with poor oral bioavailability). RIP3 knockout mice have been shown to be protective against inflammatory bowel diseases (including ulcerative colitis and Crohn's disease), psoriasis, retinal detachment induced photoreceptor necrosis, retinitis pigmentosa, bombesin induced acute pancreatitis and sepsis/systemic inflammatory response syndrome. Necrostin-1 has been shown to be effective in alleviating ischemic brain injury, retinal ischemia/reperfusion injury, Huntington's disease, renal ischemia reperfusion injury, cisplatin-induced renal injury and traumatic brain injury. Other diseases or conditions regulated at least in part by RIP1-dependent apoptosis, necrosis or cytokine production include malignancies of the blood and solid organs, bacterial and viral infections (including but not limited to tuberculosis and influenza), and lysosomal storage disorders (especially, Gaucher disease).

Provided is an effective and selective small molecule RIP1 kinase activity inhibitor, which is capable of blocking RIP1-dependent cell necrosis, and thereby providing therapeutic effect for a disease or event associated with DAMP, cell death, inflammation.

### Summary of the Invention

The present invention provides a substituted dihydropyrazole compound, as a RIPK1 inhibitor, which is advantageous for its high activity, high selectivity and low toxic/side effect and the like.

In one respect, the present invention provides a bisheterocyclic carbonyl-substituted dihydropyrazole compound, or a pharmaceutically acceptable salt, stereoisomer, solvate or prodrug thereof, the compound has a structure as represented by formula (I): 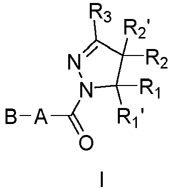 wherein,
R₁ is hydrogen, substituted or unsubstituted C₆₋₁₀ aryl, substituted or unsubstituted C₅₋₁₀ heteroaryl, substituted or unsubstituted C₃₋₆ monocyclic cycloalkyl, or substituted or unsubstituted C₃₋₆ monocyclic heterocyclyl; the "substituted" means that 1, 2 or 3 hydrogen atom(s) in the group are each replaced by a substituent independently selected from the group S1, the substituent of the group S1 is selected from the group consisting of: cyano, acetyl, hydroxy, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy), wherein the C₁₋₁₀ alkyl and C₁₋₁₀ alkoxy are unsubstituted, or substituted by 1, 2 or 3 substituent(s) each independently selected from the group S11, the substituent of the group S11 is selected from the group consisting of: acetyl, hydroxy, cyano, carboxyl, halo, C₁₋₆ alkyl, halo C₁₋₆ alkyl, C₁₋₆ alkoxy, halo C₁₋₆ alkoxy, C₃₋₆ monocyclic cycloalkyl, C₃₋₆ monocyclic heterocyclyl, phenyl, 5- or 6-membered monoheteroaryl, NRⁱR^{j}, - C(O)NRⁱR^{j}, -SO₂NRⁱR^{j}; Rⁱ and R^{j} are each independently hydrogen or C₁₋₃ alkyl;
R₁' is hydrogen, cyano, hydroxy, cyanomethyl, cyanoethyl, hydroxymethyl, hydroxyethyl, carboxyl, halo, C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy) or halo C₁₋₁₀ alkoxy (preferably, halo C₁₋₆ alkoxy, more preferably, halo C₁₋₃ alkoxy);
R₂ and R₂' are each independently hydrogen, cyano, hydroxy, cyanomethyl, cyanoethyl, hydroxymethyl, hydroxyethyl, carboxyl, halo, C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy) or C₃₋₆ monocyclic cycloalkyl;
or R₂ and R₂' together with the carbon atom attached thereto form a 3- to 6-membered saturated or partially unsaturated monocycle or a 3- to 6-membered saturated or partially unsaturated monoheterocycle;
R₃ is hydrogen, cyano, hydroxy, cyanomethyl, cyanoethyl, hydroxymethyl, hydroxyethyl, carboxyl, halo, C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy) or C₃₋₆ monocyclic cycloalkyl;
A has a structure as represented by formula (A), formula (B), formula (C) or formula (D): 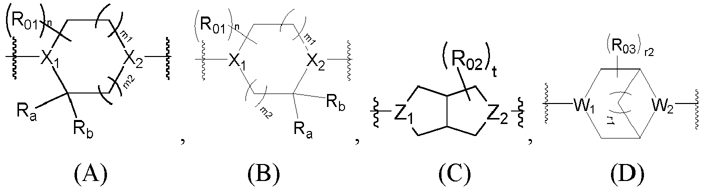 wherein X₁ is N or CR₄; X₂ is N or CR₅;
R₄ and R₅ are each independently hydrogen, hydroxy, cyano, hydroxymethyl, cyanomethyl or C₁₋₁₀ alkyl (preferably, C₁₋₆ alkyl, more preferably, C₁₋₃ alkyl);
m1 and m2 are each independently 0, 1 or 2;
(R₀₁)ₙ represents that the hydrogen(s) on the ring are replaced by n of R₀₁, n is 0, 1, 2, 3, 4, 5 or 6, each of R₀₁ are the same or different, and are independently cyano, acetyl, hydroxyl, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), halo C₁₋₁₀ alkyl (preferably, halo C₁₋₆ alkyl, more preferably, halo C₁₋₃ alkyl) or halo C₁₋₁₀ alkoxy (preferably, halo C₁₋₆ alkoxy, more preferably, halo C₁₋₃ alkoxy);
or Rₐ and R_{b} together with the carbon atom attached thereto form a 3- to 6-membered saturated or partially unsaturated monocycle or a 3- to 6-membered saturated or partially unsaturated monoheterocycle; the 3- to 6-membered saturated or partially unsaturated monocycle, 3- to 6-membered saturated or partially unsaturated monoheterocycle are unsubstituted, or substituted by 1, 2 or 3 substituent(s) independently selected from the group S2, the substituent of the group S2 is selected from the group consisting of: cyano, acetyl, hydroxy, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy), C₃₋₆ monocyclic cycloalkyl, C₃₋₆ monocyclic heterocyclyl, phenyl, 5- or 6-membered monoheteroaryl, NR^{e}R^{f}, -C(O)NR^{e}R^{f}, -SO₂NR^{e}R^{f}, wherein the C₃₋₆ monocyclic cycloalkyl, C₃₋₆ monocyclic heterocyclyl, phenyl, 5- or 6-membered monoheteroaryl, C₁₋₁₀ alkyl and C₁₋₁₀ alkoxy are unsubstituted, or substituted by 1, 2 or 3 substituent(s) each independently selected from the group S21, the substituent of the group S21 is selected from the group consisting of: acetyl, hydroxyl, cyano, carboxyl, halo, C₁₋₆ alkyl, halo C₁₋₆ alkyl, C₁₋₆ alkoxy, halo C₁₋₆ alkoxy, C₃₋₆ monocyclic cycloalkyl, C₃₋₆ monocyclic heterocyclyl, phenyl, 5- or 6-membered monoheteroaryl, NR^{g}R^{h}, - C(O)NR^{g}R^{h}, -SO₂NR^{g}R^{h}; R^{e}, R^{f}, R^{g} and R^{h} are each independently hydrogen, hydroxyethyl, hydroxymethyl or C₁₋₃ alkyl;
Z₁ is N or CR₆; Z₂ is N or CR₇;
R₆ and R₇ are each independently hydrogen, hydroxy, cyano, hydroxymethyl, cyanomethyl or C₁₋₁₀ alkyl (preferably, C₁₋₆ alkyl, more preferably, C₁₋₃ alkyl);
(R₀₂)ₜ represents that the hydrogen(s) on the ring are replaced by t of R₀₂, n is 0, 1, 2, 3, 4, 5 or 6, each of R₀₂ are the same or different, and are independently cyano, acetyl, hydroxyl, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), halo C₁₋₁₀ alkyl (preferably, halo C₁₋₆ alkyl, more preferably, halo C₁₋₃ alkyl) or halo C₁₋₁₀ alkoxy (preferably, halo C₁₋₆ alkoxy, more preferably, halo C₁₋₃ alkoxy);
W₁ is N or CR₈; W₂ is N or CR₉;
R₈ and R₉ are each independently hydrogen, hydroxy, cyano, hydroxymethyl, cyanomethyl or C₁₋₁₀ alkyl (preferably, C₁₋₆ alkyl, more preferably, C₁₋₃ alkyl);
(R₀₃)ᵣ₂ represents that the hydrogen(s) on the ring are replaced by r2 of R₀₃, r2 is 0, 1, 2, 3, 4, 5 or 6, each of R₀₃ are the same or different, and are independently cyano, acetyl, hydroxyl, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), halo C₁₋₁₀ alkyl (preferably, halo C₁₋₆ alkyl, more preferably, halo C₁₋₃ alkyl) or halo C₁₋₁₀ alkoxy (preferably, halo C₁₋₆ alkoxy, more preferably, halo C₁₋₃ alkoxy);
r1 is 0, 1, 2 or 3;
B is a substituted or unsubstituted C₆₋₁₀ aryl, a substituted or unsubstituted C₅₋₁₀ heteroaryl, a substituted or unsubstituted C₃₋₆ monocyclic heterocyclyl; the "substituted" means that 1, 2 or 3 hydrogen atom(s) in the group are replaced by a substituent independently selected from the group S3, the substituent of the group S3 is selected from the group consisting of: cyano, acetyl, hydroxy, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), C₁₋₁₀ alkylthio (preferably, C₁₋₆ alkylthio, more preferably, C₁₋₃ alkylthio), halo C₁₋₁₀ alkoxy (preferably, halo C₁₋₆ alkoxy, more preferably, halo C₁₋₃ alkoxy), NR^{a}R^{b}, -CONR^{a}R^{b}, -CONR^{a}NR^{a}R^{b}, -NR^{a}COC₁₋₁₀ alkyl (preferably, -NR^{a}COC₁₋₆ alkyl, more preferably, - NR^{a}COC₁₋₃ alkyl), -CO₂C₁₋₁₀ alkyl (preferably, -CO₂C₁₋₆ alkyl, more preferably, - CO₂C₁₋₃ alkyl), -SO₂NR^{a}R^{b}, -SO₂C₁₋₁₀ alkyl (preferably, -SO₂C₁₋₆ alkyl, more preferably, -SO₂C₁₋₃ alkyl), -CO-C₃₋₆ monocyclic heterocyclyl, -(CH₂)ᵤ-C₃₋₆ monocyclic cycloalkyl, -(CH₂)ᵤ-C₆₋₁₀ aryl (preferably, phenyl), -(CH₂)ᵤ-5- or 6-membered monoheteroaryl, -(CH₂)ᵤ-C₃₋₆ monocyclic heterocyclyl;
wherein, among the substitutes of the group S3, the C₁₋₁₀ alkyl, C₁₋₁₀ alkoxy, C₃₋₆ monocyclic cycloalkyl, C₃₋₆ monocyclic heterocyclyl, C₆₋₁₀ aryl, 5- or 6-membered monoheteroaryl are unsubstituted, or substituted by 1, 2 or 3 substituent(s) each independently selected from the group S31, the substituent of the group S31 is selected from the group consisting of: acetyl, hydroxy, cyano, carboxyl, nitro, halo, C₁₋₃ alkyl, halo C₁₋₃ alkyl, C₁₋₃ alkoxy, halo C₁₋₃ alkoxy, C₃₋₆ monocyclic cycloalkyl, C₃₋₆ monocyclic heterocyclyl, phenyl, 5- or 6-membered monoheteroaryl, NR^{c}R^{d}, - C(O)NR^{c}R^{d} , -SO₂NR^{c}R^{d};
R^{a}, R^{b}, R^{c}, R^{d} are each independently hydrogen, hydroxymethyl, hydroxyethyl, C₁₋₃ alkyl, C₃₋₆ monocyclic cycloalkyl or C₃₋₆ monocyclic heterocyclyl; wherein the C₃₋₆ monocyclic cycloalkyl is selected from the group consisting of: cyclopropyl, cyclobutyl, cyclopentyl and cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran; and the C₃₋₆ monocyclic heterocyclyl is optionally substituted by 1, 2 or 3 C₁₋₃ alkyl, acetyl;
u is 0, 1, 2, 3 or 4.

In another respect, the present invention provides a bisheterocyclic carbonyl-substituted dihydropyrazole compoud or a pharmaceutically acceptable salt, stereoisomer, solvate or prodrug thereof, the compound has a structure as represented by formula (I-1): 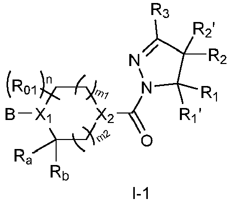 wherein,
R₁ is hydrogen, substituted or unsubstituted C₆₋₁₀ aryl, substituted or unsubstituted C₅₋₁₀ heteroaryl, substituted or unsubstituted C₃₋₆ monocyclic cycloalkyl, or substituted or unsubstituted C₃₋₆ monocyclic heterocyclyl; the "substituted" means that 1, 2 or 3 hydrogen atom(s) in the group are replaced by a substituent independently selected from the group S1, the substituent of the group S1 is selected from the group consisting of: cyano, acetyl, hydroxy, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy), wherein the C₁₋₁₀ alkyl and C₁₋₁₀ alkoxy are unsubstituted, or substituted by 1, 2 or 3 substituent(s) each independently selected from the group S11, the substituent of the group S11 is selected from the group consisting of: acetyl, hydroxy, cyano, carboxyl, halo, C₁₋₆ alkyl, halo C₁₋₆ alkyl, C₁₋₆ alkoxy, halo C₁₋₆ alkoxy, C₃₋₆ monocyclic cycloalkyl, C₃₋₆ monocyclic heterocyclyl, phenyl, 5- or 6-membered monoheteroaryl, NRⁱR^{j}, - C(O)NRⁱR^{j}, -SO₂NRⁱR^{j}; Rⁱ and R^{j} are each independently hydrogen or C₁₋₃ alkyl;
R₁' is hydrogen, cyano, hydroxy, cyanomethyl, cyanoethyl, hydroxymethyl, hydroxyethyl, carboxyl, halo, C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy) or halo C₁₋₁₀ alkoxy (preferably, halo C₁₋₆ alkoxy, more preferably, halo C₁₋₃ alkoxy);
R₂ and R₂' are each independently hydrogen, cyano, hydroxy, cyanomethyl, cyanoethyl, hydroxymethyl, hydroxyethyl, carboxyl, halo, C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy) or C₃₋₆ monocyclic cycloalkyl;
or R₂ and R₂' together with the carbon atom attached thereto form a 3- to 6-membered saturated or partially unsaturated monocycle or a 3- to 6-membered saturated or partially unsaturated monoheterocycle;
R₃ is hydrogen, cyano, hydroxy, cyanomethyl, cyanoethyl, hydroxymethyl, hydroxyethyl, carboxyl, halo, C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy) or C₃₋₆ monocyclic cycloalkyl;
X₁ is N or CR₄; X₂ is N or CR₅;
R₄ and R₅ are each independently hydrogen, hydroxy, cyano, hydroxymethyl, cyanomethyl or C₁₋₁₀ alkyl (preferably, C₁₋₆ alkyl, more preferably, C₁₋₃ alkyl);
m1 and m2 are each independently 0, 1 or 2;
(R₀₁)ₙ represents that the hydrogen(s) on the ring are replaced by n of R₀₁, n is 0, 1, 2, 3, 4, 5 or 6, each of R₀₁ are the same or different, and are independently cyano, acetyl, hydroxyl, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), halo C₁₋₁₀ alkyl (preferably, halo C₁₋₆ alkyl, more preferably, halo C₁₋₃ alkyl) or halo C₁₋₁₀ alkoxy (preferably, halo C₁₋₆ alkoxy, more preferably, halo C₁₋₃ alkoxy);
Rₐ and R_{b} together with the carbon atom attached thereto form a 3- to 6-membered saturated or partially unsaturated monocycle or a 3- to 6-membered saturated or partially unsaturated monoheterocycle; the 3- to 6-membered saturated or partially unsaturated monocycle, 3- to 6-membered saturated or partially unsaturated monoheterocycle are unsubstituted, or substituted by 1, 2 or 3 substituent(s) independently selected from the group S2, the substituent of the group S2 is selected from the group consisting of: cyano, acetyl, hydroxy, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy), C₃₋₆ monocyclic cycloalkyl, C₃₋₆ monocyclic heterocyclyl, phenyl, 5- or 6-membered monoheteroaryl, NR^{e}R^{f}, -C(O)NR^{e}R^{f}, -SO₂NR^{e}R^{f}, wherein the C₃₋₆ monocyclic cycloalkyl, C₃₋₆ monocyclic heterocyclyl, phenyl, 5- or 6-membered monoheteroaryl, C₁₋₁₀ alkyl and C₁₋₁₀ alkoxy are unsubstituted, or substituted by 1, 2 or 3 substituent(s) each independently selected from the group S21, the substituent of the group S21 is selected from the group consisting of: acetyl, hydroxyl, cyano, carboxyl, halo, C₁₋₆ alkyl, halo C₁₋₆ alkyl, C₁₋₆ alkoxy, halo C₁₋₆ alkoxy, C₃₋₆ monocyclic cycloalkyl, C₃₋₆ monocyclic heterocyclyl, phenyl, 5- or 6-membered monoheteroaryl, NR^{g}R^{h}, - C(O)NR^{g}R^{h}, -SO₂NR^{g}R^{h}; R^{e}, R^{f}, R^{g} and R^{h} are each independently hydrogen, hydroxyethyl, hydroxymethyl or C₁₋₃ alkyl;
B is a substituted or unsubstituted C₆₋₁₀ aryl, a substituted or unsubstituted C₅₋₁₀ heteroaryl, a substituted or unsubstituted C₃₋₆ monocyclic heterocyclyl; the "substituted" means that 1, 2 or 3 hydrogen atom(s) in the group are replaced by a substituent independently selected from the group S3, the substituent of the group S3 is selected from the group consisting of: cyano, acetyl, hydroxy, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), C₁₋₁₀ alkylthio (preferably, C₁₋₆ alkylthio, more preferably, C₁₋₃ alkylthio), halo C₁₋₁₀ alkoxy (preferably, halo C₁₋₆ alkoxy, more preferably, halo C₁₋₃ alkoxy), NR^{a}R^{b}, -CONR^{a}R^{b}, -CONR^{a}NR^{a}R^{b}, -NR^{a}COC₁₋₁₀ alkyl (preferably, -NR^{a}COC₁₋₆ alkyl, more preferably, - NR^{a}COC₁₋₃ alkyl), -CO₂C₁₋₁₀ alkyl (preferably, -CO₂C₁₋₆ alkyl, more preferably, - CO₂C₁₋₃ alkyl), -SO₂NR^{a}R^{b}, -SO₂C₁₋₁₀ alkyl (preferably, -SO₂C₁₋₆ alkyl, more preferably, -SO₂C₁₋₃ alkyl), -CO-C₃₋₆ monocyclic heterocyclyl, -(CH₂)ᵤ-C₃₋₆ monocyclic cycloalkyl, -(CH₂)ᵤ-C₆₋₁₀ aryl (preferably, phenyl), -(CH₂)ᵤ-5- or 6-membered monoheteroaryl, -(CH₂)ᵤ-C₃₋₆ monocyclic heterocyclyl;
wherein, among the substitutes of the group S3, the C₁₋₁₀ alkyl, C₁₋₁₀ alkoxy, C₃₋₆ monocyclic cycloalkyl, C₃₋₆ monocyclic heterocyclyl, C₆₋₁₀ aryl, 5- or 6-membered monoheteroaryl are unsubstituted, or substituted by 1, 2 or 3 substituent(s) each independently selected from the group S31, the substituent of the group S31 is selected from the group consisting of: acetyl, hydroxy, cyano, carboxyl, nitro, halo, C₁₋₃ alkyl, halo C₁₋₃ alkyl, C₁₋₃ alkoxy, halo C₁₋₃ alkoxy, C₃₋₆ monocyclic cycloalkyl, C₃₋₆ monocyclic heterocyclyl, phenyl, 5- or 6-membered monoheteroaryl, NR^{c}R^{d}, - C(O)NR^{c}R^{d} and -SO₂NR^{c}R^{d};
R^{a}, R^{b}, R^{c}, R^{d} are each independently hydrogen, hydroxymethyl, hydroxyethyl, C₁₋₃ alkyl, C₃₋₆ monocyclic cycloalkyl or C₃₋₆ monocyclic heterocyclyl; wherein the C₃₋₆ monocyclic cycloalkyl is selected from the group consisting of: cyclopropyl, cyclobutyl, cyclopentyl and cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran; and the C₃₋₆ monocyclic heterocyclyl is optionally substituted by 1, 2 or 3 C₁₋₃ alkyl, acetyl;
u is 0, 1, 2, 3 or 4.

In one embodiment of the present invention, the compound has a structure as represented by formula (1-1-1): 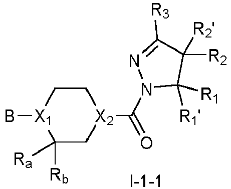 wherein, the R₁, R₁', R₂, R₂', R₃, X₁, X₂, Rₐ, R_{b} and B are as defined in formula (I-1).

In one embodiment of the present invention, the compound has a structure as represented by formula (1-1-1-1) or formula (1-1-1-2): 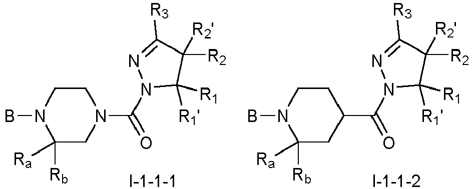 wherein, the R₁, R₁', R₂, R₂', R₃, Rₐ, R_{b} and B are as defined in formula (I-1).

In one embodiment of the present invention, the compound has a structure as represented by formula (1-1-1-3) or formula (1-1-1-4): 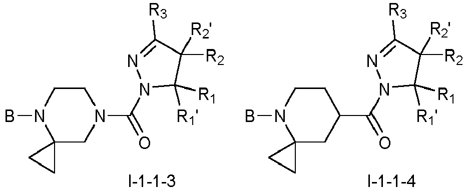 wherein, the R₁, R₁', R₂, R₂', R₃ and B are as defined in formula (I-1).

In one embodiment of the present invention, the compound has a structure as represented by formula (1-1-1-4'): 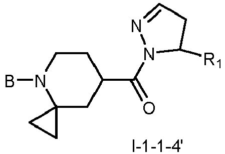 wherein, the R₁ and B are as defined in formula (I-1).

In another respect, the present invention provides a bisheterocyclic carbonyl-substituted dihydropyrazole compound, or a pharmaceutically acceptable salt, stereoisomer, solvate or prodrug thereof, the compound has a structure as represented by formula (I-a): 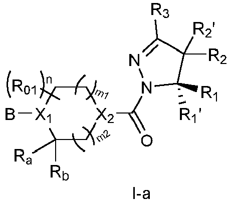 wherein,
R₁ is hydrogen, substituted or unsubstituted C₆₋₁₀ aryl, substituted or unsubstituted C₅₋₁₀ heteroaryl, substituted or unsubstituted C₃₋₆ monocyclic cycloalkyl, or substituted or unsubstituted C₃₋₆ monocyclic heterocyclyl; the "substituted" means that 1, 2 or 3 hydrogen atom(s) in the group are replaced by a substituent independently selected from the group S1, the substituent of the group S1 is selected from the group consisting of: cyano, acetyl, hydroxy, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy), wherein the C₁₋₁₀ alkyl and C₁₋₁₀ alkoxy are unsubstituted, or substituted by 1, 2 or 3 substituent(s) each independently selected from the group S11, the substituent of the group S11 is selected from the group consisting of: acetyl, hydroxy, cyano, carboxyl, halo, C₁₋₆ alkyl, halo C₁₋₆ alkyl, C₁₋₆ alkoxy, halo C₁₋₆ alkoxy, C₃₋₆ monocyclic cycloalkyl, C₃₋₆ monocyclic heterocyclyl, phenyl, 5- or 6-membered monoheteroaryl, NRⁱR^{j}, - C(O)NRⁱR^{j}, -SO₂NRⁱR^{j}; Rⁱ and R^{j} are each independently hydrogen or C₁₋₃ alkyl;
R₁' is hydrogen, cyano, hydroxy, cyanomethyl, cyanoethyl, hydroxymethyl, hydroxyethyl, carboxyl, halo, C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy) or halo C₁₋₁₀ alkoxy (preferably, halo C₁₋₆ alkoxy, more preferably, halo C₁₋₃ alkoxy);
R₂ and R₂' are each independently hydrogen, cyano, hydroxy, cyanomethyl, cyanoethyl, hydroxymethyl, hydroxyethyl, carboxyl, halo, C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy) or C₃₋₆ monocyclic cycloalkyl;
or R₂ and R₂' together with the carbon atom attached thereto form a 3- to 6-membered saturated or partially unsaturated monocycle or a 3- to 6-membered saturated or partially unsaturated monoheterocycle;
R₃ is hydrogen, cyano, hydroxy, cyanomethyl, cyanoethyl, hydroxymethyl, hydroxyethyl, carboxyl, halo, C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy) or C₃₋₆ monocyclic cycloalkyl;
X₁ is N or CR₄; X₂ is N or CR₅;
R₄ and R₅ are each independently hydrogen, hydroxy, cyano, hydroxymethyl, cyanomethyl or C₁₋₁₀ alkyl (preferably, C₁₋₆ alkyl, more preferably, C₁₋₃ alkyl);
m1 and m2 are each independently 0, 1 or 2;
(R₀₁)ₙ represents that the hydrogen(s) on the ring are replaced by n of R₀₁, n is 0, 1, 2, 3, 4, 5 or 6, each of R₀₁ are the same or different, and are independently cyano, acetyl, hydroxyl, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), halo C₁₋₁₀ alkyl (preferably, halo C₁₋₆ alkyl, more preferably, halo C₁₋₃ alkyl) or halo C₁₋₁₀ alkoxy (preferably, halo C₁₋₆ alkoxy, more preferably, halo C₁₋₃ alkoxy);
Rₐ and R_{b} together with the carbon atom attached thereto form a 3- to 6-membered saturated or partially unsaturated monocycle or a 3- to 6-membered saturated or partially unsaturated monoheterocycle; the 3- to 6-membered saturated or partially unsaturated monocycle, 3- to 6-membered saturated or partially unsaturated monoheterocycle are unsubstituted, or substituted by 1, 2 or 3 substituent(s) independently selected from the group S2, the substituent of the group S2 is selected from the group consisting of: cyano, acetyl, hydroxy, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy), C₃₋₆ monocyclic cycloalkyl, C₃₋₆ monocyclic heterocyclyl, phenyl, 5- or 6-membered monoheteroaryl, NR^{e}R^{f}, -C(O)NR^{e}R^{f}, -SO₂NR^{e}R^{f}, wherein the C₃₋₆ monocyclic cycloalkyl, C₃₋₆ monocyclic heterocyclyl, phenyl, 5- or 6-membered monoheteroaryl, C₁₋₁₀ alkyl and C₁₋₁₀ alkoxy are unsubstituted, or substituted by 1, 2 or 3 substituent(s) each independently selected from the group S21, the substituent of the group S21 is selected from the group consisting of: acetyl, hydroxyl, cyano, carboxyl, halo, C₁₋₆ alkyl, halo C₁₋₆ alkyl, C₁₋₆ alkoxy, halo C₁₋₆ alkoxy, C₃₋₆ monocyclic cycloalkyl, C₃₋₆ monocyclic heterocyclyl, phenyl, 5- or 6-membered monoheteroaryl, NR^{g}R^{h}, - C(O)NR^{g}R^{h}, -SO₂NR^{g}R^{h}; R^{e}, R^{f}, R^{g} and R^{h} are each independently hydrogen, hydroxyethyl, hydroxymethyl or C₁₋₃ alkyl;
B is a substituted or unsubstituted C₆₋₁₀ aryl, a substituted or unsubstituted C₅₋₁₀ heteroaryl, a substituted or unsubstituted C₃₋₆ monocyclic heterocyclyl; the "substituted" means that 1, 2 or 3 hydrogen atom(s) in the group are replaced by a substituent independently selected from the group S3, the substituent of the group S3 is selected from the group consisting of: cyano, acetyl, hydroxy, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), C₁₋₁₀ alkylthio (preferably, C₁₋₆ alkylthio, more preferably, C₁₋₃ alkylthio), halo C₁₋₁₀ alkoxy (preferably, halo C₁₋₆ alkoxy, more preferably, halo C₁₋₃ alkoxy), NR^{a}R^{b}, -CONR^{a}R^{b}, -CONR^{a}NR^{a}R^{b}, -NR^{a}COC₁₋₁₀ alkyl (preferably, -NR^{a}COC₁₋₆ alkyl, more preferably, - NR^{a}COC₁₋₃ alkyl), -CO₂C₁₋₁₀ alkyl (preferably, -CO₂C₁₋₆ alkyl, more preferably, - CO₂C₁₋₃ alkyl), -SO₂NR^{a}R^{b}, -SO₂C₁₋₁₀ alkyl (preferably, -SO₂C₁₋₆ alkyl, more preferably, -SO₂C₁₋₃ alkyl), -CO-C₃₋₆ monocyclic heterocyclyl, -(CH₂)ᵤ-C₃₋₆ monocyclic cycloalkyl, -(CH₂)ᵤ-C₆₋₁₀ aryl (preferably, phenyl), -(CH₂)ᵤ-5- or 6-membered monoheteroaryl, -(CH₂)ᵤ-C₃₋₆ monocyclic heterocyclyl;
wherein, among the substitutes of the group S3, the C₁₋₁₀ alkyl, C₁₋₁₀ alkoxy, C₃₋₆ monocyclic cycloalkyl, C₃₋₆ monocyclic heterocyclyl, C₆₋₁₀ aryl, 5- or 6-membered monoheteroaryl are unsubstituted, or substituted by 1, 2 or 3 substituent(s) each independently selected from the group S31, the substituent of the group S31 is selected from the group consisting of: acetyl, hydroxy, cyano, carboxyl, nitro, halo, C₁₋₃ alkyl, halo C₁₋₃ alkyl, C₁₋₃ alkoxy, halo C₁₋₃ alkoxy, C₃₋₆ monocyclic cycloalkyl, C₃₋₆ monocyclic heterocyclyl, phenyl, 5- or 6-membered monoheteroaryl, NR^{c}R^{d}, - C(O)NR^{c}R^{d} and -SO₂NR^{c}R^{d};
R^{a}, R^{b}, R^{c}, R^{d} are each independently hydrogen, hydroxymethyl, hydroxyethyl, C₁₋₃ alkyl, C₃₋₆ monocyclic cycloalkyl or C₃₋₆ monocyclic heterocyclyl; wherein the C₃₋₆ monocyclic cycloalkyl is selected from the group consisting of: cyclopropyl, cyclobutyl, cyclopentyl and cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran; and the C₃₋₆ monocyclic heterocyclyl is optionally substituted by 1, 2 or 3 C₁₋₃ alkyl, acetyl;
u is 0, 1, 2, 3 or 4.

In one embodiment of the present invention, the compound has a structure as represented by formula (I-a-1): 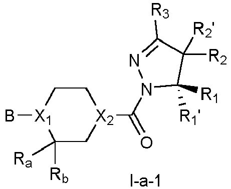 wherein, the R₁, R₁', R₂, R₂', R₃, X₁, X₂, Rₐ, R_{b} and B are as defined in formula (I-a).

In one embodiment of the present invention, the compound has a structure as represented by formula (I-a-1-1) or formula (I-a-1-2): 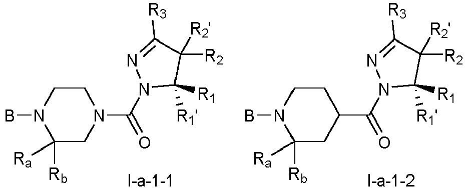 wherein, the R₁, R₁', R₂, R₂', R₃, Rₐ, R_{b} and B are as defined in formula (I-a).

In one embodiment of the present invention, the compound has a structure as represented by formula (I-a-1-3) or formula (I-a-1-4): 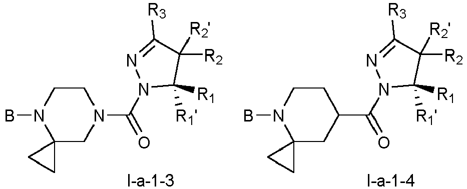 wherein, the R₁, R₁', R₂, R₂', R₃ and B are as defined in formula (I-a).

In one embodiment of the present invention, the compound has a structure as represented by formula (I-a-1-4'): 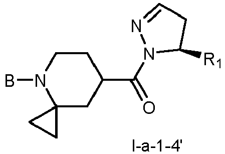 wherein, the R₁ and B are as defined in formula (I-1).

In another respect, the present invention provides a bisheterocyclic carbonyl-substituted dihydropyrazole compound, or a pharmaceutically acceptable salt, stereoisomer, solvate or prodrug thereof, the compound has a structure as represented by formula (1-2): 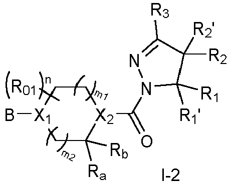 wherein,
R₁ is hydrogen, substituted or unsubstituted C₆₋₁₀ aryl, substituted or unsubstituted C₅₋₁₀ heteroaryl, substituted or unsubstituted C₃₋₆ monocyclic cycloalkyl, or substituted or unsubstituted C₃₋₆ monocyclic heterocyclyl; the "substituted" means that 1, 2 or 3 hydrogen atom(s) in the group are replaced by a substituent independently selected from the group S1, the substituent of the group S1 is selected from the group consisting of: cyano, acetyl, hydroxy, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy), wherein the C₁₋₁₀ alkyl and C₁₋₁₀ alkoxy are unsubstituted, or substituted by 1, 2 or 3 substituent(s) each independently selected from the group S11, the substituent of the group S11 is selected from the group consisting of: acetyl, hydroxy, cyano, carboxyl, halo, C₁₋₆ alkyl, halo C₁₋₆ alkyl, C₁₋₆ alkoxy, halo C₁₋₆ alkoxy, C₃₋₆ monocyclic cycloalkyl, C₃₋₆ monocyclic heterocyclyl, phenyl, 5- or 6-membered monoheteroaryl, NRⁱR^{j}, - C(O)NRⁱR^{j}, -SO₂NRⁱR^{j}; Rⁱ and R^{j} are each independently hydrogen or C₁₋₃ alkyl;
R₁' is hydrogen, cyano, hydroxy, cyanomethyl, cyanoethyl, hydroxymethyl, hydroxyethyl, carboxyl, halo, C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy) or halo C₁₋₁₀ alkoxy (preferably, halo C₁₋₆ alkoxy, more preferably, halo C₁₋₃ alkoxy);
R₂ and R₂' are each independently hydrogen, cyano, hydroxy, cyanomethyl, cyanoethyl, hydroxymethyl, hydroxyethyl, carboxyl, halo, C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy) or C₃₋₆ monocyclic cycloalkyl;
or R₂ and R₂' together with the carbon atom attached thereto form a 3- to 6-membered saturated or partially unsaturated monocycle or a 3- to 6-membered saturated or partially unsaturated monoheterocycle;
R₃ is hydrogen, cyano, hydroxy, cyanomethyl, cyanoethyl, hydroxymethyl, hydroxyethyl, carboxyl, halo, C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy) or C₃₋₆ monocyclic cycloalkyl;
X₁ is N or CR₄; X₂ is N or CR₅;
R₄ and R₅ are each independently hydrogen, hydroxy, cyano, hydroxymethyl, cyanomethyl or C₁₋₁₀ alkyl (preferably, C₁₋₆ alkyl, more preferably, C₁₋₃ alkyl);
m1 and m2 are each independently 0, 1 or 2;
(R₀₁)ₙ represents that the hydrogen(s) on the ring are replaced by n of R₀₁, n is 0, 1, 2, 3, 4, 5 or 6, each of R₀₁ are the same or different, and are independently cyano, acetyl, hydroxyl, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), halo C₁₋₁₀ alkyl (preferably, halo C₁₋₆ alkyl, more preferably, halo C₁₋₃ alkyl) or halo C₁₋₁₀ alkoxy (preferably, halo C₁₋₆ alkoxy, more preferably, halo C₁₋₃ alkoxy);
Rₐ and R_{b} together with the carbon atom attached thereto form a 3- to 6-membered saturated or partially unsaturated monocycle or a 3- to 6-membered saturated or partially unsaturated monoheterocycle; the 3- to 6-membered saturated or partially unsaturated monocycle, 3- to 6-membered saturated or partially unsaturated monoheterocycle are unsubstituted, or substituted by 1, 2 or 3 substituent(s) independently selected from the group S2, the substituent of the group S2 is selected from the group consisting of: cyano, acetyl, hydroxy, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy), C₃₋₆ monocyclic cycloalkyl, C₃₋₆ monocyclic heterocyclyl, phenyl, 5- or 6-membered monoheteroaryl, NR^{e}R^{f}, -C(O)NR^{e}R^{f}, -SO₂NR^{e}R^{f}, wherein the C₃₋₆ monocyclic cycloalkyl, C₃₋₆ monocyclic heterocyclyl, phenyl, 5- or 6-membered monoheteroaryl, C₁₋₁₀ alkyl and C₁₋₁₀ alkoxy are unsubstituted, or substituted by 1, 2 or 3 substituent(s) each independently selected from the group S21, the substituent of the group S21 is selected from the group consisting of: acetyl, hydroxyl, cyano, carboxyl, halo, C₁₋₆ alkyl, halo C₁₋₆ alkyl, C₁₋₆ alkoxy, halo C₁₋₆ alkoxy, C₃₋₆ monocyclic cycloalkyl, C₃₋₆ monocyclic heterocyclyl, phenyl, 5- or 6-membered monoheteroaryl, NR^{g}R^{h}, - C(O)NR^{g}R^{h}, -SO₂NR^{g}R^{h}; R^{e}, R^{f}, R^{g} and R^{h} are each independently hydrogen, hydroxyethyl, hydroxymethyl or C₁₋₃ alkyl;
B is a substituted or unsubstituted C₆₋₁₀ aryl, a substituted or unsubstituted C₅₋₁₀ heteroaryl, a substituted or unsubstituted C₃₋₆ monocyclic heterocyclyl; the "substituted" means that 1, 2 or 3 hydrogen atom(s) in the group are replaced by a substituent independently selected from the group S3, the substituent of the group S3 is selected from the group consisting of: cyano, acetyl, hydroxy, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), C₁₋₁₀ alkylthio (preferably, C₁₋₆ alkylthio, more preferably, C₁₋₃ alkylthio), halo C₁₋₁₀ alkoxy (preferably, halo C₁₋₆ alkoxy, more preferably, halo C₁₋₃ alkoxy), NR^{a}R^{b}, -CONR^{a}R^{b}, -CONR^{a}NR^{a}R^{b}, -NR^{a}COC₁₋₁₀ alkyl (preferably, -NR^{a}COC₁₋₆ alkyl, more preferably, - NR^{a}COC₁₋₃ alkyl), -CO₂C₁₋₁₀ alkyl (preferably, -CO₂C₁₋₆ alkyl, more preferably, - CO₂C₁₋₃ alkyl), -SO₂NR^{a}R^{b}, -SO₂C₁₋₁₀ alkyl (preferably, -SO₂C₁₋₆ alkyl, more preferably, -SO₂C₁₋₃ alkyl), -CO-C₃₋₆ monocyclic heterocyclyl, -(CH₂)ᵤ-C₃₋₆ monocyclic cycloalkyl, -(CH₂)ᵤ-C₆₋₁₀ aryl (preferably, phenyl), -(CH₂)ᵤ-5- or 6-membered monoheteroaryl, -(CH₂)ᵤ-C₃₋₆ monocyclic heterocyclyl;
wherein, among the substitutes of the group S3, the C₁₋₁₀ alkyl, C₁₋₁₀ alkoxy, C₃₋₆ monocyclic cycloalkyl, C₃₋₆ monocyclic heterocyclyl, C₆₋₁₀ aryl, 5- or 6-membered monoheteroaryl are unsubstituted, or substituted by 1, 2 or 3 substituent(s) each independently selected from the group S31, the substituent of the group S31 is selected from the group consisting of: acetyl, hydroxy, cyano, carboxyl, nitro, halo, C₁₋₃ alkyl, halo C₁₋₃ alkyl, C₁₋₃ alkoxy, halo C₁₋₃ alkoxy, C₃₋₆ monocyclic cycloalkyl, C₃₋₆ monocyclic heterocyclyl, phenyl, 5- or 6-membered monoheteroaryl, NR^{c}R^{d}, - C(O)NR^{c}R^{d} and -SO₂NR^{c}R^{d};
R^{a}, R^{b}, R^{c}, R^{d} are each independently hydrogen, hydroxymethyl, hydroxyethyl, C₁₋₃ alkyl, C₃₋₆ monocyclic cycloalkyl or C₃₋₆ monocyclic heterocyclyl; wherein the C₃₋₆ monocyclic cycloalkyl is selected from the group consisting of: cyclopropyl, cyclobutyl, cyclopentyl and cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran; and the C₃₋₆ monocyclic heterocyclyl is optionally substituted by 1, 2 or 3 C₁₋₃ alkyl, acetyl;
u is 0, 1, 2, 3 or 4.

In one embodiment of the present invention, the compound has a structure as represented by formula (1-2-1): 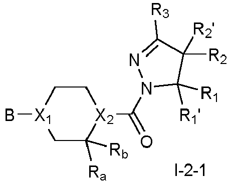 wherein, the R₁, R₁', R₂, R₂', R₃, X₁, X₂, Rₐ, R_{b} and B are as defined in formula (1-2).

In one embodiment of the present invention, the compound has a structure as represented by formula (1-2-1-1) or formula (1-2-1-2): 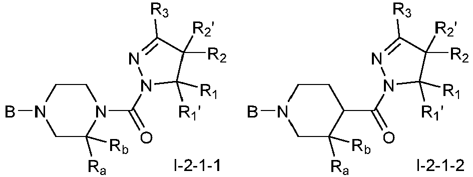 wherein, the R₁, R₁', R₂, R₂', R₃, Rₐ, R_{b} and B are as defined in formula (1-2).

In one embodiment of the present invention, the compound has a structure as represented by formula (1-2-1-3) or formula (1-2-1-4): 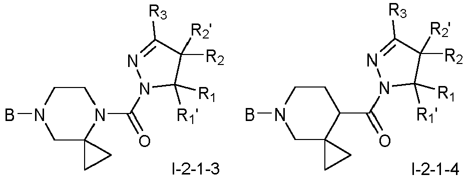 wherein, the R₁, R₁', R₂, R₂', R₃ and B are as defined in formula (1-2).

In one embodiment of the present invention, the compound has a structure as represented by formula (1-2-1-4'): 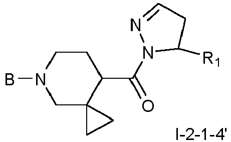 wherein, the R₁ and B are as defined in formula (1-2).

In another respect, the present invention provides a bisheterocyclic carbonyl-substituted dihydropyrazole compound, or a pharmaceutically acceptable salt, stereoisomer, solvate or prodrug thereof, the compound has a structure as represented by formula (I-b): 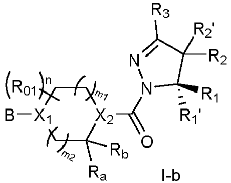 wherein,
R₁ is hydrogen, substituted or unsubstituted C₆₋₁₀ aryl, substituted or unsubstituted C₅₋₁₀ heteroaryl, substituted or unsubstituted C₃₋₆ monocyclic cycloalkyl, or substituted or unsubstituted C₃₋₆ monocyclic heterocyclyl; the "substituted" means that 1, 2 or 3 hydrogen atom(s) in the group are replaced by a substituent independently selected from the group S1, the substituent of the group S1 is selected from the group consisting of: cyano, acetyl, hydroxy, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy), wherein the C₁₋₁₀ alkyl and C₁₋₁₀ alkoxy are unsubstituted, or substituted by 1, 2 or 3 substituent(s) each independently selected from the group S11, the substituent of the group S11 is selected from the group consisting of: acetyl, hydroxy, cyano, carboxyl, halo, C₁₋₆ alkyl, halo C₁₋₆ alkyl, C₁₋₆ alkoxy, halo C₁₋₆ alkoxy, C₃₋₆ monocyclic cycloalkyl, C₃₋₆ monocyclic heterocyclyl, phenyl, 5- or 6-membered monoheteroaryl, NRⁱR^{j}, - C(O)NRⁱR^{j}, -SO₂NRⁱR^{j}; Rⁱ and R^{j} are each independently hydrogen or C₁₋₃ alkyl;
R₁' is hydrogen, cyano, hydroxy, cyanomethyl, cyanoethyl, hydroxymethyl, hydroxyethyl, carboxyl, halo, C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy) or halo C₁₋₁₀ alkoxy (preferably, halo C₁₋₆ alkoxy, more preferably, halo C₁₋₃ alkoxy);
R₂ and R₂' are each independently hydrogen, cyano, hydroxy, cyanomethyl, cyanoethyl, hydroxymethyl, hydroxyethyl, carboxyl, halo, C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy) or C₃₋₆ monocyclic cycloalkyl;
or R₂ and R₂' together with the carbon atom attached thereto form a 3- to 6-membered saturated or partially unsaturated monocycle or a 3- to 6-membered saturated or partially unsaturated monoheterocycle;
R₃ is hydrogen, cyano, hydroxy, cyanomethyl, cyanoethyl, hydroxymethyl, hydroxyethyl, carboxyl, halo, C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy) or C₃₋₆ monocyclic cycloalkyl;
X₁ is N or CR₄; X₂ is N or CR₅;
R₄ and R₅ are each independently hydrogen, hydroxy, cyano, hydroxymethyl, cyanomethyl or C₁₋₁₀ alkyl (preferably, C₁₋₆ alkyl, more preferably, C₁₋₃ alkyl);
m1 and m2 are each independently 0, 1 or 2;
(R₀₁)ₙ represents that the hydrogen(s) on the ring are replaced by n of R₀₁, n is 0, 1, 2, 3, 4, 5 or 6, each of R₀₁ are the same or different, and are independently cyano, acetyl, hydroxyl, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), halo C₁₋₁₀ alkyl (preferably, halo C₁₋₆ alkyl, more preferably, halo C₁₋₃ alkyl) or halo C₁₋₁₀ alkoxy (preferably, halo C₁₋₆ alkoxy, more preferably, halo C₁₋₃ alkoxy);
Rₐ and R_{b} together with the carbon atom attached thereto form a 3- to 6-membered saturated or partially unsaturated monocycle or a 3- to 6-membered saturated or partially unsaturated monoheterocycle; the 3- to 6-membered saturated or partially unsaturated monocycle, 3- to 6-membered saturated or partially unsaturated monoheterocycle are unsubstituted, or substituted by 1, 2 or 3 substituent(s) independently selected from the group S2, the substituent of the group S2 is selected from the group consisting of: cyano, acetyl, hydroxy, carboxyl, nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy), C₃₋₆ monocyclic cycloalkyl, C₃₋₆ monocyclic heterocyclyl, phenyl, 5- or 6-membered monoheteroaryl, NR^{e}R^{f}, -C(O)NR^{e}R^{f}, -SO₂NR^{e}R^{f}, wherein the C₃₋₆ monocyclic cycloalkyl, C₃₋₆ monocyclic heterocyclyl, phenyl, 5- or 6-membered monoheteroaryl, C₁₋₁ alkyl and C₁₋₁ alkoxy are unsubstituted, or substituted by 1, 2 or 3 substituent(s) each independently selected from the group S21, the substituent of the group S21 is selected from the group consisting of: acetyl, hydroxyl, cyano, carboxyl, halo, C₁₋₆ alkyl, halo C₁₋₆ alkyl, C₁₋₆ alkoxy, halo C₁₋₆ alkoxy, C₃₋₆ monocyclic cycloalkyl, C₃₋₆ monocyclic heterocyclyl, phenyl, 5- or 6-membered monoheteroaryl, NR^{g}R^{h},-C(O)NR^{g}R^{h}, -SO₂NR^{g}R^{h}; R^{e}, R^{f}, R^{g} and R^{h} are each independently hydrogen, hydroxyethyl, hydroxymethyl or C₁₋₃ alkyl;
B is a substituted or unsubstituted C₆₋₁₀ aryl, a substituted or unsubstituted C₅₋₁₀ heteroaryl, a substituted or unsubstituted C₃₋₆ monocyclic heterocyclyl; the "substituted" means that 1, 2 or 3 hydrogen atom(s) in the group are replaced by a substituent independently selected from the group S3, the substituent of the group S3 is selected from the group consisting of: cyano, acetyl, hydroxy, carboxyl, nitro, halo (preferably, F or Cl), C₁₋₁₀ alkyl (preferably, C₁₋₆ alkyl, more preferably, C₁₋₃ alkyl), C₁₋₁ alkoxy (preferably, C₁₋₆ alkoxy, more preferably, C₁₋₃ alkoxy), C₁₋₁₀ alkylthio (preferably, C₁₋₆ alkylthio, more preferably, C₁₋₃ alkylthio), halo C₁₋₁ alkoxy (preferably, halo C₁₋₆ alkoxy, more preferably, halo C₁₋₃ alkoxy), NR^{a}R^{b}, -CONR^{a}R^{b}, -CONR^{a}NR^{a}R^{b}, -NR^{a}COC₁₋₁₀ alkyl (preferably, -NR^{a}COC₁₋₆ alkyl, more preferably,-NR^{a}COC₁₋₃ alkyl), -CO₂C₁₋₁ alkyl (preferably, -CO₂C₁₋₆ alkyl, more preferably,-CO₂C₁₋₃ alkyl), -SO₂NR^{a}R^{b}, -SO₂C₁₋₁₀ alkyl (preferably, -SO₂C₁₋₆ alkyl, more preferably, -SO₂C₁₋₃ alkyl), -CO-C₃₋₆ monocyclic heterocyclyl, -(CH₂)ᵤ-C₃₋₆ monocyclic cycloalkyl, -(CH₂)ᵤ-C₆₋₁₀ aryl (preferably, phenyl), -(CH₂)ᵤ-5- or 6-membered monoheteroaryl, -(CH₂)ᵤ-C₃₋₆ monocyclic heterocyclyl;
wherein, among the substitutes of the group S3, the C₁₋₁₀ alkyl, C₁₋₁₀ alkoxy, C₃₋₆ monocyclic cycloalkyl, C₃₋₆ monocyclic heterocyclyl, C₆₋₁₀ aryl, 5- or 6-membered monoheteroaryl are unsubstituted, or substituted by 1, 2 or 3 substituent(s) each independently selected from the group S31, the substituent of the group S31 is selected from the group consisting of: acetyl, hydroxy, cyano, carboxyl, nitro, halo, C₁₋₃ alkyl, halo C₁₋₃ alkyl, C₁₋₃ alkoxy, halo C₁₋₃ alkoxy, C₃₋₆ monocyclic cycloalkyl, C₃₋₆ monocyclic heterocyclyl, phenyl, 5- or 6-membered monoheteroaryl, NR^{c}R^{d},-C(O)NR^{c}R^{d} and -SO₂NR^{c}R^{d};
R^{a}, R^{b}, R^{c}, R^{d} are each independently hydrogen, hydroxymethyl, hydroxyethyl, C₁₋₃ alkyl, C₃₋₆ monocyclic cycloalkyl or C₃₋₆ monocyclic heterocyclyl; wherein the C₃₋₆ monocyclic cycloalkyl is selected from the group consisting of: cyclopropyl, cyclobutyl, cyclopentyl and cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran; and the C₃₋₆ monocyclic heterocyclyl is optionally substituted by 1, 2 or 3 C₁₋₃ alkyl, acetyl;
u is 0, 1, 2, 3 or 4.

In one embodiment of the present invention, the compound has a structure as represented by formula (I-b-1): 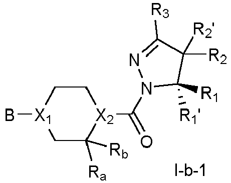 wherein, the R₁, R₁', R₂, R₂', R₃, X₁, X₂, Rₐ, R_{b} and B are as defined in formula (I-b).

In one embodiment of the present invention, the compound has a structure as represented by formula (I-b-1-1) or formula (I-b-1-2): 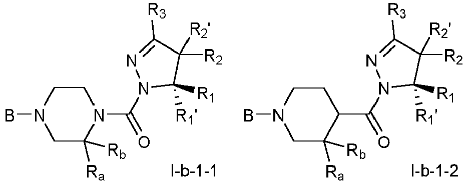 wherein, the R₁, R₁', R₂, R₂', R₃, Rₐ, R_{b} and B are as defined in formula (I-b).

In one embodiment of the present invention, the compound has a structure as represented by formula (I-b-1-3) or formula (I-b-1-4): 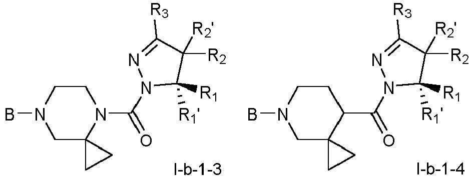 wherein, the R₁, R₁', R₂, R₂', R₃ and B are as defined in formula (I-b).

In one embodiment of the present invention, the compound has a structure as represented by formula (I-b-1-4'): 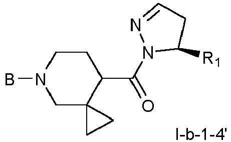 wherein, the R₁ and B are as defined in formula (I-b).

In another respect, the present invention provides a bisheterocyclic carbonyl-substituted dihydropyrazole compound, or a pharmaceutically acceptable salt, stereoisomer, solvate or prodrug thereof, the compound has a structure as represented by formula (1-3-1): 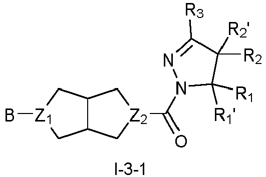 wherein,
R₁ is hydrogen, substituted or unsubstituted C₆₋₁₀ aryl, substituted or unsubstituted C₅₋₁₀ heteroaryl, substituted or unsubstituted C₃₋₆ monocyclic cycloalkyl, or substituted or unsubstituted C₃₋₆ monocyclic heterocyclyl; the "substituted" means that 1, 2 or 3 hydrogen atom(s) in the group are replaced by a substituent independently selected from the group S1, the substituent of the group S1 is selected from the group consisting of: cyano, acetyl, hydroxy, carboxyl, nitro, halo (preferably, F or Cl), C₁₋₁₀ alkyl (preferably, C₁₋₆ alkyl, more preferably, C₁₋₃ alkyl), C₁₋₁ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy), wherein the C₁₋₁₀ alkyl and C₁₋₁ alkoxy are unsubstituted, or substituted by 1, 2 or 3 substituent(s) each independently selected from the group S11, the substituent of the group S11 is selected from the group consisting of: acetyl, hydroxy, cyano, carboxyl, halo, C₁₋₆ alkyl, halo C₁₋₆ alkyl, C₁₋₆ alkoxy, halo C₁₋₆ alkoxy, C₃₋₆ monocyclic cycloalkyl, C₃₋₆ monocyclic heterocyclyl, phenyl, 5- or 6-membered monoheteroaryl, NRⁱR^{j},-C(O)NRⁱR^{j}, -SO₂NRⁱR^{j}; Rⁱ and R^{j} are each independently hydrogen or C₁₋₃ alkyl;
R₁' is hydrogen, cyano, hydroxy, cyanomethyl, cyanoethyl, hydroxymethyl, hydroxyethyl, carboxyl, halo, C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁ alkoxy (preferably, C₁₋₆ alkoxy, more preferably, C₁₋₃ alkoxy) or halo C₁₋₁₀ alkoxy (preferably, halo C₁₋₆ alkoxy, more preferably, halo C₁₋₃ alkoxy);
R₂ and R₂' are each independently hydrogen, cyano, hydroxy, cyanomethyl, cyanoethyl, hydroxymethyl, hydroxyethyl, carboxyl, halo, C₁₋₁ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy) or C₃₋₆ monocyclic cycloalkyl;
or R₂ and R₂' together with the carbon atom attached thereto form a 3- to 6-membered saturated or partially unsaturated monocycle or a 3- to 6-membered saturated or partially unsaturated monoheterocycle;
R₃ is hydrogen, cyano, hydroxy, cyanomethyl, cyanoethyl, hydroxymethyl, hydroxyethyl, carboxyl, halo, C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy) or C₃₋₆ monocyclic cycloalkyl;
Z₁ is N or CR₆; Z₂ is N or CR₇;
R₆ and R₇ are each independently hydrogen, hydroxy, cyano, hydroxymethyl, cyanomethyl or C₁₋₁₀ alkyl (preferably, C₁₋₆ alkyl, more preferably, C₁₋₃ alkyl);
B is a substituted or unsubstituted C₆₋₁₀ aryl, a substituted or unsubstituted C₅₋₁₀ heteroaryl, a substituted or unsubstituted C₃₋₆ monocyclic heterocyclyl; the "substituted" means that 1, 2 or 3 hydrogen atom(s) in the group are replaced by a substituent independently selected from the group S3, the substituent of the group S3 is selected from the group consisting of: cyano, acetyl, hydroxy, carboxyl, nitro, halo (preferably, F or Cl), C₁₋₁₀ alkyl (preferably, C₁₋₆ alkyl, more preferably, C₁₋₃ alkyl), C₁₋₁ alkoxy (preferably, C₁₋₆ alkoxy, more preferably, C₁₋₃ alkoxy), C₁₋₁₀ alkylthio (preferably, C₁₋₆ alkylthio, more preferably, C₁₋₃ alkylthio), halo C₁₋₁ alkoxy (preferably, halo C₁₋₆ alkoxy, more preferably, halo C₁₋₃ alkoxy), NR^{a}R^{b}, -CONR^{a}R^{b}, -CONR^{a}NR^{a}R^{b}, -NR^{a}COC₁₋₁₀ alkyl (preferably, -NR^{a}COC₁₋₆ alkyl, more preferably,-NR^{a}COC₁₋₃ alkyl), -CO₂C₁₋₁ alkyl (preferably, -CO₂C₁₋₆ alkyl, more preferably,-CO₂C₁₋₃ alkyl), -SO₂NR^{a}R^{b}, -SO₂C₁₋₁₀ alkyl (preferably, -SO₂C₁₋₆ alkyl, more preferably, -SO₂C₁₋₃ alkyl), -CO-C₃₋₆ monocyclic heterocyclyl, -(CH₂)ᵤ-C₃₋₆ monocyclic cycloalkyl, -(CH₂)ᵤ-C₆₋₁₀ aryl (preferably, phenyl), -(CH₂)ᵤ-5- or 6-membered monoheteroaryl, -(CH₂)ᵤ-C₃₋₆ monocyclic heterocyclyl;
wherein, among the substitutes of the group S3, the C₁₋₁₀ alkyl, C₁₋₁₀ alkoxy, C₃₋₆ monocyclic cycloalkyl, C₃₋₆ monocyclic heterocyclyl, C₆₋₁₀ aryl, 5- or 6-membered monoheteroaryl are unsubstituted, or substituted by 1, 2 or 3 substituent(s) each independently selected from the group S31, the substituent of the group S31 is selected from the group consisting of: acetyl, hydroxy, cyano, carboxyl, nitro, halo, C₁₋₃ alkyl, halo C₁₋₃ alkyl, C₁₋₃ alkoxy, halo C₁₋₃ alkoxy, C₃₋₆ monocyclic cycloalkyl, C₃₋₆ monocyclic heterocyclyl, phenyl, 5- or 6-membered monoheteroaryl, NR^{c}R^{d},-C(O)NR^{c}R^{d}, -SO₂NR^{c}R^{d};
R^{a}, R^{b}, R^{c}, R^{d} are each independently hydrogen, hydroxymethyl, hydroxyethyl, C₁₋₃ alkyl, C₃₋₆ monocyclic cycloalkyl or C₃₋₆ monocyclic heterocyclyl; wherein the C₃₋₆ monocyclic cycloalkyl is selected from the group consisting of: cyclopropyl, cyclobutyl, cyclopentyl and cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran; and the C₃₋₆ monocyclic heterocyclyl is optionally substituted by 1, 2 or 3 C₁₋₃ alkyl, acetyl;
u is 0, 1, 2, 3 or 4.

In one embodiment of the present invention, the compound has a structure as represented by formula (1-3-1-1) or formula (1-3-1-2): 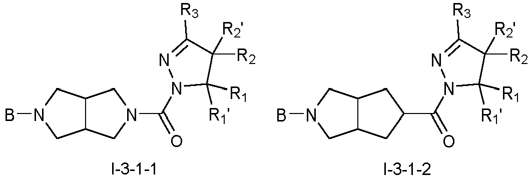 wherein, the R₁, R₁', R₂, R₂', R₃ and B are as defined in formula (1-3-1).

In one embodiment of the present invention, the compound has a structure as represented by formula (1-3-1-3), formula (1-3-1-4), formula (1-3-1-5) or formula (I-3-1-6): 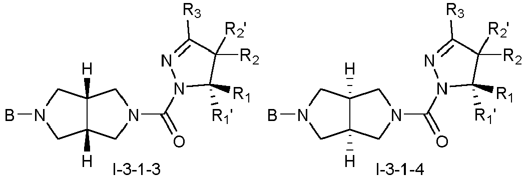 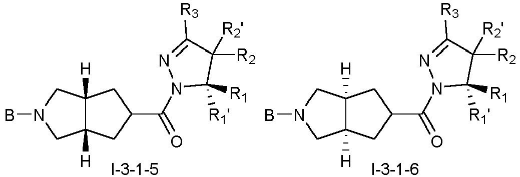 wherein, the R₁, R₁', R₂, R₂', R₃ and B are as defined in formula (1-3-1).

In one embodiment of the present invention, the compound has a structure as represented by formula (1-3-1-3'), formula (1-3-1-4'), formula (1-3-1-5') or formula I-3-1-6': 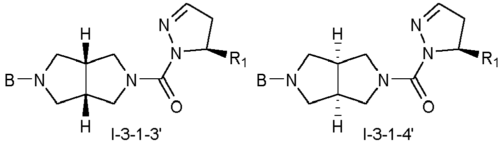 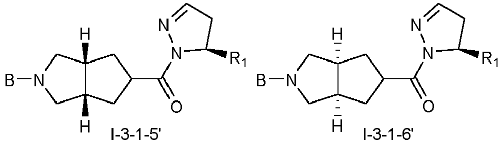 wherein, the R₁ and B are as defined in formula (1-3-1).

In one embodiment of the present invention, in formula (I-1), formula (1-1-1), formula (1-1-1-1), formula (1-1-1-2), formula (1-1-1-3), formula (1-1-1-4), formula (I-1-1-4'), formula (I-a), formula (I-a-1), formula (I-a-1-1), formula (I-a-1-2) , formula (I-a-1-3), formula (I-a-1-4), formula (I-a-1-4'), formula (1-2), formula (1-2-1), formula (1-2-1-1), formula (1-2-1-2), formula (1-2-1-3), formula (1-2-1-4), formula (I-2-1-4'), formula (I-b), formula (I-b-1), formula (I-b-1-1), formula (I-b-1-2), formula (I-b-1-3), formula (I-b-1-4) , formula (I-b-1-4'), formula (1-3-1), formula (1-3-1-1), formula (I-3-1-2) , formula (1-3-1-3), formula (I-3-1-4) , formula (1-3-1-5), formula (I-3-1-6) , formula (1-3-1-3'), formula (1-3-1-4'), formula (1-3-1-5') and formula (1-3-1-6'), B is a substituted or unsubstituted C₅₋₁₀ heteroaryl; the "substituted" means that 1 or 2 hydrogen atom(s) in the group are replaced by a substituent independently selected from the group S3, the substituent of the group S3 is selected from the group consisting of: nitro, halo (preferably, F or Cl), C₁₋₁₀ alkyl (preferably, C₁₋₆ alkyl, more preferably, C₁₋₃ alkyl), C₁₋₁₀ alkoxy (preferably, C₁₋₆ alkoxy, more preferably, C₁₋₃ alkoxy), NR^{a}R^{b}, -CONR^{a}R^{b}, -CO₂C₁₋₁₀ alkyl (preferably, -CO₂C₁₋₆ alkyl, more preferably, -CO₂C₁₋₃ alkyl);
wherein, among the substitutes of the group S3, the C₁₋₁₀ alkyl, C₁₋₁₀ alkoxy are unsubstituted, or substituted by 1 or 2 or 3 substituent(s) each independently selected from the group S31, the substituent of the group S31 is selected from the group consisting of: halo;
R^{a} and R^{b} are each independently hydrogen, hydroxymethyl, hydroxyethyl or C₁₋₃ alkyl.

In one embodiment of the present invention, in formula (I-1), formula (1-1-1), formula (1-1-1-1), formula (1-1-1-2), formula (1-1-1-3), formula (1-1-1-4), formula (I-1-1-4'), formula (I-a), formula (I-a-1), formula (I-a-1-1), formula (I-a-1-2) , formula (I-a-1-3), formula (I-a-1-4), formula (I-a-1-4'), formula (1-2), formula (1-2-1), formula (1-2-1-1), formula (1-2-1-2), formula (1-2-1-3), formula (1-2-1-4), formula (I-2-1-4'), formula (I-b), formula (I-b-1), formula (I-b-1-1), formula (I-b-1-2), formula (I-b-1-3), formula (I-b-1-4) , formula (I-b-1-4'), formula (1-3-1), formula (1-3-1-1), formula (I-3-1-2) , formula (1-3-1-3), formula (I-3-1-4) , formula (1-3-1-5), formula (I-3-1-6) , formula (1-3-1-3'), formula (I-3-1-4') , formula (1-3-1-5') and formula (I-3-1-6'), B is a substituted or unsubstituted C₅₋₁₀ heteroaryl; the "substituted" means that 1 or 2 hydrogen atom(s) in the group are replaced by a substituent independently selected from the group S3, the substituent of the group S3 is selected from the group consisting of: cyano, F, C₁₋₃ alkyl, C₁₋₃ alkoxy, NR^{a}R^{b}, -CONR^{a}R^{b} and -CO₂C₁₋₃ alkyl;
Wherein, among the substitutes of the group S3, the C₁₋₃ alkyl, C₁₋₃ alkoxy are unsubstituted;
R^{a} and R^{b} are each independently hydrogen or C₁₋₃ alkyl.

In one embodiment of the present invention, in formula (I-1), formula (1-1-1), formula (1-1-1-1), formula (1-1-1-2), formula (1-1-1-3), formula (1-1-1-4), formula (I-1-1-4'), formula (I-a), formula (I-a-1), formula (I-a-1-1), formula (I-a-1-2) , formula (I-a-1-3), formula (I-a-1-4), formula (I-a-1-4'), formula (1-2), formula (1-2-1), formula (1-2-1-1), formula (1-2-1-2), formula (1-2-1-3), formula (1-2-1-4), formula (I-2-1-4'), formula (I-b), formula (I-b-1), formula (I-b-1-1), formula (I-b-1-2), formula (I-b-1-3), formula (I-b-1-4) , formula (I-b-1-4'), formula (1-3-1), formula (1-3-1-1), formula (I-3-1-2) , formula (1-3-1-3), formula (I-3-1-4) , formula (1-3-1-5), formula (I-3-1-6) , formula (1-3-1-3'), formula (I-3-1-4') , formula (1-3-1-5') and formula (I-3-1-6'), B is a substituted or unsubstituted C₅₋₁₀ heteroaryl; the "substituted" means that 1 or 2 hydrogen atom(s) in the group are replaced by a substituent independently selected from the group S3, the substituent of the group S3 is selected from the group consisting of: cyano, F, methyl, methoxy, NR^{a}R^{b}, -CONR^{a}R^{b} and -CO₂CH₃; R^{a} and R^{b} are each independently hydrogen or methyl.

In one embodiment of the present invention, in formula (I-1), formula (1-1-1), formula (1-1-1-1), formula (1-1-1-2), formula (1-1-1-3), formula (1-1-1-4), formula (I-a), formula (I-a-1), formula (I-a-1-1), formula (I-a-1-2) , formula (I-a-1-3), formula (I-a-1-4), formula (1-2), formula (1-2-1), formula (1-2-1-1), formula (1-2-1-2), formula (1-2-1-3), formula (1-2-1-4), formula (I-b), formula (I-b-1), formula (I-b-1-1), formula (I-b-1-2), formula (I-b-1-3), formula (I-b-1-4) , formula (1-3-1), formula (1-3-1-1), formula (I-3-1-2) , formula (I-3-1-3), formula (I-3-1-4), formula (I-3-1-5) and formula (I-3-1-6), R₁ is substituted or unsubstituted phenyl, substituted or unsubstituted pyridinyl; the "substituted" means that 1 or 2 hydrogen atom(s) in the group are replaced by a substituent each independently selected from the group consisting of: halo (preferably, F or Cl); R₁' is hydrogen; R₂ is hydrogen; R₂' is hydrogen; and R₃ is hydrogen.

In one embodiment of the present invention, the substituent of the group S3 is cyano, acetyl, hydroxy, carboxyl, halo, C₁₋₃ alkyl, C₁₋₃ alkoxy, C₁₋₃ alkylthio, halo C₁₋₃ alkoxy, NR^{a}R^{b}, -CONR^{a}R^{b}, -CONHNR^{a}R^{b}, -NHCOC₁₋₃ alkyl, -CO₂C₁₋₃ alkyl,-SO₂NR^{a}R^{b}, -SO₂C₁₋₃ alkyl, -CO-C₃₋₆ monocyclic heterocyclyl, -(CH₂)ᵤ-C₃₋₆ monocyclic cycloalkyl, -(CH₂)ᵤ₋phenyl, -(CH₂)ᵤ-5- or 6-membered monoheteroaryl,-(CH₂)ᵤ-C₃₋₆ monocyclic heterocyclyl;
wherein, among the substituents of the group S3, the C₁₋₃ alkyl, C₁₋₃ alkoxy, C₃₋₆ monocyclic cycloalkyl, C₃₋₆ monocyclic heterocyclyl, phenyl, 5- or 6-membered monoheteroaryl are unsubstituted, or substituted by 1, 2 or 3 substituent(s) each independently selected from the group S31, the substituent of the group S31 is selected from the group consisitng of: acetyl, hydroxy, cyano, carboxyl, halo, C₁₋₃ alkyl, halo C₁₋₃ alkyl, C₁₋₃ alkoxy, halo C₁₋₃ alkoxy, C₃₋₆ monocyclic cycloalkyl, C₃₋₆ monocyclic heterocyclyl, phenyl, 5- or 6-membered monoheteroaryl, NR^{c}R^{d},-C(O)NR^{c}R^{d} and -SO₂NR^{c}R^{d};
R^{a}, R^{b}, R^{c}, R^{d} are each independently hydrogen, hydroxymethyl, hydroxyethyl, C₁₋₃ alkyl, C₃₋₆ monocyclic cycloalkyl or C₃₋₆ monocyclic heterocyclyl; wherein the C₃₋₆ monocyclic cycloalkyl is selected from the group consisting of: cyclopropyl, cyclobutyl, cyclopentyl and cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran; and the C₃₋₆ monocyclic heterocyclyl is optionally substituted by 1, 2 or 3 C₁₋₃ alkyl or acetyl;
u is 0, 1, 2 or 3.

In one embodiment of the present invention, among the substitutes of the groups S3 and S31, the C₃₋₆ monocyclic heterocyclyl are each independently selected from the group consisting of: aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrol-2,5-dione, furan-2(3H)-one, furan-2(5H)-one, 1,3-dioxol-2-one, oxazol-2(3H)-one, 1,3-dihydro-2H-imidazol-2-one, furan-2,5-dione, 3,6-dihydropyridin-2(1H)-one, pyridin-2,6-(1H,3H)-dione, 5,6-dihydro-2H-pyran-2-one, 3,6-dihydro-2H-pyran-2-one, 3,4-dihydro-2H-1,3-oxazine, 3,6-dihydro-2H-1,3-oxazine and 1,2,3,4-tetrahydropyrimidine.

In one embodiment of the present invention, among the substitutes of the groups S3 and S31, the C₃₋₆ monocyclic cycloalkyl are each independently selected from the group consisting of: cyclopropyl, cyclobutyl, cyclopentyl, cyclopentenyl, cyclohexyl, cyclohexenyl, cyclohexdienyl, cyclobutanone, cyclobutan-1,2-dione, cyclopentanone, cyclopentan-1,3-dione, cyclohexanone and cyclohexan-1,3-dione.

In one embodiment of the present invention, among the substitutes of the groups S3 and S31, the 5- or 6-membered monoheteroaryl are each independently selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

In one embodiment of the present invention, R₁ is substituted or unsubstituted C₆₋₁₀ aryl, substituted or unsubstituted C₅₋₁₀ heteroaryl, substituted or unsubstituted C₃₋₆ monocyclic cycloalkyl, or substituted or unsubstituted C₃₋₆ monocyclic heterocyclyl; the "substituted" means that 1, 2 or 3 hydrogen atom(s) in the group are replaced by a substituent each independently selected from the group consisting of: cyano, halo (preferably, F or Cl), C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy).

In one embodiment of the present invention, the C₆₋₁₀ aryl in R₁ is a phenyl, a 9- or 10-membered aromatic fused bicyclic ring formed by fusing a phenyl to one 5- or 6-membered monocyclic heterocyclyl ring, or a 9- or 10-membered aromatic fused bicyclic ring formed by fusing a phenyl to one 5- or 6-membered monocyclic cycloalkyl ring.

In one embodiment of the present invention, the C₅₋₁₀ heteroaryl in R₁ is a 5- or 6-membered monoheteroaryl, a 9- or 10-membered biheteroaryl formed by fusing a phenyl to a 5- or 6-membered monoheteroaryl, a 8- to 10-membered biheteroaryl formed by fusing a 5- or 6-membered monoheteroaryl to a 5- or 6-membered monoheteroaryl, a 8- to 10-membered biheteroaryl formed by fusing a 5- or 6-membered monoheteroaryl to one 5- or 6-membered monocyclic heterocyclyl ring, or a 8- to 10-membered biheteroaryl formed by fusing a 5- or 6-membered monoheteroaryl to one 5- or 6-membered monocyclic cycloalkyl ring.

In one embodiment of the present invention, the C₃₋₆ monocyclic cycloalkyl in R₁ is selected from the group consisting of: cyclopropyl, cyclobutyl, cyclopentyl, cyclopentenyl, cyclohexyl, cyclohexenyl, cyclohexdienyl, cyclobutanone, cyclobutan-1,2-dione, cyclopentanone, cyclopentan-1,3-dione, cyclohexanone, cyclohexan-1,3-dione.

In one embodiment of the present invention, the C₃₋₆ monocyclic heterocyclyl in R₁ are selected from the group consisting of: aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrol-2,5-dione, furan-2(3H)-one, furan-2(5H)-one, 1,3-dioxol-2-one, oxazol-2(3H)-one, 1,3-dihydro-2H-imidazol-2-one, furan-2,5-dione, 3,6-dihydropyridin-2(1H)-one, pyridin-2,6-(1H,3H)-dione, 5,6-dihydro-2H-pyran-2-one, 3,6-dihydro-2H-pyran-2-one, 3,4-dihydro-2H-1,3-oxazine, 3,6-dihydro-2H-1,3-oxazine and 1,2,3,4-tetrahydropyrimidine.

In one embodiment of the present invention, if the C₆₋₁₀ aryl in R₁ is a 9- or 10-membered aromatic fused bicyclic ring formed by fusing a phenyl to one 5- or 6-membered monocyclic heterocyclyl ring, the 5- or 6-membered monocyclic heterocyclyl ring is selected from the group consisting of: oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrol-2,5-dione, furan-2(3H)-one, furan-2(5H)-one, 1,3-dioxol-2-one, oxazol-2(3H)-one, 1,3-dihydro-2H-imidazol-2-one, furan-2,5-dione, 3,6-dihydropyridin-2(1H)-one, pyridin-2,6-(1H,3H)-dione, 5,6-dihydro-2H-pyran-2-one, 3,6-dihydro-2H-pyran-2-one, 3,4-dihydro-2H-1,3 -oxazine, 3,6-dihydro-2H-1,3-oxazine and 1,2,3,4-tetrahydropyrimidine.

In one embodiment of the present invention, if the C₆₋₁₀ aryl in R₁ is a 9- or 10-membered aromatic fused bicyclic ring formed by fusing a phenyl to one 5- or 6-membered monocyclic cycloalkyl ring, the 5- or 6-membered monocyclic cycloalkyl ring is selected from the group consisting of: cyclopentyl, cyclopentenyl, cyclohexyl, cyclohexenyl, cyclohexdienyl, cyclopentanone, cyclopentan-1,3-dione, cyclohexanone, cyclohexan-1,3-dione.

In one embodiment of the present invention, the C₆₋₁₀ aryl in R₁ is a phenyl.

In one embodiment of the present invention, if the C₅₋₁₀ heteroaryl in R₁ is a 5- or 6-membered monoheteroaryl, the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

In one embodiment of the present invention, if the C₅₋₁₀ heteroaryl in R₁ is a 5- or 6-membered monoheteroaryl, the 5- or 6-membered monoheteroaryl has a structure selected from the group consisting of:

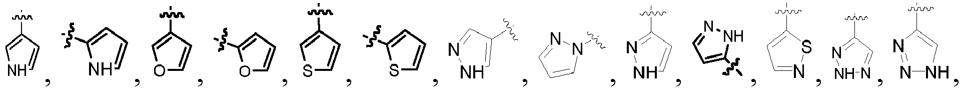

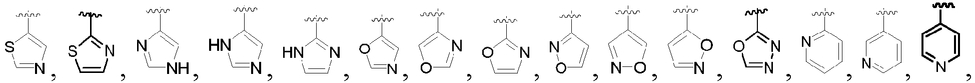

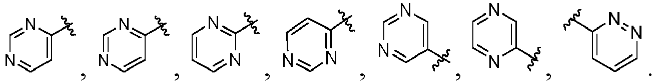

In one embodiment of the present invention, if the C₅₋₁₀ heteroaryl in R₁ is a 9- or 10-membered biheteroaryl formed by fusing a phenyl to a 5- or 6-membered monoheteroaryl, the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

In one embodiment of the present invention, if the C₅₋₁₀ heteroaryl in R₁ is a 8- to 10-membered biheteroaryl formed by fusing a 5- or 6-membered monoheteroaryl to a 5- or 6-membered monoheteroaryl, the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

In one embodiment of the present invention, if the C₅₋₁₀ heteroaryl in R₁ is a 8- to 10-membered biheteroaryl formed by fusing a 5- or 6-membered monoheteroaryl to one 5- or 6-membered monocyclic heterocyclyl ring, the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine;
the 5- or 6-membered monocyclic heterocyclyl ring is selected from the group consisting of: oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrol-2,5-dione, furan-2(3H)-one, furan-2(5H)-one, 1,3-dioxol-2-one, oxazol-2(3H)-one, 1,3-dihydro-2H-imidazol-2-one, furan-2,5-dione, 3,6-dihydropyridin-2(1H)-one, pyridin-2,6-(1H,3H)-dione, 5,6-dihydro-2H-pyran-2-one, 3,6-dihydro-2H-pyran-2-one, 3,4-dihydro-2H-1,3-oxazine, 3,6-dihydro-2H-1,3-oxazine and 1,2,3,4-tetrahydropyrimidine.

In one embodiment of the present invention, if the C₅₋₁₀ heteroaryl in R₁ is a 8- to 10-membered biheteroaryl formed by fusing a 5- or 6-membered monoheteroaryl to one 5- or 6-membered monocyclic cycloalkyl ring, the the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine;
the 5- or 6-membered monocyclic cycloalkyl ring is selected from the group consisting of: cyclopentyl, cyclopentenyl, cyclohexyl, cyclohexenyl, cyclohexdienyl, cyclopentanone, cyclopentan-1,3-dione, cyclohexanone, cyclohexan-1,3-dione.

In one embodiment of the present invention, the C₆₋₁₀ aryl in B is a phenyl, a 9- or 10-membered aromatic fused bicyclic ring formed by fusing a phenyl to one 5- or 6-membered monocyclic heterocyclyl ring, or a 9- or 10-membered aromatic fused bicyclic ring formed by fusing a phenyl to one 5- or 6-membered monocyclic cycloalkyl ring.

In one embodiment of the present invention, if the C₆₋₁₀ aryl in B is a 9- or 10-membered aromatic fused bicyclic ring formed by fusing a phenyl to one 5- or 6-membered monocyclic heterocyclyl ring, the 5- or 6-membered monocyclic heterocyclyl ring is selected from the group consisting of: oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrol-2,5-dione, furan-2(3H)-one, furan-2(5H)-one, 1,3-dioxol-2-one, oxazol-2(3H)-one, 1,3-dihydro-2H-imidazol-2-one, furan-2,5-dione, 3,6-dihydropyridin-2(1H)-one, pyridin-2,6-(1H,3H)-dione, 5,6-dihydro-2H-pyran-2-one, 3,6-dihydro-2H-pyran-2-one, 3,4-dihydro-2H-1,3 -oxazine, 3,6-dihydro-2H-1,3-oxazine and 1,2,3,4-tetrahydropyrimidine.

In one embodiment of the present invention, if the C₆₋₁₀ aryl in B is a 9- or 10-membered aromatic fused bicyclic ring formed by fusing a phenyl to one 5- or 6-membered monocyclic cycloalkyl ring, the 5- or 6-membered monocyclic cycloalkyl ring is selected from the group consisting of: cyclopentyl, cyclopentenyl, cyclohexyl, cyclohexenyl, cyclohexdienyl, cyclopentanone, cyclopentan-1,3-dione, cyclohexanone, cyclohexan-1,3-dione.

In one embodiment of the present invention, the C₅₋₁₀ heteroaryl in B is a 5- or 6-membered monoheteroaryl, a 9- or 10-membered biheteroaryl formed by fusing a phenyl to a 5- or 6-membered monoheteroaryl, a 8- to 10-membered biheteroaryl formed by fusing a 5- or 6-membered monoheteroaryl to a 5- or 6-membered monoheteroaryl, a 8- to 10-membered biheteroaryl formed by fusing a 5- or 6-membered monoheteroaryl to one 5- or 6-membered monocyclic heterocyclyl ring, or a 8- to 10-membered biheteroaryl formed by fusing a 5- or 6-membered monoheteroaryl to one 5- or 6-membered monocyclic cycloalkyl ring.

In one embodiment of the present invention, if the C₅₋₁₀ heteroaryl in R₁ and B is a 5- or 6-membered monoheteroaryl, the 5- or 6-membered monoheteroaryl are each independently selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

In one embodiment of the present invention, if the C₅₋₁₀ heteroaryl in R₁ and B is a 5- or 6-membered monoheteroaryl, the 5- or 6-membered monoheteroaryl has a structure each independently selected from the group consisting of:

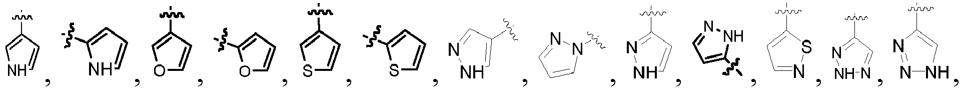

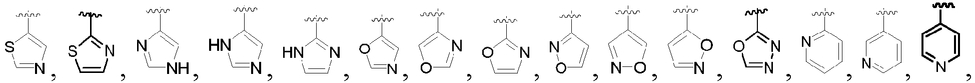

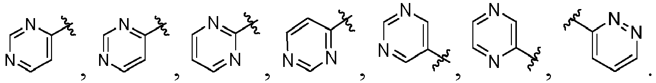

In one embodiment of the present invention, B has a structure selected from the group consisting of:

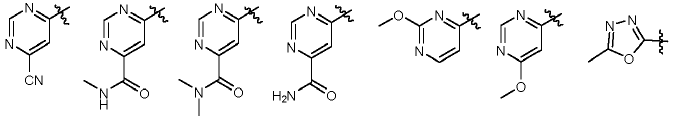

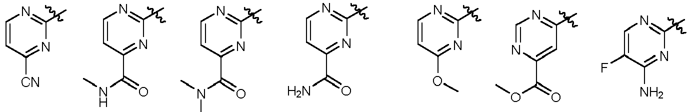

In one embodiment of the present invention, R₁ has a structure selected from the group consisting of:

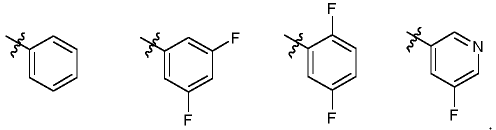

In one embodiment of the present invention, in a 9- or 10-membered biheteroaryl formed by fusing a phenyl to a 5- or 6-membered monoheteroaryl, the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

In one embodiment of the present invention, in a 8- to 10-membered biheteroaryl formed by fusing a 5- or 6-membered monoheteroaryl to a 5- or 6-membered monoheteroaryl, the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

In one embodiment of the present invention, in a 8- to 10-membered biheteroaryl formed by fusing a 5- or 6-membered monoheteroaryl to one 5- or 6-membered monocyclic heterocyclyl ring, the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine;
the 5- or 6-membered monocyclic heterocyclyl ring is selected from the group consisting of: oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H- pyrrol-2-one, 1H-pyrrol-2,5-dione, furan-2(3H)-one, furan-2(5H)-one, 1,3-dioxol-2-one, oxazol-2(3H)-one, 1,3-dihydro-2H-imidazol-2-one, furan-2,5-dione, 3,6-dihydropyridin-2(1H)-one, pyridin-2,6-(1H,3H)-dione, 5,6-dihydro-2H-pyran-2-one, 3,6-dihydro-2H-pyran-2-one, 3,4-dihydro-2H-1,3-oxazine, 3,6-dihydro-2H-1,3-oxazine and 1,2,3,4-tetrahydropyrimidine.

In one embodiment of the present invention, in a 8- to 10-membered biheteroaryl formed by fusing a 5- or 6-membered monoheteroaryl to one 5- or 6-membered monocyclic cycloalkyl ring, the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine;
the 5- or 6-membered monocyclic cycloalkyl ring is selected from the group consisting of: cyclopentyl, cyclopentenyl, cyclohexyl, cyclohexenyl, cyclohexdienyl, cyclopentanone, cyclopentan-1,3-dione, cyclohexanone, cyclohexan-1,3-dione.

In one embodiment of the present invention, the 9- or 10-membered biheteroaryl has a structure as represented by formula (a) or fomula (b): 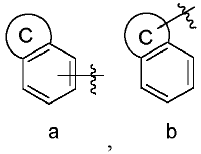 wherein, the C ring is a 5- or 6-membered monoheteroaryl; wherein the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

In one embodiment of the present invention, the C ring has structure selected from the group consisting of: 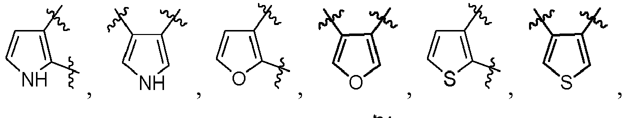 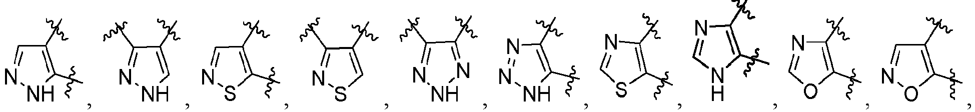 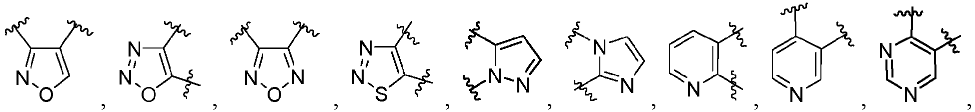 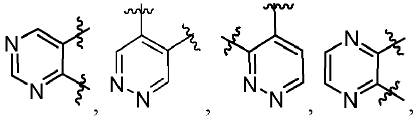 wherein the linked two ring atoms as represented by " 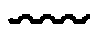 " are a pair of adjacent atoms shared as fused to another ring.

In one embodiment of the present invention, the 9- or 10-membered biheteroaryl formed by fusing a phenyl to a 5- or 6-membered monoheteroaryl is selected from the group consisting of: benzoxazole, benzisoxazole, benzimidazole, benzothiazole, benzisothiazole, benzotriazole, benzofuran, benzothiophene, indole, indazole, isoindole, quinoline, isoquinoline, quinazoline, quinoxaline and cinnoline.

In one embodiment of the present invention, the 9- or 10-membered biheteroaryl formed by fusing a phenyl to a 5- or 6-membered monoheteroaryl is selected from the group consisting of: benzo[d]isoxazole, 1H-indole, isoindole, 1H-benzo[d]imidazole, benzo[d]isothiazole, 1H-benzo[d] [1,2,3]-triazole, benzo[d]oxazole, benzo[d]thiazole, indazole, benzofuran, benzo[b]thiophene, quinoline, isoquinoline, quinazoline, quinoxaline and cinnoline.

In one embodiment of the present invention, the 8- to 10-membered biheteroaryl has a structure as represented by formula (d) or fomula (e): 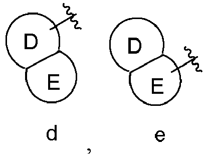 wherein, the D ring and the E ring are a 5- or 6-membered monoheteroaryl; wherein the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

In one embodiment of the present invention, the D ring and the E ring have a structure selected from the group consisting of: 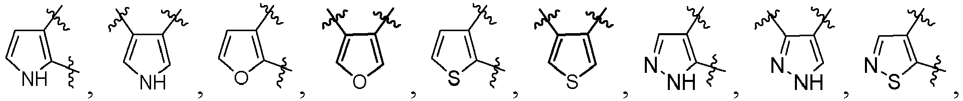 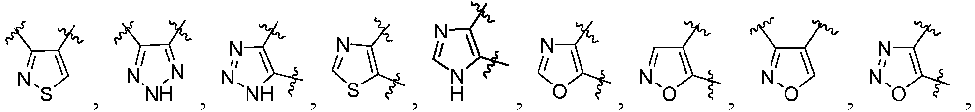 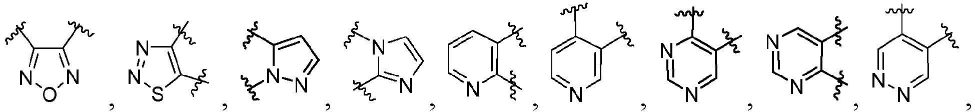 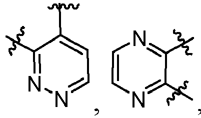 wherein the linked two ring atoms as represented by " 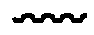 " are a pair of adjacent atoms shared as fused to another ring.

In one embodiment of the present invention, the 8- to 10-membered biheteroaryl formed by fusing a 5- or 6-membered monoheteroaryl to a 5- or 6-membered monoheteroaryl is selected from the group consisting of: pyridopyrimidine, naphthyridine, pyridopyrimidine and imidazopyridazine.

In one embodiment of the present invention, the 8- to 10-membered biheteroaryl formed by fusing a 5- or 6-membered monoheteroaryl to a 5- or 6-membered monoheteroaryl is selected from the group consisting of: pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine, pyrazolo[1,5-a]pyrimidine and imidazo[1,2-b]pyridazine.

In one embodiment of the present invention, R₁ is a substituted or unsubstituted phenyl, or a substituted or unsubstituted 5- or 6-membered monoheteroaryl, the "substituted" means that 1, 2 or 3 hydrogen atom(s) in the group are replaced by a substituent each independently selected from the group consisting of: cyano, halo (preferably, F or Cl), C₁₋₁₀ alkyl (preferably, C₁₋₆ alkyl, more preferably, C₁₋₃ alkyl), halo C₁₋₁₀ alkyl (preferably, halo C₁₋₆ alkyl, more preferably, halo C₁₋₃ alkyl), C₁₋₁₀ alkoxy (preferably, C₁₋₆ alkoxy, more preferably, C₁₋₃ alkoxy), halo C₁₋₁₀ alkoxy (preferably, halo C₁₋₆ alkoxy, more preferably, halo C₁₋₃ alkoxy).

In one embodiment of the present invention, formula (A) and formula (B) have the structures as represented by formula (A-1) and formula (B-1), respectively. 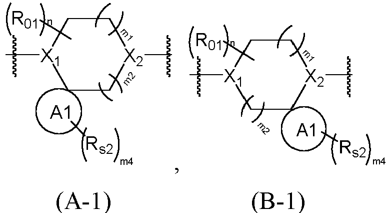
wherein R₀₁, n, m1, m2, X₁, X₂ are as defined in the specification;
the A1 ring is a 3- to 6-membered saturated monocyclic ring or a 3- to 6-membered saturated monoheterocyclyl ring;
(Rₛ₂)ₘ₄ represents that the hydrogen(s) on the A1 ring are replaced by m4 of Rₛ₂, m4 is 0, 1, 2 or 3, each of Rₛ₂ are the same or different, and are independently a substituent selected from the group S2.

In one embodiment of the present invention, A has a structure as represented by formula (A-1) or formula (B-1).

In one embodiment of the present invention, the 3- to 6-membered saturated monocyclic ring in the A1 ring is selected from the group consisting of: cyclopropyl ring, cyclobutyl ring, cyclopentyl ring and cyclohexyl ring.

In one embodiment of the present invention, the 3- to 6-membered saturated monoheterocyclyl ring in the A1 ring is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran.

In one embodiment of the present invention, formula (A) and formula (B) have the structures as represented by formula (A-2) and (B-2), respectively: 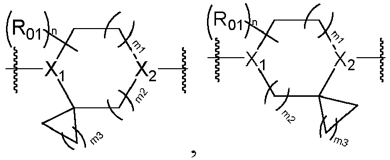 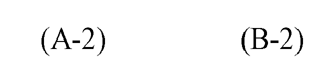 wherein R₀₁, n, m1, m2, X₁ and X₂ are as defined in the specification; m3 is 1, 2, 3 or 4.

In one embodiment of the present invention, A has a structure as represented by formula (A-2) or formula (B-2).

In one embodiment of the present invention, m3 is 1.

In one embodiment of the present invention, each of m1 and m2 is 1.

In one embodiment of the present invention, X₁ is N or CH; X₂ is N or CH. In one embodiment of the present invention, n is 0.

In one embodiment of the present invention, B has a structure as represented by: 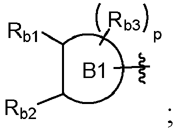
where the B1 ring is a phenyl ring, a 5- or 6-membered monoheteroaryl ring; the 5- or 6-membered monoheteroaryl ring is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine;
(R_{b3})ₚ represents that the hydrogen(s) on the ring are replaced by p of R_{b3}, p is 0, 1, 2 or 3, each of R_{b3} are the same or different, and are independently a substituent selected from the group S3;
R_{b1} and R_{b2} represent the substituents on adjacent ring atoms, and are each independently hydrogen or a substituent selected from the group S3;
or R_{b1} and R_{b2} together with the ring atoms attached thereto form a fused phenyl, a fused 5- or 6-membered monoheteroaryl ring, a fused 5- or 6-membered monocyclic heterocyclyl ring, or a fused 5- or 6-membered monocyclic cycloalkyl ring; wherein the fused 5- or 6-membered monoheteroaryl ring and the fused 5- or 6-membered monocyclic heterocyclyl ring each have 1, 2 or 3 of heteroatom(s) selected from the group consisting of N, O and S as the ring atom(s); the fused phenyl, the fused 5- or 6-membered monoheteroaryl ring, the fused 5- or 6-membered monocyclic heterocyclyl ring and fused 5- or 6-membered monocyclic cycloalkyl ring are optionally substituted by 1, 2 or 3 substituent(s) independently selected from the group S3.

In one embodiment of the present invention, the B1 ring is a phenyl ring, R_{b1} and R_{b2} represent the substituents on adjacent ring atoms, and are each independently hydrogen or a substituent selected from the group S3; or R_{b1} and R_{b2} together with the ring atoms attached thereto form a fused 5- or 6-membered monoheteroaryl ring, a fused 5- or 6-membered monocyclic heterocyclyl ring, or a fused 5- or 6-membered monocyclic cycloalkyl ring; wherein the fused 5- or 6-membered monoheteroaryl ring and the fused 5- or 6-membered monocyclic heterocyclyl ring each have 1, 2 or 3 heteroatom(s) selected from the group consisting of N, O and S as the ring atom(s); the fused 5- or 6-membered monoheteroaryl ring, the fused 5- or 6-membered monocyclic heterocyclyl ring and the fused 5- or 6-membered monocyclic cycloalkyl ring are optionally substituted by 1, 2 or 3 substituent(s) independently selected from the group S3.

In one embodiment of the present invention, the B1 ring is a 5- or 6-membered monoheteroaryl ring, wherein the 5- or 6-membered monoheteroaryl ring is selected from the group consisiting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine;

R_{b1} and R_{b2} represent the substituents on adjacent ring atoms, and are each independently hydrogen or a substituent selected from the group S3; or R_{b1} and R_{b2} together with the ring atoms attached thereto form a fused phenyl ring, a fused 5- or 6-membered monoheteroaryl ring, a fused 5- or 6-membered monocyclic heterocyclyl ring, or a fused 5- or 6-membered monocyclic cycloalkyl ring; wherein the fused 5- or 6-membered monoheteroaryl ring and the fused 5- or 6-membered monocyclic heterocyclyl ring each have 1, 2 or 3 heteroatom(s) selected from the group consisting of N, O and S as the ring atom(s); the fused phenyl ring, the fused 5- or 6-membered monoheteroaryl ring, the fused 5- or 6-membered monocyclic heterocyclyl ring and the fused 5- or 6-membered monocyclic cycloalkyl ring are optionally substituted by 1, 2 or 3 substituent(s) independently selected from the group S3.

In one embodiment of the present invention, B has a structure as represented by: 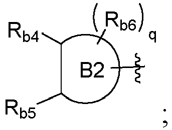
wherein the B2 ring is a 5- or 6-membered monocyclic heterocyclyl ring;
wherein the 5- or 6-membered monocyclic heterocyclyl ring is selected from the group consisting of: oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrol-2,5-dione, furan-2(3H)-one, furan-2(5H)-one, 1,3-dioxol-2-one, oxazol-2(3H)-one, 1,3-dihydro-2H-imidazol-2-one, furan-2,5-dione, 3,6-dihydropyridin-2(1H)-one, pyridin-2,6-(1H,3H)-dione, 5,6-dihydro-2H-pyran-2-one, 3,6-dihydro-2H-pyran-2-one, 3,4-dihydro-2H-1,3-oxazine, 3,6-dihydro-2H-1,3-oxazine and 1,2,3,4-tetrahydropyrimidine.
(R_{b6})_{q} represents that the hydrogen(s) on the ring are replaced by q of R_{b6}, q is 0, 1, 2 or 3, each of R_{b6} are the same or different, and are independently a substituent selected from the group S3;
R_{b4} and R_{b5} represent the substituents on adjacent ring atoms, and are each independently hydrogen or a substituent selected from the group S3;
or R_{b4} and R_{b5} together with the ring atoms attached thereto form a fused phenyl, or a fused 5- or 6-membered monoheteroaryl ring; wherein the fused 5- or 6-membered monoheteroaryl ring has 1, 2 or 3 of heteroatom(s) selected from the group consisting of N, O and S as the ring atom(s); the fused phenyl and the fused 5- or 6-membered monoheteroaryl ring are optionally substituted by 1, 2 or 3 substituent(s) independently selected from the group S3.

In one embodiment of the present invention, the fused 5- or 6-membered monoheteroaryl ring is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

In one embodiment of the present invention, the fused 5- or 6-membered monocyclic heterocyclyl ring is selected from the group consisting of: oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrol-2,5-dione, furan-2(3H)-one, furan-2(5H)-one, 1,3-dioxol-2-one, oxazol-2(3H)-one, 1,3-dihydro-2H-imidazol-2-one, furan-2,5-dione, 3,6-dihydropyridin-2(1H)-one, pyridin-2,6-(1H,3H)-dione, 5,6-dihydro-2H-pyran-2-one, 3,6-dihydro-2H-pyran-2-one, 3,4-dihydro-2H-1,3 -oxazine, 3,6-dihydro-2H-1,3-oxazine and 1,2,3,4-tetrahydropyrimidine.

In one embodiment of the present invention, the fused 5- or 6-membered monocyclic cycloalkyl ring is selected from the group consisting of: cyclopentyl, cyclopentenyl, cyclohexyl, cyclohexenyl, cyclohexdienyl, cyclopentanone, cyclopentan-1,3-dione, cyclohexanone and cyclohexan-1,3-dione.

In one embodiment of the present invention, B is substituted or unsubstituted phenyl, or substituted or unsubstituted 5- or 6-membered monoheteroaryl, the "substituted" means that 1, 2 or 3 hydrogen atom(s) in the group are replaced by a substituent independently selected from the group S3.

In one embodiment of the present invention, B is substituted or unsubstituted pyrimidine, substituted or unsubstituted pyridine, substituted or unsubstituted pyrazine, substituted or unsubstituted pyridazine, substituted or unsubstituted oxazole, substituted or unsubstituted thiazole, substituted or unsubstituted oxadiazole, substituted or unsubstituted pyrimidoimidazole, substituted or unsubstituted pyrimidopyrazole, substituted or unsubstituted pyrazo[1,5-a]pyrimidine, substituted or unsubstituted imidazo[1,2-b]pyridazine, substituted or unsubstituted quinoxaline, substituted or unsubstituted 5,7-dihydro-6H-pyrrolo[2,3-d]pyrimidin-6-one, substituted or unsubstituted 1,7-dihydro-4H-pyrazo[3,4-d]pyrimidin-4-one, substituted or unsubstituted pyrimidin-4(3H)-one; the "substituted" means that 1, 2 or 3 hydrogen atom(s) in the group are replaced by a substituent independently selected from the group S3.

In one embodiment of the present invention, B has a structure selected from the group consisting of: 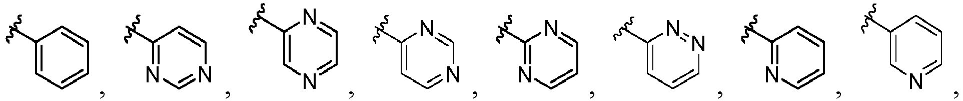 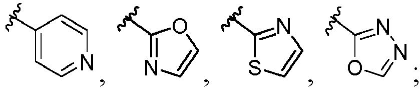 the structure is unsubstituted, or is substituted by 1, 2 or 3 substituent(s) independently selected from the group S3.

In one embodiment of the present invention, B has a structure selected from the group consisting of: 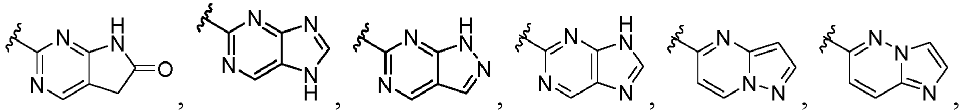 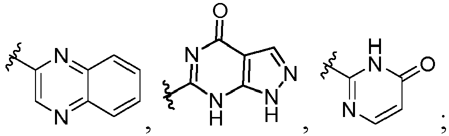 the structure is unsubstituted, or is substituted by 1, 2 or 3 substituent(s) independently selected from the group consisting of halo and C₁₋₃ alkyl.

In one embodiment of the present invention, B has a structure selected from the group consisting of:

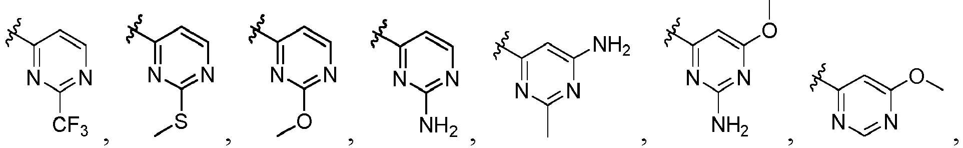

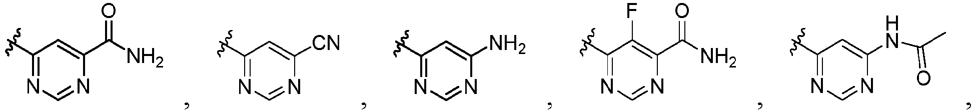

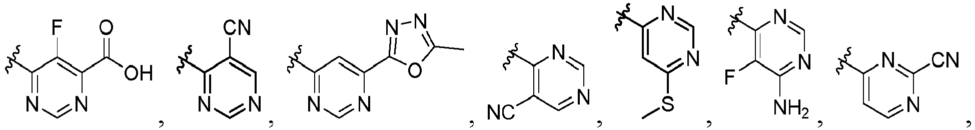

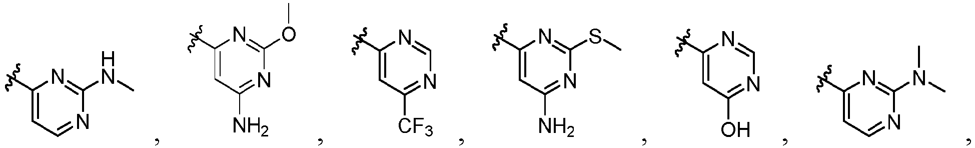

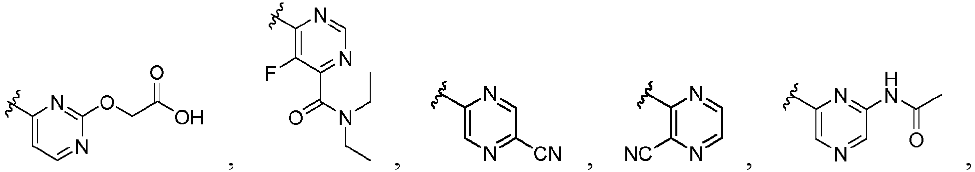

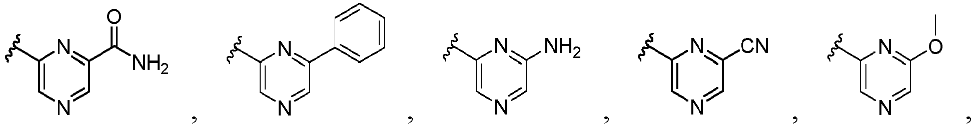

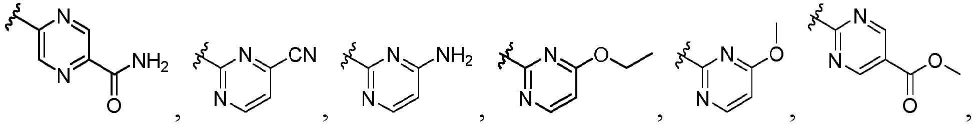

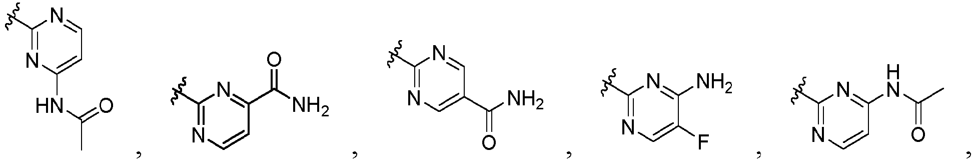

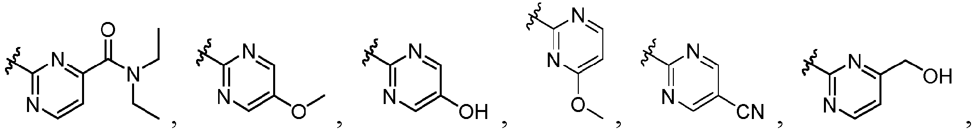

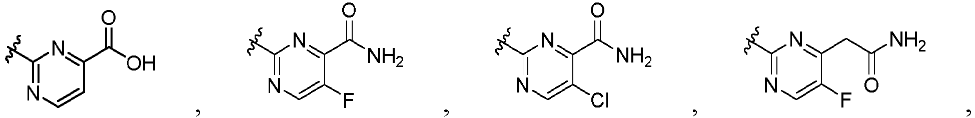

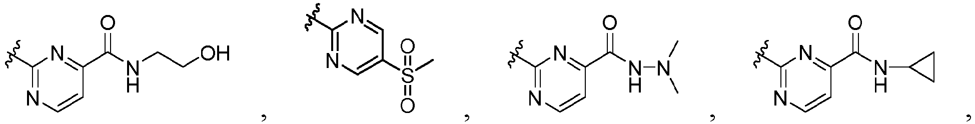

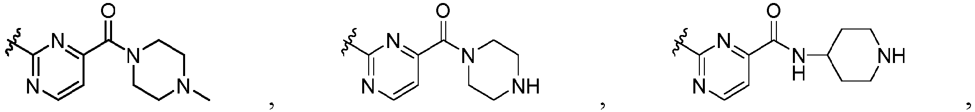

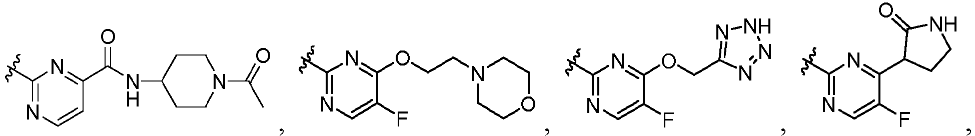

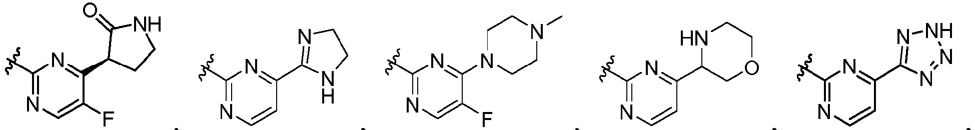

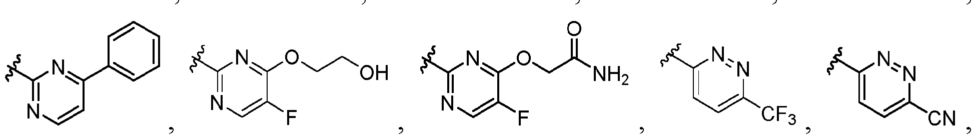

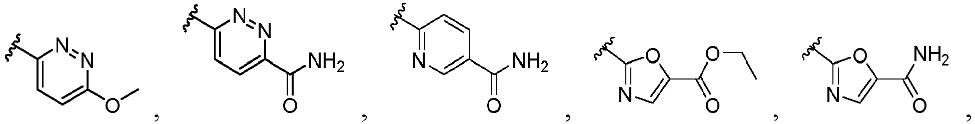

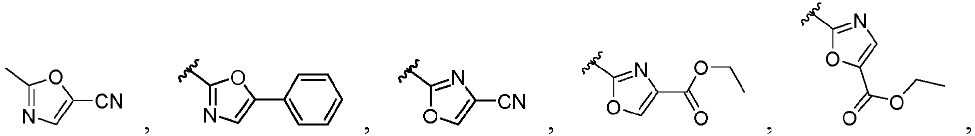

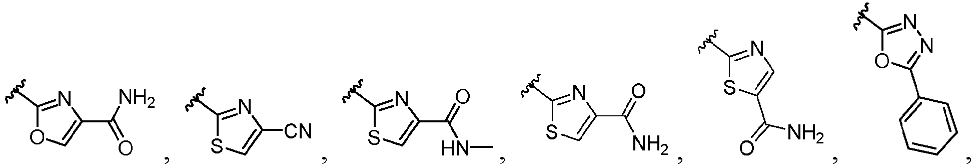

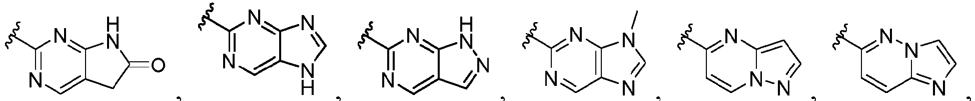

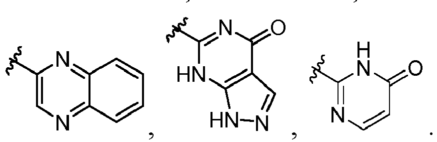

In one embodiment of the present invention, m1 and m2 is 1.

In one embodiment of the present invention, X₁ is N; X₂ is N or CH.

In one embodiment of the present invention, Rₐ and R_{b} together with the carbon atom attached thereto form a 3- to 6-membered saturated or partially unsaturated monocyclic ring selected from the group consisting of: cyclopropyl ring, cyclobutyl ring, cyclopentyl ring, cyclopentenyl ring, cyclohexyl ring, cyclohexenyl ring, cyclohexdienyl ring, cyclobutanone, cyclobutan-1,2-dione, cyclopentanone, cyclopentan-1,3-dione, cyclohexanone, cyclohexan-1,3-dione.

In one embodiment of the present invention, Rₐ and R_{b} together with the carbon atom attached thereto form a cyclopropyl ring.

In one embodiment of the present invention, Rₐ and R_{b} together with the carbon atom attached thereto form a 3- to 6-membered saturated or partially unsaturated monocyclic heterocyclyl ring selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran.

In one embodiment of the present invention, R₁' is hydrogen.

In one embodiment of the present invention, R₂' is hydrogen.

In one embodiment of the present invention, R₂ and R₂' together with the carbon atom attached thereto form a 3- to 6-membered saturated or partially unsaturated monocyclic ring selected from the group consisting of: cyclopropyl ring, cyclobutyl ring, cyclopentyl ring, cyclopentenyl ring, cyclohexyl ring, cyclohexenyl ring, cyclohexdienyl ring, cyclobutanone, cyclobutan-1,2-dione, cyclopentanone, cyclopentan-1,3-dione, cyclohexanone, cyclohexan-1,3-dione.

In one embodiment of the present invention, R₂ and R₂' together with the carbon atom attached thereto form a cyclopropyl ring.

In one embodiment of the present invention, R₂ and R₂' together with the carbon atom attached thereto form a 3- to 6-membered saturated or partially unsaturated monocyclic heterocyclyl ring selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran.

In one embodiment of the present invention, the C₃₋₆ monocyclic heterocyclyl in a substituent of the groups S11, S2 and S21 are each independently selected from the group consisting of: aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrol-2,5-dione, furan-2(3H)-one, furan-2(5H)-one, 1,3-dioxol-2-one, oxazol-2(3H)-one, 1,3-dihydro-2H-imidazol-2-one, furan-2,5-dione, 3,6-dihydropyridin-2(1H)-one, pyridin-2,6-(1H,3H)-dione, 5,6-dihydro-2H-pyran-2-one, 3,6-dihydro-2H-pyran-2-one, 3,4-dihydro-2H-1,3-oxazine, 3,6-dihydro-2H-1,3-oxazine and 1,2,3,4-tetrahydropyrimidine.

In one embodiment of the present invention, the C₃₋₆ monocyclic cycloalkyl in a substituent of the groups S11, S2 and S21 are each independently selected from the group consisting of: cyclopropyl, cyclobutyl, cyclopentyl, cyclopentenyl, cyclohexyl, cyclohexenyl, cyclohexdienyl, cyclobutanone, cyclobutan-1,2-dione, cyclopentanone, cyclopentan-1,3-dione, cyclohexanone, cyclohexan-1,3-dione.

In one embodiment of the present invention, the 5- or 6-membered monoheteroaryl in a substituent of the groups S11, S2 and S21 are each independently selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

In one embodiment of the present invention, R₁, R₂, R₃, A and B is each independently the corresponding group in the specific compounds in the Examples.

In one embodiment of the present invention, the compound of formula (I) is selected from the group consisting of the specific compounds as noted in the Examples, and is preferably a compounds selected from the group consisting of: Z-2, Z-3, Z-4, Z-5, Z-6, Z-7, Z-8, Z-9, Z-9-1, Z-9-2, Z-9-3, Z-9-4, Z-10, Z-11, Z-12, Z-13, Z-14, Z-15, Z-17, Z-18, Z-18-1, Z-18-2, Z-18-3, Z-18-4, Z-19, Z-20, Z-21, Z-22, Z-22-1, Z-22-2, Z-22-3, Z-22-4, Z-23, Z-23-1, Z-23-2, Z-23-3, Z-23-4, Z-24, Z-25, Z-26, Z-27, Z-28, Z-29, Z-30, Z-31, Z-32, Z-32-1, Z-32-2, Z-32-3-, Z-32-4, Z-33, Z-34, Z-37, Z-37-1, Z-37-2, Z-37-3, Z-37-4, Z-38, Z-38-1, Z-38-2, Z-38-3, Z-38-4, Z-39, Z-39-1, Z-39-2, Z-39-3, Z-39-4, Z-40, Z-40-1, Z-40-2, Z-41, Z-41-1, Z-41-2, Z-42, Z-42-1, Z-42-2, Z-43, Z-43-1, Z-43-2, Z-44, Z-44-1, Z-44-2, Z-45, Z-46, Z-46'-1, Z-46'-2, Z-46-1, Z-46-2, Z-47, Z-48, Z-48-1, Z-48-2, Z-48-a, Z-48-b, Z-48-c, Z-48-d, Z-49, Z-49-1, Z-49-2, Z-50, Z-51, Z-52, Z-52-1, Z-52-2, Z-53, Z-53-1, Z-53-2, Z-54, Z-55, Z-56, Z-57, Z-58, Z-59, Z-60-1, Z-60-2, Z-61, Z-62, Z-63-a, Z-63-b, Z-63-1, Z-63-2, Z-64-1, Z-64-2, Z-65-1, Z-65-2, Z-65-3, Z-65-4, Z-66, Z-67, Z-68, Z-69, Z-71, Z-70-a, Z-70-b, Z-72 and Z-73.

In one embodiment of the present invention, the compound of formula (I) is selected from the group consisting of the compounds as prepared in the Examples of the present application.

In another respect, the present invention provides a pharmaceutical composition, comprising the compound as decribed above or a pharmaceutically acceptable salt, stereoisomer, solvate or prodrug thereof; and a pharmaceutically acceptable carrier.

As used herein, the term "pharmaceutically acceptable carrier" means any formulation or carrier medium capable of delivering an effective amount of the active substance of the invention without interfering with the biological activity of the active substance and without causing adverse effects to the host or subject. Representative carriers include water, oil, vegetables and minerals, cream base, lotion base, ointment base and the like. These bases include suspension agents, viscosifiers, transdermal promoters and the like. Their formulations are well known to those skilled in the field of cosmetic or topical medicine.

In an embodiment of the present invention, the pharmaceutical compoisiton may be administered in any form of oral, spray inhalation, rectal administration, nasal administration, buccal administration, topical administration, parenteral administration, such as, subcutaneous, intravenous, intramuscular, intraperitoneal, intrathecal, intraventricular, intrasternal and intracranial injection or infusion, or administered by means of an explanted reservoir. Among these, oral, intraperitoneal or intravenous administration is preferred. When administered orally, the compound of the present invention may be formulated into any orally acceptable dosage form, including but not limited to tablets, capsules, aqueous solutions or aqueous suspensions. Carriers used in tablets typically include lactose and cornstarch. Lubricants such as magnesium stearate may also be added. Diluents used in capsules typically include lactose and dried corn starch. Aqueous suspensions are typically formulated by mixing an active ingredient with appropriate emulsifiers and suspension agents. Sweeteners, fragrances or colorants may be added to the oral dosage form as required. When topically administered, especially to the affected surface or organ readily accessible by topical application, such as eye, skin, or lower intestinal neuropathy, the compound of the present invention may be formulated into different topical dosage forms depending on the surface or organs. When topically administered to eyes, the compound of the present invention may be formulated into a dosage form of micronized suspension or solution using an isotonic sterile saline of a certain pH as the carrier, in which preservatives such as benzyl alkoxide chloride may or may not be added. For ocular administration, the compound may be formulated into a form of cream, such as, Vaseline cream. When administered topically to skin, the compound of the present invention may be formulated into a suitable dosage form of ointment, lotion or cream, in which an active ingredient is suspended or dissolved in one or more carriers. The carriers useful in an ointment formulation include but not limited to: mineral oils, liquid vaseline, white vaseline, propylene glycol, polyoxyethylene, polypropylene oxide, emulsified wax and water. The carriers useful in a lotion or cream include but not limited to: mineral oils, sorbitan monostearate, Tween 60, Cetyl ester wax, hexadecenyl aryl alcohol, 2-octyldodecanol, benzyl alcohol and water. The compound of the present invention may be administered in a dosage form of sterile injections, including sterile aqueous injection or oil suspension or sterile injection solution. Useful carriers and solvents include water, Ringer's solution and isotonic sodium chloride solution. Further, sterilized non-volatile oils can also be used as solvents or suspension media, such as monotriglycerides or diglycerides

In another respect, the present invention provides use of the dihydropyrazole compound as described above or a pharmaceutically acceptable salt, stereoisomer, solvate or prodrug thereof in the preparation of a medicament for treating and/or preventing a disease.

In an embodiment of the present invention, the diease is inflammatory bowel disease, ulcerative colitis, Crohn's disease, psoriasis, rheumatoid arthritis, NASH and heart failure.

In another respect, the present invention provides use of the dihydropyrazole compound as described above or a pharmaceutically acceptable salt, stereoisomer, solvate or prodrug thereof in the preparation of a selective RIPK1 inhibitor, the selective RIPK1 inhibitor is useful in treating a RIPK1-related disease or disorder.

In another respect, the present invention provides a method for treating a RIPK1-related disease or disorder, comprising the step of administering to a subject in need thereof a therapeutically effective amount of the dihydropyrazole compound as described above, or a pharmaceutically acceptable salt, stereoisomer, solvate or prodrug thereof, or any combination thereof, or the above pharmaceutical compostition.

In one embodiment of the present invention, the RIPK1-related disease or disorder includes, but are not limited to: inflammatory disease such as Crohn's disease and ulcerative colitis, inflammatory bowel disease, asthma, graft versus host disease, chronic obstructive pulmonary disease; autoimmune diseases such as Graves disease, rheumatoid arthritis, systemic lupus erythematosus, psoriasis; destructive bone diseases such as bone resorption diseases, osteoarthritis, osteoporosis, multiple myeloma-related bone diseases; hyperplastic diseases such as acute myeloid leukemia, chronic myelogenous leukemia; angiogenesis disorders such as angiogenesis disorders, include solid tumors, ocular neovascularization and infantile hemangiomas; infectious diseases such as sepsis, septic shock and shigellosis; neurodegenerative diseases such as Alzheimer's disease, Parkinson's disease, amyotrophic lateral sclerosis, cerebral ischemia or neurodegenerative diseases caused by a traumatic injury, tumors and viral diseases such as metastatic melanoma, Kaposi's sarcoma, multiple myeloma, HIV infection and CMV retinitis, AIDS.

In one embodiment of the present invention, the RIPK1-related disease or disorder includes, but is not limited to, pancreatitis (acute or chronic), asthma, allergies, adult respiratory distress syndrome, chronic obstructive pulmonary disease, glomerulonephritis, rheumatoid arthritis, systemic lupus erythematosus, scleroderma, Hashimoto's thyroiditis, Graves's disease, autoimmune gastritis, diabetes, autoimmune hemolytic anemia, autoimmune neutropenia, thrombocytopenia, atopic dermatitis, chronic active hepatitis, myasthenia gravis, amyotrophic lateral sclerosis, multiple sclerosis, inflammatory bowel disease, ulcerative colitis, Crohn's disease, psoriasis, graft versus host disease, inflammatory responses induced by endotoxins, tuberculosis, atherosclerosis, muscular degeneration, cachexia, psoriatic arthritis, Reiter's syndrome, gout, traumatic arthritis, rubella arthritis, acute synovitis, pancreatic beta cell disease; diseases characterized by extensive neutrophil infiltration; rheumatoid spondylitis, gouty arthritis and other arthritis conditions, cerebral malaria, chronic pulmonary inflammation, silicosis, pulmonary sarcomatosis, bone resorption diseases, allograft rejection, fever and myalgia induced by infections, cachexia secondary to infections, luteoid formation, scar tissue formation, ulcerative colitis, fever, influenza, osteoporosis, osteoarthritis, acute myeloid leukemia, chronic myelogenous leukemia, metastatic melanoma, Kaposi's sarcoma, multiple myeloma, sepsis, septic shock and Shigellosis; Alzheimer's disease, Parkinson's disease, cerebral ischemia or neurodegenerative diseases caused by traumatic injury; angiogenesis disorders, including solid tumors, ocular neovascularization, and infantile hemangiomas; viral diseases, including acute hepatitis infections (including Hepatitis A, B and C), HIV infection and CMV retinitis, AIDS, ARC or malignant tumor and herpes; stroke, myocardial ischemia, stroke heart attack, ischemia of organs, vascular proliferation, heart and kidney reperfusion injury, thrombosis, cardiac hypertrophy, the platelet aggregation induced by thrombin, entotoxemia and/or toxic shock syndrome, prostaglandin-endoperoxide synthase-2-related disorders and pemphigus vulgaris.

In one embodiment of the present invention, the RIPK1-related disease or disorder is selected from the group consisting of inflammatory bowel disease, Crohn's disease and ulcerative colitis, allograft rejection, rheumatoid arthritis, psoriasis, ankylosing spondylitis, psoriatic arthritis and pemphigus vulgaris. Alternatively, preferred diseases are selected from ischemia reperfusion injuries, including cerebral ischemia reperfusion injury induced by stroke and myocardial ischemia reperfusion injury induced by myocardial infarction.

In another respect, the present invention provides use of the dihydropyrazole compound as described above or a pharmaceutically acceptable salt, stereoisomer, solvate or prodrug thereof in the preparation of a medicament for prevending and/or treating tumor or cancer, the tumor or cancer is selected from the group consisting of: colorectal cancer, multiple myeloma, lung cancer, bone cancer, head or neck cancer, pancreatic cancer, bile duct cancer, prostate cancer, skin cancer, skin or intraocular malignant melanoma, uterine cancer, ovarian cancer, rectal cancer, anal region cancer, stomach cancer, testicular cancer, breast cancer, uterine cancer, fallopian tube cancer, endometrial cancer, cervical cancer, vaginal cancer, vulva cancer, Hodgkin's disease, non-Hodgkin's lymphoma, esophageal cancer, small intestinal carcinoma, endocrine system cancer, thyroid cancer, parathyroid carcinoma, adrenal cancer, soft tissue sarcoma, urethra cancer, penile cancer, chronic or acute leukemia, including acute myeloid leukemia, chronic myeloid leukemia, acute lymphocytic leukemia, chronic lymphocytic leukemia, pediatric solid tumor, lymphocytic lymphoma, bladder cancer, renal or ureteral cancer, renal pelvis cancer, central nervous system (CNS) tumor, primary CNS lymphoma, tumor angiogenesis, spinal tumor, brainstem glioma, pituitary adenoma, Kaposi's sarcoma, epidermoid carcinoma, squamous cell carcinoma, T-cell lymphoma, environmentally induced cancers, including asbestos-induced cancers, and a combination of the cancers.

In another respect, the present invention provides a method for treating tumor or cancer, comprising the step of administering to a subject in need thereof a therapeutically effective amount of the dihydropyrazole compound as described above, or a pharmaceutically acceptable salt, stereoisomer, solvate or prodrug thereof, or any combination thereof, or the above pharmaceutical compostition.

As used herein, the term "subject" refers to an aminal, especially a mammal, preferably, a human being.

As used herein, the term "effective amount" or "therapeutically effective amount" refers to the sufficient amount of a drug or agent that is non-toxic but has the desired effect. In an embodiment of the present invention, when treating a patient in accordance with the present invention, the amount of a given drug depends on a number of factors, such as the particular dosage regimen, the type of disease or disorder and its severity, and the uniqueness of the subject or the host in need of treatment (e.g., body weight), however, depending on the particular circumstances, including, for example, the particular drug that has been employed, the route of administration, the condition being treated, and the subject or host being treated, the dosage administered can be decided by methods routinely known in the art. Generally, for use in the treatment for an adult, the dosage administered will typically range from 0.02 to 5000 mg/day, for example from about 1 to 1500 mg/day. The desired dose may conveniently be presented as a single dose, or concurrently (or in a short period of time) or in divided doses at appropriate intervals, such as two, three, four or more divided doses per day. It will be understood by those skilled in the art that although the above dosage ranges are given, the specific effective amount can be appropriately adjusted depending on the condition of the patient and in connection with the diagnosis of the physician.

As used therein, the term "pharmaceutically acceptable salts" refers to salts of the the compound of the compound which are pharmaceutically acceptable, and have the pharmacological activity of the parent compound. The type of pharmaceutical acceptable salts includes: acid addition salts formed with inorganic acids (such as hydrochloric acid, hydrobromic acid, sulfuric acid, nitric acid, phosphoric acid, and the like); organic acids (such as acetic acid, propionic acid, hexanoic acid, cyclopentanepropionic acid, glycolic acid, pyruvic acid, trifluoroacetic acid, formic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, benzenesulfonic acid, naphthalene sulfonic acid, camphor sulfonic acid, gluconic acid, glutamic acid, hydroxynaphthalamic acid, salicylic acid, stearic acid, muconic acid and the like); or salts formed when an acidic proton present in the parent compound either is replaced by a metal ion such as an alkali metal ion or alkaline earth ion, or coordinates with an organic base such as ethanolamine, diethanolamine, triethanolamine, N-methylglucamine, and the like. The pharmaceutically acceptable salts of the present invention can be synthesized from the parent compound containing acid radicals or base radicals by conventional chemical methods. In general, such salts are prepared by the reaction of these compounds in a form of free acid or base with a stoichiometric amount of the appropriate base or acid in water or an organic solvent or a mixture of the both. In general, non-aqueous media such as ether, ethyl acetate, ethanol, isopropanol or acetonitrile are preferred. In addition to salt forms, the compounds provided herein also exist in prodrug forms. The prodrugs of the compounds described herein are readily chemically altered under physiological conditions to be converted into the compounds of the invention. In addition, prodrugs can be converted to the compounds of the present invention by chemical or biochemical methods in an *in vivo* environment.

As used herein, the term "solvent compound" and "solvate" refers to a substance formed by combining the compound of the invention with a pharmaceutically acceptable solvent. Pharmaceutical acceptable solvates include water, ethanol, acetic acid and the like. The solvates include stoichiometric solvates and non-stoichiometric solvates, preferably hydrates. Certain compounds of the present invention may be present in unsolvated or solvated forms, including hydrated forms. In general, solvated forms are equivalent to unsolvated forms and both are included within the scope of the present invention.

The present compound as represented by formula (I) may contain one or more chiral center(s), and may be present in various optically active forms. Where the comound contains one chiral center, the compound comprises enantiomers. The present invention comprises both of the isomers and a mixture of the isomers, such as, a racemic mixture. The enatiomers may be subjected to chiral resolution according to a method known in the art, such as, crystallization, chiral chromatography or the like. Where the comound contains more than one chiral center, diastereoisomers may be present. The present invention comprises an optially pure specific isomer subjected to chiral resolution and a mixture of diastereoisomers. The diastereoisomers may be subjected to chiral resolution according to a method known in the art, such as, crystallization, preparative chromatography or the like. The term "stereoisomers" include both conformational and configurational isomers, of which configurational isomers mainly include cis-trans isomers and optical isomers. The compound of the present invention may be present in a stereoisomeric form, and thereby cover all possible stereoisomeric forms, including but not limited to, cis-trans isomers, tautomers, enantiomers, diastereomers, atropisomers and the like. The compound of the present invention may also be present in a form of any combination or any mixture of the steroisomers as described above, such as, a mesomer, a racemate, mixture of equal amounts of atropisomer or the like. Exemplified is a single enantiomer, a single non-enantiomer, or a mixture thereof, or a sigle atropisomer or a mixtue thereof. Where the compound of the invention contains an olefinic double bond, it includes a cis-isomer and trans-isomer, and any combination thereof, unless otherwise specified.

As used herein, the term "alkyl" refers to a liner or branched saturated aliphatic hydrocarbon group having 1 to 20 carbon atoms. The term "C₁₋₁₀ alkyl" refers to a liner or branched alkyl group having 1 to 10 carbon atoms, more preferably 1, 2, 3, 4, 5 or 6 carbon atoms, i.e., C₁₋₆ alkyl, more preferably, C₁₋₄ alkyl, the most preferably, C₁₋₃ alkyl. Specific examples include, but are not limited to, methyl, ethyl, n-propyl, iso-propyl, n-butyl, iso-butyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and various branched isomers thereof.

As used herein, the term "alkoxy" refers to a group having a structure of -O-alkyl, wherein the alkyl is defined as above. The term "C₁₋₁₀ alkoxy" refers to an alkoxy group having 1 to 10 carbon atoms, preferably, C₁₋₆ alkoxy, more preferably, C₁₋₄ alkoxy, the most preferably, C₁₋₃ alkoxy. Specific examples include, but are not limited to, methoxy, ethoxy, n-propoxy, iso-propoxy, n-butoxy, tert-butoxy, iso-butoxy, n-pentoxy and the like.

As used herein, the term "alkylthio" refers to a group having a structure of -S-alkyl, wherein the alkyl is defined as above. The term "C₁₋₁₀ alkylthio" refers to an alkylthio group having 1 to 10 carbon atoms, preferably, C₁₋₆ alkylthio, more preferably, C₁₋₄ alkylthio, more preferably, C₁₋₃ alkylthio. Specific examples include, but are not limited to, methylthio, ethylthio, propylthio, iso-propylthio, butylthio, tert-butylthio, iso-butylthio, pentylthio and the like.

As used herein, the term "alkenyl" refers to an alkyl as defined above having one or more carbon-carbon double bond at any position of the chain. The term "C₂₋₈ alkenyl" refers to an alkenyl group having 2 to 8 carbon atoms and at least one carbon-carbon double bonds, prefereably, an alkenyl group having 2 to 6 carbon atoms and 1 to 2 carbon-carbon double bonds, i.e., C₂₋₆ alkenyl, more preferably, an alkenyl group having 2 to 4 carbon atoms and 1 to 2 carbon-carbon double bonds, i.e., C₂₋₄ alkenyl. Specific examples include, but are not limited to, vinyl, 1-propenyl, 2-propenyl, 1-, 2- or 3-butenyl, pentenyl, hexenyl, butadienyl, and the like.

As used herein, the term "alkynyl" refers to an alkyl as defined above having one or more carbon-carbon triple bonds at any position of the chain. The term "C₂₋₈ alkynyl" refers to an alkynyl group having 2 to 8 carbon atoms and at least one carbon-carbon triple bonds, prefereably, an alkynyl group having 2 to 6 carbon atoms and 1 to 2 carbon-carbon triple bonds, i.e., C₂₋₆ alkynyl, more preferably, an alkynyl group having 2 to 4 carbon atoms and 1 to 2 carbon-carbon triple bonds, i.e., C₂₋₄ alkynyl. Specific examples include, but are not limited to, ethynyl, 1-propynyl, 2-propynyl, 1-, 2- or 3-butynyl, and the like.

As used herein, the term "halogen" refers to fluoro, chloro, bromo and iodine.

As used herein, the term "haloalkyl" refers to an alkyl as defined above which is substituted by one or more (1, 2, 3, 4 or 5) halogens. The term "halo C₁₋₁ alkyl" refers to a haloalkyl having 1 to 10 carbon atoms, preferably, halo C₁₋₆ alkyl, more preferably, halo C₁₋₄ alkyl, most preferably, halo C₁₋₃ alkyl. Specific examples include, but are not limited to, chloromethyl, dichloromethyl, trichloromethyl, chloroethyl, 1,2-dichloroethyl, trichloroethyl, bromoethyl, fluoromethyl, difluoromethyl, trifluoromethyl, fluoroethyl, difluoroethyl, trifluoroethyl, and the like.

As used herein, the term "haloalkoxy" refers to an alkoxy as defined above which is substituted by one or more (1, 2, 3, 4 or 5) halogens. The term "halo C₁₋₁₀ alkoxy" refers to a haloalkoxy having 1 to 10 carbon atoms, preferably, halo C₁₋₆ alkoxy, more preferably, halo C₁₋₄ alkoxy, most preferably, halo C₁₋₃ alkoxy. Specific examples include, but are not limited to, trifluoromethoxy, trifluoroethoxy, fluoromethoxy, fluoroethoxy, difluoromethoxy, difluoroethoxy, and the like.

As used herein, the term "cycloalkyl" and "cycloalkyl ring" are used exchangeably to refer to a saturated or partially unsaturated monocyclic or polycyclic cyclic hydrocarbyl substituent. The cycloalkyl ring contains 3 to 20 carbon atoms (C₃₋₂₀), preferably, 3 to 12 carbon atoms (C₃₋₁₂), more preferably, 3 to 10 carbon atoms (C₃₋₁₀), the most preferably, 3 to 6 carbon atoms (C₃₋₆). A ring carbon atom in the cycloalkyl may be optionally replaced by 1, 2 or 3 oxo group(s) to form a cyclic ketone structure. In a situation of a monocyclic cycloalkyl, the monocyclic cycloalkyl is saturated or partially unsaturated, and is preferably a monocyclic cycloalkyl containing 3 to 8 ring carbon atoms (i.e., 3- to 8-memered or C₃₋₈), preferably, 3 to 6 ring carbon atoms. Non-limiting examples of a monocyclic cycloalkyl include cyclopropyl, cyclobutyl, cyclopentyl, cyclopentenyl, cyclohexyl, cyclohexenyl, cyclohexdienyl, cycloheptyl, cycloheptatrienyl, cyclooctyl, cyclobutanone, cyclobutan-1,2-dione, cyclopentanone, cyclopentan-1,3-dione, cyclohexanone, cyclohexan-1,3-dione and the like. A polycyclic cycloalkyl includes spirocycloalkyl, fused cycloalkyl and bridged cycloalkyl. The cycloalkyl ring may be fused to an aryl ring, a heteroaryl ring or a heterocyclyl ring, wherein the ring attached to the parent structure is the cycloalkyl ring. Non-limiting examples include indanyl, tetralyl, benzocycloheptyl and the like. The cycloalkyl may be optionally substituted or unsubstituted. When substituted, the substituent(s) are preferably one or more substituents as described in the present disclosure.

As used herein, the term "heterocyclyl" and "heterocyclyl ring" are used exchangeably to refer to a saturated or partially unsaturated monocyclic or polycyclic cyclic hydrocarbyl containing 3 to 20 ring atoms (i.e., 3- to 20-membered or C₃₋₂₀), wherein one or more (preferably, 1 to 4) ring atoms are heteroatoms selected from the group consisting of nitrogen, oxygen or S(O)ₘ (wherein m is an integer from 0 to 2), but excluding a cyclic moity of -O-O-, -O-S- or -S-S-, and the remaining ring atoms are carbon atoms. The nitrogen atom may be substituted or unsubstituted (i.e., N or NR, R is hydrogen or another substituent as defined herein). The carbon atom in the ring of the heterocyclyl may be optionally replaced by 1, 2 or 3 oxo group(s) to form a structure of a cyclic ketone, cyclic lactone or cyclic lactam. Preferably, the heterocyclyl contains 3 to 12 ring atoms, more preferably, 3 to 10 ring atoms, wherein 1 to 4 of which are heteroatoms.

In certain embodiments of the present invention, the "heterocyclyl" refers to a monocyclic heterocyclyl, wherein the monocyclic heterocyclyl is saturated or partially unsaturated. Preferred is a monocyclic heterocyclyl containing 3 to 8 ring atoms (i.e., 3- to 8-membered or C₃₋₈), wherein 1 to 3 of which are heteroatoms. More preferred is a monocyclic heterocyclyl containing 3 to 6 ring atoms (i.e., 3- to 6-membered or C₃₋₆), wherein 1 to 2 of which are heteroatoms. The most preferred is a monocyclic heterocyclyl containing 5 or 6 ring atoms (i.e., 5 or 6-membered or C₅₋₆), wherein 1 to 2 of which are heteroatoms. If the heteroatom is a nitrogen atom, the nitrogen atom may be substituted or unsubstituted (i.e., N or NR, R is hydrogen or another substituent as defined herein). If the heteroatom is a sulfur atom, the sufur atom may be optionally oxidized (i.e., S(O)ₘ, m is an integer from 0 to 2). The carbon atom in the ring of the monocyclic heterocyclyl may be optionally replaced by 1, 2 or 3 oxo group(s) to form a structure of a cyclic ketone, cyclic lactone or cyclic lactam. Non-limiting examples of a monocyclic heterocyclyl include: aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimid-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrol-2,5-dione, furan-2(3H)-one, furan-2(5H)-one, 1,3-dioxol-2-one, oxazole-2(3H)-one, 1,3-dihydro-2H-imidazole-2-one, furan-2,5-dione, 3,6-dihydropyridin-2(1H)-one, pyridin-2,6-(1H,3H)-dione, 5,6-dihydro-2H-pyran-2-one, 3,6-dihydro-2H-pyran-2-one, 3,4-dihydro-2H-1,3 -oxazine, 3,6-dihydro-2H-1,3-oxazine, 1,2,3,4-tetrahydroopyrimidine and the like. The adjacent two ring atoms in the above monocyclic heterocyclyl, including C-C, N-C, may be optionally fused to a cyclokyl, heterocyclyl, aryl or heteroaryl, such as, the monocyclic cylcoalkyl ring, monocyclic heterocyclyl ring, monoaryl ring or 5- or 6-membered monoheteroaryl ring as defined herein, to form a fused polycyclyl. The adjacent two ring atoms in the above monocyclic heterocyclyl fused to another ring are preferably C-C. In certain embodiments of the present invention, the "heterocyclyl" refers to a polycyclic heterocyclyl, including spiroheterocyclyl, fused heterocyclyl and bridged heterocyclyl. In the present invention, each of the above types of heterocyclyl may be optionally substituted or unsubstituted. If substituted, the substituent(s) are preferably one or more substituents as described in the present disclosure.

As used herein, the terms "aryl", "aryl ring" and "aromatic ring" are used interchangeably to refer to an all-carbon monocyclyl, an all-carbon polycyclyl (a ring is linked to another by a covalent bond, non-fused) or an all-carbon fused polycyclyl (i.e., a pair of adjacent carbon atoms are shared between the rings) group having 6 to 14 ring atoms (i.e., 6- to 14-membered or C₆₋₁₄), wherein at least one ring is aromatic in the ring system, that is, has a π electron conjugated system. Preferred is an aryl having 6 to 10 ring atoms (i.e., 6- to 10-membered or C₆₋₁₀). Each of the rings in he ring system contains 5 or 6 ring atoms. The terms of "aryl" and "aryl ring" may be used interchangeably. In certain embodiments of the present invention, the "aryl" refers to a monocyclic aryl or polycyclic aryl ring. Non-limiting examples include phenyl, biphenylyl, and the like. In certain embodiments of the present invention, the "aryl" refers to an aromatic fused polycyclyl, which is a polycyclic group formed by fusing a monocyclic aryl ring to one or more monocyclic aryl ring. Non-limiting examples include naphthyl, anthryl, and the like. In certain embodiments of the present invention, the "aryl" refers to an aromatic fused polycyclyl (preferably, a 9- or 10-membered aromatic fused polycyclyl). The aromatic fused polycyclyl is a polycyclic group formed by fusing a monocyclic aryl ring (such as phenyl) to one or more non-aromatic rings, wherein the ring attached to the parent structure is an aromatic or non-aromatic ring. The non-aromatic ring includes, but is not limited to, a 3- to 6-membered monocyclic heterocyclyl ring, preferably, a 5- or 6-membered monocyclic heterocyclyl ring (the ring carbon atom in the monocyclic heterocyclyl ring may be replaced by 1 or 2 oxo groups to form a structure of cyclic lactam or cyclic lactone), a 3- to 6-membered monocyclic cycloalkyl ring, preferably, a 5- or 6-membered monocyclic cycloalkyl ring (the ring carbon atom in the monocyclic cycloalkyl ring may be replaced by 1 or 2 oxo goups to form a structure of cyclic ketone), and the like. The above polycyclic group formed by fusing a monocyclic aryl ring to one or more non-aromatic rings may be linked to other moiety or the parent structure through a nitrogen atom or carbon atom. The ring attached to the parent structure is an aromatic or non-aromatic ring.

In the present disclosure, the 9- or 10-membered aromatic fused bicyclic ring formed by fusing a phenyl to one 5- or 6-membered monocyclic heterocyclyl ring means that the adjacent two substitutes in the phenyl together with the ring atoms attached thereto form a fused 5- or 6-membered monocyclic heterocyclyl ring defined as above. The formed 9- or 10-membered aromatic fused bicyclic ring may also be referred to as a 9- or 10-membered phenylheterocycloalkyl ring.

In the present disclosure, the 9- or 10-membered aromatic fused bicyclic ring formed by fusing a phenyl to one 5- or 6-membered monocyclic cycloalkyl ring means that the adjacent two substitutes in the phenyl together with the ring atoms attached thereto form a fused 5- or 6-membered monocyclic cycloalkyl ring defined as above. The formed the 9- or 10-membered aromatic fused bicyclic ring may also be referred to as a 9- or 10-membered phenylcycloalkyl ring. The non-limiting examples include:

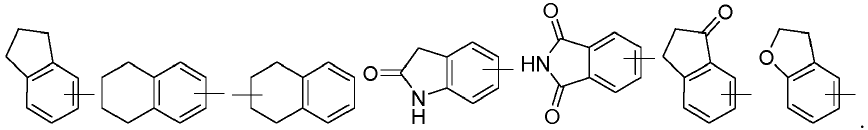

In the present invention, each of the above types of aryl may be substituted or unsubstituted. If substituted, the substituent(s) are preferably one or more substituents as described in the present disclosure.

As use herein, the terms "heteroaryl", "heteroaryl ring" and "heteroarylcycle" are used exchangably to refer to a monocyclic or fused polycyclic (i.e., a pair of adjacent carbon atoms are shared between the rings) group containing 1 to 4 heteroatoms, which has 5 to 14 ring atoms (i.e., 5- to 14-memered or C₅₋₁₄), preferably, 5 to 10 ring atoms (i.e., 5- to 10-memered or C₅₋₁₀), more preferably, 5, 6, 8, 9 or 10 ring atoms, wherein the heteroatom is selected from the group consisting of nitrogen, oxygen and sulfur, wherein the nitrogen and sulfur atoms may be optionally oxidized, and the nitrogen atom may be optionally quaternized. The heteroaryl group has 6, 10 or 14 π electrons as shared in the ring system. In the ring system at least one ring is aromatic. The terms "heteroaryl" and "heteroaryl ring" may be used exchangably.

In certain embodiments of the present invention, the "heteroaryl" refers to a monoheteroaryl ring (preferably, 5- or 6-membered monoheteroaryl ring). Non-limiting examples of the monoheteroaryl include thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine and the like. In certain embodiments of the present invention, the "heteroaryl" refers to a fused polycyclic heteroaryl ring (preferably, 8- to 10- membered biheteroaryl ring). The fused polycyclic heteroaryl ring includes both a polycyclic group (prefererably, 9- or 10-membered biheteroaryl ring) formed by fusing a monoaryl ring (such as phenyl) to a monoheteroaryl ring (preferably, 5- or 6-membered monoheteroaryl ring), and a polycyclic group (prefererably, 8- to 10-membered biheteroaryl ring) formed by fusing a monoheteroaryl (preferably, 5- or 6-membered monoheteroaryl) to a monoheteroaryl (preferably, 5- or 6-membered monoheteroaryl). Any two adjacent ring atoms in the above monoheteroaryl ring, including C-C, N-C, N-N, may be fused to the cycloalkyl, heterocyclyl, aryl or heteroaryl such as the monocyclic cycloalkyl ring, monocyclic heterocyclyl ring, monoaryl ring, 5- or 6-membered monoheteroaryl ring and the like as define in the present disclosure, to form a fused polycyclyl. The two adjacent ring atoms in the monoheteroaryl ring, which are fused to another ring to form a fused ring, are preferably C-C, and include in a non-limiting way the forms of:

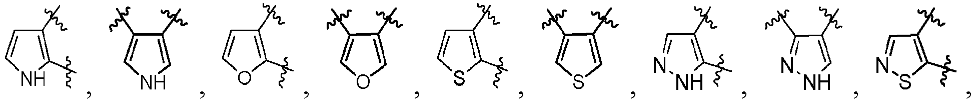

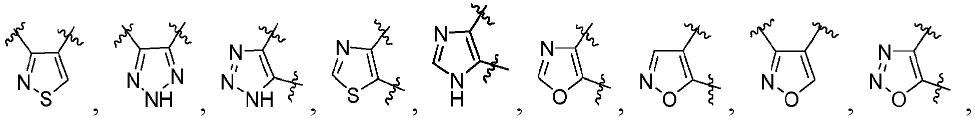

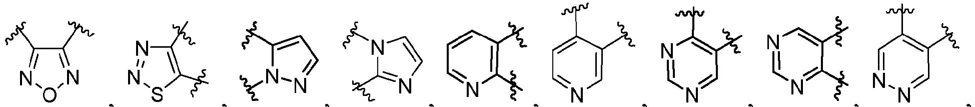

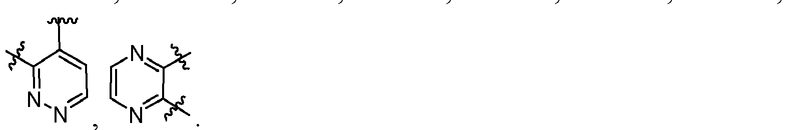

Non-limiting examples of fused polycyclic heteroaryl ring include benzo[d]isoxazole, 1H-indole, isoindole, 1H-benzo[d]imidazole, benzo[d]isothiazole, 1H-benzo[d][1,2,3]triazole, benzo[d]oxazole, benzo[d]thiazole, indazole, benzofuran, benzo[b]thiophene, quinoline, isoquinoline, quinazoline, quinoxaline, cinnoline, pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine, pyrazolo[1,5-a]pyrimidine, imidazolo[1,2-b]pyridazine, and the like.

The above monoheteroaryl, the polycyclic group formed by fusing a monocyclic aryl ring to a monoheteroaryl ring, or the polycyclic group formed by fusing a monoheteroaryl to a monoheteroaryl may be linked to other moiety or the parent structure through a nitrogen atom or carbon atom. In the case of a polycyclic group, the ring linked to the parent structure is a heteroaryl ring, an aryl ring, a monocyclic cycloalkyl ring or a monocyclic heterocyclyl ring. Non-limiting examples include:

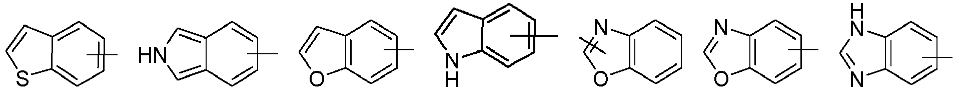

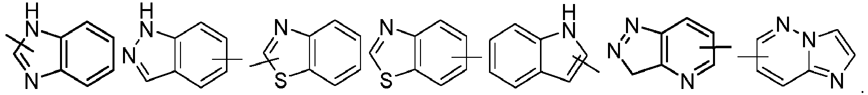

In certain embodiments of the present invention, the "heteroaryl" refers to a fused polycyclic heteroaryl group (preferably, 8- to 10-membered biheteroaryl ring). The fused polycyclic heteroaryl ring is a polycyclic group formed by fusing a monoheteroaryl ring (preferably, 5- or 6-membered monoheteroaryl ring) to one or more non-aromatic rings, wherein the ring linked to the parent structure is a heteroaryl ring or a non-aromatic ring, the non-aromatic ring includes, but is not limited to, 3- to 6- membered (preferably, 5- or 6-membered) monocyclic heterocyclyl ring (the ring carbon atom in the monocyclic heterocyclyl ring may be replaced by 1 to 2 oxo groups, to form a structure of cyclic lactam or cyclic lactone), 3- to 6-membered (preferably, 5- or 6-membered) monocyclic cycloalkyl ring (the ring carbon atom in the monocyclic cycloalkyl ring may be replace by 1 to 2 oxo, to form a structure of cyclic ketone) and the like. The polycyclic group formed by fusing a monoheteroaryl ring to one or more non-aromatic ring may be linked to other moiety or the parent structure through a nitrogen atom or carbon atom, and the ring linked to the parent structure is a heteroaryl ring or non-aromatic ring.

In the present disclosure, the 8- to 10-membered biheteroaryl formed by fusing a 5- or 6-membered monoheteroaryl to one 5- or 6-membered monocyclic heterocyclyl ring means that the adjacent two substitutes in the 5- or 6-membered monoheteroaryl together with the ring atoms attached thereto form a fused 5- or 6-membered monocyclic heterocyclyl ring as defined above. The formed 8- to 10-membered biheteroaryl may also be referred to as 8- to 10-membered heteroaryl heterocycloalkyl ring.

In the present disclosure, the 8- to 10-membered fused biheteroaryl formed by fusing a 5- or 6-membered monoheteroaryl to one 5- or 6-membered monocyclic cycloalkyl ring means that the adjacent two substitutes in the 5- or 6-membered monoheteroaryl together with the ring atoms attached thereto form a fused 5- or 6-membered monocyclic cycloalkyl ring as defined above. The formed the 8- to 10-membered biheteroaryl may also be referred to as 8- to 10-membered heteroaryl cycloalkyl ring. Non-limiting examples include:

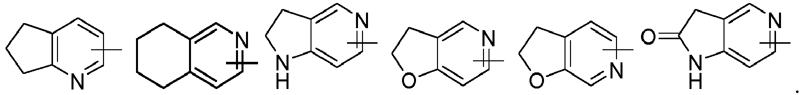

In the present invention, each of the above types of heteroaryl may be substituted or unsubstituted. When substituted, the substituent(s) are preferably one or more substituents as described in the present disclosure.

As used herein, the "3- to 6-membered saturated or partially unsaturated monocyclic ring" refers to a saturated or partially unsaturated all-carbon monocyclic group having 3 to 6 ring atoms. The ring carbon atoms in the monocyclic ring may be optionally replaced by 1, 2 or 3 oxo groups, to form a structure of cyclic ketone. Examples of 3- to 6-membered saturated or partially unsaturated monocyclic ring include (but are not limited to): cyclopropyl ring, cyclobutyl ring, cyclopentyl ring, cyclopentenyl ring, cyclohexyl ring, cyclohexenyl ring, cyclohexdienyl ring, cyclobutanone, cyclobutan-1,2-dione, cyclopentanone, cyclopentan-1,3-dione, cyclohexanone, cyclohexan-1,3-dione, and the like.

As used herein, the "3- to 6-membered saturated or partially unsaturated monoheterocyclyl ring" means that 1, 2 or 3 carbon atoms in the 3- to 6-membered monocyclic ring are replaced by a heteroatom selected from the group consisting of nitrogen, oxygen or S(O)ₜ (wherein t is an integer from 0 to 2), but excluding a cyclic moity of -O-O-, -O-S- or -S-S-, and the remaining ring atoms are carbon; and is preferably 4- to 6-membered, more preferably, 5- to 6-membered. The ring carbon atom in the monoheterocyclyl ring may be optionally replaced by 1, 2 or 3 oxo group(s) to form a structure of a cyclic ketone, cyclic lactone or cyclic lactam. Examples of 3- to 6-membered saturated or partially unsaturated monoheterocyclyl ring include (but are not limited to) aziridine, oxirane, azetidine, oxetane, tetrahydrofuran, tetrahydrothiophene, tetrahydropyrrole, piperidine, pyrroline, oxazolidine, piperazine, dioxolane, dioxane, morpholine, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, and the like.

As used herein, the term "hydroxyl" refers to -OH group.

As used herein, the term "hydroxymethyl" refers to -CH₂OH, and "hydroxyethyl" refers to -CH₂CH₂OH or -CH(OH)CH₃.

As used herein, the term "cyanomethyl" refers to -CH₂CN, and "cyanoethyl" refers to -CH₂CH₂CN or -CHCNCH₃.

As used herein, the term "halo" refers to fluoro, chloro, bromo or iodo.

As used herein, the term "amino" refers to -NH₂.

As used herein, the term "cyano" refers to -CN.

As used herein, the term "nitro" refers to -NO₂.

As used herein, the term "benzyl"refers to -CH₂-phenyl.

As used herein, the term "oxo group"refers to =O.

As used herein, the term "carboxyl"refers to -C(O)OH.

As used herein, the term "carboxylate group"refers to -C(O)O(alkyl) or-C(O)O(cycloalkyl).

As used herein, the term "acetyl"refers to -COCH₃.

As used herein, the term "substituted" means that any one or more hydrogen atoms on a particular atom are replaced with substituents, including deuterium and hydrogen variants, as long as the valence of a particular atom is normal and the substituted compound is stable. When the substituent is an oxo group (i.e., =O), it means that two hydrogen atoms are replaced. Replacement of an oxo group does not occur on aromatic groups. The term "optionally substituted" means that it may or may not be substituted. Unless otherwise specified, the type and number of substituents may be arbitrary on the basis of being chemically achievable.

When any variant (e.g., R) occurs more than once in the constitution or structure of a compound, its definition in each case is independent. Thus, for example, if a group is substituted by 0-2 of R, the group may optionally be substituted with up to two R, and R in each case has an independent option. In addition, combinations of substituents and/or variants thereof are permissible only if such combinations result in stable compounds.

### Detailed Description of the Invention

The compound of the present invention can be prepared by a variety of synthetic methods well known to those skilled in the art, including the specific embodiments listed below, the embodiments obtained by combining those listed below with other chemical synthesis methods, and the equivalent alternatives well known to those skilled in the art. Preferred embodiments include, but are not limited to, the Examples of the present invention.

It is to be understood that the absolute configuration of a stereoisomer obtained by seperation in the Examples below is arbitrarily assigned.

The present invention is illustrated in details by means of the Examples, but is not meant to be unfavorably limited thereto. In the present disclosure, the present invention has been described in details, wherein specific embodiments thereof are also disclosed. It will be apparent to those skilled in the art that various changes and modifications can be made to the specific embodiments of the present invention without departing from the spirit and scope of the present invention. If specific conditions are not indicated in the Examples, it shall be carried out in accordance at conventional conditions or those recommended by the manufacturer. The reagents or instruments without their manufacturers indicated are all conventional products that can be purchased commercially.

In the present disclosure, the room temperature refers to about 20-25°C.

Method for measuring the retention time of the product obtained in the Examples (LCMS method): column: ZORBAX Extend-C18, 2.1×50mm, 5µm; mobile phase A: H₂O (0.02% FA); mobile phase B: ACN (0.02% FA); grident: mobile phase B, from 20% to 95% to 20%; running time: 3 min; flow rate: 1.5 ml/min; column temperature: 40°C.

Abbreviations: ACN: acetonitrile; IPA: isopropanol; AMMN: 7 M ammonia solution in methanol; DEA: diethylamine; FA: formic acid, Hex: hexane, EtOH: ethanol, HATU: 2-(7-azabenzotriazole)-N,N,N',N'-tetramethyluronium hexafluorophosphate.

### Example 2: Preparation of 6-(8-(5-phenyl-4,5-dihydro-1H-pyrazole-1-carbonyl)-3-azabicyclo[3.2.1]oct-3-yl)pyrimidine-4-carbonitrile (racemate, Z-2) and 6-(8-(5-phenyl-4,5-dihydro-1H-pyrazole-1-carbonyl)-3-azabicyclo[3.2.1]oct-3-yl)pyrimidine-4-carbonitrile (racemate, Z-3)

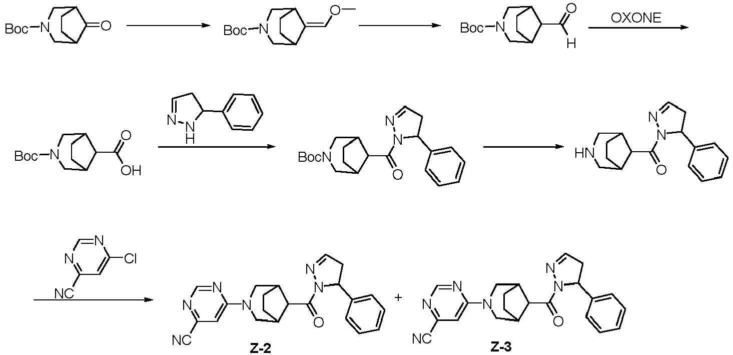

Step 1: tert-butyl 8-oxo-3-azabicyclo[3.2.1]octane-3-carboxylate (8.8 g, 39.1 mmol) and (methoxymethyl)triphenylphosphine chloride (24.1 g, 70.4 mmol) were dissolved in tetrahydrofuran (150 mL). The reaction mixture was cooled to 0°C, to which potassium tert-butoxide (7.88 g, 70.4 mmol) was added. The reaction mixture was slowly warmed to 25°C, and stirred for 18 h. The resultant reaction mixture was quenched with a diluted solution of ammonium chloride solution, and extracted with dichloromethane (150 mL × 2). The organic phase was washed with saline, dried over anhydrous magnesium sulfate, and rotary evaporated to remove the solvent. The resultant residue was subjected to column chromotography (petroleum ether/ethyl acetate=5/1) to give tert-butyl 8-methoxymethylene-3-azabicyclo[3.2.1]octane-3-carboxylate (7.25 g, yield: 73%). ES-API:[M+H]⁺= 254.2.

Step 2: tert-butyl 8-methoxymethylene-3-azabicyclo[3.2.1]octane-3-carboxylate (7.25 g, 28.7 mmol), p-toluenesulfonic acid monohydrate (5.72 g, 30.1 mmol) and water (1.03 g, 57.3 mmol) were added to acetone (150 mL). The mixture was stirred at 25°C for 18 h. The resultant reaction mixture was used in the next step of reaction without further treatment. ES-API:[M+H]⁺= 240.1.

Step 3: A potassium monopersulfate complex salt (OXONE) (36.7 g, 57.32 mmol) and water (7 mL) were added to the reaction mixture from the previous step. The reaction mixture was reacted at 25°C for 2 h. The resultant reaction mixture was extracted with dichloromethane (150 mL × 2). The organic phases were combined, washed with saline, dried over anhydrous magnesium sulfate, and rotary evaporated to remove the solvent to give 3-(tert-butoxycarbonyl)-3-azabicyclo[3.2.1]octane-8-carboxylic acid (3.3 g, yield: 45%)ES-API:[M+H]⁺= 256.1.

Step 4: A mixture of 3-(tert-butoxycarbonyl)-3-azabicyclo[3.2.1]octane-8-carboxylic acid (100 mg, 0.39 mmol), 5-phenyl-4,5-dihydro-1H-pyrazole (286 mg, 1.96 mmol), HATU (179 mg, 0.47 mmol) and triethylamine (79 mg, 0.78 mmol) in dichloromethane (2 mL) was stirred at 25°C for 2 h. The resultant reaction mixture was washed with saline, dried over anhydrous magnesium sulfate, and rotary evaporated to remove the solvent. The residue was subjected to column chromatography (petroleum ether/ethyl acetate=3/1) to give tert-butyl 8-(5-phenyl-4,5-dihydro-1H-pyrazole-1-carbonyl)-3-azabicyclo[3.2.1]octane-3-carboxylate (45 mg, yield: 30%), ES-API:[M+H]⁺= 384.2.

Step 5: A mixture of tert-butyl 8-(5-phenyl-4,5-dihydro-1H-pyrazole-1-carbonyl)-3-azabicyclo[3.2.1]octane-3-carboxylate and hydrochloride (a solution in tetrahydrofuran, 3.5 M, 150 m) was stirred at 25°C for 2 h. The resultant solution was rotary evaporated to remove the solvent, to give (3-azabicyclo[3.2.1]oct-8-yl)(5-phenyl-4,5-dihydro-1H-pyrazol-1-yl)ketone hydrochloride (50 mg, crude product), which was directly used in the next step of reaction. ES-API: [M+H]⁺= 284.2.

Step 6: A mixture of (3-azabicyclo[3.2.1]oct-8-yl)(5-phenyl-4,5-dihydro-1H-pyrazol-1-yl)ketone hydrochloride (50 mg, 0.156 mmol), 4-chloro-6-cyanopyrimidine (22 mg, 0.16 mmol), potassium carbonate(43 mg, 0.31 mmol) and triethylamine(32 mg, 0.31 mmol) in N,N-dimethylformamide (2 mL) was stirred at 25°C for 2 h. The resultant solution was quenched with water, and extracted with ethyl acetate (5 mL × 2). The organic phases were combined, washed with saline, dried over anhydrous magnesium sulfate, and rotary evaporated to remove the solvent. The crude product of 6-(8-(5-phenyl-4,5-dihydro-1H-pyrazole-1-carbonyl)-3-azabicyclo[3.2.1]oct-3-yl)pyrimidine-4-carbonitrile was separated by preparative HPLC (column: Ultimate XB-C18,50^{∗}250 mm,10 um; mobile phase system: A: pure water; B: pure acetonitrile; flow rate:80 ml/min, B/A=20%-90%; column temperature: room temperature) to give 2 racemates:
Z-2 (retention time: 39.1 min): A racemic peak of 6-(8-(5-phenyl-4,5-dihydro-1H-pyrazole-1-carbonyl)-3-azabicyclo[3.2.1]oct-3-yl)pyrimidine-4-carbonitrile (peak 1, 7.0 mg, yield: 12%), ES-API:[M+H]⁺= 387.2. ¹H NMR(400 MHz, CDCl₃) δ 8.53 (s, 1H), 7.32-7.15 (m, 3H),6.99 (s, 1H), 6.70 (s, 1H), 5.38 (dd, *J* = 11.9, 5.0 Hz, 1H), 4.33 (s, 1H), 3.88-3.18(m,6H), 2.87-2.69 (m, 4H), 1.58 (d, *J* = 18.2 Hz, 2H).
Z-3 (retention time: 40.46 min): Another racemic peak of 6-(8-(5-phenyl-4,5-dihydro-1H-pyrazole-1-carbonyl)-3-azabicyclo[3.2.1]oct-3-yl)pyrimidine-4-carbonitrile (peak 2, 3.5 mg, yield: 6%). ES-API: [M+H]⁺= 387.2. ¹H NMR(400 MHz, CDCl₃) δ 8.59 (d, *J* = 1.1 Hz, 1H), 7.33-7.12 (m, 5H), 6.98 (s, 1H), 6.81 (s, 1H), 5.36 (dd, *J* = 12.0, 5.0 Hz, 1H), 3.43-3.18 (m, 3H), 2.88 - 2.69 (m, 3H), 2.01-1.66 (m, 5H), 1.44 (d, *J* = 9.0 Hz, 2H).

### Example 3: Preparation of (3-(6-methoxypyrimidin-4-yl)-3-azabicyclo[3.2.1]oct-8-yl)(5-phenyl-4,5-dihydro-1H-pyrazol-1-yl)ketone (Z-4)

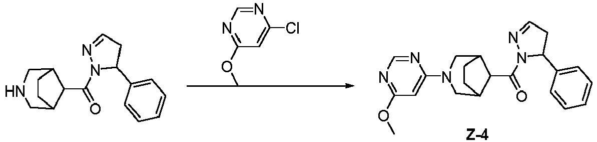

Step 1: A reaction mixture of (3-azabicyclo[3.2.1]oct-8-yl)(5-phenyl-4,5-dihydro-1H-pyrazol-1-yl)ketone (70 mg, 0.25 mmol), 4-chloro-6-methoxypyrimidine (30 mg, 0.2 mmol) and diisopropylethylamine (67 mg, 0.52 mmol) in N,N-dimethylformamide (1mL) was reacted at 70°C for 16 h. Thereafter, the reaction mixture was diluted with ethyl acetate (20 mL). The organic phase was washed with water (30 mL). The separated organic phase was washed with saline, dried over anhydrous sodium sulfate, and rotary evaporated to remove the solvent. The residue was separated by preparative HPLC (column: Ultimate XB-C18, 50^{∗}250 mm, 10 um; mobile phase system: A: pure water; B: pure acetonitrile; flow rate: 80 ml/min, B/A=20%-100%; column temperate: room temperatue) to give (3-(6-methoxypyrimidin-4-yl)-3-azabicyclo[3.2.1]oct-8-yl)(5-phenyl-4,5-dihydro-1H-pyrazol-1-yl)ketone (Z-4, 1.7 mg, yield: 6%). ES-API:[M+H]⁺= 392.1. ¹H NMR(400 MHz, CDCl₃) δ 8.26 (s, 1H), 7.31-7.15 (m, 5H), 6.96 (s, 1H), 5.67 (s, 1H), 5.39 (dd, *J* = 11.9, 4.9 Hz, 1H), 3.87 (s, 3H), 3.55 -3.23 (m, 4H), 2.79-2.68 (m, 3H), 1.87 - 1.81 (m, 2H), 1.60 (d, *J* = 7.2 Hz, 4H).

### Example 4: Preparation of (3-(2-methoxypyrimidin-4-yl)-3-azabicyclo [3.2.1]oct-8-yl)(5-phenyl-4,5-dihydro-1H-pyrazol-1-yl)ketone (Z-5)

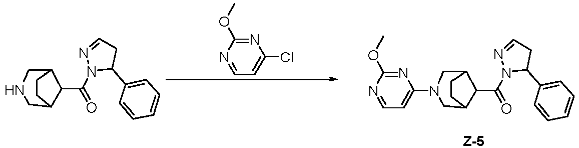

Step 1: A reaction mixture of (3-azabicyclo[3.2.1]oct-8-yl)(5-phenyl-4,5-dihydro-1H-pyrazol-1-yl)ketone (70 mg, 0.25 mmol) and 2-methoxy-4-chloropyrimidine (30 mg, 0.2 mmol), diisopropylethylamine (67 mg, 0.52 mmol) in N,N-dimethylformamide (1 mL) was reacted at 70°C for 16 h. Thereafter, the reaction mixture was diluted with ethyl acetate (20 mL), and washed with water (30 mL). The organic phase was washed with saline, dried over anhydrous sodium sulfate, and rotary evaporated to remove the solvent. The residue was separated by preparative HPLC (column: Ultimate XB-C18,50^{∗}250 mm,10 um; mobile phase system: A: pure water; B: pure acetonitrile; flow rate: 80 ml/min, B/A=20%-100%; column temperature: room temperature) to give (3-(2-methoxypyrimidin-4-yl)-3-azabicyclo[3.2.1]oct-8-yl)(5-phenyl-4,5-dihydro-1H-pyrazol-1-yl)ketone (Z-5, 6.1 mg, yield: 7%). ES-API:[M+H]⁺= 392.1. ¹H NMR(400 MHz, CDCl₃) δ 7.93 (d, *J* = 6.2 Hz, 1H), 7.31-7.13 (m, 5H), 6.97 (d, *J* = 1.8 Hz, 1H), 6.06 (d, *J* = 6.2 Hz, 1H), 5.39 (dd, *J* = 11.9, 4.9 Hz, 1H), 3.90 (s, 3H), 3.46- 3.40 (m, 3H), 2.85 - 2.61 (m, 3H), 1.85 (s, 2H), 1.60-1.52 (m, 5H).

### Example 5: Preparation of 6-(8-(5-phenyl-4,5-dihydro-1H-pyrazole-1-carbonyl)-3-azabicyclo[3.2.1]oct-3-yl)pyrimidine-4-carboxamide (Z-6)

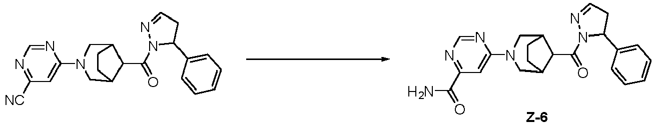

Step 1: The crude product of 6-(8-(5-phenyl-4,5-dihydro-1H-pyrazole-1-carbonyl)-3-azabicyclo[3.2.1]oct-3-yl)pyrimidine-4-carbonitrile (15 mg, 0.04 mmol) and sodium hydroxide (18 mg, 0.5 mmol) were dissolved in water (0.5 mL) and acetonitrile (0.1 mL). Hydrogen peroxide (0.14 mL) was added to the reaction mixture. The resultant reaction mixture was stirred at the room temperature for 20 h. Then, sodium sulphite (20 mg) was added, and the resultant mixture was stirred for 10 minutes. The resultant reaction mixture was diluted with dichloromethane (20 mL), and washed with water (30 mL). The organic phase was washed with saline, dried over anhydrous sodium sulfate, and rotary evaporated to remove the solvent. The residue was separated by preparative thin layer chromatography (petroleum ether/ethyl acetate=1/1) to give 6-(8-(5-phenyl-4,5-dihydro-1H-pyrazole-1-carbonyl)-3-azabicyclo[3.2.1]oct-3-yl)pyrimidine-4-carboxamide (Z-6, 3.2 mg, yield: 20%). ES-API:[M+H]⁺= 405.1. ¹H NMR(400 MHz, CDCl₃) δ 8.50 (s, 1H), 7.79 (s, 1H), 7.32- 7.13 (m, 6H), 6.98 (d, *J* = 1.8 Hz, 1H), 5.51 (s, 1H), 5.39 (dd, *J* = 11.9, 4.9 Hz, 1H), 3.47-3.27 (m, 4H), 2.86 - 2.61 (m, 3H), 1.86 (s, 2H), 1.55 (s, 4H).

### Example 6: Preparation of 6-(8-(5-(3,5-difluorophenyl)-4,5-dihydro-1H-pyrazole-1-carbonyl)-3-azabicyclo[3.2.1]oct-3-yl)pyrimidine-4-carbonitrile (Z-7)

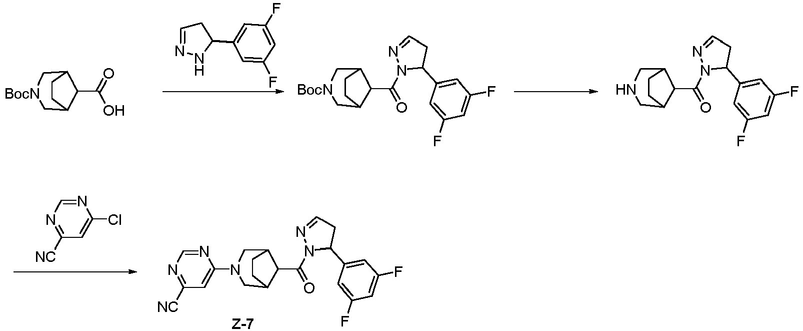

Step 1: A reaction mixture of 3-(tert-butoxycarbonyl)-3-azabicyclo[3.2.1]octane-8-carboxylic acid (100 mg, 0.392 mmol), 5-(3,5-difluorophenyl)-4,5-dihydro-1H-pyrazole (357 mg, 1.96 mmol), HATU (179 mg, 0.471 mmol) and diisopropylethylamine (101 mg, 0.784 mmol) in dichloromethane(2 mL) was stirred at 25°C for 2 h. The resultant reation solution was washed with saline, dried over anhydrous magnesium sulfate, and rotary evaporated to remove the solvent. The resultant residue was subjected to column chromatography (petroleum ether/ethyl acetate=3/1) to give tert-butyl 8-(5-(3,5-difluorophenyl)-4,5-dihydro-1H-pyrazole-1-carbonyl)-3-azabicyclo[3.2.1]octane-3-carboxylate (130 mg, yield: 40%). ES-API: [M+H]⁺= 420.2.

Step 2: A reaction mixture of tert-butyl 8-(5-(3,5-difluorophenyl)-4,5-dihydro-1H-pyrazole-1-carbonyl)-3-azabicyclo[3.2.1]octane-3-carboxylate (130 mg, 0.31 mmol) and hydrochloric acid (3.5 M, a solution in tetrafuran, 5 mL) was reacted at 25°C for 0.5 h. The solvent was removed to give (3-azabicyclo[3.2.1]oct-8-yl)(5-(3,5-difluorophenyl)-4,5-dihydro-1H-pyrazol-1-yl) ketone hydrochloride (70 mg, crude product), which was directly used in the next step of reaction without purufication. ES-API:[M+H]⁺=320.2.

Step 3: A mixture of 3-azabicyclo[3.2.1]oct-8-yl)(5-(3,5-difluorophenyl)-4,5-dihydro-1H-pyrazol-1-yl)ketone hydrochloride (70 mg, 0.156 mmol), 4-chloro-6-cyanopyrimidine (22 mg, 0.156 mmol), potassium carbonate (43 mg, 0.312 mmol), triethylamine (32 mg, 0.312 mmol) in N,N-dimethylformamide (2 mL) was stirred at 25°C for 1 h. The resultant reaction mixture was quenched with water, and extracted with ethyl acetate (5 mL × 2). The organic phase was washed with saline, dried over anhydrous magnesium sulfate, and rotary evaporated to remove the solvent. The residue was separated by preparative HPLC (column: Ultimate XB-C18,50^{∗}250 mm, 10 um; mobile phase system: A: pure water; B: pure acetonitrile; flow rate: 80 ml/min, B/A=20%-100%; column temperature: room temperature) to give 6-(8-(5-(3,5-difluorophenyl)-4,5-dihydro-1H-pyrazole-1-carbonyl)-3-azabicyclo[3.2.1]oct-3-yl)pyrimidine-4-carbonitrile (Z-7, 8.9 mg, yield: 14%). ES-API:[M+H]+ =423.2. ¹H NMR(400 MHz, CDCl₃) δ 8.54 (s, 1H), 6.99 (d, *J* = 1.8 Hz, 1H), 6.73-6.60 (m, 4H), 5.34 (dd, *J* = 11.9, 5.0 Hz, 1H), 4.36 (bs, 1H), 3.78-3.13 (m, 5H), 2.88 - 2.53 (m, 3H), 1.59 (d, *J* = 11.4 Hz, 4H).

### Example 7: Preparation of (3-(4-amino-5-fluoropyrimidin-2-yl)-3-azabicyclo[3.2.1]oct-8-yl)(5-phenyl-4,5-dihydro-1H-pyrazol-1-yl)ketone (Z-8)

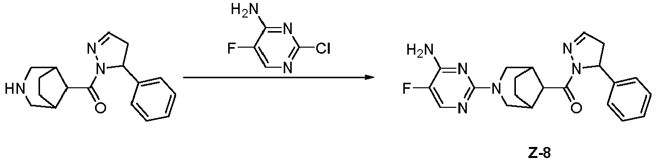

Step 1: Diisopropylethylamine (67 mg, 0.52 mmol) was added to a mixture of (3-azabicyclo[3.2.1]oct-8-yl)(5-phenyl-4,5-dihydro-1H-pyrazol-1-yl) ketone (70 mg, 0.25 mmol) and 2-chloro-5-fluoropyrimidine-4-amine (30 mg, 0.2 mmol) in N,N-dimethylformamide (1 mL). The reaction mixture was reacted at 125°C for 16 h. The resultant reaction mixture was diluted with ethyl acetate (20 mL), and washed with water (30 mL). The organic phase was washed with saline, dried over anhydrous sodium sulfate, and rotary evaporated to remove the solvent. The residue was separated by preparative thin layer charomatography (petroleum ether/ethyl acetate=2/1) to give (3-(4-amino-5-fluoropyrimidin-2-yl)-3-azabicyclo[3.2.1]oct-8-yl)(5-phenyl-4,5-dihydro-1H-pyrazol-1-yl) ketone (Z-8, 1.9 mg, yield: 2%). ES-API:[M+H]⁺= 395.2. ¹H NMR(400 MHz, CDCl₃) δ 7.78 (d, *J* = 3.1 Hz, 1H), 7.33 - 7.13 (m, 5H), 6.94 (s, 1H), 5.39 (dd, *J* = 11.9, 4.9 Hz, 1H), 4.69 (bs, 2H), 3.98- 3.27 (m, 5H), 3.16 (t, *J* = 4.2 Hz, 1H), 2.77-2.64 (m, 3H), 1.61 (d, *J* = 6.6 Hz, 4H).

### Example 8: Preparation of 6-(5-(5-phenyl-4,5-dihydro-1H-pyrazole-1-carbonyl)hexahydrocyclopenta[C]pyrrol-2(1H)-yl)pyrimidine-4-carbonitrile (Z-9), 6-((3aR,5s,6aS)-5-((S)-5-phenyl-4,5-dihydro-1H-pyrazole-1-carbonyl)hexahydrocyclopenta[c]pyrrol-2(1H)-yl)pyrimidine-4-carbonitrile (Z-9-1), 6-((3aR,5r,6aS)-5-((S)-5-phenyl-4,5-dihydro-1H-pyrazole-1-carbonyl)hexahydrocyclopenta[c]pyrrol-2(1H)-yl)pyrimidine-4-carbonitrile (Z-9-2), 6-((3aR,5s,6aS)-5-((R)-5-phenyl-4,5-dihydro-1H-pyrazole-1-carbonyl)hexahydrocyclopenta[c]pyrrol-2(1H)-yl)pyrimidine-4-carbonitrile (Z-9-3) and 6-((3aR,5r,6aS)-5-((R)-5-phenyl-4,5-dihydro-1H-pyrazole-1-carbonyl)hexahydrocyclopenta[c]pyrrol-2(1H)-yl)pyrimidine-4-carbonitrile (Z-9-4)

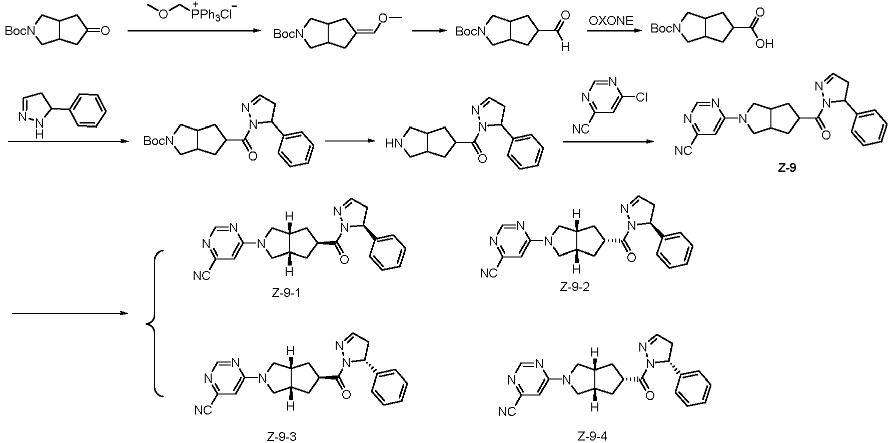

Step 1: Potassium tert-butoxide (797 mg, 7.11 mmol) was added to a mixture of tert-butyl (3aR,6aS)-5-oxohexahydrocyclopenta[C]pyrrole-2(1H)-carboxylate (800 mg, 3.556 mmol) and (methoxymethyl)triphenylphosphine chloride (2.189 g, 6.4 mmol) in tetrahydrofuran (15 mL) at 0 °C. The resultant reaction was reacted at 25°C for 18 h. Thereafter, the reaction mixture was quenched with a diluted aqueous solution of ammonium chloride. The resultant mixture was extracted with dichloromethane (15 mL × 2). The organic phase was washed with saline, dried over anhydrous magnesium sulfate, and rotary evaporated to remove the solvent. The residue was subjected to column chromatography (petroleum ether/ethyl acetate=5/1) to give tert-butyl (3aR,6aS)-5-methoxymethylenehexahydrocyclopenta[C]pyrrole-2(1H)-carboxylate (400 mg, 45%). ES-API:[M+H]⁺= 254.2.

Step 2: A reaction mixture of tert-butyl (3aR,6aS)-5-methoxymethylenehexahydrocyclopenta[C]pyrrole-2(1H)-carboxylate (400 mg, 1.6 mmol), p-toluenesulfonic acid monohydrate (320 mg, 1.68 mmol) and water (56 mg, 0.32 mmol) in acetone (10 mL) was stirred at 25°C for 18 h. The resultant rereaction solution was directly used in the next step of reaction. ES-API: [M+H]+= 240.1.

Step 3: Potassium monopersulfate complex salt (OXONE, 5.36 g, 8.36 mmol) and water (10 mL) were added to the reaction mixture obtained in the Step 2, which was reacted at 25°C for 2 h. The resultant reaction mixture was extracted with dichloromethane (15 mL × 2). The organic phase was washed with saline, dried over anhydrous magnesium sulfate, and rotary evaporated to remove the solvent, to give (3aR,6aS)-2-(tert-butoxycarbonyl)octahydropenta[c]pyrrole-5-carboxylic acid (370 mg, yield: 87%). ES-API: [M+H]⁺= 256.1.

Step 4: A mixture of (3aR,6aS)-2-(tert-butoxycarbonyl)octahydropenta[c] pyrrole-5-carboxylic acid (370 mg, 1.45 mmol), 5-phenyl-4,5-dihydropyrazole (1.06 g, 7.25 mmol), HATU (661 mg, 1.74 mmol) and triethylamine(293 mg, 2.9 mmol) in dichloromethane (10 mL) was reacted at 25°C for 2 h. The resultant reaction mixture was washed with saline, dried over anhydrous magnesium sulfate, and rotary evaporated to remove the solvent. The residue was objected to column chromatography (petroleum ether/ethyl acetate=3/1) to give tert-butyl (3aR,6aS)-5-(5-phenyl-4,5-dihydro-1H-pyrazole-1-carbonyl)hexahydrocyclopenta[C]pyrrole-2(1H)-carboxylate (200 mg, yield: 36%). ES-API: [M+H]+= 384.2.

Step 5: A mixture of tert-butyl (3aR,6aS)-5-(5-phenyl-4,5-dihydro-1H-pyrazole-1-carbonyl)hexahydrocyclopenta[C]pyrrole-2(1H)-carboxylate (200 mg, 0.522 mmol) and hydrochloric acid (a solution in tetrahydrofuran, 3.5 M, 15 mL) was stirred at 25°C for 2 h. The resultant reaction mixture was rotary evaporated to remove the solvent to give (3aR,6aS)-(octahydrocyclopenta[c]pyrrol-5-yl)(5-phenyl-4,5-dihydro-1H-pyrazol-1-yl)ketone hydrochloride (210 mg, crude product), which was directly used in the next step of reaction without purification. ES-API: [M+H]⁺= 284.2.

Step 6: A reaction mixture of (3aR,6aS)-(octahydrocyclopenta[c]pyrrol-5-yl)(5-phenyl-4,5-dihydro-1H-pyrazol-1-yl) ketone hydrochloride (210 mg, 0.657 mmol), 4-chloro-6-cyanopyrimidine (91.7 mg, 0.657 mmol), potassium carbonate (181 mg, 1.314 mmol) and triethylamine (133 mg, 1.314 mmol) in N,N-dimethylformamide (2 mL) was reacted at 25°C for 2 h. The resultant reaction mixture was quenched with water, and extracted with ethyl acetate (5 mL × 2). The organic phase was washed with saline, dried over anhydrous magnesium sulfate, and rotary evaporated to remove the solvent. The residue was purified by preparative HPLC (column: Ultimate XB-C18, 50^{∗}250 mm, 10 um; mobile phase system: A: pure water; B: pure acetonitrile; flow rate: 80 ml/min, B/A=20%-100%; column temperatue: room temperatue) to give 6-(3aR,6aS)-(5-(5-phenyl-4,5-dihydro-1H-pyrazole-1-carbonyl)hexahydrocyclopenta[c]pyrrol-2(1H)-yl)pyrimidine-4-carbonitrile (Z-9, 45 mg, 18%). ES-API:[M+H]⁺= 387.2. ¹H NMR(400 MHz, CDCl₃) δ 8.56 (dd, *J =* 18.6, 1.2 Hz, 1H), 7.36 -6.95 (m, 6H), 6.68 (s, 1H), 6.52 (s, 1H), 5.34 (dt, *J* = 11.1, 5.3 Hz, 1H), 3.79-2.74 (m, 9H),1.84 (s, 2H).

Step 7: The Z-9 as obtained in the above step was separated by SFC (chromatography column type: AD-H (4.6^{∗}100 mm 5 um); mobile phase: CO₂; co-solvent: 45%MeOH (added with a solution of 0.2% ammonia in methanol); flow rate: 4 ml/min, T=40 °C), to give 4 isomers having a single configuration (the structures of the 4 isomers having a single configuration are each arbitrarily assigned).

Isomer 1 (retention time: 1.37 min), arbitrarily assigned as Z-9-1, 6-((*3aR,5s,6aS*)-5-((*S*)-5-phenyl-4,5-dihydro-1H-pyrazole-1-carbonyl)hexahydrocyclopenta[c]pyrrol-2(1H)-yl)pyrimidine-4-carbonitrile (4.8 mg, purity: 100%, ee value: 100%). ES-API:[M+H]⁺= 387.2.

Isomer 2 (retention time: 1.75 min), arbitrarily assigned as Z-9-2, 6-((*3aR,5r,6aS*)-5-((*S*)-5-phenyl-4,5-dihydro-1H-pyrazole-1-carbonyl)hexahydrocyclopenta[c]pyrrol-2(1H)-yl)pyrimidine-4-carbonitrile (4.5 mg, purity: 97.5%, ee value: 95.1%). ES-API:[M+H]⁺= 387.2.

Isomer 3 (retention time: 2.04 min), arbitrarily assigned as Z-9-3, 6-((*3aR,5s,6aS)*-5-((*R*)-5-phenyl-4,5-dihydro-1H-pyrazole-1-carbonyl)hexahydrocyclopenta[c]pyrrol-2(1H)-yl)pyrimidine-4-carbonitrile (4.9 mg, purity: 92.7%, ee value: 85.3%). ES-API:[M+H]⁺= 387.2.

Isomer 4 (retention time: 5.24 min), arbitrarily assigned as Z-9-4, 6-((*3aR,5r,6aS)*-5-((*R*)-5-phenyl-4,5-dihydro-1H-pyrazole-1-carbonyl)hexahydrocyclopenta[c]pyrrol-2(1H)-yl)pyrimidine-4-carbonitrile (4.8 mg, purity: 100%, ee value: 100%). ES-API:[M+H]⁺= 387.2.

### Example 9: Preparation of 6-(5-phenyl-4,5-dihydro-1H-pyrazole-1-carbonyl)hexahydrocyclopenta[C]pyrrol-2(1H)-yl)pyrimidine-4-carboxamide (Z-10)

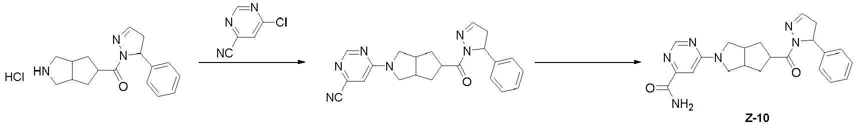

Step 1: Diisopropylethylamine (79 mg, 0.62 mmol) was added to a mixture of (octahydrocyclopenta[c]pyrrol-5-yl)(5-phenyl-4,5-dihydro-1H-pyrazol-1-yl) ketone hydrochloride (70 mg, 0.25 mmol), 4-chloro-6-cyanopyrimidine (38 mg, 0.27 mmol) in acetonitrile (2 mL). The reaction mixture was reacted at 100°C for 2 h, diluted with dichloromethane (20 mL), and washed with water (30 mL). The organic phase was washed with saline, dried over anhydrous sodium sulfate, and rotary evaporated to remove the solvent. The residue was subjected to chlomn chromatography (petroleum ether/ethyl acetate=21/1) to give 6-(5-(5-phenyl-4,5-dihydro-1H-pyrazole-1-carbonyl)hexahydrocyclopenta[C]pyrrol-2(1H)-yl)pyrimidine-4-carbonitrile (Z-10, 60 mg, yield: 63%).

Step 2: Hydrogen peroxide (0.54 mL) was added to a mixture of 6-(5-(5-phenyl-4,5-dihydro-1H-pyrazole-1-carbonyl)hexahydrocyclopenta[C]pyrrol-2(1H)-yl) pyrimidine-4-carbonitrile (60 mg, 0.15 mmol) and sodium hydroxide (75 mg, 1.8 mmol) in water/acetonitrile (2 mL/1.5 mL). The reaction mixture was stirred at the room temperatue for 2 h. The reaction was quenched with sodium sulphite (20 mg), and stirred for a further 10 minutes. The resultant reaction mixture was diluted with dichloromethane (20 mL), and washed with water (30 mL). The organic phase was washed with saline, dried over anhydrous sodium sulfate, and rotary evaporated to remove the solvent. The residue was separated by preparative thin layer chromatography (ethyl acetate) to give 6-(5-phenyl-4,5-dihydro-1H-pyrazole-1-carbonyl)hexahydrocyclopenta[C]pyrrol-2(1H)-yl)pyrimidine-4-carboxamide (Z-10, 42.7 mg, yield: 68%). ES-API:[M+H]⁺= 405.2. ¹H NMR(400 MHz, CDCl₃) δ 8.52 (d, *J* = 17.5 Hz, 1H), 7.80 (d, *J* = 6.7 Hz, 1H), 7.32-6.93 (m, 6H), 5.84 - 5.71 (m, 1H), 5.33 (ddd, *J* = 12.0, 4.9, 1.9 Hz, 1H), 3.83 -3.35 (m, 5H), 2.95-2.79 (m, 3H), 2.22-1.71 (m, 5H).

### Example 10: Preparation of 6-(5-(5-(3,5-difluorophenyl)-4,5-dihydro-1H-pyrazole-1-carbonyl)hexahydrocyclopenta[C]pyrrol-2(1H)-yl)pyrimidine-4-carbonitrile (Z-11)

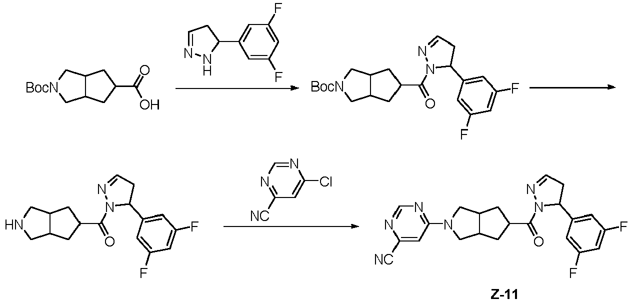

Step 1: 5-(3,5-difluorophenyl)-4,5-dihydro-1H-pyrazole (278 mg, 1.53 mmol), HATU (233 mg, 0.61 mmol) and triethylamine (103 mg, 1.02 mmol) were added to 2-(tert-butoxycarbonyl)octahydrocyclopenta[c]pyrrole-5-carboxylic acid (130 mg, 0.51 mmol) in dichloromethane (5 mL). The reaction mixture was stirred at the room temperature for 3 h. Then, the reaction was quenched with water. The aqueous phase was extracted with dichloromethane (25 mL). The organic phases were combined, washed with saline, dried over anhydrous sodium sulfate, and rotary evaporated to remove the solvent. The residue was subjected to column chromatography (petroleum ether/ethyl acetate=100/0 to 3/2) to give tert-butyl 5-(5-(3,5-difluorophenyl)-4,5-dihydro-1H-pyrazole-1-carbonyl)hexahydrocyclopenta[C] pyrrole-2(1H)-carboxylate (110 mg, yield: 53%). ES-API: [M+H]⁺= 420.2.

Step 2: 5-(5-(3,5-difluorophenyl)-4,5-dihydro-1H-pyrazole-1-carbonyl) hexahydrocyclopenta[C]pyrrole-2(1H)-carboxylate (110 mg, 0.51 mmol) was dissolved in hydrochloric acid (a solution in 1,4-dioxane, 6 mL). The reaction mixture was stirred at the room temperature for 2 h, and rotary evaporated to remove the solvent to give (5-(3,5-difluorophenyl)-4,5-dihydro-1H-pyrazol-1-yl)(octahydrocyclopenta[c]pyrrol-5-yl)ketone hydrochloride (89 mg, crude product). ES-API:[M+H]⁺= 320.1.

Step 3: (5-(3,5-difluorophenyl)-4,5-dihydro-1H-pyrazol-1-yl)(octahydrocyclo-penta[C]pyrrol-5-yl) ketone hydrochloride (89 mg, 0.25 mmol) was dissolved in tetrahydrofuran (1 mL), into which triethylamine (50 mg, 0.50 mmol) was added dropwise. Then, potassium carbonate (69 mg, 0.50 mmol), 4-chloro-6-cyanopyrimidine (35 mg, 0.25 mmol) and N,N-dimethylformamide (2 mL) were added. The reaction mixture was reacted at 70°C for 18 h. Thereafter, the reaction mixture was diluted with ethyl acetate (15 mL), and washed with water (10 mL). The organic phase was washed with saline, dried over anhydrous sodium sulfate, and rotary evaporated to remove the solvent. The residue was separated with preparative thin layer chromatography (petroleum ether/ethyl acetate=1/1) to give 6-(5-(5-(3,5-difluorophenyl)-4,5-dihydro-1H-pyrazole-1-carbonyl)hexahydrocyclopenta[C]pyrrol-2(1H)-yl)pyrimidine-4-carbonitrile (Z-11, 5 mg, yield: 24%). ES-API:[M+H]⁺= 423.1. ¹H NMR (400 MHz, CD₃OD) δ 8.45 (dd, *J* = 15.3, 1.2 Hz, 1H), 7.12-6.72 (m, 5H), 5.34 (dt, *J* = 11.7, 5.0 Hz, 1H), 3.86-3.50 (m, 5H), 2.93-2.78 (m, 3H), 2.34-1.72 (m, 5H).

### Example 11: Preparation of (2-(6-methoxypyrimidin-4-yl)octahydrocyclo penta[c]pyrrol-5-yl)(5-phenyl-4,5-dihydro-1H-pyrazol-1-yl) ketone (Z-12)

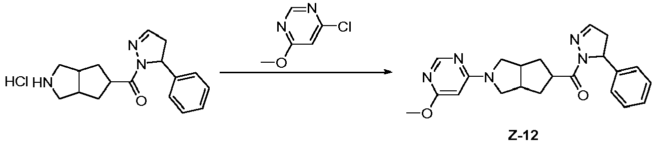

Step 1: (octahydrocyclopenta[c]pyrrol-5-yl)(5-phenyl-4,5-dihydro-1H-pyrazol-1-yl) ketone hydrochloride (188 mg, 0.59mmol) was dissolved in tetrahydrafuran (1 mL), into which triethylamine (119 mg, 1.18mmol) was added dropwise. Then, potassium carbonate (162 mg, 1.18 mmol), 4-chloro-6-methoxypyrimidine (85 mg, 0.59mmol) and N,N-dimethylformamide (1 mL) were added to the solution. The reaction mixture was stirred at 70°C for 18 h, diluted with ethyl acetate (15 mL), and washed with water (10 mL). The organic phase was washed with saline, dried over anhydrous sodium sulfate, and rotary evaporated to remove the solvent. The residue was separated by preparative thin layer chromatography (petroleum ether/ethyl acetate=1/1) to give (2-(6-methoxypyrimidin-4-yl)octahydrocyclopenta[c]pyrrol-5-yl)(5-phenyl-4,5-dihydro-1H-pyrazol-1-yl) ketone (Z-12, 30 mg, yield: 15%). ES-API: [M+H]⁺= 392.2. ¹H NMR(400 MHz, CDCl₃) δ 8.29 (d, *J* = 15.7 Hz, 1H), 7.38-6.89 (m, 6H), 5.58-5.52 (d, *J* = 15.7 Hz, 1H), 5.34 (dd, *J* = 11.9, 4.9 Hz, 2H), 3.89 (d, *J* = 10.1 Hz, 3H), 3.78-3.28 (m, 4H), 2.98-2.74 (m, 3H), 2.32-2.07 (m, 2H), 2.04-1.60 (m, 4H).

### Example 12: Preparation of (2-(2-methoxypyrimidin-4-yl)octahydro cyclopenta[c]pyrrol-5-yl)(5-phenyl-4,5-dihydro-1H-pyrazol-1-yl) ketone (Z-13)

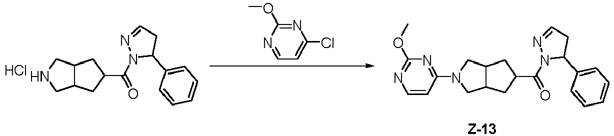

Step 1: (octahydrocyclopenta[c]pyrrol-5-yl)(5-phenyl-4,5-dihydro-1H-pyrazol-1-yl) ketone hydrochloride (120 mg, 0.38 mmol) was dissolved in tetrahydrafuran (1 mL), into which triethylamine (76 mg, 0.75 mmol) was added dropwise. Then, potassium carbonate (104 mg, 0.75 mmol), 2-methoxy-4-chloropyrimidine (54 mg, 0.38 mmol) and N,N-dimethylformamide (2 mL) was added to the solution. The reaction mixture was stirred at 70°C for 18 h, diluted with ethyl acetate (15 mL), and washed with water (10 mL). The organic phase was washed with saline, dried over anhydrous sodium sulfate, and rotary evaporated to remove the solvent. The residue was separated by preparative thin layer chromatography (petroleum ether/ethyl acetate=1/1) to give (2-(2-methoxypyrimidin-4-yl)octahydrocyclopenta[c]pyrrol-5-yl)(5-phenyl-4,5-dihydro-1H-pyrazol-1-yl) ketone (Z-13, 25 mg, yield: 15%). ES-API:[M+H]⁺= 392.2. ¹H NMR(400 MHz, CDCl₃) δ 7.95 (dd, *J* = 19.0, 5.9 Hz, 1H), 7.35-6.94 (m, 6H), 5.93 (dd, *J* = 24.1, 6.0 Hz, 1H), 5.34 (dd, *J* = 12.0, 4.9 Hz, 1H), 3.91 (d, *J* = 10.7 Hz, 3H), 3.83 - 1.54 (m, 13H).

### Example 13: Preparation of (2-(4-amino-5-fluoropyrimidin-2-yl) octahydrocyclopenta[c]pyrrol-5-yl)(5-phenyl-4,5-dihydro-1H-pyrazol-1-yl) ketone (Z-14)

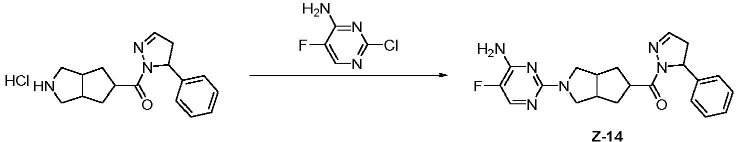

Step 1: (octahydrocyclopenta[c]pyrrol-5-yl)(5-phenyl-4,5-dihydro-1H-pyrazol-1-yl) ketone hydrochloride (70 mg, 0.22 mmol) was dissolved in N,N-dimethylformamide (5 mL), into which diisopropylethylamine (70 mg, 0.55 mmol) and 2-chloro-5-fluoropyrimidine-4-amine (38 mg, 0.26 mmol) were added dropwise. The reaction mixture was stirred at 100°C for 18 h, diluted with ethyl acetate (15 mL), and washed with water (10 mL). The organic phase was washed with saline, dried over anhydrous sodium sulfate, and rotary evaporated to remove the solvent. The residue was separated by preparative thin layer chromatography (petroleum ether/ethyl acetate=3/2) to give (2-(4-amino-5-fluoropyrimidin-2-yl)octahydro cyclopenta[c]pyrrol-5-yl)(5-phenyl-4,5-dihydro-1H-pyrazol-1-yl) ketone (Z-14, 4.5 mg, yield: 5%). ES-API:[M+H]⁺= 393.2. ¹H NMR(400 MHz, CDCl₃) δ 7.81 (d, *J* = 16.0 Hz, 1H), 7.25-6.91 (m, 5H), 5.34 (d, *J* = 11.5 Hz, 1H), 4.77 (bs, 2H), 3.96-3.14 (m, 5H), 2.85-2.76(m, 4H), 2.26-1.54 (m, 4H).

### Example 14: Preparation of (5-(2-methoxypyrimidin-4-yl)hexahydropyrrolo [3,4-e]pyrrol-2(1H)-yl)(5-phenyl-4,5-dihydro-1H-pyrazol-yl) ketone (Z-15)

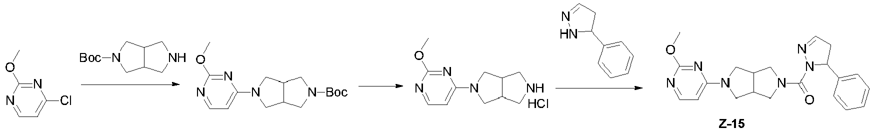

Step 1: 6mL of anhydrous N,N-dimethylformamide, 4-chloro-2-methoxypyrimidine (200vmg, 1.383vmmol), tert-butyl hexahydropyrrolo[3,4-c]pyrrole-2(1H)-carboxylate (294 mg, 1.383 mmol), and finally potassium carbonate (382 mg, 2.767 mmol) were added to a 25 mL single-necked round-bottomed flask under the protection of nitrogen gas. The reaction mixture was stirred at the room temperature. As monitored by TLC [PE:EA=1:1, V/V] when the spot for the starting materials disappeared, the reaction was terminated. 100 mL of ethyl acetate was added to the reaction system. The resultant mixture was washed with sodium chloride for 5 times (60 mL×5). The phase of ethyl acetate was dried over anhydrous sodium sulfate, and then filtered. The filtrate was was spin-dried to give the target compound of tert-butyl 5-(2-methoxypyrimidin-4-yl)hexahydropyrrolo[3,4-c]pyrrole-2(1H)-carboxylate (0.5 g, yield: 75%). ES-API:[M+H]⁺=320.9.

Step 2: 3 mL of a solution of hydrochloric acid (4 M) in dioxane and tert-butyl 5-(2-methoxypyrimidin-4-yl)hexahydropyrrolo[3,4-c]pyrrole-2(1H)-carboxylate (200 mg, 0.625 mmol) were added to a 50 mL single-necked round-bottomed flask under the protection of nitrogen gas. The reaction mixture was stirred at the room temperature overnight. As monitored by TLC [PE:EA=1:1, V/V] when the spot for the starting materials disappeared, the reaction was terminated. The resultant reaction mixture was spin-dried to remove the solvent to give a crude product of the target compound of 2-(2-methoxypyrimidin-4-yl)octahydropyrrolo[3,4-c]pyrrole hydrochloride (208 mg, crude). ES-API:[M+H]⁺=221.2.

Step 3: N,N'-carbonyldiimidazole (56 mg, 0.345 mmol) and 2 mL of dichloromethane were added to a 50 mL single-necked round-bottomed flask under the protection of nitrogen gas. The resultant mixture was stirred at the room temperature for 12 h. Then, a solution of 2-(2-methoxypyrimidin-4-yl)octahydropyrrolo[3,4-c]pyrrole hydrochloride (104 mg, 0.312 mmol) in dichloromethane (2mL) was added to the above solution. The resultant mixture was stirred at the room temperature for 48 h. After the reaction was completed, the reaction mixture was spin-dried. The crude product was purified by preparative HPLC (column: Ultimate XB-C18,50^{∗}250 mm, 10 um; mobile phase system: A: pure water; B: pure acetonitrile; flow rate: 80 ml/min, B/A=20%-100%; column temperature: room temperature) to give the target compound of (5-(2-methoxypyrimidin-4-yl) hexahydropyrrolo[3,4-c]pyrrol-2(1H)-yl)(5-phenyl-4,5-dihydro-1H-pyrazol-1-yl) ketone (Z-15, 44 mg, yield: 35%). ES-API:[M+H]⁺=393.3. ¹H NMR(400 MHz, DMSO-*d6*) δ 7.93 (d, *J* = 5.9 Hz, 1H), 7.26 (t, *J* = 7.4 Hz, 2H), 7.18 (t, *J* = 7.1 Hz, 3H), 6.93 (s, 1H), 6.10 (d, *J* = 5.9 Hz, 1H), 5.15 (dd, *J* = 11.8, 9.0 Hz, 1H), 3.73 (d, *J* = 8.5 Hz, 4H), 3.35 (d, *J* = 2.2 Hz, 5H), 3.29 - 2.79 (m, 4H), 2.58 - 2.47 (m, 2H).

### Example 16: Preparation of 6-(5-(5-(3,5-difluorophenyl)-4,5-dihydro-1H-pyrazole-1-carbonyl)hexahydrocyclopenta[c]pyrrol-2(1H)-yl)pyrimidine-4-carboxamide (Z-17)

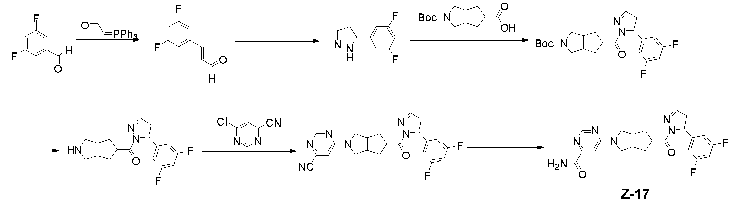

Step 1: 3,5-difluorobenzaldehyde (5 g, 35.2 mmol) and (formylmethylene)triphenylphosphorane (11.8 g, 38.7 mmol) were dissolved in tetrahydrofuran (30 mL). The resultant solution was reacted at 80°C for 16 h, concentrated at a reduced pressure, and subjected to column chromatography (petroleum ether/ethyl acetate: 10/1) to give 3-(3,5-difluorophenyl)acraldehyde (5.2 g, yield: 88%). ES-API:[M+H]⁺= 169.0.

Step 2: 3-(3,5-difluorophenyl)acraldehyde (2.56 g, 15.2 mmol) was dissolved in ethanol (5 mL). After adding the solution dropwise to hydrazine hydrate (1.1 mL) and acetic acid (1.5 mL) at a temperature of 50°C, the reaction mixture was reacted at a constant temperature of 80°C for 16 h. The resultant reaction mixture was concentrated at a reduced pressure, and entrained with toluene for three times (10 mL × 3) to give 5-(3,5-difluorophenyl)-4,5-dihydro-1H-pyrazole(3 g, crude product), which was directly used in the next step. ES-API:[M+H]⁺= 183.0.

Step 3: 2-(tert-butoxycarbonyl)octacyclopenta[c]pyrrole-5-carboxylic acid (1.4 g, 5.5 mmol), 5-(3,5-difluorophenyl)-4,5-dihydro-1H-pyrazole (1 g, 5.5 mmol), N,N-diisopropylethylamine (1.77 g, 13.8 mol) and 2-(7-azabenzotriazole)-N,N,N',N'-tetramethyluronium hexafluorophosphate (HATU, 2.28 g, 6.0 mmol) were to dichloromethane (25 mL). The reaction was carried out at 25°C for 16 h. The resultant reaction mixture was extracted with dichloromethane, and subjected to column chromatography (petroleum ether/ethyl acetate: 10%-45%) to give tert-butyl 5-(5-(3,5-difluorophenyl)-4,5-dihydro-1H-pyrazole-1-carbonyl)hexahydrocyclo penta [c]pyrrole-2(1H)-carboxylate (650 mg, yield: 28%). ES-API:[M+H-56]⁺= 364.0.

Step 4: tert-butyl 5-(5-(3,5-difluorophenyl)-4,5-dihydro-1H-pyrazole-1-carbonyl)hexahydrocyclopenta[c]pyrrole-2(1H)-carboxylate (650 mg, 1.55 mmol) was dissolved in dichloromethane(10 mL). Then, trifluoroacetic acid (2 mL) was added. The reaction was carried out at 25°C for 4 h. The resultant reaction mixture was concentrated at a reduced pressure to give (5-(3,5-difluorophenyl)-4,5-dihydro-1H-pyrazol-1-yl)(octacyclopenta[c]pyrrol-5-yl) ketone trifluoroacetate (520 mg, crude product). ES-API:[M+H]⁺= 320.0.

Step 5: (5-(3,5-difluorophenyl)-4,5-dihydro-1H-pyrazol-1-yl)(octacyclopenta[c] pyrrol-5-yl) ketone trifluoroacetate (300 mg,0.94 mmol), 6-chloropyrimidine-4-carbonitrile (131 mg, 0.94 mmol) and potassium carbonate (389 mg, 2.82 mmol) were added to N,N-dimethylformamide (5 mL). The reaction was carried out at a heating temperature of 50°C for 2 h. The resultant reaction mixture was extracted with ethyl acetate, and subjected to column chromatography (petroleum ether/ethyl acetate: 10%-40%) to give 6-(5-(5-(3,5-difluorophenyl)-4,5-dihydro-1H-pyrazole-1-carbonyl)hexahydrocyclopenta[c]pyrrol-2(1H)-yl)pyrimidine-4-carbonitrile as a yellow oil (200 mg, yield: 50%). ES-API:[M+H]⁺= 423.1.

Step 6: 6-(5-(5-(3,5-difluorophenyl)-4,5-dihydro-1H-pyrazole-1-carbonyl) hexahydrocyclopenta[c]pyrrol-2(1H)-yl)pyrimidine-4-carbonitrile (200 mg, 0.47 mmol) was dissolved in acetonitrile/water (5 mL/0.5 mL). Sodium hydroxide (56 mg, 1.41 mmol) and hydrogen peroxide (1 mL) were added. The reaction mixture was stirred at the room temperature for 2 h, extracted with ethyl acetate, washed with a solution of sodium sulphite, and separated by preparative HPLC (column: Ultimate XB-C18,50^{∗}250 mm, 10um; mobile phase system: A: pure water; B: pure acetonitrile; flow rate: 80 ml/min, B/A=20%-100%; column temperature: room temperature) to give 6-(5-(5-(3,5-difluorophenyl)-4,5-dihydro-1H-pyrazole-1-carbonyl)hexahydrocyclopenta[c]pyrrol-2(1H)-yl)pyrimidine-4-carboxamide (Z-17, 103 mg, yield: 50%). ES-API:[M+H]⁺= 441.2. ¹H NMR(300 MHz, CDCl₃) δ 8.56 (d, *J* = 11.3 Hz, 1H), 7.98 (bs, 2H), 7.16- 6.94 (m, 2H), 6.69 (dd, *J* = 7.7, 3.9 Hz, 3H), 5.67 (s, 1H), 5.32 (dd, *J* = 10.9, 4.1 Hz, 1H), 3.74-3.45 (m, 6H), 3.06 - 2.72 (m, 3H), 2.29-1.82 (m, 3H).

### Example 17: Preparation of 2-(5-(5-(3,5-difluorophenyl)-4,5-dihydro-1H-pyrazole-1-carbonyl)hexahydrocyclopenta[c]pyrrol-2(1H)-yl)pyrimidine-4-carboxamide (Z-18), 2-((3aR, 5s,6aS)-5-((S)-5-(3,5-difluorophenyl)-4,5-dihydro-1H-pyrazole-1-carbonyl)hexahydrocyclopenta[c]pyrrol-2(1H)-yl)pyrimidine-4-carboxamide (Z-18-1), 2-((3aS,5s,6aS)-5-((R)-5-(3,5-difluorophenyl)-4,5-dihydro-1H-pyrazole-1-carbonyl)hexahydrocyclopenta[c]pyrrol-2(1H)-yl) pyrimidine-4-carboxamide (Z-18-2), 2-((3aR,5r,6aS)-5-((S)-5-(3,5-difluorophenyl)-4,5-dihydro-1H-pyrazole-1-carbonyl)hexahydrocyclopenta[c]pyrrol-2(1H)-yl)pyrimidine-4-carboxamide (Z-18-3), 2-((3aR,5r,6aR)-5-((R)-5-(3,5-difluorophenyl)-4,5-dihydro-1H-pyrazole-1-carbonyl)hexahydrocyclopenta[c]pyrrol-2(1H)-yl)pyrimidine-4-carboxamide (Z-18-4)

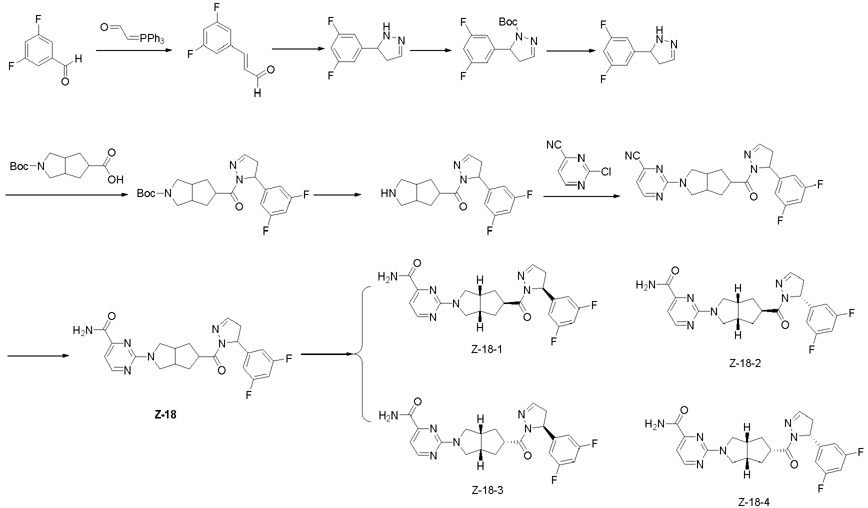

Step 1: 3,5-difluorobenzaldehyde (20.0 g, 140 mmol) and (formylmethylene)triphenylphosphorane (20 g, 140 mmol) were dissolved in tetrahydrofuran (250 mL). The resultant solution was heated to 80°C, and reacted under reflux overnight. The reaction mixture was concentrated at a reduced pressure. The crude product was subjected to column chromatography (petroleum ether/ethyl acetate: 10/1) to give 3-(2,5-difluorophenyl)acraldehyde (yellow solid, 20 g, yield: 84%). ES-API:[M+H]⁺= 169.0.

Step 2: 3-(2,5-difluorophenyl)acraldehyde (25 g, 150 mmol) was dissolved in ethanol (500 mL). After adding the solution dropwise to hydrazine hydrate (17.5 g, 350 mmol) at 50°C over about 2 h, the reaction was carried out at a constant temperature of 50°C overnight. The resultant reaction mixture was spin-dried, and entrained with toluene for three times to give 5-(3,5-difluorophenyl)-4,5-dihydro-1H-pyrazole (32 g, yield: 100%), which was directly used in the next step. ES-API:[M+H]⁺= 183.0.

Step 3: 5-(3,5-difluorophenyl)-4,5-dihydro-1H-pyrazole (32 g, 176 mmol) was dissolved in tetrahydrofuran (80 mL). Then, di-tert-butyl dicarbonate (76 g, 351 mmol) was added. The reaction was carried out at the room temperature overnight. The resultant reaction mixture was concentrated at a reduced pressure. The crude product was subjected to column chromatography (petroleum ether/ethyl acetate: 3/1), and recrystallized from acetonitrile to give tert-butyl 5-(3,5-difluorophenyl)-4,5-dihydro-1H-pyrazole-1-carboxylate (pale yellow solid, 8 g, 19%). ES-API:[M-55]⁺= 227.0.

Step 4: tert-butyl 5-(3,5-difluorophenyl)-4,5-dihydro-1H-pyrazole-1-carboxylate (700 mg, 2.48 mmol) was dissolved in dichloromethane (7 mL). Then, trifluoroacetic acid was added (2.5 mL). The reaction mixture was reacted at the room temperature for 0.5 h, and concentrated at a reduced pressure to give 5-(3,5-difluorophenyl)-4,5-dihydro-1H-pyrazole (700 mg, yield: 100%), which was directly used in the next step. ES-API:[M+H]⁺= 183.1.

Step 5: (3aR,6aS)-2-(tert-butoxycarbonyl)octacyclopenta[c]pyrrole-5-carboxylic acid (696 mg, 2.73 mmol) was dissolved in dichloromethane. Then, triethylamine (1 mL), 2-(7-azabenzotriazole)-N,N,N',N'-tetramethyluronium hexafluorophosphate (1.56 g, 4.09 mol) and 5-(3,5-difluorophenyl)-4,5-dihydro-1H-pyrazole (700 mg, 2.48 mmol) were added. The reaction was carried out at the room temperature overnight. The resultant reaction mixture was extacted with ethyl acetate, and concentrated at a reduced pressure. The crude product was subjected to column chromatography (petroleum ether/ethyl acetate: 3/2) to give tert-butyl (3aR,6aS)-5-(5-(3,5-difluorophenyl)-4,5-dihydro-1H-pyrazole-1-carbonyl)hexahydrocyclopenta[c] pyrrole-2(1H)-carboxylate (brown liquid, 650 mg, yield: 57%). ES-API:[M-55]⁺= 364.1.

Step 6: tert-butyl (3aR,6aS)-5-(5-(3,5-difluorophenyl)-4,5-dihydro-1H-pyrazole-1-carbonyl)hexahydrocyclopenta[c]pyrrole-2(1H)-carboxylate (650 mg, 1.55 mmol) was dissolved in dichloromethane (15 mL). Then, trifluoroacetic acid (4 mL) was added. The mixture was stirred for reaction at the room temperature for 40 minutes. The resultant reaction mixture was concentrated at a reduced pressure to give a crude product of ((5-(3,5-difluorophenyl)-4,5-dihydro-1H-pyrazol-1-yl) (3aR,6aS)-octahydrocyclopenta[c]pyrrol-5-yl) ketone trifluoroacetate (500 mg, yield: 100%).

Step 7: The crude product of ((5-(3,5-difluorophenyl)-4,5-dihydro-1H-pyrazol-1-yl) (3aR,6aS)-octahydrocyclopenta[c]pyrrol-5-yl)ketone trifluoroacetate (500 mg, 1.55 mmol) was dissolved in N,N-dimethylformamide (5 mL). Then, 2-chloropyrimidine-4-carbonitrile (260 mg, 1.86 mmol) and potassium carbonate (642 mg, 4.65 mmol) were added. The reaction was carried out at 50°C overnight. The resultant reaction mixture was extracted with ethyl acetate, and was concentrated at a reduced pressure. The crude product was subjected to column chromatography (petroleum ether/ethyl acetate: 1/1) to give 2-(3aR,6aS)-(5-(5-(3,5-difluorophenyl)-4,5-dihydro-1H-pyrazole-1-carbonyl)hexahydrocyclopenta[c]pyrrol-2(1H)-yl) pyrimidine-4-carbonitrile (513 mg, yield: 78%). ES-API:[M+H]⁺= 423.1.

Step 8: 2-(3aR,6aS)-(5-(5-(3,5-difluorophenyl)-4,5-dihydro-1H-pyrazole-1-carbonyl)hexahydrocyclopenta[c]pyrrol-2(1H)-yl)pyrimidine-4-carbonitrile (513 mg, 1.2 mmol) was dissolved in acetonitrile/water (9 mL, V/V=2/1 ). Then, sodium hydroxide (72 mg, 1.8 mmol) and hydrogen peroxide (3 mL, 30% aqueous solution) were added. The reaction was carried out at the room temperature for 1 h. The resultant reaction mixture was extacted with ethyl acetate, and concnetated at a reduced pressure. The crude product was subjected to column chromatography (petroleum ether/ethyl acetate: 1/5) to give 2-(3aR,6aS)-(5-(5-(3,5-difluorophenyl)-4,5-dihydro-1H-pyrazole-1-carbonyl)hexahydrocyclopenta[c]pyrrol-2(1H)-yl) pyrimidine-4-carboxamide (Z-18, white solid, 210 mg, yield: 40%). ES-API:[M+H]⁺= 441.2. ¹H NMR(400 MHz, CDCl₃) δ 8.52 (dd, *J* = 15.3, 4.9 Hz, 1H), 7.67 (s, 1H), 7.25 (d, *J* = 5.1 Hz, 3H), 7.02 - 6.86 (m, 1H), 6.67 (dd, *J* = 10.1, 7.5 Hz, 3H), 5.49 (s, 1H), 5.30 (dd, *J* = 12.0, 4.9 Hz, 1H), 3.87 - 3.66 (m, 3H), 3.59 (d, *J* = 11.5 Hz, 2H), 3.42 (dd, *J* = 18.9, 12.0 Hz, 1H), 2.85 (s, 1H), 2.77 (dd, *J* = 18.9, 4.7 Hz, 1H), 2.26 (ddd, *J* = 33.7, 18.4, 10.4 Hz, 2H), 1.80 (s, 2H).

Step 9: The Z-18 (210 mg) as obtained in the above step was subjected to a chiral resolution (mobile phase: n-hexane (0.1%DEA):ethanol (0.1%DEA)=60:40); column: IE (4.6^{∗}250mm 5um); flow rate: 1.0 ml/min; room temperature: 40°C) , to give 4 isomers having a single configuration (the structures of the 4 isomers having a single configuration are each arbitrarily assigned):
Isomer 1 (retention time: 12.589 min), arbitrarily assigned as a compound of Z-18-1, 2-((3aR,5s,6aS)-5-((S)-5-(3,5-difluorophenyl)-4,5-dihydro-1H-pyrazole-1-carbonyl)hexahydrocyclopenta[c]pyrrol-2(1H)-yl)pyrimidine-4-carboxamide (14 mg, purity: 100%, ee value: 100%). ES-API: [M+H]⁺= 441.1.
Isomer 2 (retention time: 14.751 min), arbitrarily assigned as a compound of Z-18-2, 2-((3aS,5s,6aS)-5-((R)-5-(3,5-difluorophenyl)-4,5-dihydro-1H-pyrazole-1-carbonyl)hexahydrocyclopenta[c]pyrrol-2(1H)-yl)pyrimidine-4-carboxamide (76 mg, purity: 100%, ee value: 99.8%). ES-API:[M+H]⁺= 441.1.
Isomer 3 (retention time: 18.265 min), arbitrarily assigned as a compound of Z-18-3, 2-((3aR,5r,6aS)-5-((S)-5-(3,5-difluorophenyl)-4,5-dihydro-1H-pyrazole-1-carbonyl)hexahydrocyclopenta[c]pyrrol-2(1H)-yl)pyrimidine-4-carboxamide (13 mg, purity: 100%, ee value: 100%). ES-API: [M+H]⁺= 441.1.
Isomer 4 (retention time: 25.228 min), arbitrarily assigned as a compound of Z-18-4, 2-((3aR,5r,6aR)-5-((R)5-(3,5-difluorophenyl)-4,5-dihydro-1H-pyrazole-1-carbonyl)hexahydrocyclopenta[c]pyrrol-2(1H)-yl)pyrimidine-4-carboxamide (72 mg, purity: 100%, ee value: 100%). ES-API: [M+H]⁺= 441.1.

### Example 18: Preparation of 6-(5-(5-(2,5-difluorophenyl)-4,5-dihydro-1H-pyrazole-1-carbonyl)hexahydrocyclopenta[c]pyrrol-2(1H)-yl)pyrimidine-4-carbonitrile (Z-19)

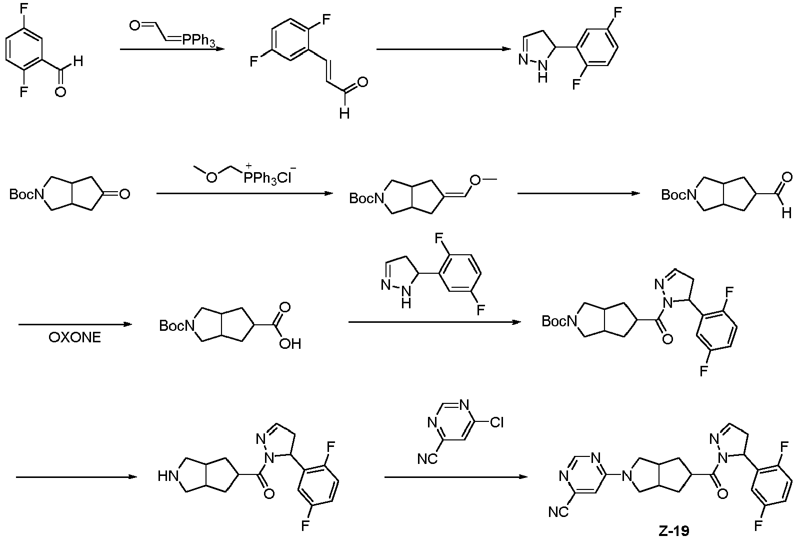

Step 1: 2,5-difluorobenzaldehyde (10.16 g, 0.072 mol) and (formylmethylene)triphenylphosphorane (21.77g, 0.072 mol) were dissolved in tetrahydrofuran (200 mL). The resultant solution was heated to 80°C, and reacted under reflux overnight. The reaction mixture was concentrated at a reduced pressure, and subjected to column chromatography (petroleum ether/ethyl acetate: 10/1) to give 3-(2,5-difluorophenyl)acraldehyde (11 g, yield: 91%). ES-API:[M+H]⁺= 169.0.

Step 2: 3-(2,5-difluorophenyl)acraldehyde (3 g, 0.018 mol) was dissolved in tert-butanol (40 mL). After adding the solution dropwise to hydrazine hydrate (3 mL) at 50°C over about 1 h, the reaction was carried out at a constant temperature of 50°C overnight. The resultant reaction mixture was spin-dried, and entrained with toluene for three times to give 5-(2,5-difluorophenyl)-4,5-dihydro-1H-pyrazole (3.3 g, yield: 100%), which was directly used in the next step. ES-API:[M+H]⁺= 183.0.

Step 3: potassium tert-butoxide (102.3 g, 0.91 mol) and (methoxymethyl) triphenylphosphonium chloride (415.6 g, 1.22 mol) were added to tetrahydrofuran (2 L). After the mixture was stirred for 0.5 h, N-tert-butoxycarbonyl-hexahydro-5-oxocyclopenta[C]pyrrole (45.6 g, 0.2 mol) was added. Thereafter, the reaction was carried out at the room temperature for 1 h. The resultant reaction mixture was concentrated at a reduced pressure, and subjected to column chromatography (petroleum ether/ethyl acetate: 5/1), to give tert-butyl 5-(methoxymethylene) hexahydrocyclopenta[c]pyrrole-2(1H)-carboxylate (40 g, yield: 90%).

Step 4: tert-butyl 5-(methoxymethylene)hexahydrocyclopenta[c]pyrrole-2(1H)-carboxylate (40 g,0.2 mol) was dissolved in tetrahydrofuran (1 L). Then, p-toluenesulfonic acid monohydrate (39.9 g, 0.21 mol) and water (3.8 g, 0.4 mol) were added. The reaction was carried out at 25 °C for 2 h. The resultant reaction mixture was used in the next step.

Step 5: water (200 mL) and potassium monopersulfate complex salt (247 g, 0.4 mol) were added to the above reaction mixture. The reaction was carried out at 25°C for 2 h. The resultant reaction mixture was spin-dried at a low temperature to remove tetrahydrofuran. Dichloromethane (1 L) was added. the resultant solution was washed with water, dried, concentrated at a reduced pressure, and subjected to column chromatography (methanol/dichloromethane: 0-10%) to give 2-(tert-butoxycarbonyl)octahydrocyclopenta[c]pyrrole-5-carboxylic acid (13 g, yield: 26%). ES-API: [M-H]⁺=254.

Step 6: 2-(tert-butoxycarbonyl)octahydrocyclopenta[c]pyrrole-5-carboxylic acid (3.67 g, 0.014 mol), 5-(2,5-difluorophenyl)-4,5-dihydro-1H-pyrazole (3.28 g, 0.018 mol), N,N-diisopropylethylamine (4.6 g, 0.036 mol) and 2-(7-azabenzotriazole)-N,N,N',N'-tetramethyluronium hexafluorophosphate (8.2 g, 0.022 mol) were added to N,N-dimethylformamide (100 mL). The reaction was carried out at 25°C for 2 h. The resultant reaction mixture was extracted with ethyl acetate, and subjected to column chromatography (petroleum ether/ethyl acetate: 10%-45%) to give tert-butyl 5-(5-(2,5-difluorophenyl)-4,5-dihydro-1H-pyrrol-1-carbonyl)hexahydrocyclopenta[c] pyrrole-2(1H)-carboxylate (2 g, yield: 34%). ES-API:[M+H]⁺= 420.0.

Step 7: tert-butyl 5-(5-(2,5-difluorophenyl)-4,5-dihydro-1H-pyrrol-1-carbonyl) hexahydrocyclopenta[c]pyrrole-2(1H)-carboxylate (523 mg, 1.25 mmol) was dissolved in dichloromethane (2 mL). Then, trifluoroacetic acid (2 mL) was added. The reaction was carried out at 25°C for 2 h. The resultant reaction mixture was spin-dried to give (5-(2,5-difluorophenyl)-4,5-dihydro-1H-pyrazol-1-yl)(octahydro cyclopenta[c]pyrrol-5-yl)ketone trifluoroacetate. ES-API:[M+H]⁺= 320.0.

Step 8: (5-(2,5-difluorophenyl)-4,5-dihydro-1H-pyrrol-1-yl)(hexahydrocyclo penta[c]pyrrol-5-yl)ketone trifluoroacetate (399 mg, 1.25 mmol), 6-chloropyrimidine-4-carbonitrile (174 mg, 1.25 mmol) and potassium carbonate (345 mg, 2.5 mmol) were added to N,N-dimethylformamide (7 mL). The reaction was carried out at a heating temperature of 50°C for 2 h. The resultant reaction mixture was extracted with ethyl acetate, and subjected to column chromatography (petroleum ether/ethyl acetate: 10%-45%) to give 6-(5-(5-(2,5-difluorophenyl)-4,5-dihydro-1H-pyrazole-1-carbonyl)hexahydrocyclopenta[c]pyrrol-2(1H)-yl) pyrimidine-4-carbonitrile (Z-19, 560 mg, yield: 100%), 200 mg of which was used to give 90 mg of the product. ES-API:[M+H]⁺= 423.1. ¹H NMR(400 MHz, CDCl₃) δ 8.60 (d, *J* = 16.2 Hz, 1H), 7.09-6.91 (m, 3H), 6.76 (m, *J* = 8.3, 5.6, 3.0 Hz, 1H), 6.62 (d, *J* = 24.3 Hz, 1H), 5.54 (dd, *J* = 12.0, 5.3 Hz, 1H), 3.82-2.82 (m, 9H), 2.25-1.60 (m, 4H).

### Example 19: Preparation of 6-(5-(5-(2,5-difluorophenyl)-4,5-dihydro-1H-pyrazole-1-carbonyl)hexahydrocyclopenta[c]pyrrol-2(1H)-yl)pyrimidine-4-carboxamide (Z-20)

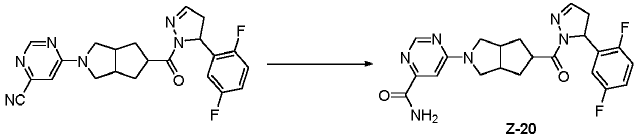

Step 1: 6-(5-(5-(2,5-difluorophenyl)-4,5-dihydro-1H-pyrazole-1-carbonyl) hexahydrocyclopenta[c]pyrrol-2(1H)-yl)pyrimidine-4-carbonitrile (Z-19, 400 mg, 0.95 mmol) was dissolved in acetonitrile/ water (4 mL/2mL). Then, sodium hydroxide (42 mg, 1.04 mmol) and hydrogen peroxide (2mL) were added. The reaction mixture was stirred at the room temperature for 2 h, extracted with ethyl acetate, and washed with a solution of sodium sulphite. The organic phase was concentrated. The crude product was separated by preparative HPLC (column: Ultimate XB-C18,50^{∗}250 mm,10 um; mobile phase system: A: pure water; B: pure acetonitrile; flow rate: 80 ml/min, B/A=20%-100%; column temperature: room temperature) to give 6-(5-(5-(2,5-difluorophenyl)-4,5-dihydro-1H-pyrazole-1-carbonyl)hexahydrocyclopenta[c]pyrrol-2(1H)-yl)pyrimidine-4-carboxamide (Z-20, 155 mg, yield: 41%). ES-API:[M+H]⁺= 441.2. ¹H NMR(400 MHz, CDCl₃) δ 8.56 (dd, J = 15.3, 1.1 Hz, 1H), 7.84 (s, 1H), 7.14- 6.72 (m, 5H), 5.64 (s, 1H), 5.54 (dt, J = 10.8, 5.3 Hz, 1H), 3.95 -3.46 (m, 6H), 3.02-2.81 (3, 3H), 2.35 -1.83 (m, 4H).

### Example 20: Preparation of (2-(4-amino-5-fluoropyrimidin-2-yl)octohydrocyclopenta[c]pyrrol-5-yl)(5-(2,5-difluorophenyl)-4,5-dihydro-1H-pyrazol-1-yl) ketone (Z-21)

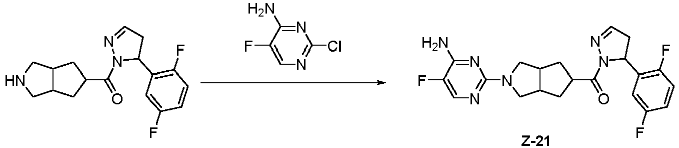

Step 1: (5-(2,5-difluorophenyl)-4,5-dihydro-1H-pyrrol-1-yl) (hexahydrocyclopenta[c]pyrrol-5-yl) ketone trifluoroacetate (252 mg, 0.79 mmol), 2-chloro-5-fluoropyrimidine-4-amine (117 mg, 0.79 mmol) and potassium carbonate (218 mg, 1.58 mmol) were added to N,N-dimethylformamide (5 mL). The reaction was carried out at a heating temperature of 110°C for 5 h. The resultant reaction mixture was extracted with ethyl acetate, and subjected to column chromatography (petroleum ether/ethyl acetate: 40%-90%) to give (2-(4-amino-5-fluoropyrimidin-2-yl)octahydrocyclopenta[c]pyrrol-5-yl)(5-(2,5-difluorophenyl)-4,5-dihydro-1H-pyrazol-1-yl) ketone (Z-21, 95 mg, yield: 28%). ES-API:[M+H]⁺= 431.2. ¹H NMR(400 MHz, CDCl₃) δ 7.86 (d, *J* = 3.2 Hz, 1H), 7.03-6.75 (m,4H), 5.55 (dd, *J* = 12.2, 5.2 Hz, 1H), 4.80 (bs, 2H), 3.82- 3.32 (m, 6H), 2.93 (s, 2H), 2.79 (dd, *J* = 18.7, 5.4 Hz, 1H), 2.15- 1.83 (m, 4H).

### Example 21: Preparation of 2-(5-(5-(2,5-difluorophenyl)-4,5-dihydro-1H-pyrazole-1-carbonyl)hexahydrocyclopenta[c]pyrrol-2(1H)-yl)pyrimidine-4-carboxamide (Z-22), 2-((3aR,5s,6aS)-5-((S)-5-(2,5-difluorophenyl)-4,5-dihydro-1H-pyrazole-1-carbonyl)hexahydrocyclopenta[c]pyrrol-2(1H)-yl)pyrimidine-4-carboxamide (Z-22-1), 2-((3aR,5s,6aS)-5-((R)-5-(2,5-difluorophenyl)-4,5-dihydro-1H-pyrazole-1-carbonyl)hexahydrocyclopenta[c]pyrrol-2(1H)-yl) pyrimidine-4-carboxamide (Z-22-2), 2-((3aR,5r,6aS)-5-((S)-5-(2,5-difluorophenyl)-4,5-dihydro-1H-pyrazole-1-carbonyl) hexahydrocyclopenta[c]pyrrol-2(1H)-yl)pyrimidine-4-carboxamide (Z-22-3), 2-((3aR,5r,6aS)-5-((R)-5-(2,5-difluorophenyl)-4,5-dihydro-1H-pyrazole-1-carbonyl)hexahydrocyclopenta[c]pyrrol-2(1H)-yl)pyrimidine-4-carboxamide (Z-22-4)

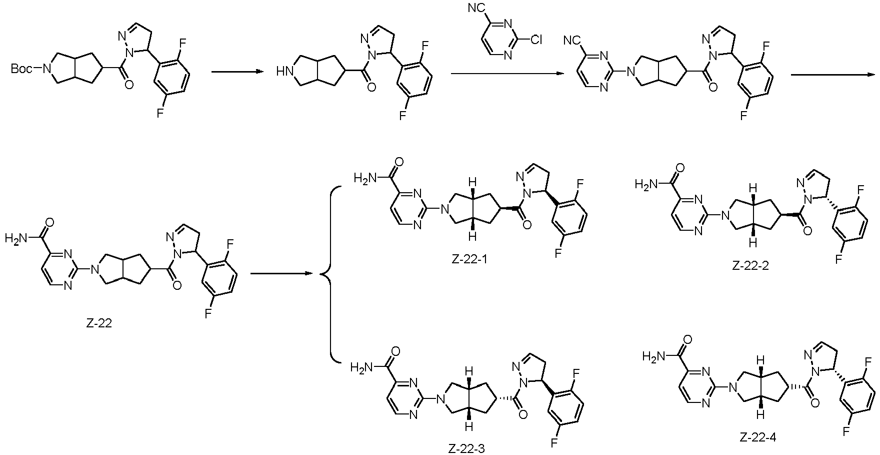

Step 1: tert-butyl (3aR,6aS)-5-(5-(2,5-difluorophenyl)-4,5-dihydro-1H-pyrazole-1-carbonyl)hexahydrocyclopenta[c]pyrrole-2(1H)-carboxylate (400 mg, 0.95 mmol) was dissolved in dichloromethane (2 mL). Then, trifluoroacetic acid (2 mL) was added. The reaction was carried out at 25°C for 2 h. The resultant reaction mixture was spin-dried to give (5-(2,5-difluorophenyl)-4,5-dihydro-1H-pyrazol-1-yl) (3aR,6aS)-(octahydrocyclopenta[c]pyrrol-5-yl)ketone trifluoroacetate (303 mg, yield: 100%). ES-API:[M+H]⁺= 320.0.

Step 2: (5-(2,5-difluorophenyl)-4,5-dihydro-1H-pyrazol-1-yl) (3aR,6aS)-(octahydrocyclopenta[c]pyrrol-5-yl)ketone trifluoroacetate (303 mg, 0.95 mmol), 2-chloropyrimidine-4-carbonitrile (133 mg, 0.95 mmol) and potassium carbonate (393 mg, 2.85 mmol) were added to N,N-dimethylformamide (10 mL). The reaction mixture was heated to 50°C, and reacted for 2 h. Thereafter, the reaction mixture was extracted with ethyl acetate, and subjected to column chromatography (petroleum ether/ethyl acetate: 10%~40%) to give 2-(3aR,6aS)-(5-(5-(2,5-difluorophenyl)-4,5-dihydro-1H-pyrazole-1-carbonyl)hexahydrocyclopenta[c]pyrrol-2(1H)-yl)pyrimidine-4-carbonitrile as a yellow oil (330 mg, yield: 82%). ES-API: [M+H]⁺= 423.1.

Step 3: 2-(3aR,6aS)-(5-(5-(2,5-difluorophenyl)-4,5-dihydro-1H-pyrazole-1-carbonyl)hexahydrocyclopenta[c]pyrrol-2(1H)-yl)pyrimidine-4-carbonitrile (320 mg, 0.76 mmol) was dissolved in acetonitrile/water (4mL/2mL). Then, sodium hydroxide (34 mg, 0.84 mmol) and hydrogen peroxide (1 mL) were added. The reaction mixture was stirred at the room temperature for 2 h, extracted with ethyl acetate, and washed with a solution of sodium sulphite. The organic phase was concentrated. The crude product was subjected to preparative HPLC (column: Ultimate XB-C18,50^{∗}250 mm,10 um; mobile phase system: A: pure water; B: pure acetonitrile; flow rate: 80 ml/min, B/A=20%-100%; column temperature: room temperature) to give 2-(3aR,6aS)-(5-(5-(2,5-difluorophenyl)-4,5-dihydro-1H-pyrazole-1-carbonyl) hexahydrocyclopenta[c]pyrrol-2(1H)-yl)pyrimidine-4-carboxamide (Z-22, 172 mg, yield: 59%). ES-API:[M+H]⁺= 441.2. ¹H NMR(400 MHz, CDCl₃) δ 8.56 (dd, *J* = 15.3, 1.1 Hz, 1H), 7.84 (s, 1H), 7.14 (dd, *J* = 19.2, 1.0 Hz, 1H), 7.07 - 6.89 (m, 3H), 6.79 - 6.72 (m, 1H), 5.64 (s, 1H), 5.54 (m, *J* = 10.8, 5.3 Hz, 1H), 3.95 - 3.69 (m, 3H), 3.46 (dd, *J* = 18.1, 12.7 Hz, 3H), 3.02 (s, 2H), 2.81 (dd, *J* = 18.9, 5.2 Hz, 1H), 2.35 - 2.14 (m, 2H), 2.05 - 1.95 (m, 1H), 1.93 - 1.83 (m, 1H).

Step 4: the Z-22 as obtained in the above step was subjected to a chiral resolution with the method as described below (column: CHIRALPAKAD-3(AD30CD-SK010) 0.46 cm I.D. × 15 cm L; mobile phase: EtOH=100%; flow rate: 1.0 ml/min; column temperature: 35°C; HPLC instrument: Shimadzu LC-20AT CP-HPLC-09) to give 4 isomers having a single configuration:
Isomer 1: arbitrarily assigned as Z-22-1 (1 mg; LCMS retention time: 1.70 min; purity: 92.3%; ee value: 84.6%; ¹H NMR (400 MHz, CDCl₃) δ 8.54 (d, *J* = 4.8 Hz, 1H), 7.71 (s, 1H), 7.01 (dt, *J* = 9.1, 4.3 Hz, 1H), 6.94 (s, 2H), 6.73 (d, *J* = 8.7 Hz, 1H), 5.56 - 5.51 (m, 2H), 3.81 (d, *J* = 7.1 Hz, 3H), 3.47 (s, 2H), 2.98 (s, 2H), 2.22 (t, *J* = 7.3 Hz, 2H), 2.02 (s, 2H).
Isomer 2: arbitrarily assigned as Z-22-2 (2.4 mg; LCMS retention time: 1.72 min; purity: 93.8%; ee value:: 87.6%); ¹H NMR (400 MHz, CDCl₃) δ 8.50 (d, *J* = 4.8 Hz, 1H), 7.69 (s, 1H), 7.23 (d, *J* = 4.8 Hz, 1H), 7.00 (dt, *J* = 9.2, 4.6 Hz, 1H), 6.90 (ddd, *J* = 12.1, 7.9, 3.5 Hz, 1H), 6.73 (ddd, *J* = 8.7, 5.7, 3.0 Hz, 1H), 5.52 (dd, *J =* 11.9, 5.5 Hz, 2H), 3.72 (q, *J* = 5.6, 3.9 Hz, 3H), 3.58 (d, *J* = 11.6 Hz, 3H), 2.87 - 2.81 (m, 2H), 2.29 (ddd, *J* = 33.7, 13.2, 6.5 Hz, 2H), 1.82 (s, 2H).
Isomer 3: arbitrarily assigned as Z-22-3 (2.3 mg; LCMS retention time: 1.73 min; purity: 96.2%; ee value:: 91.4%); ¹H NMR (400 MHz, CDCl₃) δ 8.49 (d, *J* = 4.8 Hz, 1H), 7.69 (s, 1H), 7.23 (d, *J* = 4.8 Hz, 1H), 7.03 - 6.97 (m, 1H), 6.90 (dd, *J* = 8.3, 4.1 Hz, 1H), 6.73 (ddd, *J* = 8.8, 5.8, 3.1 Hz, 1H), 5.52 (dd, *J* = 12.1, 5.4 Hz, 2H), 3.73 (p, *J* = 4.5 Hz, 3H), 3.62 - 3.41 (m, 3H), 2.85 (dd, *J* = 7.8, 4.4 Hz, 2H), 2.36 - 2.19 (m, 2H), 1.82 (s, 2H).
Isomer 4: arbitrarily assigned as Z-22-4 (1.1 mg; LCMS retention time: 1.70 min; purity: 95.6%; ee value: 91.2%); ¹H NMR (400 MHz, CDCl₃) δ 8.53 (d, *J* = 4.8 Hz, 1H), 7.71 (s, 1H), 7.01 (td, *J* = 9.1, 4.2 Hz, 1H), 6.93 (d, *J* = 8.0 Hz, 2H), 6.75 (d, *J* = 8.7 Hz, 1H), 5.53 (dd, *J* = 12.2, 5.4 Hz, 2H), 3.82 (t, *J* = 7.8 Hz, 3H), 3.47 (t, *J* = 11.4 Hz, 3H), 2.98 (s, 2H), 2.23 - 2.18 (m, 2H), 2.01 (d, *J* = 6.6 Hz, 2H).

### Example 22: Preparation of (2-(4-amino-5-fluoropyrimidin-2-yl)octahydrocyclopenta[c]pyrrol-5-yl)(5-(3,5-difluorophenyl)-4,5-dihydro-1H-pyrazol-1-yl) ketone (Z-23), ((3aR,5s,6aS)-2-(4-amino-5-fluoropyrimidin-2-yl) octahydrocyclopenta[c]pyrrol-5-yl)((S)-5-(3,5-difluorophenyl)-4,5-dihydro-1H-pyrazol-1-yl) ketone(Z-23-1), ((3aR,5s,6aS)-2-(4-amino-5-fluoropyrimidin-2-yl) octahydrocyclopenta[c]pyrrol-5-yl)((R)-5-(3,5-difluorophenyl)-4,5-dihydro-1H-pyrazol-1-yl) ketone (Z-23-2), ((3aR,5r,6aS)-2-(4-amino-5-fluoropyrimidin-2-yl) octahydrocyclopenta[c]pyrrol-5-yl)((S)-5-(3,5-difluorophenyl)-4,5-dihydro-1H-pyrazol-1-yl) ketone (Z-23-3), ((3aR,5r,6aS)-2-(4-amino-5-fluoropyrimidin-2-yl) octahydrocyclopenta[c]pyrrol-5-yl)((R)-5-(3,5-difluorophenyl)-4,5-dihydro-1H-pyrazol-1-yl) ketone (Z-23-4)

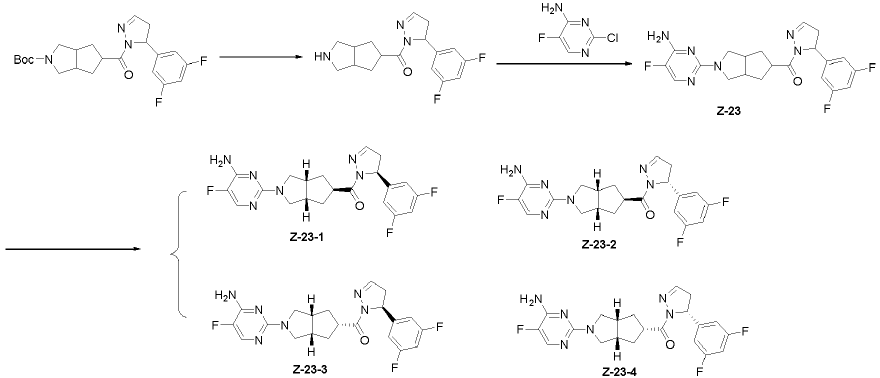

Step 1: tert-butyl (3aR,6aS)-5-(5-(3,5-difluorophenyl)-4,5-dihydro-1H-pyrazole-1-carbonyl)hexahydrocyclopenta[c]pyrrole-2(1H)-carboxylate (650 mg, 1.55 mmol) was dissolved in dichloromethane (10 mL). Then, trifluoroacetic acid was added (2 mL). The reaction was carried out at 25°C for 4 h. The resultant reaction mixture was concentrated at a reduced pressure to give a crude product of (3aR,6aS)-(5-(3,5-difluorophenyl)-4,5-dihydro-1H-pyrazol-1-yl) (octahydrocyclopenta[c]pyrrol-5-yl) ketone trifluoroacetate (520 mg, crude product). ES-API:[M+H]⁺= 320.0.

Step 2: (3aR,6aS)-(5-(3,5-difluorophenyl)-4,5-dihydro-1H-pyrrol-1-yl) (hexahydrocyclopenta[c]pyrrol-5-yl) ketone trifluoroacetate (300 mg, 0.94 mmol), 2-chloro-5-fluoropyrimidine-4-amine (139 mg, 0.94 mmol) and potassium carbonate (389 mg, 2.82 mmol) were added to N,N-dimethylformamide (8 mL). The reaction mixture was heated to 100°C, and reacted for 16 h. The resultant reaction mixture was extracted with ethyl acetate, and subjected to preparative HPLC to give (3aR,6aS)-(2-(4-amino-5-fluoropyrimidin-2-yl) (octahydrocyclopenta[c]pyrrol-5-yl)(5-(3,5-difluorophenyl)-4,5-dihydro-1H-pyrazol-1-yl) ketone (Z-23, 160 mg, yield: 40%). ES-API:[M+H]⁺= 431.2. ¹H NMR(300 MHz, CDCl₃) δ 7.84 ( m, 1H), 6.95 (m, 1H), 6.80 - 6.56 (m, 3H), 5.43 - 5.28 (m, 1H), 4.83 (bs, 2H), 3.86 - 3.31 (m, 6H), 2.78 - 2.75 (m, 2H), 2.28-2.25 (m, 2H), 1.92 - 1.69 (m, 3H).

Step 3: the Z-23 (150 mg) as obtained in the above step was subjected to a chiral resolution (column: CHIRALPAK IG(IG00CE-UC011)0.46 cm I.D.×25 cm L; mobile phase: MeOH=100%; flow rate: 1.0 ml/min; column temperature: 35 °C; HPLC instrument: Shimadzu LC-20AT CP-HPLC-06) to give 4 isomers having a single configuration (the structures of the 4 isomers having a single configuration are each arbitrarily assigned):
Isomer 1: arbitrarily assigned as Z-23-1, ((3aR,5s,6aS)-2-(4-amino-5-fluoropyrimidin-2-yl) octahydrocyclopenta[c]pyrrol-5-yl)((S)-5-(3,5-difluorophenyl)-4,5-dihydro-1H-pyrazol-1-yl) ketone (9.5 mg, LCMS retention time: 5.60 min; purity: 99.29%; ee value: 98.517%); ES-API:[M+H]⁺= 431.2. ¹H NMR (300 MHz, CDCl₃) δ 7.85 (s, 1H), 6.91 (s, 1H), 6.69 - 6.66 (m, 3H), 5.32 - 5.28 (m, 1H), 4.78 (bs, 2H), 3.80 - 3.68 (m, 3H), 3.44 - 3.31 (m, 3H),2.90 - 2.71 (m, 3H), 2.15-1.84 (m, 4H).
Isomer 2: arbitrarily assigned as Z-23-2, ((3aR,5s,6aS)-2-(4-amino-5-fluoropyrimidin-2-yl) octahydrocyclopenta[c]pyrrol-5-yl)((R)-5-(3,5-difluorophenyl)-4,5-dihydro-1H-pyrazol-1-yl) ketone (9.6 mg, LCMS retention time: 6.24 min; purity: 99.39%; ee value: 99.092%); ES-API:[M+H]⁺= 431.2. ¹H NMR (300 MHz, CDCl₃) δ 7.85 (s, 1H), 6.91 (s, 1H), 6.69-6.66 (m, 3H), 5.32-5.28 (m, 1H), 4.80 (bs, 2H), 3.80-3.68 (m, 3H), 3.44-3.31 (m, 3H), 2.90-2.71 (m, 3H), 2.15-1.84 (m, 4H).
Isomer 3: arbitrarily assigned as Z-23-3, ((3aR,5r,6aS)-2-(4-amino-5-fluoropyrimidin-2-yl) octahydrocyclopenta[c]pyrrol-5-yl)((S)-5-(3,5-difluorophenyl)-4,5-dihydro-1H-pyrazol-1-yl) ketone (64.6 mg, LCMS retention time: 7.27 min; purity: 99.10%; ee value: 99.569%); ES-API:[M+H]⁺= 431.2. ¹H NMR (300 MHz, CDCl₃) δ 7.81 (s, 1H), 6.93 (s, 1H), 6.68-6.65 (m, 3H), 5.32-5.28 (m, 1H), 4.74 (bs, 2H), 3.65-3.37 (m, 6H), 2.78-2.72 (m, 3H), 2.28-2.19 (m, 2H), 1.78-1.73 (m, 2H).
Isomer 4: arbitrarily assigned as Z-23-4, ((3aR,5r,6aS)-2-(4-amino-5-fluoropyrimidin-2-yl)octahydrocyclopenta[c]pyrrol-5-yl)((R)-5-(3,5-difluorophenyl)-4,5-dihydro-1H-pyrazol-1-yl) ketone (56 mg, LCMS retention time: 16.01 min; purity: 99.15%; ee value: 100%); ES-API:[M+H]⁺= 431.2. ¹H NMR (300 MHz, CDCl₃) δ 7.80 ( s, 1H), 6.93 (s, 1H), 6.68 - 6.65 (m, 3H), 5.32 - 5.28 (m, 1H), 4. 75 (bs, 2H), 3.63 - 3.37 (m, 6H), 2.78 - 2.72 (m, 3H), 2.28-2.19 (m, 2H), 1.78-1.73 (m, 2H).

### Example 23: Preparation of 2-(5-(5-(2,5-difluorophenyl)-4,5-dihydro-1H-pyrazole-1-carbonyl)hexahydrocyclopenta[c]pyrrol-2(1H)-yl)pyrimidine-4-carbonitrile (Z-24)

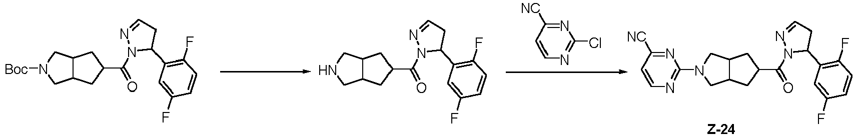

Step 1: tert-butyl 5-(5-(2,5-difluorophenyl)-4,5-dihydro-1H-pyrazole-1-carbonyl)hexahydrocyclopenta[c]pyrrole-2(1H)-carboxylate (213 mg, 0.51 mmol) was dissolved in dichloromethane (1 mL). Then, trifluoroacetic acid was added (1 mL). The reaction was carried out at 25°C for 2 h. The resultant reaction mixture was spin-dried to give (5-(2,5-difluorophenyl)-4,5-dihydro-1H-pyrazol-1-yl) (octahydrocyclopenta[c]pyrrol-5-yl) ketone trifluoroacetate (163 mg, yield: 100%). ES-API:[M+H]⁺= 320.0.

Step 2: (5-(2,5-difluorophenyl)-4,5-dihydro-1H-pyrazol-1-yl) (octahydro cyclopenta[c]pyrrol-5-yl) ketone trifluoroacetate (163 mg, 0.51 mmol), 2-chloropyrimidine-4-carbonitrile (72 mg, 0.51 mmol) and potassium carbonate (140 mg, 1.02 mmol) were added to N,N-dimethylformamide (2 mL). The reaction mixture was heated to 50°C, and reacted for 2 h. The resultant reaction mixture was extracte with ethyl acetate, and subjected to column chromatography (petroleum ether/ethyl acetate: 10%-40%) to give 2-(5-(5-(2,5-difluorophenyl)-4,5-dihydro-1H-pyrazole-1-carbonyl)hexahydrocyclopenta[c]pyrrol-2(1H)-yl)pyrimidine-4-carbonitrile as a yellow oil (Z-24, 105 mg, yield: 52%). ES-API:[M+H]⁺= 423.1. ¹H NMR(400 MHz, CDCl₃) δ 8.41 (dd, *J* = 16.3, 4.7 Hz, 1H), 7.01 (m, *J* = 9.2, 4.4 Hz, 1H), 6.97 - 6.86 (m, 2H), 6.77 - 6.68 (m, 2H), 5.51 (m, *J* = 9.7, 4.8 Hz, 1H), 3.88-3.67 (m, 3H), 3.48 - 3.40 (m, 2H), 2.95 (s, 2H), 2.88 - 2.69 (m, 2H), 2.27 - 2.09 (m, 2H), 1.97 (s, 1H), 1.89 - 1.80 (m, 1H).

### Example 24: 6-(8-(3-(3,5-difluorophenyl)-4,5-dihydro-1H-pyrazole-1-carbonyl)-5-aza-spiro[2.5]oct-5-yl)pyrimidine-4-carbonitrile (racemate, Z-25) and 6-(8-(3-(3,5-difluorophenyl)-4,5-dihydro-1H-pyrazole-1-carbonyl)-5-aza-spiro[2.5]oct-5-yl)pyrimidine-4-carbonitrile (racemate, Z-26)

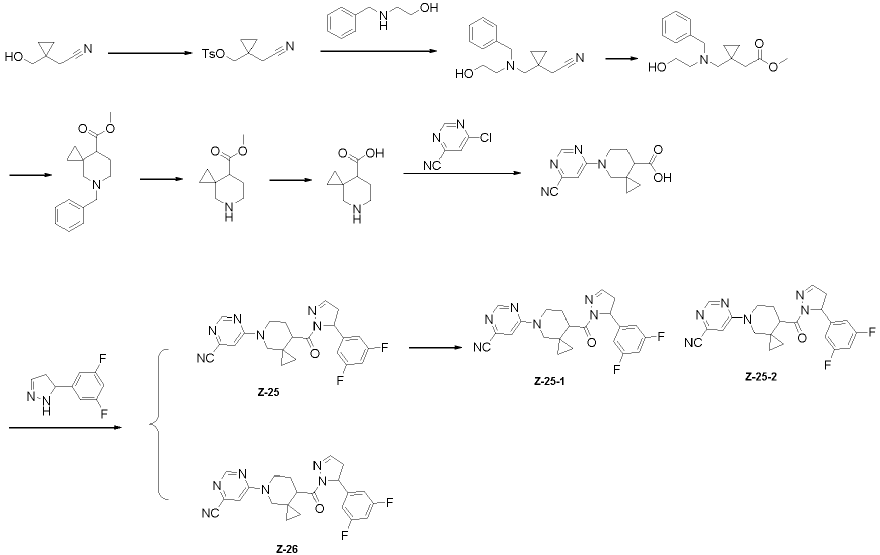

Step 1: p-toluensulfonyl chloride (20.76 g, 108.9 mmol) was dissolved in dichloromethane (40 mL). The obtained solution was slowly added to a solultion of 2-(1-(hydoxymethyl)cyclopropyl) acetonitrile (11 g, 99 mmol) and triethylene diamine (13.88 g, 123.7 mmol) in dichloromethane (135 mL) at a condition of icebath (0-5 °C). The mixture was stirred at the room temperature for 1 h. Thereafter, ethyl ether (100 mL) was added. The resultant mixture was filtered. The filter cake was washed with ethyl ether (50 mL), and the filtrate was washed with 0.5% hydrochloric acid (30 mL). The organic phases were combined, dried over sodium sulfate, concentrate at a reduced pressure, and subjected to column chromatography (ethyl acetate/petroleum ether: 0-40%) to give (1-(cyanomethyl)cyclopropyl)methyl 4-toluenesulfonate (22 g, yield: 83%). ES-API:[M+H]⁺= 266.1.

Step 2: (1-(cyanomethyl)cyclopropyl)methyl 4-toluenesulfonate (20 g, 75.3 mmol) was dissolved in anhydrous tetrahydrofuran (15 mL). The obtained solution was slowly added dropwise at a condition of 70°C to a solution of 2-(benzylamino)ethanol (22.7 g, 150.6 mmol), anhydrous potassium carbonate (10.4 g, 75.3 mmol) and sodium iodide (1.13 g, 7.53 mmol) in anhydrous dimethylsulfoxide (75 mL). The reaction was carried out at 70°C under the protection of nitrogen gas for 16 h. After being cooled, the reaction mixture was added to water (300 mL), and extracted with ethyl ether (150 mL × 3). The organic phases were combined, dried over sodium sulfate, and concentrated at a reduced pressure. The crude product was subjected to column chromatography (ethyl acetate/petroleum ether: 0-30%), to give 2-(1-((benzyl(2-hydoxyethyl)amino)methyl)cyclopropyl)acetonitrile (10 g, yield: 50%). ES-API:[M+H]⁺= 245.1.

Step 3: 2-(1-((benzyl(2-hydoxyethyl)amino)methyl)cyclopropyl)acetonitrile (10 g, 40.9 mmol) and potassium hydroxide (36.6 g, 654.4 mmol) were added to water (150 mL). The reaction was carried out under reflux for 48 h. Thereafter, the reaction system was cooled to the room temperature. Concentrated hydrochloric acid was added to adjust the pH to 3-4. The resultant mixture was concentrated under a reduced pressure. The residue was added to a solution of hydrochloric acid in methanol (100 mL). The mixture was heated under reflux overnight. After being cooled to the room temperature, the resultant mixture was concentrated at a reduced pressure. 0.5 M sodium hydroxide was added to adjust the pH to 9-10. The resultant mixture was extracted with ethyl acetate (150 mL × 3). The organic phases were combined, dried over sodium sulfate, concentrated at a reduced pressure, and subjected to column chromatography (methanol/dichloromethane: 0-10%), to give methyl 2-(1-((benzyl(2-hydroxyethyl)amino)methyl)cyclopropyl)acetate (6 g, yield: 53%). ES-API:[M+H]⁺= 278.1.

Step 4: methyl 2-(1-((benzyl(2-hydroxyethyl)amino)methyl)cyclopropyl)acetate (1 g, 3.6 mmol) was dissolved in 10 mL of dichloromethane. Then, sulfoxide chloride (0.86 g, 7.2 mmol) was slowly added to the reaction mixture. The resultant reaction mixture was stirred at the room temperature for 2 h, and concentrated at a reduced pressure to give a crude product, which was directly used in the next step of reaction. The crude product was dissolved in anhydrous dimethylsulfoxide (8 mL). The obtained solution was added to a solution of potassium tert-butoxide (1 g, 9.0 mmol) in tetrahydrofuran (35 mL). The reaction mixture was stirred at the room temperature under the protection of nitrogen gas for 4 h. The resultant reaction mixture was added to water (40 mL), and extracted with ethyl ether for three times (30 mL × 3). The organic phases were combined, dried over sodium sulfate, and concentrated at a reduced pressure. The crude product was subjected to column chromatography (methanol/dichloromethane: 0-5%) to give methyl 5-benzyl-5-aza-spiro[2.5]octane-8-carboxylate (500 mg, yield: 54%). ES-API:[M+H]⁺= 260.1.

Step 5: methyl 5-benzyl-5-aza-spiro[2.5]octane-8-carboxylate (500 mg, 1.72 mmol) was dissolved in 20 mL of methanol. Then, 10% palladium on carbon (150 mg 0.3 w/w) was added. The reaction mixture was stirred at the room temperature under an atmosphere of hydrogen gas for 16 h, and filtered. The filtrate was concentrated at a reduced pressure to give methyl 5-aza-spiro[5-]octane-8-carboxylate (280 mg, yield: crude product), which was directely used in the nexte step of reaction. ES-API:[M+H]⁺= 170.1.

Step 6: methyl 5-aza-spiro[5-]octane-8-carboxylate (290 mg, 1.70 mmol) was added to methanol (5 mL). Then, a solution of sodium hydroxide (2 M, 4 mL) was added dropwise. The reaction was carried out at the room temperature for 24 h. After the reaction was completed, diluted hydrochloric acid (2 M) was added to adjust the pH to 6-7. The resultant solution was concentrated at a reduced pressure to give 5-aza-spiro[2.5]octane-8-carboxylic acid (800 mg, yield: crude product, containing sodium chloride salt). ES-API: [M+H]⁺= 156.1.

Step 7: 5-aza-spiro[2.5]octane-8-carboxylic acid (800 mg, 1.70 mmol), N,N-diisopropylethylamine (671 mg, 5.2 mmol) and 6-chloropyrimidine-4-carbonitrile (236 mg,1.70 mmol) were added to acetonitrile (10 mL). The reaction mixture was stirred at 50°C for 2 h. Thereafter, water (10 mL) was added to the reaction mixture. The resultant mixture was extracted with ethyl acetate (50 mL × 3). The organic phase was dried over anhydrous sodium sulfate, and concentrated at a reduced pressure. The crude product was subjected to column chromatography (methanol/dichloromethane: 0-5%) to give 5-(6-cyanopyrimidin-4-yl)-5-aza-spiro[2.5]octane-8-carboxylic acid (150 mg, yield: 34%). ES-API: [M+H]⁺ =259.1.

Step 8: 5-(6-cyanopyrimidin-4-yl)-5-aza-spiro[2.5]octane-8-carboxylic acid (150 mg, 0.58 mmol) was dissolved in dichloromethane (10 mL). Then, sulfoxide chloride (2 mL) was added. The reaction mixture was stirred at the room temperature for 2 h, and concentrated at a reduced pressure. The concentrated crude product was dissolved in dichloromethane (10 mL). The obtained solution was added to a solution of 5-(3,5-difluorophenyl)-4,5-dihydro-1H-pyrazole (106 mg,0.58 mmol) and N,N-diisopropylethylamine (374 mg,2.9 mmol) in dichloromethane (5 mL). The reaction mixture was stirred at the room temperature for 2 h. Ethyl acetate (100 mL) was added. The resultant mixture was washed with water (20 mL × 2). The organic phases were combined, dried over anhydrous sodium sulfate, and concentrated under a reduced pressure. The crude product was purified by thin layer chromatography (PE/EA=2/1) to give 2 racemates;
Z-25 (having a Rf of 0.25): a racemate of 6-(8-(3-(3,5-difluorophenyl)-4,5-dihydro-1H-pyrazole-1-carbonyl)-5-aza-spiro[2.5]oct-5-yl)pyrimidine-4-carbonitrile (4.6 mg, yield: 1.8%, LCMS retention time: 1.92 min). ES-API: [M+H]⁺ =423.1. ¹H NMR(300 MHz, CDCl₃) δ 8.55 (s, 1H), 6.99 (s, 1H), 6.85 - 6.63 (m, 4H), 5.37 (dd, *J* = 12.0, 5.1 Hz, 1H), 3.97 (d, *J* = 12.9 Hz, 1H), 3.68 - 3.39 (m, 2H), 3.14 (m, 1H), 2.84-2.80 (m, 1H), 2.20 - 1.77 (m, 4H), 0.75 - 0.35 (m, 4H).
Z-26 (having a Rf of 0.3): a recemate of 6-(8-(3-(3,5-difluorophenyl)-4,5-dihydro-1H-pyrazole-1-carbonyl)-5-aza-spiro[2.5]oct-5-yl)pyrimidine-4-carbonitrile (4.1 mg, yield: 1.7%, LCMS retention time: 1.93 min). ES-API: [M+H]⁺ =423.1. ¹H NMR(300 MHz, CDCl₃) δ 8.53 (s, 1H), 7.08 - 6.96 (m, 1H), 6.95 - 6.63 (m, 4H), 5.37 (dd, *J* = 11.9, 5.3 Hz, 1H), 3.82 (br, 2H), 3.48 (m, 1H), 3.17 (t, *J* = 4.9 Hz, 1H), 2.82 (m, 1H), 2.04 (m, 4H), 1.00 - 0.35 (m, 4H).

Step 9: the compound of Z-25 as obtained above (35 mg) was subjected to a chiral resolution (mobile phase: CO₂, co-solvent: MeOH (containing a solution of 0.2% ammonia in methanol)); column: AD-H (4.6^{∗}100 mm 5 um); flow rate: 1.0 ml/min; T: 40.3 °C) to give 2 single isomers:
Isomer Z-25-1: an isomer having a single configuration of 6-(8-(3-(3,5-difluorophenyl)-4,5-dihydro-1H-pyrazole-1-carbonyl)-5-aza-spiro[2.5]oct-5-yl)pyrimidine-4-carbonitrile (LCMS retention time: 0.96 min, 16 mg, P: 92%, ee value: 100%). ES-API:[M+H]⁺= 423.2.
Isomer Z-25-2: an isomer having a single configuration of 6-(8-(3-(3,5-difluorophenyl)-4,5-dihydro-1H-pyrazole-1-carbonyl)-5-aza-spiro[2.5]oct-5-yl)pyrimidine-4-carbonitrile (LCMS retention time: 1.73 min, 15 mg, P: 100%, ee value: 100%). ES-API:[M+H]⁺= 423.2.

### Example 25: Preparation of 6-(5-(5-(5-fluoropyridin-3-yl)-4,5-dihydro-1H-pyrazole-1-carbonyl)hexahydrocyclopenta[c]pyrrol-2(1H)-yl)pyrimidine-4-carbonitrile (Z-27)

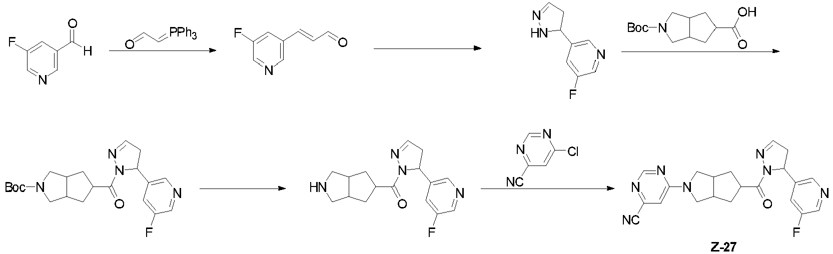

Step 1: 5-fluoropyridine-3-carboxaldehyde (15 g, 120 mmol) and 2-(triphenylphosphoranylene) acetaldehyde (38.3 g, 126 mmol) were dissolved in tetrahydrofuran (200 mL). The solution was heated to 50°C overnight. Thereafter, the reaction solution was spin-dried, and subjected to column chromatography (petroleum ether/ethyl acetate=5/1) to give 3-(5-fluoropyridin-3-yl)acraldehyde (17 g, yield: 94%). ES-API:[M+H]⁺= 152.0.

Step 2: hydrazine hydrate (434 mg, 6.95 mmol) was dissolved in ethanol (10 mL). Then, acetic acid (596 mg, 9.93 mmol) was added. The mixture was heated to 45°C. After 3-(5-fluoropyridin-3-yl)acraldehyde (1 g, 6.62 mmol) was added in batches to the reaction mixture over 30 minutes, the reaction was kept at the temperature of 100°C. The reaction was carried out overnight. The resultant reaction mixture was spin-dried, into which water was added. Sodium bicarbonate was added to adjust the pH to about 7. The resultant mixtue was extracted with dichloromethane. The organic phase was washed with saline, dried over anhydrous sodium sulfate, and concentrated under a reduced pressure, to give 3-(4,5-dihydro-1H-pyrazol-5-yl)-5-fluoropyridine (1.1 g, yield: crude product), which was directly used in the next step. ES-API: [M+H]⁺= 166.0.

Step 3: 2-(tert-butoxycarbonyl)octahydrocyclopenta[c]pyrrole-5-carboxylic acid (1.7 g, 6.67 mmol), 3-(4,5-dihydro-1H-pyrazol-5-yl)-5-fluoropyridine (1.1 g, 6.67 mol), N,N-diisopropylethylamine (1.72 g, 13.34 mmol) and 2-(7-azabenzotriazole)-N,N,N',N'-tetramethyluronium hexafluorophosphate (HATU, 3.04 g, 8 mmol) were added to dichloromethane (100 mL). The reaction was carried out at 25°C for 2 h. The organic phase was washed with water and saturated saline solution, dried over anhydrous sodium sulfate, spin-dried, and subjected to column chromatography (petroleum ether/ethyl acetate=1/1) to give tert-butyl 5-(5-(5-fluoropyridin-3-yl)-4,5-dihydro-1H-pyrazole-1-carbonyl)hexahydrocyclopenta[c]pyrrole-2(1H)-carboxylate (2.3 g, yield: 86%). ES-API:[M+H]⁺= 403.2.

Step 4: tert-butyl 5-(5-(5-fluoropyridin-3-yl)-4,5-dihydro-1H-pyrazole-1-carbonyl)hexahydrocyclopenta[c]pyrrole-2(1H)-carboxylate (2.3 g, 5.72 mmol) was dissolved in dichloromethane (10 mL). Then, trifluoroacetic acid (10 mL) was added. The reaction was carried out at 25°C for 2 h. The resultant reaction mixture was spin-dried to give (5-(5-fluoropyridin-3-yl)-4,5-dihydro-1H-pyrazol-1-yl)(octahydrocyclopenta[c]pyrrol-5-yl) ketone trifluroacetate (crude product). ES-API:[M+H]⁺= 303.1.

Step 5: 5-(5-fluoropyridin-3-yl)-4,5-dihydro-1H-pyrazol-1-yl) (octahydrocyclopenta[c]pyrrol-5-yl) ketone trifluroacetate (1 g, 2.4 mmol), 6-chloropyrimidine-4-carbonitrile (368 mg, 2.64 mmol) and potassium carbonate (728 mg, 5.28 mmol) were added to N,N-dimethylformamide (10 mL). The mixture was heated to 50°C and reacted for 2 h. The resultant reaction mixture was extracted with ethyl acetate, and subjected to column chromatography (petroleum ether/ethyl acetate=1/1) to give a crude product. The crude product was futher purified by preparative HPLC (column: Ultimate XB-C18, 50^{∗}250 mm, 10 um; mobile phase system: A: pure water; B: pure acetonitrile; flow rate: 80 ml/min, B/A=20%-100%; column temperature: room temperature) to give 6-(5-(5-(5-fluoropyridin-3-yl)-4,5-dihydro-1H-pyrazole-1-carbonyl)hexahydrocyclopenta[c]pyrrol-2(1H)-yl) pyrimidine-4-carbonitrile (Z-27, 160 mg, yield: 16%). ES-API: [M+H]⁺= 406.2. ¹H NMR (400 MHz, CDCl₃) δ 8.63 - 8.52 (m, 1H), 8.45 - 8.24 (m, 2H), 7.20 (d, *J* = 8.6 Hz, 1H), 7.08 - 6.95 (m, 1H), 6.66 - 6.52 (m, 1H), 5.38 (dd, *J* = 12.0, 5.1 Hz, 1H), 4.00 - 3.42 (m, 6H), 3.28-1.83 (m, 7H).

### Example 26: Preparation of 6-(5-(5-(5-fluoropyridin-3-yl)-4,5-dihydro-1H-pyrazole-1-carbonyl)hexahydrocyclopenta[c]pyrrol-2(1H)-yl)pyrimidine-4-carboxamide (racemate, Z-28) and 6-(5-(5-(5-fluoropyridin-3-yl)-4,5-dihydro-1H-pyrazole-1-carbonyl)hexahydrocyclopenta[c]pyrrol-2(1H)-yl)pyrimidine-4-carboxamide (racemate, Z-29)

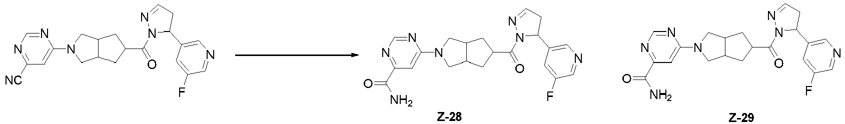

Step 1: 6-(5-(5-(5-fluoropyridin-3-yl)-4,5-dihydro-1H-pyrazole-1-carbonyl) hexahydrocyclopenta[c]pyrrol-2(1H)-yl)pyrimidine-4-carbonitrile (Z-27, 140 mg, 0.346 mmol) was dissolved in acetonitrile/ water (1.5 mL/4.5 mL). Then, sodium hydroxide (15 mg, 0.381 mmol) was added. Next, 30% hydrogen peroxide (1.5 mL) was added in an ice-water bath. The reaction was carried out at 25°C for 2 h. The resultant reaction was quenched with a solution of sodium sulphite. The resultant mixture was extracted with dichloromethane, dried over sodium sulfate, and concentrated at a reduced pressure to give a crude product. The crude product was purified by preparative HPLC (column: Ultimate XB-C18, 50^{∗}250 mm, 10 um; mobile phase system: A: pure water; B: pure acetonitrile; flow rate: 80 ml/min, B%=20%-100%, column temperature: room temperature), to give 2 racemates:
Z-28 (retention time: 14.69 min): a racemate of 6-(5-(5-(5-fluoropyridin-3-yl)-4,5-dihydro-1H-pyrazole-1-carbonyl)hexahydrocyclopenta[c]pyrrol-2(1H)-yl)pyrimidine-4-carboxamide (26.6 mg, yield: 18%). ES-API:[M+H]⁺= 424.2. ¹H NMR(400 MHz, CDCl₃) δ 8.51 (d, *J* = 1.2 Hz, 1H), 8.37 (d, *J* = 2.7 Hz, 1H), 8.29 (d, *J* = 1.7 Hz, 1H), 7.79 (s, 1H), 7.17 (dt, *J* = 8.9, 2.3 Hz, 1H), 7.09 (d, *J* = 1.3 Hz, 1H), 6.99 (d, *J* = 1.7 Hz, 1H), 5.56 (s, 1H), 5.38 (dd, *J* = 12.1, 5.2 Hz, 1H), 3.87 - 3.30 (m, 6H), 2.82 (ddd, *J* = 18.9, 5.2, 1.8 Hz, 3H), 2.28 (tq, *J* = 14.1, 7.3, 6.7 Hz, 2H), 1.77 (s, 2H).
Z-29 (retention time: 16.43 min): another racemate of 6-(5-(5-(5-fluoropyridin-3-yl)-4,5-dihydro-1H-pyrazole-1-carbonyl)hexahydrocyclopenta[c]pyrrol-2(1H)-yl)pyrimidine-4-carboxamide (18 mg, yield: 12%). ES-API:[M+H]⁺= 424.2. ¹H NMR(400 MHz, CDCl₃) δ 8.54 (dd, *J* = 15.3, 1.2 Hz, 1H), 8.38 (dd, *J* = 5.9, 2.7 Hz, 1H), 8.30 (d, *J* = 7.2 Hz, 1H), 7.80 (s, 1H), 7.21 - 7.06 (m, 2H), 7.02 - 6.95 (m, 1H), 5.57 (d, *J* = 10.5 Hz, 1H), 5.38 (dt, *J* = 12.1, 4.7 Hz, 1H), 3.95 - 3.20 (m, 5H), 3.06-2.76 (m, 3H), 2.38 - 1.77 (m, 4H).

### Example 27: Preparation of (2-(4-amino-5-fluoropyrimidin-2-yl)octahydrocyclopenta[c]pyrrol-5-yl)(5-(5-fluoropyridin-3-yl)-4,5-dihydro-1H-pyrazol-1-yl) ketone (Z-30)

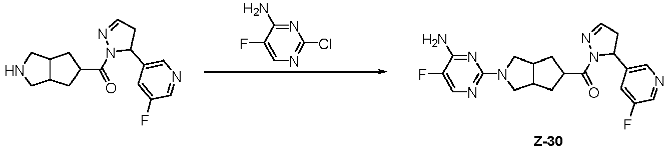

Step 1: 5-(5-fluoropyridin-3-yl)-4,5-dihydro-1H-pyrazol-1-yl) (octahydrocyclopenta[c]pyrrol-5-yl) ketone trifluoroacetate (500 mg, 1.2 mmol), 2-chloro-5-fluoropyrimidine-4-amine (194 mg, 1.32 mmol) and potassium carbonate (364 mg, 2.64 mmol) were added to N,N-dimethylformamide (10 mL). The mixture was heated to 115°C and reacted for 14 h. The resultant reaction mixture was extracted with ethyl acetate, and subjected to column chromatography (petroleum ether/ethyl acetate: 1/1) to give a crude product. The crude product was purified by preparative HPLC (column: Ultimate XB-C18, 50^{∗}250 mm, 10 um; mobile phase system: A: pure water; B: pure acetonitrile; flow rate: 80 ml/min, B/A=20%-100%; column temperature: room temperature) to give (2-(4-amino-5-fluoropyrimidin-2-yl)octahydrocyclopenta[c]pyrrol-5-yl)(5-(5-fluoropyridin-3-yl)-4,5-dihydro-1H-pyrazol-1-yl) ketone as an off-white solid (Z-30, 7 mg, yield: 1.4%). ES-API: [M+H]⁺= 414.2. ¹H NMR(400 MHz, CDCl₃) δ 8.37 (s, 1H), 8.29 (s, 1H), 7.80 (d, *J* = 3.2 Hz, 1H), 7.16 (d, *J* = 8.9 Hz, 1H), 6.97 (t, *J* = 1.7 Hz, 1H), 5.39 (dd, *J* = 12.1, 5.1 Hz, 1H), 4.73 (s, 2H), 3.59 (dt, *J* = 11.4, 5.2 Hz, 3H), 3.45 (d, *J* = 11.7 Hz, 3H), 2.84 - 2.75 (m, 2H), 2.30 - 2.16 (m, 2H), 2.01 (d, *J* = 6.0 Hz, 1H), 1.82 - 1.66 (m, 2H).

### Example 28: Preparation of 2-(5-(5-(5-fluoropyridin-3-yl)-4,5-dihydro-1H-pyrazole-1-carbonyl)hexahydrocyclopenta[c]pyrrol-2(1H)-yl)pyrimidine-4-carbonitrile (Z-31)

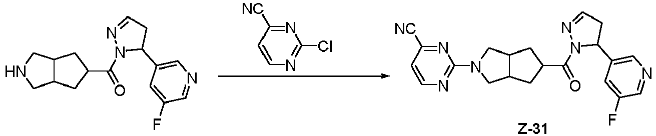

5-(5-fluoropyridin-3-yl)-4,5-dihydro-1H-pyrazol-1-yl) (octahydrocyclopenta[c] pyrrol-5-yl) ketone trifluoroacetate (900 mg, 2.4 mmol), 2-fluoropyrimidine-4-carbonitrile (331 mg, 2.38 mmol) and potassium carbonate (728 mg, 5.28 mmol) were added to N,N-dimethylformamide (10 mL). The mixture was heated to 50°C and reacted for 2 h. The resultant reaction mixture was extracted with ethyl acetate, and subjected to column chromatography (petroleum ether/ethyl acetate: 1/1) to give a crude product. The crude product was purified by preparative HPLC (column: Ultimate XB-C18, 50^{∗}250 mm, 10 um; mobile phase system: A: pure water; B: pure acetonitrile; flow rate: 80 ml/min, B/A=20%-100%; column temperature: room temperature) to give 2-(5-(5-(5-fluoropyridin-3-yl)-4,5-dihydro-1H-pyrazole-1-carbonyl)hexahydrocyclopenta[c]pyrrol-2(1H)-yl)pyrimidine-4-carbonitrile (Z-31, 120 mg, yield: 14%). ES-API:[M+H]⁺= 406.2. ¹H NMR(400 MHz, CDCl₃) δ 8.47 - 8.34 (m, 2H), 8.30 (s, 1H), 7.18 (d, *J* = 8.5 Hz, 1H), 6.98 (d, *J* = 6.6 Hz, 1H), 6.72 (dd, *J* = 14.3, 4.7 Hz, 1H), 5.39 (dd, *J* = 12.1, 4.9 Hz, 1H), 3.80 - 3.64 (m, 3H), 3.57 - 3.42 (m, 3H), 3.01 - 2.76 (m, 3H), 2.28 (s, 2H), 1.75 (s, 2H).

### Example 29: Preparation of 2-(5-(5-(3,5-difluorophenyl)-4,5-dihydro-1H-pyrazole-1-carbonyl)hexahydrocyclopenta[c]pyrrol-2(1H)-yl)pyrimidine-4-carbonitrile (Z-32), 2-((3aR,5s,6aS)-5-((S)-5-(3,5-difluorophenyl)-4,5-dihydro-1H-pyrazole-1-carbonyl)hexahydrocyclopenta[c]pyrrol-2(1H)-yl)pyrimidine-4-carbonitrile (Z-32-1), 2-((3aR,5s,6aS)-5-((R)-5-(3,5-difluorophenyl)-4,5-dihydro-1H-pyrazole-1-carbonyl)hexahydrocyclopenta[c]pyrrol-2(1H)-yl)pyrimidine-4-carbonitrile (Z-32-2), 2-((3aR,5r,6aS)-5-((S)-5-(3,5-difluorophenyl)-4,5-dihydro-1H-pyrazole-1-carbonyl)hexahydrocyclopenta[c]pyrrol-2(1H)-yl)pyrimidine-4-carbonitrile (Z-32-3), 2-((3aR,5r,6aS)-5-((R)-5-(3,5-difluorophenyl)-4,5-dihydro-1H-pyrazole-1-carbonyl)hexahydrocyclopenta[c]pyrrol-2(1H)-yl)pyrimidine-4-carbonitrile (Z-32-4)

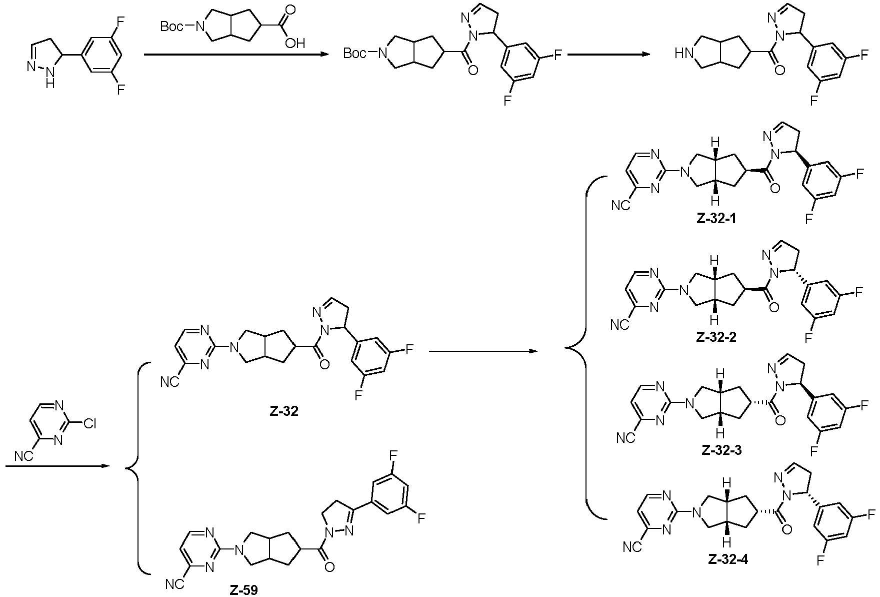

Step 1: (3aR,6aS)-2-(tert-butoxycarbonyl)octahydrocyclopenta[c]pyrrole-5-carboxylic acid (1.4 g, 5.5 mmol), 5-(3,5-difluorophenyl)-4,5-dihydro-1H-pyrazole (1 g, 5.5 mmol), N,N-diisopropylethylamine (1.77 g, 13.8 mol) and 2-(7-azabenzotriazole)-N,N,N',N'-tetramethyluronium hexafluorophosphate (2.28 g, 6.0 mmol) were added to dichloromethane (25 mL). The reaction was carried out at 25°C for 16 h. The resultant reaction mixture was extracted with dichloromethane, and subjected to column chromatography (petroleum ether/ethyl acetate: 10%-45%) to give tert-butyl (3aR,6aS)-5-(5-(3,5-difluorophenyl)-4,5-dihydro-1H-pyrazole-1-carbonyl)hexahydrocyclopenta[c]pyrrole-2(1H)-carboxylate as a yellow oil (0.7 g, yield: 30%). ES-API:[M+H-56]⁺= 364.0.

Step 2: tert-butyl (3aR,6aS)-5-(5-(3,5-difluorophenyl)-4,5-dihydro-1H-pyrazole-1-carbonyl)hexahydrocyclopenta[c]pyrrole-2(1H)-carboxylate (700 mg, 1.67 mmol) was dissolved in dichloromethane (10 mL). Then, trifluoroacetic acid (2 mL) was added. The reaction was carried out at 25°C for 4 h. The resultant reaction mixture was concentrated at a reduced pressure to give a crude product of (5-(3,5-difluorophenyl)-4,5-dihydro-1H-pyrazol-1-yl) (3aR,6aS)-(octahydrocyclopenta[c] pyrrol-5-yl) ketone trifluoroacetate (600 mg, yield: crude product). ES-API:[M+H]⁺= 320.0.

Step 3: (5-(3,5-difluorophenyl)-4,5-dihydro-1H-pyrazol-1-yl) (3aR,6aS)-(octahydrocyclopenta[c]pyrrol-5-yl) ketone trifluoroacetate (400 mg,1.25 mmol), 2-chloropyrimidine-4-carbonitrile (174 mg, 1.25 mmol) and potassium carbonate (518 mg, 3.75 mmol) were added to N,N-dimethylformamide (10 mL). The mixture was heated to 50°C and reacted for 2 h. The resultant reaction mixture was extracted with ethyl acetate and concentrated at a reduced pressure to give a crude product. The crude product was subjected to preparative HPLC (column: Ultimate XB-C18, 50^{∗}250 mm, 10 um; mobile phase system: A: pure water; B: pure acetonitrile; flow rate: 80 ml/min, B/A=20%-100%; column temperature: room temperature) to give 2-(3aR,6aS)-(5-(5-(3,5-difluorophenyl)-4,5-dihydro-1H-pyrazole-1-carbonyl)hexahydrocyclopenta[c]pyrrol-2(1H)-yl)pyrimidine-4-carbonitrile as a white solid (Z-32, 290 mg, yield: 55%). ES-API:[M+H]⁺= 423.1. ¹H NMR(400 MHz, CDCl₃) δ 8.39 (d, *J* = 4.7 Hz, 1H), 7.26 (s, 0H), 6.94 (d, *J* = 1.7 Hz, 1H), 6.70 (d, *J* = 4.7 Hz, 1H), 6.69 - 6.62 (m, 2H), 5.29 (dd, *J* = 12.0, 5.0 Hz, 1H), 3.69-3.37 (m, 7H), 2.84-2.73 (m, 3H), 2.76 (m, 1H), 2.38 - 2.18 (m, 2H), 1.87-1.62(m, 2H), and
2-(3aR,6aS)-(5-(3-(3,5-difluorophenyl)-4,5-dihydro-1H-pyrazole-1-carbonyl) hexahydrocyclopenta[c]pyrrol-2(1H)-yl)pyrimidine-4-carbonitrile (Z-59, 40 mg, yield: 7.6%) as a white solid. ES-API:[M+H]⁺= 423.1. ¹H NMR(400 MHz, CDCl₃) δ 8.42 (d, *J* = 4.6 Hz, 1H), 7.23 (dt, *J* = 6.4, 2.1 Hz, 2H), 6.87 (tt, *J* = 8.7, 2.3 Hz, 1H), 6.72 (d, *J* = 4.7 Hz, 1H), 4.06 (t, *J* = 10.3 Hz, 2H), 3.82 - 3.69 (m, 3H), 3.61 (dd, *J* = 12.0, 3.6 Hz, 2H), 3.17 (dd, *J* = 11.0, 9.5 Hz, 2H), 2.87 (dt, *J* = 7.1, 3.4 Hz, 2H), 2.40 - 2.23 (m, 2H), 1.86 (s, 2H).

Step 4: the Z-32 (200 mg) as obtained in the above step was subjected to a chiral resolution (column: CHIRALPAK IG (IG00CE-UC011) 0.46 cm I.D. ×25 cm L; mobile phase: MeOH/CAN = 90/10(V/V); flow rate: 1.0 ml/min; column temperatue: 35°C; HPLC instrument: Shimadzu LC-20AT CP-HPLC-06) to give 4 isomers having a single configuration (the structures of the 4 isomers having a single configuration are each arbitrarily assigned):
Isomer 1 (retention time: 6.480 min); arbitrarily assigned as Z-32-1; ee value: 89.423%; purity: 96.50%; ES-API:[M+H]⁺= 423.1. ¹H NMR (400 MHz, CDCl₃) δ 8.39 (d, *J* = 4.7 Hz, 1H), 6.94 (s, 1H), 6.75 - 6.66 (m, 4H), 5.30 (dd, *J* = 12.0, 5.0 Hz, 1H), 3.69-3.37 (m,6H), 2.97-2.73 (m, 3H), 2.18-1.62(m, 4H).
Isomer 2(retention time: 7.426 min); arbitrarily assigned as Z-32-2; ee value: 100%; purity: 99.76%; ES-API:[M+H]⁺= 423.1. ¹H NMR (400 MHz, CDCl₃) δ 8.39 (d, *J* = 4.7 Hz, 1H), 6.94 (s, 1H), 6.75 - 6.66 (m, 4H), 5.29 (dd, *J* = 12.0, 5.0 Hz, 1H), 3.72-3.38 (m,7H), 2.86-2.73 (m, 3H), 2.38-2.18(m,2H),1.82-1.62(m, 2H).
Isomer 3 (retention time: 8.701 min); arbitrarily assigned as Z-32-3; ee value: 98.217%; purity: 91.84%; ES-API:[M+H]⁺= 423.1. ¹H NMR (400 MHz, CDCl₃) δ 8.43 (d, *J* = 4.7 Hz, 1H), 6.93 (s, 1H), 6.75 - 6.66 (m, 4H), 5.29 (dd, *J* = 12.0, 5.0 Hz, 1H), 3.82-3.76 (m,3H), 3.46-3.38 (m,3H), 2.79-2.73 (m, 3H), 2.17-1.63(m, 4H).
Isomer 4 (retention time: 14.054 min); arbitrarily assigned as Z-32-4: ee value: 100%; purity: 98.16%; ES-API:[M+H]⁺= 423.1. ¹H NMR (400 MHz, CDCl₃) δ 8.39 (d, *J* = 4.7 Hz, 1H), 6.95 (s, 1H), 6.75 - 6.66 (m, 4H), 5.31 (dd, *J* = 12.0, 5.0 Hz, 1H), 3.72-3.38 (m,6H), 2.85-2.73 (m, 3H), 2.26-2.18(m, 2H), 1.77-1.62(m, 2H).

### Example 30: 2-(5-(5-(5-fluoropyridin-3-yl)-4,5-dihydro-1H-pyrazole-1-carbonyl)hexahydrocyclopenta[c]pyrrol-2(1H)-yl)pyrimidine-4-carboxamide (racemate, Z-33) and 2-(5-(5-(5-fluoropyridin-3-yl)-4,5-dihydro-1H-pyrazole-1-carbonyl)hexahydrocyclopenta[c]pyrrol-2(1H)-yl)pyrimidine-4-carboxamide (racemate, Z-34)

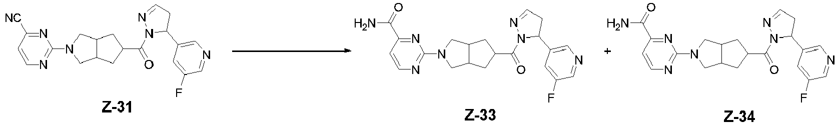

Step 1: 2-(5-(5-(5-fluoropyridin-3-yl)-4,5-dihydro-1H-pyrazole-1-carbonyl)hexahydrocyclopenta[c]pyrrol-2(1H)-yl)pyrimidine-4-carbonitrile (Z-31, 100 mg, 0.247 mmol) was dissolved in acetonitrile/ water (1 mL/3 mL). Then, sodium hydroxide (10 mg, 0.272 mmol) was added. Next, 30% hydrogen peroxide (1 mL) was added in an ice-water bath. The reaction was carried out at 25°C for 2 h. There reaction was quenched with a solution of sodium sulphite. The resultant reaction mixture was extracted with dichloromethane, dried over sodium sulfate, and concentrated at a reduced pressure, to give a crude product. The crude product was purified by preparative HPLC (column: Ultimate XB-C18, 50^{∗}250mm, 10um; mobile phase system: A: pure water; B: pure acetonitrile; flow rate: 80 ml/min, B%=20%-100%, column temperature: room temperature) to give 2 racemate:
Z-33 (retention time: 18.83 min): a racemate of 2-(5-(5-(5-fluoropyridin-3-yl)-4,5-dihydro-1H-pyrazole-1-carbonyl)hexahydrocyclopenta[c]pyrrol-2(1H)-yl)pyrimidine-4-carboxamide (7.3 mg, yield: 7%). ES-API:[M+H]⁺= 424.2. ¹H NMR (400 MHz, CDCl₃) δ 8.53 (d, *J* = 4.8 Hz, 1H), 8.39 (d, *J* = 2.7 Hz, 1H), 8.31 (s, 1H), 7.71 (s, 1H), 7.21 - 7.14 (m, 1H), 6.97 (d, *J* = 1.8 Hz, 1H), 5.51 (s, 1H), 5.40 (dd, *J* = 12.1, 5.2 Hz, 1H), 3.82 (dt, *J* = 16.1, 7.8 Hz, 3H), 3.48 (dd, *J* = 17.5, 12.2 Hz, 3H), 2.96-2.79(m, 3H), 2.15-1.86 (m, 4H).
Z-34 (retention time: 20.82 min): another racemate of 2-(5-(5-(5-fluoropyridin-3-yl)-4,5-dihydro-1H-pyrazole-1-carbonyl)hexahydrocyclopenta[c]pyrrol-2(1H)-yl)pyrimidine-4-carboxamide (20.2 mg). ES-API: [M+H]⁺= 424.2. ¹H NMR (400 MHz, CDCl₃) δ 8.49 (d, *J* = 4.8 Hz, 1H), 8.36 (d, *J* = 2.7 Hz, 1H), 8.29 (s, 1H), 7.68 (s, 1H), 7.23 (d, *J* = 4.8 Hz, 1H), 7.20 - 7.14 (m, 1H), 5.48 (s, 1H), 5.39 (dd, *J* = 12.1, 5.1 Hz, 1H), 3.78 - 3.63 (m, 3H), 3.59 - 3.42 (m, 3H), 2.84-2.79 (m, 3H), 2.27-1.58 (m, 4H).

### Example 33: (2-(2-methoxypyrimidin-4-yl)octahydrocyclopenta[c]pyrrol-5-yl)(5-phenyl-4,5-dihydro-1H-pyrazol-yl) ketone (Z-37), ((3aR,5s,6aS)-2-(2-methoxypyrimidin-4-yl)octahydrocyclopenta[c]pyrrol-5-yl)((S)-5-phenyl-4,5-dihydro-1H-pyrazol-1-yl) ketone (Z-37-1), ((3aR,5s,6aS)-2-(2-methoxypyrimidin-4-yl)octahydrocyclopenta[c]pyrrol-5-yl)((R)-5-phenyl-4,5-dihydro-1H-pyrazol-1-yl) ketone (Z-37-2), ((3aR,5r,6aS)-2-(2-methoxypyrimidin-4-yl)octahydrocyclopenta[c]pyrrol-5-yl)((S)-5-phenyl-4,5-dihydro-1H-pyrazol-1-yl) ketone (Z-37-3), ((3aR,5r,6aS)-2-(2-methoxypyrimidin-4-yl)octahydrocyclopenta[c]pyrrol-5-yl)((R)-5-phenyl-4,5-dihydro-1H-pyrazol-1-yl) ketone (Z-37-4)

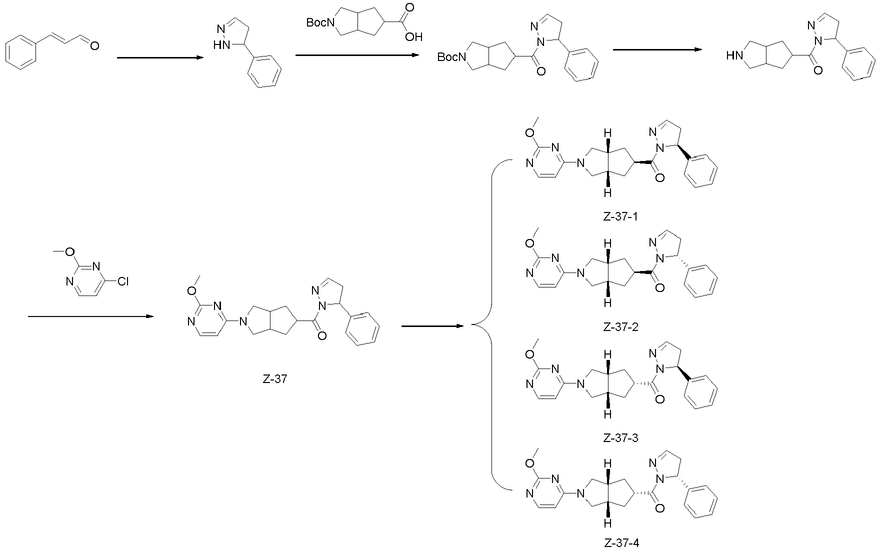

Step 1: hydrazine hydrate (30 mL, 620 mmol) was added to a 250 mL round-bottomed flask, and was heated under reflux. A solution obtained by dissolving cinnamic aldehyde (20 mL, 158.8 mmol) in n-butanol (30 mL) was slowly added dropwise to the hydrazine hydrate. The mixture was refluxed overnight. The resultant mixture was concentrated at a reduced pressure. Water (100 ml) was added to the residue. The mixture was extracted with 200 mL of dichloromethane for three times. The combined organic phase was washed with water, dried over anhydrous sodium sulfate, and concentrated at a reduced pressure. The residue was purified by combiflash to give 5-phenyl-4,5-dihydro-1H-pyrazole as a yellow oil (14 g, yield: 60%). ES-API:[M+H]⁺= 147.0.

Step 2: 2-(7-azabenzotriazol-1-yl)-N,N,N',N'-tetramethyluronium hexafluorophosphate (1072 mg, 2.82 mmol) was added in batches to a mixture of (3aR,6aS)-2-(tert-butoxycarbonyl)octahydrocyclopenta[c]pyrrole-5-carboxylic acid (600 mg, 2.350 mmol), 5-phenyl-4,5-dihydro-1H-pyrazole (687 mg, 4.700 mmol), triethylamine (475 mg, 4.700 mmol) and dichloromethane (15 mL). The mixture was stirred at the room temperatue for 3 h. The resultant reaction mixture was concentrated at a reduced pressure to give a crude product. The product was purified by combiflash to give tert-butyl 5-(3aR,6aS)-(5-phenyl-4,5-dihydro-1H-pyrazole-1-carbonyl)hexahydrocyclopenta[c]pyrrole-2(1H)-carboxylate as an oily liquid (620 mg, yield: 69%). ES-API: [M+H]⁺= 384.2.

Step 3: tert-butyl 5-(3aR,6aS)-(5-phenyl-4,5-dihydro-1H-pyrazole-1-carbonyl)hexahydrocyclopenta[c]pyrrole-2(1H)-carboxylate (620 mg, 1.617 mmol) was dissolved in 1,4-dioxane (10 mL). Then, a 4 M solution of hydrochlorid acid in dioxane (10 mL) was slowly added dropwise at the room temperature. The mixture was stirred at the room temperature overnight, and then concentrated at a reduced pressure to give (3aR,6aS)-(octahydrocyclopenta[c]pyrrol-5-yl)(5-phenyl-4,5-dihydro-1H-pyrazol-1-yl)ketone hydrochloride (540 mg, yield: 100%), ES-API: [M+H]⁺= 284.1.

Step 4: potassium carbonate (263 mg, 1.908 mmol) was added to a mixture of (3aR,6aS)-(octahydrocyclopenta[c]pyrrol-5-yl)(5-phenyl-4,5-dihydro-1H-pyrazol-1-yl) ketone hydrochloride (270 mg, 0.954 mmol), 4-chloro-2-methoxypyrimidine (137 mg, 0.954 mmol) and N,N-dimethylformamide (20 ml). The reaction mixture was heated to 70°C and stirred overnight, and then concentrated at a reduced pressure to remove the solvent. The crude product was purified by column chromatography to give (2-(3aR,6aS)-(2-methoxypyrimidin-4-yl)octahydrocyclopenta[c]pyrrol-5-yl)(5-phenyl-4,5-dihydro-1H-pyrazol-1-yl) ketone as an off-white solid (Z-37, 270 mg, yield: 72%).

Step 5: the Z-37 as obtained in the above step was subjected to a chiral resolution (column: OJ-H 4.6^{∗}100 mm 5 um; mobile phase: CO2, co-solvent: MeOH (containing a 0.2% solution of ammonia in methanol); flow rate: 1.8; column temperature: 30) to give 4 isomers having a single configuration (the structures of the 4 isomers having a single configuration are each arbitrarily assigned):
Isomer 1 (retention time: 0.67 min); arbitrarily assigned as Z-37-1 (64.2 mg); ES-API: [M+H]⁺= 392.2; ¹H NMR (500 MHz, DMSO-d6) δ 7.92 (d, *J*= 6.0 Hz, 1 H), 7.30 (t, *J*1= 7.0 Hz, *J*2= 15.0 Hz, 2 H), 7.22 (t, *J*1= 7.5 Hz, *J*2= 17.0 Hz, 2 H), 7.09 (d, *J*= 7.5 Hz, 2 H), 6.10 (d, *J*= 5.5 Hz, 1 H), 5.29 (dd, *J*1= 5.0 Hz, *J*2= 12.0 Hz, 1 H), 3.76 (s, 3 H), 3.70- 3.29 (m, 6 H), 2.77 (bs, 2 H), 2.69- 2.64 (m, 1 H), 2.23- 2.10 (m, 2 H), 1.61- 1.51 (m, 2 H). ES-API: [M+H]⁺= 392.2. ee value: 100%.
Isomer 2 (retention time: 0.93 min); arbitrarily assigned as Z-37-2 (61.1 mg); ES-API: [M+H]⁺= 392.2; ¹H NMR (500 MHz, DMSO-d6) δ 7.92 (d, *J*= 6.0 Hz, 1 H), 7.30 (t, *J*1= 8.0 Hz, *J2=* 15.5 Hz, 2 H), 7.22 (t, *J*1= 7.5 Hz, *J*2= 17.0 Hz, 2 H), 7.09 (d, *J*= 7.5 Hz, 2 H), 6.10 (d, *J*= 5.5 Hz, 1 H), 5.29 (dd, *J*1= 5.0 Hz, *J2=* 12.0 Hz, 1 H), 3.76 (s, 3 H), 3.70- 3.29 (m, 6 H), 2.77 (bs, 2 H), 2.69- 2.64 (m, 1 H), 2.23- 2.10 (m, 2 H), 1.61- 1.51 (m, 2 H). ES-API: [M+H]⁺= 392.2. ee value: 100%.
Isomer 3 (retention time: 1.58 min); arbitrarily assigned as Z-37-3 (40.7 mg); ES-API: [M+H]⁺= 392.2; ¹H NMR (500 MHz, DMSO-d6) δ 7.96 (d, *J*= 6.0 Hz, 1 H), 7.32 (t, *J*1= 7.5 Hz, *J*2= 15.5 Hz, 2 H), 7.23 (t, *J*1= 7.0 Hz, *J*2= 14.5 Hz, 1 H), 7.18 (s, 1 H), 7.10 (d, *J*= 7.5 Hz, 2 H), 6.15 (d, *J*= 5.5 Hz, 1 H), 5.30 (dd, *J*1= 4.5 Hz, *J*2= 12.0 Hz, 1 H), 3.79 (s, 3 H), 3.70- 3.44 (m, 4 H), 3.29- 3.25 (m, 2 H), 2.84 (bs, 2 H), 2.68- 2.63 (m, 1 H), 2.02- 1.91 (m, 2 H), 1.86- 1.82 (m, 1 H), 1.76- 1.72 (m, 1 H). ES-API: [M+H]⁺= 392.2. ee value: 100%.
Isomer 4 (retention time: 3.37 min); arbitrarily assigned as Z-37-4 (40.5 mg). ES-API:[M+H]⁺= 392.2; ¹H NMR (500 MHz, DMSO-d6) δ 7.96 (d, *J*= 6.5 Hz, 1 H), 7.32 (t, *J*1= 8.0 Hz, *J*2= 15.5 Hz, 2 H), 7.23 (t, *J*1= 7.0 Hz, *J*2= 14.5 Hz, 1 H), 7.18 (s, 1 H), 7.10 (d, *J*= 7.0 Hz, 2 H), 6.15 (d, *J*= 6.0 Hz, 1 H), 5.30 (dd, *J*1= 5.0 Hz, *J*2= 12.0 Hz, 1 H), 3.79 (s, 3 H), 3.70- 3.44 (m, 4 H), 3.29- 3.25 (m, 2 H), 2.83 (bs, 2 H), 2.68- 2.63 (m, 1 H), 2.00- 1.92 (m, 2 H), 1.86- 1.81 (m, 1 H), 1.76- 1.72 (m, 1 H). ES-API: [M+H]⁺= 392.2. ee value: 100%.

### Example 34: 6-(5-(5-(3,5-difluorophenyl)-4,5-dihydro-1H-pyrazole-1-carbonyl)hexahydrocyclopenta[c]pyrrol-2(1H)-yl)pyrimidine-4-carbonitrile (Z-38), 6-((3aR,5s,6aS)-5-((S)-5-(3,5-difluorophenyl)-4,5-dihydro-1H-pyrazole-1-carbonyl)hexahydrocyclopenta[c]pyrrol-2(1H)-yl)pyrimidine-4-carbonitrile (Z-38-1), 6-((3aR,5s,6aS)-5-((R)-5-(3,5-difluorophenyl)-4,5-dihydro-1H-pyrazole-1-carbonyl)hexahydrocyclopenta[c]pyrrol-2(1H)-yl)pyrimidine-4-carbonitrile (Z-38-2), 6-((3aR,5r,6aS)-5-((S)-5-(3,5-difluorophenyl)-4,5-dihydro-1H-pyrazole-1-carbonyl)hexahydrocyclopenta[c]pyrrol-2(1H)-yl)pyrimidine-4-carbonitrile (Z-38-3), 6-((3aR,5r,6aS)-5-((R)-5-(3,5-difluorophenyl)-4,5-dihydro-1H-pyrazole-1-carbonyl)hexahydrocyclopenta[c]pyrrol-2(1H)-yl)pyrimidine-4-carbonitrile (Z-38-4)

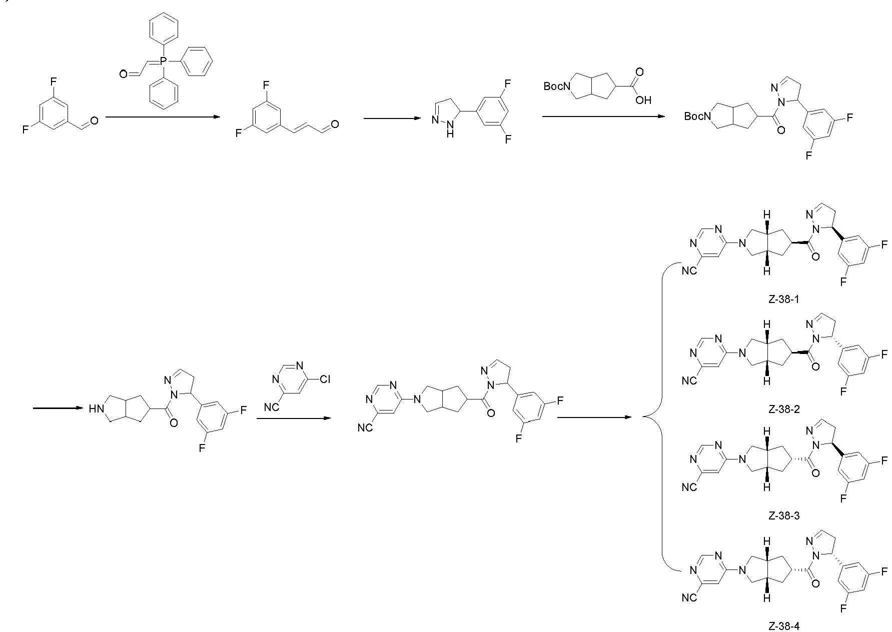

Step 1: 3,5-difluorobenzaldehyde (4.237 g, 29.817 mmol) was dissolved in 30 mL tetrahydrofuran. 2-(triphenylphosphoranylene)acetaldehyde (10g, 32.859 mmol) was added under the protection of nigrogen gas. The reaction mixture was heated to 80°C and stirred overnight. The resultant mixture was concentrated at a reduced pressure. The crude product was purified by combiflash to give 3-(3,5-difluorophenyl)acraldehyde as a yellow oil (4.0 g, yield: 80 %). ES-API:[M+H]⁺= 169.0.

Step 2: hydrazine hydrate (1.7 mL, 35.625 mmol), ethanol (10 mL) and acetic acid (2.3 mL, 40.469 mmol) were sequentially added to a 100 mL round-bottomed flask. The mixture was heated to 45°C. To the reaction mixture, 3-(3,5-difluorophenyl)acraldehyde (4g, 23.75 mmol) was added in batches. Thereafter, the mixture was warmed to 45°C and reacted for 21 h. The resultant mixture was concentrated at a reduced pressure. The residue was purified by column chromatography to give 5-(3,5-difluorophenyl)-4,5-dihydro-1H-pyrazole as a yellow oil (2.8 g, yield: 65%). ES-API:[M+H]⁺= 183.1.

Step 3: 2-(7-azabenzotriazol-1-yl)-N,N,N',N'-tetramethyluronium hexafluorophosphate (358 mg, 0.941 mmol) was added in batches to a mixture of (3aR,6aS)-2-(tert-butoxycarbonyl)octahydrocyclopenta[c]pyrrole-5-carboxylic acid (200 mg, 0.784 mmol), 5-(3,5-difluorophenyl)-4,5-dihydro-1H-pyrazole (285 mg, 1.568 mmol), triethylamine (158 mg, 1.568 mmol) and dichloromethane (10 mL). The mixture was stirred at the room temperatue overnight. The resultant reaction mixture was concentrated at a reduced pressure. The crude product was purified by combiflash to give tert-butyl 5-(3aR,6aS)-(5-(3,5-difluorophenyl)-4,5-dihydro-1H-pyrazole-1-carbonyl)hexahydrocyclopenta[c]pyrrole-2(1H)-carboxylate as a pale yellow oily liquid (377 mg, yield: 99%). ES-API: [M+H]⁺= 420.2.

Step 4: tert-butyl 5-(3aR,6aS)-(5-(3,5-difluorophenyl)-4,5-dihydro-1H-pyrazole-1-carbonyl)hexahydrocyclopenta[c]pyrrole-2(1H)-carboxylate (377 mg, 0.899 mmol) was dissolved in 1,4-dioxane (10 mL). Then, a 4M solution of hydrochlorid acid in dioxane (10 mL) was slowly added dropwise at the room temperature. The mixture was stirred at the room temperature overnight, and then concentrated at a reduced pressure to give ((5-(3,5-difluorophenyl)-4,5-dihydro-1H-pyrazol-1-yl) (3aR,6aS)-(octahydrocyclopenta[c]pyrrol-5-yl)ketone hydrochloride (277 mg, yield: 99%), ES-API:[M+H]⁺= 320.1.

Step 5: potassium carbonate (242 mg, 1.756 mmol) was added to a mixture of ((5-(3,5-difluorophenyl)-4,5-dihydro-1H-pyrazol-1-yl)(3aR,6aS)-(octahydrocyclopenta[c]pyrrol-5-yl) ketone hydrochloride (277 mg, 0.867 mmol), 6-chloropyrimidine-4-carbonitrile (122 mg, 0.878 mmol) and N,N-dimethylformamide (10 ml). The reaction mixture was heated to 70°C and stirred overnight, and then concentrated at a reduced pressure to remove the solvent. The crude product was purified by column chromatography to give (3aR,6aS)-6-(5-(5-(3,5-difluorophenyl)-4,5-dihydro-1H-pyrazole-1-carbonyl)hexahydrocyclopenta[c]pyrrol-2(1H)-yl) pyrimidine-4-carbonitrile as an off-white solid (Z-38, 98 mg, yield: 26%).

Step 6: the Z-38 as obtained in the above step was subjected to a chiral resolution (column: IE-5-5-30MIN (4.6^{∗}250mm 5um); mobile phase: n-hexane (0.1%DEA):EtOH(0.1%DEA)=50:50; flow rate: 1.0 ml/min; column temperature= 40 °C) to give 4 isomers having a single configuration (the structures of the 4 isomers having a single configuration are each arbitrarily assigned):
Isomer 1 (retention time: 10.501 min); arbitrarily assigned as Z-38-1 (15.3 mg); ES-API:[M+H]⁺= 423.1; ¹H NMR (500 MHz, DMSO-d6) δ 8.54 (s, 1 H), 7.19 (d, *J* = 5.0 Hz, 2 H), 7.13- 7.10 (m, 1 H), 6.83 (d, *J* = 6.5 Hz, 2 H), 5.35- 5.31 (m, 1 H), 3.72-3.63 (m, 3 H), 3.49- 3.29 (m, 3 H), 2.90- 2.85 (m, 2 H), 2.73 (dd, *J*1 = 4.5 Hz, *J*2 = 18.0 Hz, 1 H), 2.01- 1.98 (m, 2 H), 1.89- 1.84 (m, 1 H), 1.78- 1.73 (m, 1 H). ES-API: [M+H]⁺= 423.1. ee value: 100%.
Isomer 2 (retention time: 12.57 min); arbitrarily assigned as Z-38-2 (23.0 mg); ES-API:[M+H]⁺= 423.1; ¹H NMR (500 MHz, DMSO-d6) δ 8.52 (s, 1 H), 7.22 (s, 1 H), 7.16 (s, 1 H), 7.12- 7.08 (m, 1 H), 6.82 (d, *J* = 7.0 Hz, 2 H), 5.34- 5.31 (m, 1 H), 3.69- 3.54 (m, 3 H), 3.50- 3.29 (m, 3 H), 2.85- 2.71 (m, 3 H), 2.27- 2.21 (m, 1 H), 2.17- 2.11 (m, 1 H), 1.61- 1.55 (m, 2 H). ES-API: [M+H]⁺= 423.1. ee value: 100%.
Isomer 3 (retention time: 17.551 min); arbitrarily assigned as Z-38-3 (11.3 mg); ES-API:[M+H]⁺= 423.1; ¹H NMR (500 MHz, DMSO-d6) δ 8.55 (s, 1 H), 7.21 (s, 1 H), 7.19 (s, 1 H), 7.14- 7.11 (m, 1 H), 6.84- 6.83 (m, 2 H), 5.35- 5.32 (m, 1 H), 3.74-3.63 (m, 3 H), 3.50- 3.29 (m, 3 H), 2.91- 2.86 (m, 2 H), 2.76- 2.71 (m, 1 H), 2.00-1.99 (m, 2 H), 1.89- 1.84 (m, 1 H), 1.79- 1.74 (m, 1 H). ES-API: [M+H]⁺= 423.1. ee value: 100%.
Isomer 4 (retention time: 21.515 min); arbitrarily assigned as Z-38-4 (19.6 mg). ES-API:[M+H]⁺= 423.1; ¹H NMR (500 MHz, DMSO-d6) δ 8.51 (s, 1 H), 7.22 (s, 1 H), 7.16 (s, 1 H), 7.12- 7.08 (m, 1 H), 6.82 (d, *J* = 6.5 Hz, 2 H), 5.34- 5.31 (m, 1 H), 3.71- 3.54 (m, 3 H), 3.50- 3.29 (m, 3 H), 2.85- 2.71 (m, 3 H), 2.27- 2.21 (m, 1 H), 2.17- 2.11 (m, 1 H), 1.61- 1.55 (m, 2 H). ES-API: [M+H]⁺= 423.1. ee value: 100%.

### Example 35: (2-(4-amino-5-fluoropyrimidin-2-yl) octahydrocyclopenta[c]pyrrol-5-yl)(5-phenyl-4,5-dihydro-1H-pyrazol-1-yl) ketone (Z-39), ((3aR,5s,6aS)-2-(4-amino-5-fluoropyrimidin-2-yl) octahydrocyclopenta[c]pyrrol-5-yl)((S)-5-phenyl-4,5-dihydro-1H-pyrazol-1-yl) ketone (Z-39-1), ((3aR,5s,6aS)-2-(4-amino-5-fluoropyrimidin-2-yl) octahydrocyclopenta[c]pyrrol-5-yl)((R)-5-phenyl-4,5-dihydro-1H-pyrazol-1-yl) ketone (Z-39-2), ((3aR,5r,6aS)-2-(4-amino-5-fluoropyrimidin-2-yl)octahydrocyclopenta[c]pyrrol-5-yl)((S)-5-phenyl-4,5-dihydro-1H-pyrazol-1-yl) ketone (Z-39-3), ((3aR,5r,6aS)-2-(4-amino-5-fluoropyrimidin-2-yl)octahydrocyclopenta[c]pyrrol-5-yl)((R)-5-phenyl-4,5-dihydro-1H-pyrazol-1-yl) ketone (Z-39-4)

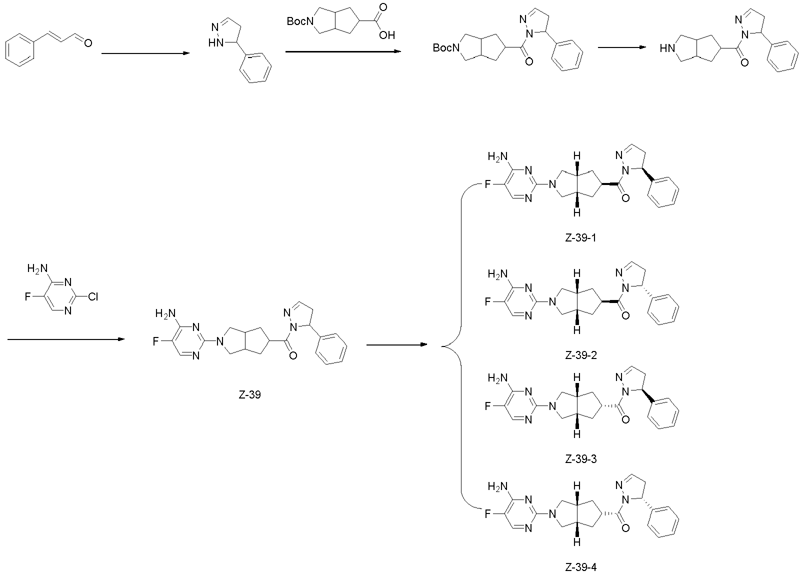

Step 1: hydrazine hydrate (30 mL, 620 mmol) was added to a 250 mL round-bottomed flask, and was heated under reflux. A solution obtained by dissolving cinnamic aldehyde (20 mL, 158.8 mmol) in n-butanol (30 mL) was slowly added dropwise to the hydrazine hydrate. The mixture was refluxed overnight. The resultant reaction mixture was concentrated at a reduced pressure. Water (100 ml) was added to the residue. The resultant mixture was extracted with 200 mL of dichloromethane for three times. The combined organic phase was washed with water, dried over anhydrous sodium sulfate, and concentrated at a reduced pressure. The residue was purified by combiflash to give 5-phenyl-4,5-dihydro-1H-pyrazole as a yellow oil (14 g, yield: 60%). ES-API:[M+H]⁺= 147.0.

Step 2: 2-(7-azabenzotriazol-1-yl)-N,N,N',N'-tetramethyluronium hexafluorophosphate (554 mg, 1.459 mmol) was added in batches to a mixture of 2-(3aR,6aS)-(tert-butoxycarbonyl)octahydrocyclopenta[c]pyrrole-5-carboxylic acid (310 mg, 1.216 mmol), 5-phenyl-4,5-dihydro-1H-pyrazole (355 mg, 2.431 mmol), triethylamine (246 mg, 2.431 mmol) and dichloromethane (15 mL). The mixture was stirred at the room temperatue for 3 h. The resultant reaction mixture was concentrated at a reduced pressure. The crude product was purified by combiflash to give tert-butyl 5-(3aR,6aS)-(5-phenyl-4,5-dihydro-1H-pyrazole-1-carbonyl) hexahydrocyclopenta[c]pyrrole-2(1H)-carboxylate as an oily liquid (388 mg, yield: 83%). ES-API:[M+H]⁺= 384.2.

Step 3: tert-butyl 5-(3aR,6aS)-(5-phenyl-4,5-dihydro-1H-pyrazole-1-carbonyl)hexahydrocyclopenta[c]pyrrole-2(1H)-carboxylate (388 mg, 1.013 mmol) was dissolved in 1,4-dioxane (10 mL). Then, a 4M solution of hydrochlorid acid in dioxane (10 mL) was slowly added dropwise at the room temperature. The mixture was stirred at the room temperature overnight, and then concentrated at a reduced pressure to give (3aR,6aS)-(octahydrocyclopenta[c]pyrrol-5-yl)(5-phenyl-4,5-dihydro-1H-pyrazol-1-yl) ketone hydrochloride (403 mg, yield: 100%), ES-API:[M+H]⁺= 284.1.

Step 4: triethylamine (245 mg, 2.424 mmol) was added to a mixture of (3aR,6aS)-(octahydrocyclopenta[c]pyrrol-5-yl)(5-phenyl-4,5-dihydro-1H-pyrazol-1-yl) ketone hydrochloride (343 mg, 1.212 mmol), 2-chloro-5-fluoropyrimidine-4-amine (125 mg, 0.848 mmol) and chlorobenzene (15 ml). The reaction mixture was heated under reflux overnight, and then concentrated at a reduced pressure to remove the solvent. The crude product was purified by column chromatography to give (2-(4-amino-5-fluoropyrimidin-2-yl)octahydrocyclopenta[c]pyrrol-5-yl)(5-phenyl-4,5-dihydro-1H-pyrazol-1-yl) ketone as an off-white solid (Z-39, 62.5 mg, yield: 13%).

Step 5: the Z-39 as obtained in the above step was subjected to a chiral resolution (column: IE-5-5-30MIN (4.6^{∗}250mm 5um); mobile phase: n-hexane (0.1%DEA):EtOH(0.1%DEA)=50:50; flow rate: 1.0; column temperature=40°C) to give 4 isomers having a single configuration (the structures of the 4 isomers having a single configuration are each arbitrarily assigned):
Isomer 1 (retention time: 7.70 min); arbitrarily assigned as Z-39-1 (14.79 mg); ¹H NMR (500 MHz, DMSO-d6) δ 7.77 (d, *J*= 4.0 Hz, 1 H), 7.31 (t, *J*1= 7.5 Hz, *J*2= 15.5 Hz, 2 H), 7.24- 7.22 (m, 1 H), 7.18- 7.16 (m, 1 H), 7.10 (d, *J*= 7.0 Hz, 2 H), 6.75 (s, 2 H), 5.31- 5.28 (m, 1 H), 3.67- 3.43 (m, 4 H), 3.24- 3.19 (m, 2 H), 2.77 (bs, 2 H), 2.68- 2.63 (m, 1 H), 2.02- 1.87 (m, 2 H), 1.82- 1.78 (m, 1 H), 1.72- 1.69 (m, 1 H). ES-API: [M+H]⁺= 395.1. ee value: 97.75%.
Isomer 2 (retention time: 8.65 min); arbitrarily assigned as Z-39-2 (10.65 mg); ¹H NMR (500 MHz, DMSO-d6) δ 7.77 (d, *J*= 3.5 Hz, 1 H), 7.31 (t, *J*1= 7.5 Hz, *J*2= 15.5 Hz, 2 H), 7.23 (t, *J*1= 7.0 Hz, *J*2= 14.5 Hz, 1 H), 7.17 (s, 1 H), 7.10 (d, *J*= 7.5 Hz, 2 H), 6.75 (s, 2 H), 5.31- 5.28 (m, 1 H), 3.67- 3.63 (m, 1 H), 3.57- 3.43 (m, 3 H), 3.24- 3.19 (m, 2 H), 2.77 (bs, 2 H), 2.67- 2.63 (m, 1 H), 1.99- 1.87 (m, 2 H), 1.82-1.78 (m, 1 H), 1.73- 1.69 (m, 1 H). ES-API: [M+H]⁺= 395.1. ee value: 96.52%.
Isomer 3 (retention time: 11.53 min); arbitrarily assigned as Z-39-3 (14.20 mg); ¹H NMR (500 MHz, DMSO-d6) δ 7.74 (d, *J*= 4.0 Hz, 1 H), 7.31 (t, *J*1= 7.5 Hz, *J*2= 15.0 Hz, 2 H), 7.23- 7.19 (m, 2 H), 7.09 (d, *J*= 7.5 Hz, 2 H), 6.70 (s, 2 H), 5.31- 5.27 (m, 1 H), 3.61- 3.56 (m, 1 H), 3.50- 3.40 (m, 3 H), 3.30- 3.29 (m, 2 H), 2.68- 2.63 (m, 3 H), 2.20- 2.06 (m, 2 H), 1.56- 1.46 (m, 2 H). ES-API: [M+H]⁺= 395.1. ee value: 100%.
Isomer 4 (retention time: 25.39 min); arbitrarily assigned as Z-39-4 (13.72 mg); ¹H NMR (500 MHz, DMSO-d6) δ 7.74 (d, *J*= 4.0 Hz, 1 H), 7.31 (t, *J*1= 7.5 Hz, *J*2= 15.0 Hz, 2 H), 7.23- 7.19 (m, 2 H), 7.09 (d, *J*= 7.5 Hz, 2 H), 6.70 (s, 2 H), 5.31- 5.27 (m, 1 H), 3.61- 3.40 (m, 4 H), 3.30- 3.29 (m, 2 H), 2.69- 2.63 (m, 3 H), 2.20- 2.06 (m, 2 H), 1.56- 1.46 (m, 2 H). ES-API: [M+H]⁺= 395.1. ee value: 95.98%.

### Example 36: Preparation of (5-(6-methoxypyrimidin-4-yl)-5-aza-spiro[2.5]oct-8-yl)(5-phenyl-4,5-dihydro-1H-pyrazol-1-yl) ketone (racemate, Z-40-1) and (5-(6-methoxypyrimidin-4-yl)-5-aza-spiro[2.5]oct-8-yl)(5-phenyl-4,5-dihydro-1H-pyrazol-1-yl) ketone (racemate, Z-40-2)

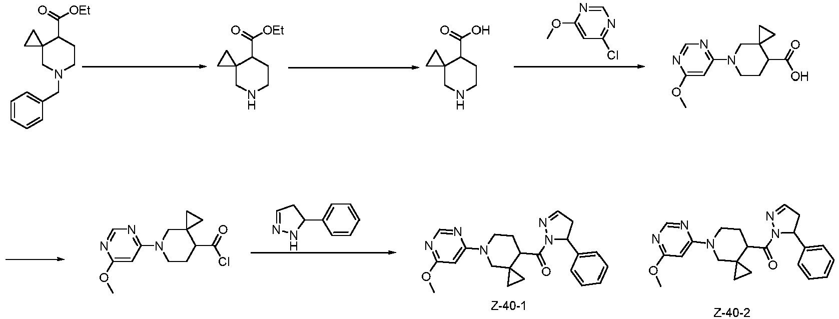

Step 1: ethyl 5-benzyl-5-aza-spiro[2.5]octane-8-carboxylate (6 g, 21.98 mmol) was dissolved in 20 mL of methanol. Then, 10% palladium on carbon (1.8 g, 0.3 w/w) was added. The mixture was stirred at the room temperature under an atmosphere of hydrogen gas for 24 h, and filtered. To the filtrate, further 10% palladium on carbon (1.8 g, 0.3 w/w) was added. The mixture was stirred at the room temperature under an atmosphere of hydrogen gas for 24 h, and filtered. The filtrate was concentrated at a reduced pressure to give ethyl 5-aza-spiro[5-]octane-8-carboxylate (4.36 g, yield: crude product), which was directly used in the next step of reaction. ES-API:[M+H]⁺= 184.2.

Step 2: ethyl 5-aza-spiro[5-]octane-8-carboxylate (4.36 g, 23.7 mmol) was added to methanol (5 mL) and water (4 mL). Then, sodium hydroxide (1.15 g, 28.4 mmol) was added. The reaction was carried out at the room temperature for 24 h. After reaction was completed, the reaction mixture was extracted with dichloromethane. The pH of the combined aqueous phases was adjusted with diluted hydrochloric acid (2M) to 6-7. The resultant solution was concentrated at a reduced pressure to give 5-aza-spiro[2.5]octane-8-carboxylic acid (5 g, yield: crude product, containing sodium chloride salt). ES-API: [M+H]⁺= 156.1.

Step 3: 5-aza-spiro[2.5]octane-8-carboxylic acid (300 mg, 1.92 mmol), potassium carbonate (829 mg, 6.00 mmol) and 4-chloro-6-methoxypyrimidine (300 mg, 2.30 mmol) were added to acetonitrile (8 mL). The mixture was stirred at 50°C for 2 h. Thereafter, water (10 mL) was added to the reaction mixture. The resultant solution was extracted with dichloromethane. The pH value of the combined aqueous phases was adjusted with diluted hydrochloric acid (2M) to 6-7. The resultant solution was further extracted with dichloromethane. The organic phases were combined and concentrated at a reduced pressure to give 5-(6-methoxypyrimidin-4-yl)-5-aza-spiro[2.5]octane-8-carboxylic acid (150 mg, yield: crude product). ES-API: [M+H]⁺ =264.1.

Step 4: 5-(6-methoxypyrimidin-4-yl)-5-aza-spiro[2.5]octane-8-carboxylic acid (150 mg, 0.57 mmol) was dissolved in dichloromethane (8 mL). Then, sulfoxide chloride (0.5 mL) was added. The mixture was stirred at the room temperatue for 0.5 h. The resultant reaction mixture was concentrated at a reduced pressure to give 5-(6-methoxypyrimidin-4-yl)-5-aza-spiro[2.5]octane-8-carbonyl chloride (100 mg, yield: crude product), which was directly used in the next step of reactoin. ES-API: [M+H]⁺ =278.1(qunced with methanol).

Step 5: 5-(6-methoxypyrimidin-4-yl)-5-aza-spiro[2.5]octane-8-carbonyl chloride (100 mg, 0.35 mmol) was dissolved in dichloromethane (8 mL). Then, N,N-diisopropylethylamine (1 mL) and 5-(3,5-difluorophenyl)-4,5-dihydro-1H-pyrazole (130 mg, 0.89 mmol) were added. The mixture was stirred at the room temperature for 2 h. Thereafter, the reaction mixture was extracted with dichloromethane, dried, and concentrated at a reduced pressure. The crude product was subjected to thin layer chromatography (petroleum ether/ethyl acetate=1/1) to give 2 racemates:
Z-40-1 (the collected fraction having a Rf of 0.6): a racemate of (5-(6-methoxypyrimidin-4-yl)-5-aza-spiro[2.5]oct-8-yl)(5-phenyl-4,5-dihydro-1H-pyrazol-1-yl) ketone (11.4 mg, yield: 1.8%, LCMS retention time: 1.74 min); ES-API: [M+H]⁺ =392.2. ¹H NMR (400 MHz, CDCl₃) δ 8.25 (s, 1H), 7.39 - 7.21 (m, 3H), 7.14 (d, *J* = 7.1 Hz, 2H), 6.94 (s, 1H), 5.71 (s, 1H), 5.38 (d, *J* = 16.4 Hz, 1H), 3.88 (s, 3H), 3.83 (s, 1H), 3.42 (dd, *J* = 18.9, 11.6 Hz, 2H), 3.29 (d, *J* = 12.5 Hz, 1H), 3.12 (s, 1H), 2.92 - 2.69 (m, 1H), 2.02 (s, 2H), 1.25 (s, 1H), 0.67 - 0.42 (m, 3H), 0.33 (d, *J* = 8.6 Hz, 1H).
Z-40-2 (the collected fraction having a Rf of 0.3): another racemate of (5-(6-methoxypyrimidin-4-yl)-5-aza-spiro[2.5]oct-8-yl)(5-phenyl-4,5-dihydro-1H-pyrazol-1-yl) ketone (11.8 mg, yield: 1.8%, LCMS retention time: 1.75min); ES-API: [M+H]⁺ =392.2. ¹H NMR(400 MHz, CDCl₃ ) δ 8.25 (s, 1H), 7.40 - 7.21 (m, 3H), 7.16 (d, *J* = 7.4 Hz, 2H), 6.93 (s, 1H), 5.72 (s, 1H), 5.44 - 5.30 (m, 1H), 3.88 (s, 3H), 3.82 - 3.67 (m, 2H), 3.42 (dd, *J =* 18.9, 12.1 Hz, 1H), 3.31 (d, *J* = 13.3 Hz, 1H), 3.21 (t, *J* = 5.2 Hz, 1H), 2.81 (dd, *J =* 19.0, 5.0 Hz, 1H), 2.06 - 1.91 (m, 2H), 1.26 (d, *J* = 7.2 Hz, 1H), 0.55 (ddt, *J* = 14.9, 9.1, 4.5 Hz, 2H), 0.49 - 0.37 (m, 2H).

### Example 37: Preparation of (5-(2-methoxypyrimidin-4-yl)-5-aza-spiro[2.5]oct-8-yl)(5-phenyl-4,5-dihydro-1H-pyrazol-1-yl) ketone (racemate, Z-41-1) and (5-(2-methoxypyrimidin-4-yl)-5-aza-spiro[2.5]oct-8-yl)(5-phenyl-4,5-dihydro-1H-pyrazol-1-yl) ketone (racemate, Z-41-2)

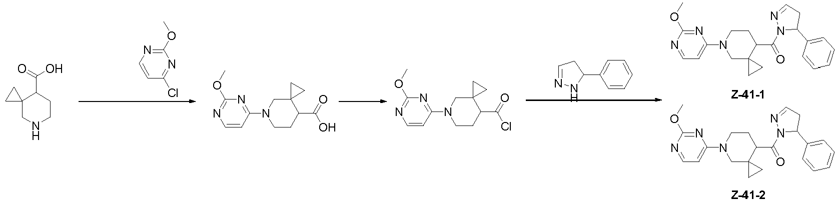

Step 1: 5-aza-spiro[2.5]octane-8-carboxylic acid (300 mg, 1.92 mmol), potassium carbonate (829 mg, 6.00 mmol) and 4-chloro-2-methoxypyrimidine (270 mg, 2.30 mmol) were added to acetonitrile (6 mL). The mixture was stirred at 50°C for 16 h. Water (10 mL) was added to the reaction mixture. The resultant mixture was extracted with dichloromethane. The pH value of the combined aqueous phases was adjuste with diluted hydrochloric acid (2M) to 6-7. The combined aqueous phases was concentrated at a reduced pressure to give 5-(2-methoxypyrimidin-4-yl)-5-aza-spiro[2.5]octane-8-carboxylic acid (2.4 g, crude product, containing salt). ES-API: [M+H]⁺ =264.1

Step 2: 5-(2-methoxypyrimidin-4-yl)-5-aza-spiro[2.5]octane-8-carboxylic acid (2.4 g, crude product, containing salt) was dissolved in dichloromethane (8 mL). Then, sulfoxide chloride (0.5 mL) was added. The mixture was stirred at the room temperature for 0.5 h. The resultant reacton mixture was concentrated at a reduced pressure to give 5-(2-methoxypyrimidin-4-yl)-5-aza-spiro[2.5]octane-8-carbonyl chloride (2.4 g, crude product), which was directly used in the next step of reaction. ES-API: [M+H]⁺ =278.1 (quenched with methanol)

Step 3: 5-(2-methoxypyrimidin-4-yl)-5-aza-spiro[2.5]octane-8-carbonyl chloride (2.4 g, crude product, containing salt) was dissolved in dichloromethane (10 mL). Then, N,N-diisopropylethylamine (2 mL) and 5-(3,5-difluorophenyl)-4,5-dihydro-1H-pyrazole (150 mg, 1.02 mmol) were added. The mixture was stirred at the room temperature for 2 h. The resultant reaction mixture was extracted with dichloromethane, dried, and concentrated at a reduced pressure. The crude product was subjected to thin layer chromatography (petroleum ether/ethyl acetate=1/2) to give 2 racemates:
Z-41-1 (the collected fraction having a Rf of 0.4): a recemate of (5-(2-methoxypyrimidin-4-yl)-5-aza-spiro[2.5]oct-8-yl)(5-phenyl-4,5-dihydro-1H-pyrazol-1-yl) ketone (2.9 mg, yield: 0.7%, LCMS retention time: 1.51 min); ES-API:[M+H]⁺ =392.2. ¹H NMR(400 MHz, CDCl₃) δ 7.94 (d, *J* = 6.1 Hz, 1H), 7.32 (t, *J* = 7.4 Hz, 2H), 7.27 (s, 1H), 7.18 - 7.09 (m, 2H), 6.94 (d, *J* = 1.9 Hz, 1H), 6.07 (d, *J* = 6.2 Hz, 1H), 5.38 (dd, *J* = 12.0, 4.9 Hz, 1H), 3.88 (s, 3H), 3.51 - 3.34 (m, 3H), 3.11 (t, *J* = 5.1 Hz, 1H), 2.80 (ddd, *J* = 18.9, 4.9, 1.7 Hz, 1H), 2.01 (dq, *J* = 9.0, 4.7 Hz, 3H), 0.64 - 0.47 (m, 3H), 0.34 (d, *J* = 9.3 Hz, 1H).
Z-41-2 (the collected fraction having a Rf of 0.3): another racemate of (5-(2-methoxypyrimidin-4-yl)-5-aza-spiro[2.5]oct-8-yl)(5-phenyl-4,5-dihydro-1H-pyrazol-1-yl) ketone (6.4 mg, yield: 1.5%, LCMS retention time: 1.49 min); ES-API:[M+H]⁺ =392.2. ¹H NMR(400 MHz, CDCl₃) δ 7.94 (d, *J* = 6.1 Hz, 1H), 7.32 (dd, *J* = 8.1, 6.5 Hz, 2H), 7.27 (d, *J* = 5.2 Hz, 1H), 7.16 (dd, *J* = 7.0, 1.8 Hz, 2H), 6.93 (d, *J* = 1.8 Hz, 1H), 6.08 (d, *J* = 6.2 Hz, 1H), 5.37 (dd, *J* = 12.0, 5.1 Hz, 1H), 3.88 (s, 3H), 3.83 - 3.72 (m, 2H), 3.42 (ddd, *J* = 18.9, 12.0, 1.7 Hz, 2H), 3.20 (t, *J* = 5.2 Hz, 1H), 2.81 (ddd, *J* = 18.9, 5.1, 1.8 Hz, 1H), 1.99 (tt, *J* = 11.0, 7.0 Hz, 3H), 0.56 (ddt, *J* = 16.9, 9.5, 4.9 Hz, 2H), 0.43 (dtt, *J* = 17.6, 9.5, 4.4 Hz, 2H).

### Example 38: Preparation of (5-(4-amino-5-fluoropyrimidin-2-yl)-5-aza-spiro[2.5]oct-8-yl)(5-phenyl-4,5-dihydro-1H-pyrazol-1-yl) ketone (racemate, Z-42-1) and (5-(4-amino-5-fluoropyrimidin-2-yl)-5-aza-spiro[2.5]oct-8-yl)(5-phenyl-4,5-dihydro-1H-pyrazol-1-yl) ketone (racemate, Z-42-2)

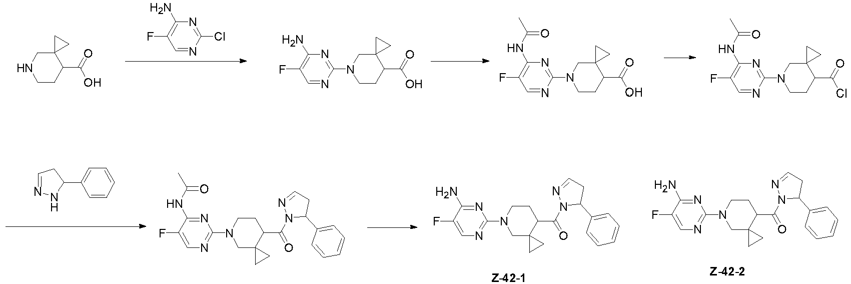

Step 1: 5-aza-spiro[2.5]octane-8-carboxylic acid (2 g, crude product, containing about 50% sodium chloride) was dissolved in N,N-dimethylformamide (10 mL). Then, potassium carbonate (5.29 g, 38.14 mmol) and 2-chloro-5-fluoropyrimidine-4-amine (1 g, 6.75 mmol) were added. The mixture was stirred at 100°C for reaction over night. The resultant reaction mixture was extracted with ethyl acetate. The aqueous phases were combined. The pH of the aqueous phase was adjusted with diluted hydrochloric acid (2N) to 5-6. The aqueous phase was further extracted with ethyl acetate, dried, and concentrated at a reduced pressure to give 5-(4-amino-5-fluoropyrimidin-2-yl)-5-aza-spiro[2.5]octane-8-carboxylic acid (600 mg, yield: 39%). ES-API:[M+H]⁺= 207.1.

Step 2: 5-(4-amino-5-fluoropyrimidin-2-yl)-5-aza-spiro[2.5]octane-8-carboxylic acid (600 mg, 2.25 mmol) was dissolved in tetrahydrofuran (6 mL). Then, triethylamine (800 mg, 7.92 mmol), 4-dimethylaminopyridine (25 mg, 0.2 mmol) and acetic anhydride (459 mg, 4.5 mmol) were added. The reaction was carried out at 60°C overnight. The reaction mixture was quenched with water, extracted with dichloromethane, dried, and concentrated at a reduced pressure to give 5-(4-acetamido-5-fluoropyrimidin-2-yl)-5-aza-spiro[2.5]octane-8-carboxylic acid (800 mg, yield: 100%). The crude product was directly used in the next step of reaction. ES-API:[M+H]⁺= 309.1.

Step 3: 5-(4-acetamido-5-fluoropyrimidin-2-yl)-5-aza-spiro[2.5]octane-8-carboxylic acid (350 mg, 1.14 mmol) was dissolved in dichloromethane (5 mL). Then, sulfoxide chloride (0.5 mL) was added. The reaction mixture was reacted at the room temperature for 30 minutes. The resultant reaction mixture was concentrated at a reduced pressure to give 5-(4-acetamido-5-fluoropyrimidin-2-yl)-5-aza-spiro[2.5]octane-8-carbonyl chloride (350 mg, yield: 100%). The crude product was directly used in the next step of reaction. ES-API:[M+H]⁺= 323.1.

Step 4: 5-(4-acetamido-5-fluoropyrimidin-2-yl)-5-aza-spiro[2.5]octane-8-carbonyl chloride (150 mg, 0.46 mmol) was dissolved in dichloromethane (5 mL). Then, N,N-diisopropylethylamine (0.5 mL) and 5-phenyl-4,5-dihydro-1H-pyrazole (200 mg, 1.36 mmol) were added. The reaction was carried out at the toom temperature for 2 h. The resultant reaction mixture was extracted with dichloromethane, dried, and concentrated at a reduced pressure to give a crude product of N-(5-fluoro-2-(8-(5-phenyl-4,5-dihydro-1H-pyrazole-1-carbonyl)-5-aza-spiro[2.5]octan-5-yl)pyrimidin-4-yl) acetamide (350 mg, yield: 100%). The crude product was directly used in the next step of reaction. ES-API:[M+H]⁺= 437.2.

Step 5: N-(5-fluoro-2-(8-(5-phenyl-4,5-dihydro-1H-pyrazole-1-carbonyl)-5-aza-spiro[2.5]octan-5-yl)pyrimidin-4-yl) acetamide (350 mg, 0.8 mmol) was dissolved in methanol/water (4mL/2mL). Then, sodium hydroxide (100 mg, 2.4 mmol) was added. The reaction was carried out overnight. The resultant reacton solution was extracted with ethyl acetate, dried, and concentrated at a reduced pressure. The crude product was purified by thin layer chromatography (petroleum ether/ethyl acetate=1/1) to give 2 racemates:
Z-42-1 (the collected fraction having a Rf of 0.6): a racemate of (5-(4-amino-5-fluoropyrimidin-2-yl)-5-aza-spiro[2.5]oct-8-yl)(5-phenyl-4,5-dihydro-1H-pyrazol-1-yl) ketone (1.4 mg, yield: 1.3 %, LCMS retention time: 1.51 min). ES-API: [M+H]⁺= 395.2. ¹H NMR (400 MHz, CDCl₃) δ 7.76 (s, 1H), 7.41 - 7.30 (m, 2H), 7.16 (d, *J* = 7.3 Hz, 3H), 6.94 (s, 1H), 5.36 (d, *J* = 11.0 Hz, 1H), 4.67 (s, 2H), 3.87 (s, 2H), 3.74 (d, *J* = 13.3 Hz, 1H), 3.51 - 3.31 (m, 2H), 3.24 (s, 1H), 2.90 - 2.68 (m, 1H), 1.93 (d, *J* = 11.7 Hz, 2H), 0.51 (s, 2H), 0.46 - 0.31 (m, 2H).
Z-42-2 (the fraction having a Rf of 0.4): another racemate of (5-(4-amino-5-fluoropyrimidin-2-yl)-5-aza-spiro[2.5]oct-8-yl)(5-phenyl-4,5-dihydro-1H-pyrazol-1-yl) ketone (0.7 mg, yield: 0.7%, LCMS retention time: 1.52 min). ES-API: [M+H]⁺= 395.2. ¹H NMR (400 MHz, CDCl₃) δ 7.79 (s, 1H), 7.46 (s, 2H), 7.36 (q, *J* = 6.1 Hz, 3H), 6.86 (s, 1H), 4.70 (s, 2H), 3.88 (m, 3H), 3.18 (m, 1H), 3.24 (m, 2H), 2.30 (m, 2H), 1.93 (d, *J* = 11.7 Hz, 2H),0.60 - 0.48 (m, 3H), 0.47 - 0.28 (m, 1H).

### Example 39: Preparation of 6-(8-(5-phenyl-4,5-dihydro-1H-pyrazole-1-carbonyl)-5-aza-spiro[2.5]oct-5-yl)pyrimidine-4-carboxamide (racemate, Z-43-1) and 6-(8-(5-phenyl-4,5-dihydro-1H-pyrazole-1-carbonyl)-5-aza-spiro[2.5]oct-5-yl)pyrimidine-4-carboxamide (racemate, Z-43-2)

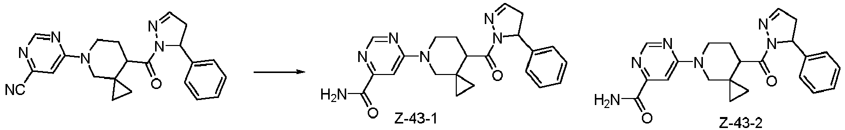

Step 1: 6-(8-(5-phenyl-4,5-dihydro-1H-pyrazole-1-carbonyl)-5-aza-spiro[2.5]oct-5-yl)pyrimidine-4-carbonitrile (20 mg, 0.05 mmol) was dissolved in acetonitrile (3 mL). Sodium hydroxide (2 N, 0.1 mL) was added under an ice-water bath. Then, 30% hydrogen peroxide (0.5 mL) was added. The reaction was carried out at 20°C for 2 h. The organic phase was washed with a solution of sodium sulphite and a saline solution, dried over anhydrous sodium sulfate, and concentrated at a reduced pressure. The crude product was subjected to thin layer chromatography (dichloromethane/methanol=10/1) to give 2 racemates:
Z-43-1 (the collected fraction having a Rf of 0.4): a racemate of 6-(8-(5-phenyl-4,5-dihydro-1H-pyrazole-1-carbonyl)-5-aza-spiro[2.5]oct-5-yl)pyrimidine-4-carboxamide, (2.7 mg, yield: 13.4%, LCMS retention time: 1.60 min), ES-API:[M+H]⁺= 405.2. ¹H NMR (400 MHz, CDCl₃) δ 8.47 (s, 1H), 7.92 (s, 1H), 7.33 (t, *J* = 7.5 Hz, 3H), 7.14 (d, *J* = 7.4 Hz, 3H), 6.95 (s, 1H), 5.63 (s, 1H), 5.38 (dd, *J* = 12.2, 4.8 Hz, 1H), 3.97 (d, *J* = 13.1 Hz, 1H), 3.58 - 3.36 (m, 3H), 3.09 (d, *J* = 5.0 Hz, 1H), 2.81 (dd, *J* = 19.3, 4.7 Hz, 1H), 2.14 - 1.94 (m, 3H), 0.58 (d, *J* = 21.8 Hz, 4H);
Z-43-2 (the collected fraction having a Rf of 0.6): another racemate of 6-(8-(5-phenyl-4,5-dihydro-1H-pyrazole-1-carbonyl)-5-aza-spiro[2.5]oct-5-yl)pyrimidine-4-carboxamide, (1.6 mg, yield: 8.6%, LCMS retention time: 1.59 min), ES-API: [M+H]⁺= 405.2. ¹H NMR (400 MHz, CDCl₃) δ 8.47 (s, 1H), 7.92 (s, 1H), 7.33 (t, *J* = 7.5 Hz, 3H), 7.14 (d, *J* = 7.4 Hz, 3H), 6.95 (s, 1H), 5.63 (s, 1H), 5.38 (dd, *J* = 12.2, 4.8 Hz, 1H), 3.97 (d, *J* = 13.1 Hz, 1H), 3.58 - 3.36 (m, 3H), 3.09 (d, *J* = 5.0 Hz, 1H), 2.81 (dd, *J* = 19.3, 4.7 Hz, 1H), 2.14 - 1.94 (m, 3H), 0.58 (d, *J* = 21.8 Hz, 4H).

### Example 40: Preparation of 6-(8-(5-(3,5-difluorophenyl)-4,5-dihydro-1H-pyrazole-1-carbonyl)-5-aza-spiro[2.5]oct-5-yl)pyrimidine-4-carboxamide (racemate, Z-44-1) and 6-(8-(5-(3,5-difluorophenyl)-4,5-dihydro-1H-pyrazole-1-carbonyl)-5-aza-spiro[2.5]oct-5-yl)pyrimidine-4-carboxamide (racemate, Z-44-2)

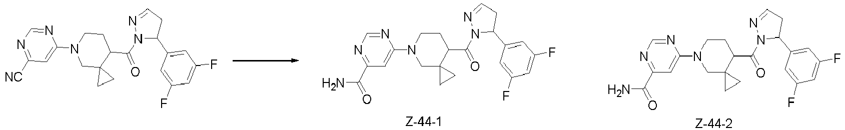

Step 1: 6-(8-(5-(3,5-difluorophenyl)-4,5-dihydro-1H-pyrazole-1-carbonyl)-5-aza-spiro[2.5]oct-5-yl)pyrimidine-4-carbonitrile (30 mg, 0.0678 mmol) was dissolved in acetonitrile (3 mL). Sodium hydroxide (2 N, 0.1 mL) was added under an ice-water bath. Then, 30% hydrogen peroxide (0.5 mL) was added. The reaction was carried out at 20°C for 2 h. The organic phase was washed with a solution of sodium sulphite and a saline solution, dried over anhydrous sodium sulfate, and concentrated at a reduced pressure. The crude product was subjected to thin layer chromatography (dichloromethane/methanol=10/1) to give 2 racemates:
Z-44-1 (the collected fraction having a Rf of 0.5): a racemate of 6-(8-(5-(3,5-difluorophenyl)-4,5-dihydro-1H-pyrazole-1-carbonyl)-5-aza-spiro[2.5]oct-5-yl)pyrimidine-4-carboxamide (7.3 mg, yield: 24.4%, LCMS retention time: 1.65 min), ES-API: [M+H]⁺= 441.1. ¹H NMR (400 MHz, CDCl₃) δ 8.48 (s, 1H), 7.86 (s, 1H), 7.29 (s, 1H), 6.95 (s, 1H), 6.77 - 6.59 (m, 3H), 5.61 (s, 1H), 5.34 (dd, *J* = 12.0, 5.0 Hz, 1H), 3.94 (d, *J* = 13.2 Hz, 1H), 3.61 (s, 1H), 3.44 (dd, *J* = 18.9, 12.0 Hz, 1H), 3.12 (d, *J* = 5.4 Hz, 1H), 2.78 (dd, *J* = 18.6, 4.9 Hz, 1H), 2.11 - 1.96 (m, 2H), 1.26 (s, 2H), 0.67 - 0.30 (m, 4H).
Z-44-2 (the collected fraction having a Rf of 0.7): another racemate of 6-(8-(5-(3,5-difluorophenyl)-4,5-dihydro-1H-pyrazole-1-carbonyl)-5-aza-spiro[2.5]oct-5-yl)pyrimidine-4-carboxamide (8.6 mg, yield: 41.7%, LCMS retention time: 1.64 min), ES-API: [M+H]⁺= 441.1. ¹H NMR (400 MHz, CDCl₃) δ 8.48 (s, 1H), 7.86 (s, 1H), 7.29 (s, 1H), 6.95 (s, 1H), 6.77 - 6.59 (m, 3H), 5.61 (s, 1H), 5.34 (dd, *J* = 12.0, 5.0 Hz, 1H), 3.94 (d, *J* = 13.2 Hz, 1H), 3.61 (s, 1H), 3.44 (dd, *J* = 18.9, 12.0 Hz, 1H), 3.12 (d, *J* = 5.4 Hz, 1H), 2.78 (dd, *J* = 18.6, 4.9 Hz, 1H), 2.11 - 1.96 (m, 2H), 1.26 (s, 2H), 0.67 - 0.30 (m, 4H).

### Example 41: Preparation of 5-(4-amino-5-fluoropyrimidin-2-yl)-5-aza-spiro[2.5]oct-8-yl)(5-(3,5-difluorophenyl)-4,5-dihydro-1H-pyrazol-1-yl) ketone (Z-45)

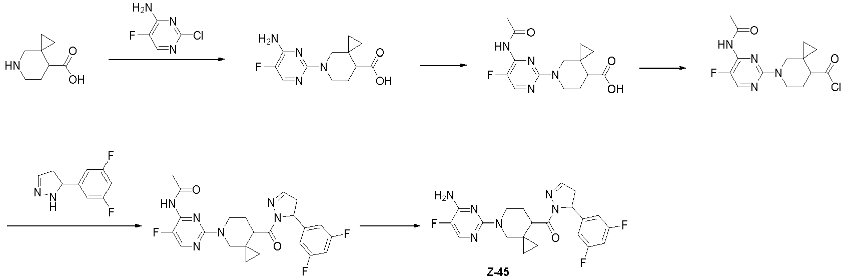

Step 1: 5-aza-spiro[2.5]octane-8-carboxylic acid (2 g, crude product, containing 50% sodium chloride) was dissolved in N,N-dimethylformamide (10 mL). Then, potassium carbonate (5.29 g, 38.14 mmol) and 2-chloro-5-fluoropyrimidine-4-amine (1 g, 6.75 mmol) were added. The mixtue was stirred at 100°C overnight. The resultant reaction mixture was extracted with ethyl acetate. The aqueous phases were combined. The pH value of the aqueous phase was adjusted to 5-6 with diluted hydrochloric acid (2N). The resultant solution was further extracted with ethyl acetate. The organic phase was dried, and concentrated at a reduced pressure to give 5-(4-amino-5-fluoropyrimidin-2-yl)-5-aza-spiro[2.5]octane-8-carboxylic acid (600 mg, yield: 39%). ES-API:[M+H]⁺= 207.1.

Step 2: 5-(4-amino-5-fluoropyrimidin-2-yl)-5-aza-spiro[2.5]octane-8-carboxylic acid (600 mg, 2.25 mmol) was dissolved in tetrahydrofuran (6 mL). Then, triethylamine (800 mg, 7.92 mmol), 4-dimethylaminopyridine (25 mg, 0.2 mmol) and acetic anhydride (459 mg, 4.5 mmol) were added. The reaction was carried out at 60°C overnight. The reaction mixture was quenched with water, extracted with dichloromethane, dried, and concentrated at a reduced pressure to give 5-(4-acetamido-5-fluoropyrimidin-2-yl)-5-aza-spiro[2.5]octane-8-carboxylic acid (800 mg, yield: 100%). The crude product was directly used in the next step of the reaction. ES-API:[M+H]⁺= 309.1.

Step 3: 5-(4-acetamido-5-fluoropyrimidin-2-yl)-5-aza-spiro[2.5]octane-8-carboxylic acid (350 mg, 1.14 mmol) was dissolved in dichloromethane (5 mL). Then, sulfoxide chloride (0.5 mL) was added. The reaction mixture was reacted at the room temperature for 30 minutes. The resultant reaction mixture was concentrated at a reduced pressure to give 5-(4-acetamido-5-fluoropyrimidin-2-yl)-5-aza-spiro[2.5]octane-8-carbonyl chloride (350 mg, yield: 100%). The crude product was directly used in the next step of the reaction. ES-API:[M+H]⁺= 323.1.

Step 4: 5-(4-acetamido-5-fluoropyrimidin-2-yl)-5-aza-spiro[2.5]octane-8-carbonyl chloride (200 mg, 0.61 mmol ) was dissolved in dichloromethane (5 mL). Then, N,N-diisopropylethylamine (0.5 mL) and 5-(3,5-difluorophenyl)-4,5-dihydro-1H-pyrazole (200 mg, 1.0 mmol) were added. The reaction was carried out at the room temperature for 2 h. The resultant reaction mixture was extracted with dichloromethane, dried, and concentrated at a reduced pressure to give a crude product of N-(2-(8-(5-(3,5-difluorophenyl)-4,5-dihydro-1H-pyrazole-carbonyl-5-aza-spiro[2.5]oct-5-yl)-5-fluoropyrimidin-4-yl)acetamide (400 mg, yield: 100%). The crude product was directly used in the next step of the reaction. ES-API:[M+H]⁺= 473.2.

Step 5: N-(2-(8-(5-(3,5-difluorophenyl)-4,5-dihydro-1H-pyrazole-carbonyl-5-aza-spiro[2.5]oct-5-yl)-5-fluoropyrimidin-4-yl) acetamide (400 mg, 0.84 mmol) was dissolved in methanol/water (4mL/2mL). Then, sodium hydroxide (120 mg, 3 mmol) was added. The reaction was carried out at the room temperature overnight. The resultant reaction mixtue was extracted with ethyl acetate, dried, and concentrated at a reduced pressure. The crude product was purified by thin layer chromatography (petroleum ether/ethyl acetate=1/1, Rf: 0.3) to give 5-(4-amino-5-fluoropyrimidin-2-yl)-5-aza-spiro[2.5]oct-8-yl)(5-(3,5-difluorophenyl)-4,5-dihydro-1H-pyrazol-1-yl) ketone (Z-45, 5.3 mg, yield: 4%, LCMS retention time: 1.46 min). ES-API:[M+H]⁺= 431.1. ¹H NMR (400 MHz, CDCl₃) δ 7.77 (s, 1H), 6.92 (s, 1H), 6.76 - 6.65 (m, 3H), 5.33 (dd, *J* = 12.0, 4.8 Hz, 1H), 4.69 (s, 2H), 4.05 - 3.93 (m, 1H), 3.85 - 3.66 (m, 2H), 3.56 - 3.34 (m, 2H), 3.17 (s, 1H), 2.76 (dd, *J* = 19.0, 5.1 Hz, 1H), 1.93 (d, *J* = 5.2 Hz, 2H), 0.64 - 0.44 (m, 3H), 0.37 (d, *J* = 6.7 Hz, 1H).

### Example 42 Preparation of 2-(8-(5-(3,5-difluorophenyl)-4,5-dihydro-1H-pyrazole-1-carbonyl)-5-aza-spiro[2.5] oct-5-yl)pyrimidine-4-carbonitrile (racemate, Z-46-1) and 2-(8-(5-(3,5-difluorophenyl)-4,5-dihydro-1H-pyrazole-1-carbonyl)-5-aza-spiro [2.5] oct-5-yl)pyrimidine-4-carbonitrile (racemate, Z-46-2)

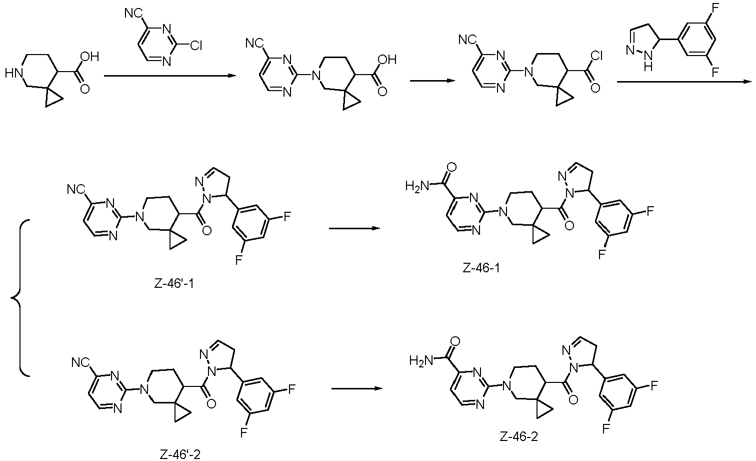

Step 1: The crude product of 5-aza-spiro[2.5]octane-8-carboxylic acid (200 mg, 1.28 mmoL), potassium carbonate (532 mg, 5.2 mmoL) and 2-fluoropyrimidine-4-carbonitrile (179 mg,1.28 mmol) were added to 5 mL of N, N-dimethylformamide. The mixture was stirred at 50°C for 2 h. Thereafter, 10 mL of water was added to the reaction mixture. The resultant mixture was back-extracted with ethyl acetate/ petroleum ether=3/1. The aqueous phase was adjusted to have a pH value of 5-6, and was extracted with ethyl acetate. The organic phase was washed with water, dried over anhydrous sodium sulfate, and concentrated at a reduced pressure to give 5-(4-cyanopyrimidin-2-yl)-5-aza-spiro[2.5]octane-8-carboxylic acid (395 mg, crude product). ES-API: [M+H]⁺ =259.1.

Step 2: 5-(4-cyanopyrimidin-2-yl)-5-aza-spiro[2.5]octane-8-carboxylic acid (200 mg, 0.77 mmol) was dissolved in dichloromethane (5 mL). Then, thionyl chloride (461 mg, 11.63 mmol) was added. The reaction was carried out at 20°C for 1 h. The resultant reaction mixture was spin-dried to remove the solvent to give 5-(4-cyanopyrimidin-2-yl)-5-aza-spiro[2.5]octane-8-carbonyl chloride (200 mg, crude product).

Step 3: 5-(4-cyanopyrimidin-2-yl)-5-aza-spiro[2.5]octane-8-carbonyl chloride (200 mg, crude product) was dissolved in dichloromethane (2 mL). The solution was added dropwise to a solution of 3-5-(3,5-difluorophenyl)-4,5-dihydro-1H-pyrazole (180 mg,0.99 mmol), N,N-diisopropylethylamine (460 mg, 3.9 mmol) in dichloromethane (1 mL). The reaction was carried out at the room temperature for 16 h. Thereafter, ethyl acetate (20 mL) was added. The resultant mixture was washed with water twice (20 mL^{∗}2). The organic phases were combined, dried over sodium sulfate, concentrated at a reduced pressure, and subjected to thin layer chromatography (petroleum ether/ethyl acetate=5/1) to give 2 raemates:
Z-46'-1: a racemate of 2-(8-(5-(3,5-difluorophenyl)-4,5-dihydro-1H-pyrazole-1-carbonyl)-5-aza-spiro[2.5]oct-5-yl)pyrimidine-4-carbonitrile; Rf: 0.5; (25 mg, yield: 7.6%); ES-API:[M+H]⁺= 423.1. and
Z-46'-2: a racemate of 2-(8-(5-(3,5-difluorophenyl)-4,5-dihydro-1H-pyrazole-1-carbonyl)-5-aza-spiro[2.5]oct-5-yl)pyrimidine-4-carbonitrile; Rf: 0.3; (30 mg, yield: 9.2%); ES-API:[M+H]⁺= 423.1.

Step 4: Z-46'-1 (25 mg, 0.06 mmol) was dissolved in water/acetonitrile (1.5 mL/0.5 mL). Sodium hydroxide (4 mg, 0.09 mmol) was added in an ice-water bath. Then, 30% hydrogen peroxide (0.5 mL) was added. The reaction was carried out at 20°C for 2 h. The organic phase was washed with a solution of sodium sulphite and a saline solution, dried over anhydrous sodium sulfate, concentrated at a reduced pressure, and subjected to thin layer chromatography (dichloromethane/methanol =50/1, Rf=0.6) to give Z-46-1 as a racemate: a racemate of 2-(8-(5-(3,5-difluorophenyl)-4,5-dihydro-1H-pyrazole-1-carbonyl)-5-aza-spiro[2.5]oct-5-yl)pyrimidine-4-carboxamide (white solid, 6.3 mg, yield: 24%). ES-API:[M+H]⁺= 441.1. ¹H NMR (400 MHz, CDCl₃) δ 8.46 (d, *J* = 4.7 Hz, 1H), 7.55 (s, 1H), 7.19 (d, *J* = 4.7 Hz, 1H), 6.95 (s, 1H), 6.75 - 6.63 (m, 3H), 5.62 (s, 1H), 5.35 (dd, *J* = 12.0, 4.9 Hz, 1H), 4.27 - 4.14 (m, 1H), 3.96 (d, *J* = 13.3 Hz, 1H), 3.75 - 3.59 (m, 2H), 3.44 (dd, *J* = 18.9, 12.1 Hz, 1H), 3.16 (d, *J* = 5.2 Hz, 1H), 2.77 (dd, *J* = 18.8, 4.9 Hz, 1H), 2.04 (p, *J* = 5.3, 4.9 Hz, 2H), 0.56 (q, *J* = 10.3 Hz, 3H), 0.37 - 0.28 (m, 1H).

Step 5: Z-46'-2 (30 mg, 0.07 mmol) was added to water/acetonitrile (1.5 mL/0.5 mL). Sodium hydroxide (4.3 mg, 0.1 mmol) was added in an ice-water bath. Then, 30% hydrogen peroxide (0.5 mL) was added. The reaction was carried out at 20°C for 2 h. The organic phase was washed with a solution of sodium sulphite and a saline solution, dried over anhydrous sodium sulfate, concentrated at a reduced pressure, and subjected to thin layer chromatography (dichloromethane/methanol =50/1, Rf=0.4) to give Z-46-2 as a racemate: a racemate of 2-(8-(5-(3,5-difluorophenyl)-4,5-dihydro-1H-pyrazole-1-carbonyl)-5-aza-spiro[2.5]oct-5-yl)pyrimidine-4-carboxamide (white solid, 1.3 mg, Y: 4%). ES-API:[M+H]⁺=441.1. ¹H NMR (400 MHz, CDCl₃) δ 8.46 (d, *J* = 4.7 Hz, 1H), 7.55 (s, 1H), 7.19 (d, *J* = 4.7 Hz, 1H), 6.95 (s, 1H), 6.75 - 6.63 (m, 3H), 5.62 (s, 1H), 5.35 (dd, *J* = 12.0, 4.9 Hz, 1H), 4.27 - 4.14 (m, 1H), 3.96 (d, *J* = 13.3 Hz, 1H), 3.75 - 3.59 (m, 2H), 3.44 (dd, *J* = 18.9, 12.1 Hz, 1H), 3.16 (d, *J* = 5.2 Hz, 1H), 2.77 (dd, *J* = 18.8, 4.9 Hz, 1H), 2.04 (p, *J* = 5.3, 4.9 Hz, 2H), 0.56 (q, *J* = 10.3 Hz, 3H), 0.37 - 0.28 (m, 1H).

### Example 43 Preparation of 6-(4-(5-(3,5difluorophenyl)4,5-dihydro-1H-pyrazole-1-carbonyl)-4,7-diaza-spiro[2.5]octan-7-yl)pyrimidine-4-carbonitrile (Z-47)

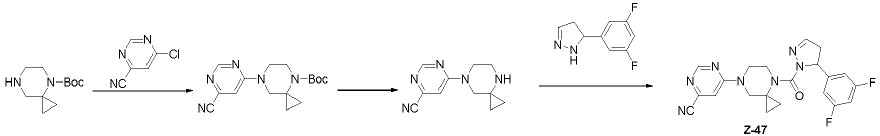

Step 1: tert-butyl 4,7-diaza-spiro[2.5]octane-4-carboxylate (250 mg, 1.18 mmol) was dissolved in 12 mL of acetonitrile. Potassium carbonate (488 mg, 3.54 mmol) and 6-cyano-4-chloropyrimidine (197 mg, 1.42 mmol) were added. The reaction was carried out at 50°C overnight. The resultant reaction mixture was extracted with dichloromethane, dried, concentrated at a reduced pressure to give tert-butyl 7-(6-cyanopyrimidin-4-yl)-4,7-diaza-spiro[2.5]octane-4-carboxylate (400 mg, crude product), which was directly used in the next step of the reaction. ES-API:[M+H]⁺= 316.1.

Step 2: tert-butyl 7-(6-cyanopyrimidin-4-yl)-4,7-diaza-spiro[2.5]octane-4-carboxylate (140 mg, 0.46 mmol) was dissolved in dichloromethane (5 mL). Trifluoroacetic acid (2 mL) was added. The mixture was stirred at the room temperature for reaction for 0.2 h. The resultant reaction mixture was concentrated at a reduced pressure to give 6-(4,7-diaza-spiro[2.5]octan-7-yl)pyrimidine-4-carbonitrile (100 mg, yield: crude product), which was directly used in the next step of the reaction. ES-API:[M+H]⁺= 216.1.

Step 3: carbonyldiimidazole (45 mg, 0.27 mmol) was dissolved in super anhydrous dioxane (3 mL) under an atmosphere of nigrogen gas. Then, 5-(3,5-difluorophenyl)-4,5-dihydro-1H-pyrazole (50 mg, 0.27 mmol) was added. The mixture was stirred at the room temperature for reaction for 0.5 h. A solution of 6-(4,7-diaza-spiro[2.5]octan-7-yl)pyrimidine-4-carbonitrile (50 mg, 0.23 mmol) and triethylamine (34 mg, 0.34 mmol) in dioxane (3 mL) was added. Thereafter, the reaction was carried out at 100°C overnight. The resultant reaction mixture was extracted with ethyl acetate, dried, and concentrated at a reduced pressure. The crude product was subjected to thin layer chromatography (petroleum ether/ethyl acetate=1/1, Rf=0.4) to give 6-(4-(5-(3,5-difluorophenyl)4,5-dihydro-1H-pyrazole-1-carbonyl)-4,7-diaza-spiro[2.5]octan-7-yl)pyrimidine-4-carbonitrile (Z-47, 3.6 mg, yield: 3.6%, LCMS retention time: 1.78 min). ¹H NMR (400 MHz, CDCl₃) δ 8.57 (s, 1H), 6.87 (s, 1H), 6.74 (dd, *J* = 20.8, 8.8 Hz, 4H), 5.29 (dd, *J* = 11.7, 9.1 Hz, 1H), 4.25 (d, *J* = 13.9 Hz, 1H), 3.75 (d, *J* = 12.4 Hz, 1H), 3.53 (t, *J* = 11.6 Hz, 2H), 3.43 - 3.17 (m, 1H), 2.73 (dd, *J* = 18.6, 8.9 Hz, 2H), 1.21 (d, *J* = 31.9 Hz, 5H), 1.06 (d, *J =* 8.5 Hz, 2H), 0.72 (dd, *J* = 17.3, 9.4 Hz, 2H).

### Example 44 Preparation of 6-(8-(5-(5-fluoropyridin-3-yl)-4,5-dihydro-1H-pyrazole-1-carbonyl)-5-aza-spiro[2.5]oct-5-yl)pyrimidine-4-carbonitrile (racemate, Z-48-1) and 6-(8-(5-(5-fluoropyridin-3-yl)-4,5-dihydro-1H-pyrazole-1-carbonyl)-5-aza-spiro[2.5]oct-5-yl)pyrimidine-4-carbonitrile (racemate, Z-48-2)

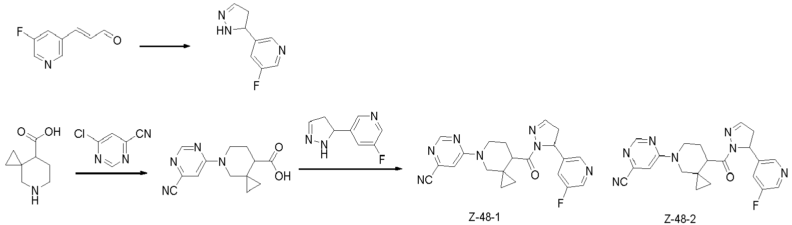

Step 1: hydrazine hydrate (869 mg, 13.91 mmol) was dissolved in ethanol (20 mL). Then, acetic acid (1.19 g, 19.86 mmol) was added. The mixture was added to 45°C. 3-(5-Fluoropyridin-3-yl)acraldehyde (2 g, 13.24 mmol) was added in batches. The mixture was heated to 100°C for reaction for 16 h. The resultant reaction mixture was cooled, concentrated at a reduced pressure to romove the solvent. The residue was dissolved in dichloromethane. Sodium bicarbonate was added to adjust the pH to 7-8. The organic phase was washed with saturated saline solution, dried over anhydrous sodium sulfate, and concentrated at a reduced pressure to give 3-(4,5-dihydro-1H-pyrazol-5-yl)-5-fluoropyridine (2.2 g, crude product). ES-API:[M+H]⁺= 166.1.

Step 2: 5-aza-spiro[2.5]octane-8-carboxylic acid (2 g, 12.9 mmol), 6-chloropyrimidine-4-carbonitrile (1.79 g,12.9 mmol) and potassium carbonate (3.56 g, 25.8 mmol) were dissolved in N,N-dimethyl formamide (20 mL). The mixture was heated to 50°C for reaction for 2 h. The resultant reaction mixture was cooled to the room temperature. Then, water was added to the reaction mixture. The resultant mixture was back-extracted with ethyl acetate/ petroleum ether=3/1. The aqueous phase was adjusted to have a pH value of 5-6, and was extracted with ethyl acetate. The organic phase was washed with saline, dried over anhydrous sodium sulfate, and concentrated at a reduced pressure to give 5-(6-cyanopyrimidin-4-yl)-5-aza-spiro[2.5]octane-8-carboxylic acid (2.05 g, 61.6%). ES-API:[M+H]⁺= 259.1.

Step 3: 5-(6-cyanopyrimidin-4-yl)-5-aza-spiro[2.5]octane-8-carboxylic acid (600 mg, 2.33 mmol) was dissolved in dichloromethane (6 mL). Then, thionyl chloride (1.38 g, 11.63 mmol) was added. The reaction was carried out at 20°C for 1 h. The resultant reaction mixture was spin-dried to remove the solvent. The residue was dissolved in dichloromethane. The resultant solution was added dropwise to a solution of 3-(4,5-dihydro-1H-pyrazol-5-yl)-5-fluoropyridine (384 mg, 2.33 mmol) and N,N-diisopropylethylamine (601 mg, 4.66 mmol) in dichloromethane. The reaction was carried out for 1 h. The organic phase was washed with 0.5N diluted hydrochloric acid, washed with saline, dried over anhydrous sodium sulfate, and concentrated at a reduced pressure. The crude product of 6-(8-(5-(5-fluoropyridin-3-yl)-4,5-dihydro-1H-pyrazole-1-carbonyl)-5-aza-spiro[2.5]oct-5-yl)pyrimidine-4-carbonitrile was subjected to thin layer chromatography (ethyl acetate=100%) to give 2 racemates:
Z-48-1 (the collected fraction having a Rf of 0.5): a racemate of 6-(8-(5-(5-fluoropyridin-3-yl)-4,5-dihydro-1H-pyrazole-1-carbonyl)-5-aza-spiro[2.5]oct-5-yl)pyrimidine-4-carbonitrile (36 mg, yield: 3.8%, LCMS retention time: 1.70 min). ¹H NMR (400 MHz, CDCl₃) δ 8.51 (s, 1H), 8.41 (s, 1H), 8.31 (s, 1H), 7.20 (s, 1H), 7.01 (s, 1H), 6.75 (s, 1H), 5.43 (dd, *J =* 12.2, 5.0 Hz, 1H), 3.91 (d, *J* = 13.0 Hz, 1H), 3.52 (dd, *J* = 18.9, 12.0 Hz, 2H), 3.11 (s, 1H), 2.85 (dd, *J* = 18.4, 5.0 Hz, 1H), 2.03 (s, 2H), 0.63 (d, *J* = 9.2 Hz, 1H), 0.56 (s, 2H), 0.39 (s, 1H).
Z-48-2 (the collected fraction having a Rf of 0.4): another racemate of 6-(8-(5-(5-fluoropyridin-3-yl)-4,5-dihydro-1H-pyrazole-1-carbonyl)-5-aza-spiro[2.5]oct-5-yl)pyrimidine-4-carbonitrile (32 mg, yield: 3.4%, LCMS retention time: 1.71 min), ES-API:[M+H]⁺= 406.2. ¹H NMR (400 MHz, CDCl₃) δ 8.51 (s, 1H), 8.41 (s, 1H), 8.33 (s, 1H), 7.21 (d, *J* = 8.9 Hz, 1H), 7.00 (s, 1H), 6.75 (s, 1H), 5.43 (dd, *J* = 12.1, 5.2 Hz, 1H), 3.77 (s, 3H), 3.52 (dd, *J* = 18.9, 12.2 Hz, 1H), 3.14 (d, *J* = 5.1 Hz, 1H), 2.85 (dd, *J* = 18.8, 5.2 Hz, 1H), 2.14 - 1.92 (m, 2H), 0.66 (d, *J* = 9.3 Hz, 1H), 0.55 (s, 2H), 0.43 (s, 1H).

### Example 44.1 Preparation of 6-((R)-8-((S)-(5-(5-fluoropyridin-3-yl)-4,5-dihydro-1H-pyrazole-1-carbonyl)-5-aza-spiro[2.5]oct-5-yl)pyrimidine-4-carbonitrile (Z-48-a), 6-((R)-8-((R)-(5-(5-fluoropyridin-3-yl)-4,5-dihydro-1H-pyrazole-1-carbonyl)-5-aza-spiro[2.5]oct-5-yl)pyrimidine-4-carbonitrile (Z-48-b), 6-((S)-8-((R)-(5-(5-fluoropyridin-3-yl)-4,5-dihydro-1H-pyrazole-1-carbonyl)-5-aza-spiro[2.5]oct-5-yl)pyrimidine-4-carbonitrile (Z-48-c), 6-((S)-8-((S)-(5-(5-fluoropyridin-3-yl)-4,5-dihydro-1H-pyrazole-1-carbonyl)-5-aza-spiro[2.5]oct-5-yl)pyrimidine-4-carbonitrile (Z-48-d)

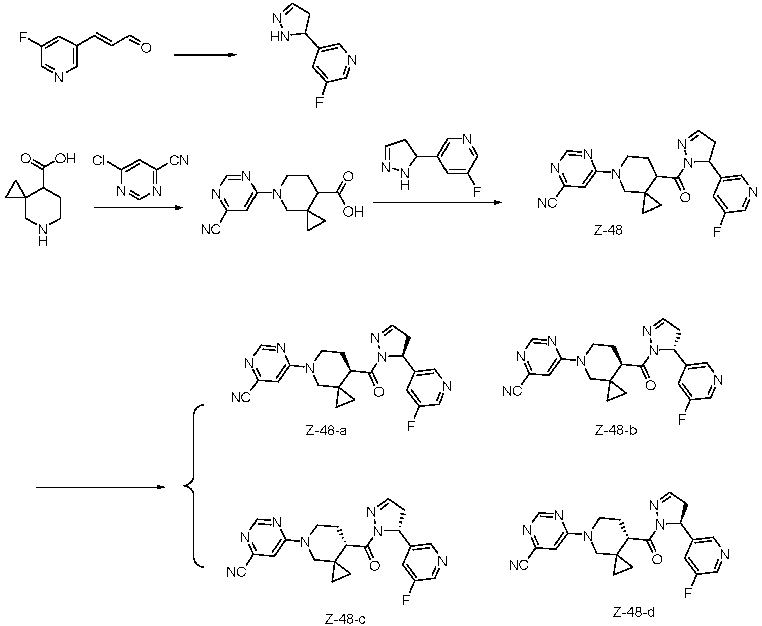

Step 1: hydrazine hydrate (869 mg, 13.91 mmol) was dissolved in ethanol (20 mL). Then, acetic acid (1.19 g, 19.86 mmol) was added. The mixture was heated to 45°C, to which 3-(5-fluoropyridin-3-yl)acraldehyde (2 g, 13.24 mmol) was added in batches. The mixture was heated to 100°C for reaction for 16 h. The resultant reaction mixture was cooled, concentrated at a reduced pressure to remove the solvent. The residue was dissolved in dichloromethane. Sodium bicarbonate was added to adjust the pH to 7-8. The organic phase was washed with saturated saline solution, dried over anhydrous sodium sulfate, and concentrated at a reduced pressure to give 3-(4,5-dihydro-1H-pyrazol-5-yl)-5-fluoropyridine (2.2 g, crude product). ES-API: [M+H]⁺= 166.1.

Step 2: 5-aza-spiro[2.5]octane-8-carboxylic acid (2 g, 12.9 mmol), 6-chloropyrimidine-4-carbonitrile (1.79 g,12.9 mmol) and potassium carbonate (3.56 g, 25.8 mmol) were dissolved in N,N-dimethyl formamide (20 mL). The mixture was heated to 50°C for reaction for 2 h. The resultant reaction mixture was cooled to the room temperature. Then, water was added to the reaction mixture. The resultant mixture was back-extracted with ethyl acetate/ petroleum ether=3/1. The aqueous phase was adjusted to have a pH value of 5-6, and was extracted with ethyl acetate. The organic phase was washed with saline, dried over anhydrous sodium sulfate, and concentrated at a reduced pressure to give 5-(6-cyanopyrimidin-4-yl)-5-aza-spiro[2.5]octane-8-carboxylic acid (2.05 g, 61.6%). ES-API:[M+H]⁺= 259.1.

Step 3: 5-(6-cyanopyrimidin-4-yl)-5-aza-spiro[2.5]octane-8-carboxylic acid (600 mg, 2.33 mmol) was dissolved in dichloromethane (6 mL). Then, thionyl chloride (1.38 g, 11.63 mmol) was added. The reaction was carried out at 20°C for 1 h. The resultant reaction mixture was spin-dried to remove the solvent. The residue was dissolved in dichloromethane. The resultant solution was added dropwise to a solution of 3-(4,5-dihydro-1H-pyrazol-5-yl)-5-fluoropyridine (384 mg, 2.33 mmol) and N,N-diisopropylethylamine (601 mg, 4.66 mmol) in dichloromethane. The reaction was carried out at the room temperature for 1 h. The organic phase was washed with 0.5N diluted hydrochloric acid, washed with saline, dried over anhydrous sodium sulfate, and concentrated at a reduced pressure. The crude product was purified to give 6-(8-(5-(5-fluoropyridin-3-yl)-4,5-dihydro-1H-pyrazole-1-carbonyl)-5-aza-spiro[2.5]oct-5-yl)pyrimidine-4-carbonitrile (Z-48, 150 mg, yield: 15.9%, LCMS retention time: 1.63 min). ES-API: [M+H]⁺= 406.1. HY1107, ¹H NMR (400 MHz, CDCl₃) δ 8.51 (s, 1H), 8.41 (s, 1H), 8.31 (s, 1H), 7.20 (s, 1H), 7.01 (s, 1H), 6.75 (s, 1H), 5.43 (dd, *J* = 12.2, 5.0 Hz, 1H), 3.91 (d, *J* = 13.0 Hz, 1H), 3.52 (dd, *J* = 18.9, 12.0 Hz, 2H), 3.11 (s, 1H), 2.85 (dd, *J* = 18.4, 5.0 Hz, 1H), 2.03 (s, 2H), 0.63 (d, *J* = 9.2 Hz, 1H), 0.56 (s, 2H), 0.39 (s, 1H).

Step 4: the Z-48 (125 mg) as obtained above was subjected to a chiral resolution (mobile phase: Hex:EtOH:AMMN=80:20:0.2); column: Column: IB (4.6^{∗}250mm 5um) ; flow rate: 1.0 ml/min; column temperature: 30°C) to give 4 isomers having a single configuration:
Isomer Z-48-a (retention: 11.823 min): arbitrarily assigned as 6-((R)-8-((S)-(5-(5-fluoropyridin-3-yl)-4,5-dihydro-1H-pyrazole-1-carbonyl)-5-aza-spiro[2.5]oct-5-yl)pyrimidine-4-carbonitrile (33 mg, purity: 100%, ee value: 100%)). ES-API:[M+H]⁺= 406.1.
Isomer Z-48-b (retention time: 13.177 min): arbitrarily assigned as 6-((R)-8-((R)-(5-(5-fluoropyridin-3-yl)-4,5-dihydro-1H-pyrazole-1-carbonyl)-5-aza-spiro[2.5]oct-5-yl)pyrimidine-4-carbonitrile (30 mg, purity: 93%, ee value: 98.3%). ES-API:[M+H]⁺= 406.1.
Isomer Z-48-c (retention time: 14.566 min): arbitrarily assigned as 6-((S)-8-((R)-(5-(5-fluoropyridin-3-yl)-4,5-dihydro-1H-pyrazole-1-carbonyl)-5-aza-spiro[2.5]oct-5-yl)pyrimidine-4-carbonitrile (4.0 mg, purity: 100%, ee value: 98.9%). ES-API:[M+H]⁺= 406.1.
Isomer Z-48-d (retention time: 15.122 min): arbitrarily assigned as 6-((S)-8-((S)-(5-(5-fluoropyridin-3-yl)-4,5-dihydro-1H-pyrazole-1-carbonyl)-5-aza-spiro[2.5]oct-5-yl)pyrimidine-4-carbonitrile (51 mg, purity: 100%, ee value: 99.5%). ES-API:[M+H]⁺= 406.1.

### Example 45 Preparation of 6-(8-(5-(5-fluoropyridin-3-yl)-4,5-dihydro-1H-pyrazole-1-carbonyl)-5-aza-spiro[2.5]oct-5-yl)pyrimidine-4-carboxamide (racemate, Z-49-1) and 6-(8-(5-(5-fluoropyridin-3-yl)-4,5-dihydro-1H-pyrazole-1-carbonyl)-5-aza-spiro[2.5]oct-5-yl)pyrimidine-4-carboxamide (racemate, Z-49-2)

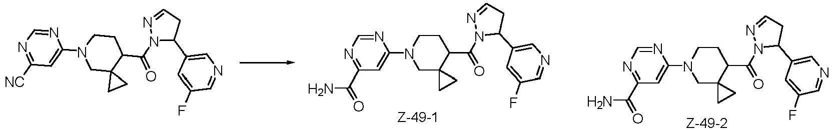

Step 1: the crude product of 6-(8-(5-(5-fluoropyridin-3-yl)-4,5-dihydro-1H-pyrazole-1-carbonyl)-5-aza-spiro[2.5]oct-5-yl)pyrimidine-4-carbonitrile (33 mg, 0.081 mmol) was dissolved in water/acetonitrile (1.5/0.5 mL). Sodium hydroxide (3.6 mg, 0.09 mmol) was added in an ice-water bath. Then, 30% hydrogen peroxide (0.5 mL) was added. The reaction was carried out at 20°C for 2 h. The organic phase was washed with a solution of sodium sulphite and a saline solution, dried over anhydrous sodium sulfate, concentrated at a reduced pressure, and subjected to thin layer chromatography (dichloromethane/methanol =10/1) to give 2 racemates:
Z-49-1 (the collected fraction having a Rf of 0.4): a recemate of 6-(8-(5-(5-fluoropyridin-3-yl)-4,5-dihydro-1H-pyrazole-1-carbonyl)-5-aza-spiro[2.5]oct-5-yl)pyrimidine-4-carboxamide (15.2 mg, yield: 44.3%, LCMS retention time: 1.47 min), ES-API:[M+H]⁺= 424.2. ¹H NMR (400 MHz, CDCl₃) δ 8.47 (s, 1H), 8.39 (d, *J* = 2.7 Hz, 1H), 8.30 (s, 1H), 7.80 (d, *J* = 4.5 Hz, 1H), 7.27 (s, 1H), 7.18 (d, *J* = 8.8 Hz, 1H), 6.99 (s, 1H), 5.79 (d, *J* = 4.7 Hz, 1H), 5.42 (dd, *J* = 12.1, 5.0 Hz, 1H), 3.90 (d, *J* = 13.2 Hz, 2H), 3.54 (ddd, *J* = 31.0, 16.1, 9.4 Hz, 3H), 3.12 (t, *J* = 4.9 Hz, 1H), 2.83 (dd, *J* = 19.0, 5.0 Hz, 1H), 2.02 (dt, *J* = 8.1, 4.6 Hz, 2H), 0.57 (q, *J* = 8.2, 5.9 Hz, 3H), 0.34 (d, *J* = 8.6 Hz, 1H).
Z-49-2 (the collected fraction having a Rf of 0.3): another racemate of 6-(8-(5-(5-fluoropyridin-3-yl)-4,5-dihydro-1H-pyrazole-1-carbonyl)-5-aza-spiro[2.5]oct-5-yl)pyrimidine-4-carboxamide (14.5 mg, yield: 49.6%, LCMS retention time: 1.48 min), ES-API:[M+H]⁺= 424.2. ¹H NMR (400 MHz, CDCl₃) δ 8.48 (s, 1H), 8.40 (d, *J* = 2.8 Hz, 1H), 8.32 (s, 1H), 7.81 (s, 1H), 7.28 (s, 1H), 7.20 (d, *J* = 9.0 Hz, 1H), 6.99 (s, 1H), 5.70 (s, 1H), 5.43 (dd, *J =* 12.1, 5.2 Hz, 1H), 3.79 (s, 3H), 3.51 (dd, *J* = 18.8, 12.1 Hz, 2H), 3.14 (d, *J* = 5.0 Hz, 1H), 2.84 (dd, *J* = 18.9, 5.2 Hz, 1H), 1.99 (p, *J* = 4.8 Hz, 2H), 0.56 (tdd, *J* = 18.0, 9.6, 5.7 Hz, 3H), 0.39 (q, *J* = 7.5, 6.7 Hz, 1H).

### Examples 46-47

The compounds Z-50 to Z-51 can be prepared according to a method similar to that used in Example 24, wherein the starting materials for each of the compounds is commercially available, or may be prepared according to a prior art method well-known to a skilled person in the art. Similar methods for preparing an intermediate may be readily obtained by a skilled person according to the prior art methods.

| Example No. | No. | Structural formula | MS (M+H)⁺ |
|---|---|---|---|
| 46 | Z-50 | 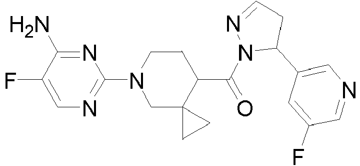 | 414.1 |
| 47 | Z-51 | 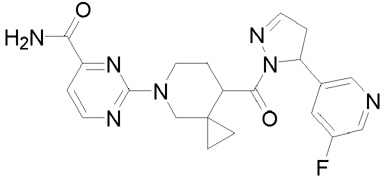 | 424.1 |

**Example 48 Preparation of 6-(8-(5-(2,5-difluorophenyl)-4,5-dihydro-1H-pyrazole-1-carbonyl)-5-aza-spiro[2.5]oct-5-yl)pyrimidine-4-carbonitrile (racemate, Z-52-1)** 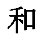 **6-(8-(5-(2,5-difluorophenyl)-4,5-dihydro-1H-pyrazole-1-carbonyl)-5-aza-spiro[2.5]oct-5-yl)pyrimidine-4-carbonitrile (racemate, Z-52-2)**

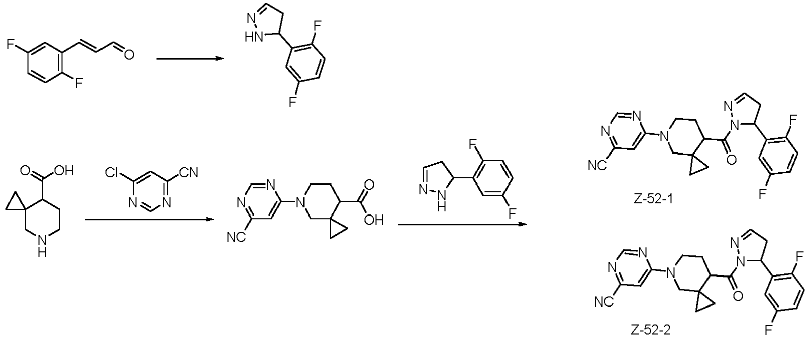

Step 1: hydrazine hydrate (195 mg, 3.12 mmol) was dissolved in tert-butanol (2 mL). The solution was heated to 70°C. A solution of 3-(2,5-difluorophenyl)acraldehyde (500 mg, 2.98 mmol) in tert-butanol (3 mL) was added dropwise. The reaction was carried out at 70°C for 4 h. The resultant reaction mixture was cooled, concentrated at a reduced pressure to remove the solvent to give 5-(2,5-difluorophenyl)-4,5-dihydro-1H-pyrazole (550 mg, crude product). ES-API:[M+H]⁺= 183.1.

Step 2: 5-aza-spiro[2.5]octane-8-carboxylic acid (2 g, 12.9 mmol), 6-chloropyrimidine-4-carbonitrile (1.79 g,12.9 mmol) and potassium carbonate (3.56 g, 25.8 mmol) was dissolved in N,N-dimethyl formamide (20 mL). The solution was heated to 50°C for reaction for 2 h. The resultant reaction mixture was cooled to the room temperature. Then, water was added. The resultant mixture was back-extracted with ethyl acetate/ petroleum ether=3/1. The aqueous phase was adjusted to have a pH value of 5-6, and was extracted with ethyl acetate. The organic phase was washed with saline, dried over anhydrous sodium sulfate and concentrated at a reduced pressure. The crude product was purified to give 5-(6-cyanopyrimidin-4-yl)-5-aza-spiro[2.5]octane-8-carboxylic acid (2.05 g, 61.6%). ES-API:[M+H]⁺= 259.1.

Step 3: 5-(6-cyanopyrimidin-4-yl)-5-aza-spiro[2.5]octane-8-carboxylic acid (500 mg, 1.94 mmol) was dissolved in dichloromethane (6 mL). Then, thionyl chloride (1.15 g, 9.69 mmol) was added. The reaction was carried out at 20°C for 1 h. The resultant reaction mixture was spin-dried to remove the solvent. The residue was dissolved in dichloromethane. The resultant solution was added dropwise to a solution of 5-(2,5-difluorophenyl)-4,5-dihydro-1H-pyrazole (550 mg, 3.02 mmol) and N,N-diisopropylethylamine (501 mg, 3.88 mmol) in dichloromethane. The reaction was carried out for 1 h. The organic phase was washed with 0.5N diluted hydrochloric acid, washed with saline, dried over anhydrous sodium sulfate, and concentrated at a reduced pressure. The crude product of 6-(8-(5-(2,5-difluorophenyl)-4,5-dihydro-1H-pyrazole-1-carbonyl)-5-aza-spiro[2.5]oct-5-yl)pyrimidine-4-carbonitrile was subjected to thin layer chromatography (ethyl acetate=1/1) to give 2 racemates:
Z-52-1 (the collected portion having a Rf of 0.5): a racemate of 6-(8-(5-(2,5-difluorophenyl)-4,5-dihydro-1H-pyrazole-1-carbonyl)-5-aza-spiro[2.5]oct-5-yl)pyrimidine-4-carbonitrile; (6.6 mg, yield: 0.81%, LCMS retention time: 1.85 min). ¹H NMR (400 MHz, CDCl₃) δ 8.51 (s, 1H), 7.09 - 6.90 (m, 3H), 6.75 (s, 2H), 5.55 (dd, *J* = 12.2, 5.2 Hz, 1H), 3.95 (d, *J* = 13.0 Hz, 1H), 3.56 - 3.40 (m, 2H), 3.11 (s, 1H), 2.82 (dd, *J* = 19.1, 5.2 Hz, 1H), 2.20 - 1.89 (m, 2H), 1.25 (s, 2H), 0.59 (d, *J* = 24.9 Hz, 3H), 0.38 (s, 1H).
Z-52-2 (the collected portion having a Rf of 0.4): another racemate of 6-(8-(5-(2,5-difluorophenyl)-4,5-dihydro-1H-pyrazole-1-carbonyl)-5-aza-spiro[2.5]oct-5-yl)pyrimidine-4-carbonitrile; (10.3 mg, yield: 1.26%, LCMS retention time: 1.84 min), ES-API:[M+H]⁺= 423.2. ¹H NMR (400 MHz, CDCl₃) δ 8.51 (s, 1H), 7.04 (q, *J* = 7.8, 6.8 Hz, 1H), 6.96 (s, 2H), 6.78 (d, *J* = 9.9 Hz, 2H), 5.55 (dd, *J* = 12.2, 5.3 Hz, 1H), 3.81 (s, 2H), 3.47 (dd, J = 19.0, 12.3 Hz, 1H), 3.16 (s, 1H), 2.82 (dd, J = 18.4, 5.3 Hz, 1H), 2.02 (s, 2H), 1.25 (s, 2H), 0.72 (d, *J* = 9.3 Hz, 1H), 0.57 (s, 2H), 0.47 (d, J = 8.0 Hz, 1H).

### Example 49 Preparation of 6-(8-(5-(2,5-difluorophenyl)-4,5-dihydro-1H-pyrazole-1-carbonyl)-5-aza-spiro[2.5]oct-5-yl)pyrimidine-4-carboxamide (racemate, Z-53-1) and 6-(8-(5-(2,5-difluorophenyl)-4,5-dihydro-1H-pyrazole-1-carbonyl)-5-aza-spiro[2.5]oct-5-yl)pyrimidine-4-carboxamide (racemate, Z-53-2)

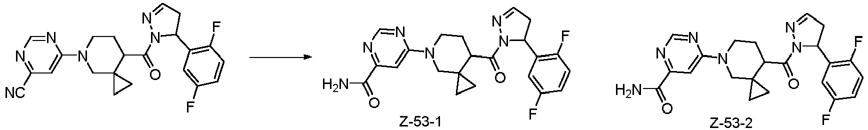

Step 1: The crude product of 6-(8-(5-(2,5-difluorophenyl)-4,5-dihydro-1H-pyrazole-1-carbonyl)-5-aza-spiro[2.5]oct-5-yl)pyrimidine-4-carbonitrile (5 mg, 0.012 mmol) was dissolved in water/acetonitrile (0.75/0.25 mL). Sodium hydroxide (0.5 mg, 0.013 mmol) was added in an ice-water bath. Then, 30% hydrogen peroxide (0.25 mL) was added. The reaction was carried out at 20°C for 2 h. The organic phase was washed with a solution of sodium sulphite and a saline solution, dried over anhydrous sodium sulfate, and concentrated at a reduced pressure. The crude product was subjected to thin layer chromatography (dichloromethane/methanol =10/1) to give 2 racemates:
Z-53-1 (the collected fraction having a Rf of 0.5): a racemate of 6-(8-(5-(2,5-difluorophenyl)-4,5-dihydro-1H-pyrazole-1-carbonyl)-5-aza-spiro[2.5]oct-5-yl)pyrimidine-4-carboxamide (LCMS retention time: 1.62 min); ES-API:[M+H]⁺= 441.2. ¹H NMR (400 MHz, CDCl₃) δ 8.49 (s, 1H), 7.27 (s, 2H),7.04-6.97 (m, 3H), 6.75 (s, 1H) 5.65 - 5.53 (m, 2H), 3.97 (m, 1H), 3.60 (m, 1H), 3.42 (m, 1H), 3.12 (m, 1H), 2.84 (m, 2H), 2.08 (m, 2H), 1.37 (m, 2H),0.88 (m, 1H), 0.62 (m, 2H), 0.37 (m, 1H).
Z-53-2 (the collected fraction having a Rf of 0.4): another racemate of 6-(8-(5-(2,5-difluorophenyl)-4,5-dihydro-1H-pyrazole-1-carbonyl)-5-aza-spiro[2.5]oct-5-yl)pyrimidine-4-carboxamide (LCMS retention time: 1.63 min); ES-API:[M+H]⁺= 441.2. ¹H NMR (400 MHz, CDCl₃) δ 8.48 (s, 1H), 6.96 (s, 2H), 5.64 - 5.52 (m, 2H), 3.86 (s, 2H), 3.47 (dd, *J* = 19.1, 12.2 Hz, 1H), 3.17 (s, 1H), 2.81 (d, *J* = 18.5 Hz, 1H), 2.02 (d, *J* = 5.9 Hz, 2H), 1.31 (d, *J* = 19.3 Hz, 2H), 0.69 (d, *J* = 8.9 Hz, 1H), 0.58 (s, 2H), 0.44 (s, 1H).

**Example 50** 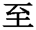 **54**

The compounds Z-54 to Z-55 can be prepared according to a method similar to that used in Example 24, and the compounds Z-56 to Z-58 can be prepared according to a method similar to that used in Example 8, wherein the starting materials for each of the compounds is commercially available, or may be prepared according to a prior art method well-known to a skilled person in the art. Similar methods for preparing an intermediate may be readily obtained by a skilled person according to the prior art methods.

| Example No. | No. | Structural formula | MS (M+H)⁺ |
|---|---|---|---|
| 50 | Z-54 | 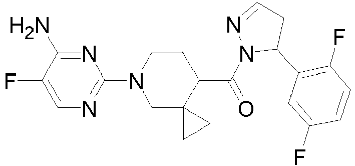 | 431.1 |
| 51 | Z-55 | 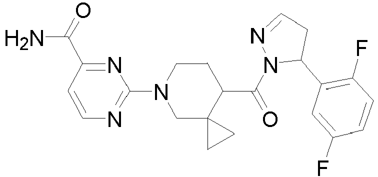 | 441.1 |

### Example 55 Preparation of 6-(8-(5-phenyl-4,5-dihydro-1H-pyrazole-1-carbonyl)-5-aza-spiro[2.5]oct-5-yl)pyrimidine-4-carbonitrile (racemate, Z-60-1) and 6-(8-(5-phenyl-4,5-dihydro-1H-pyrazole-1-carbonyl)-5-aza-spiro[2.5]oct-5-yl)pyrimidine-4-carbonitrile (racemate, Z-60-2)

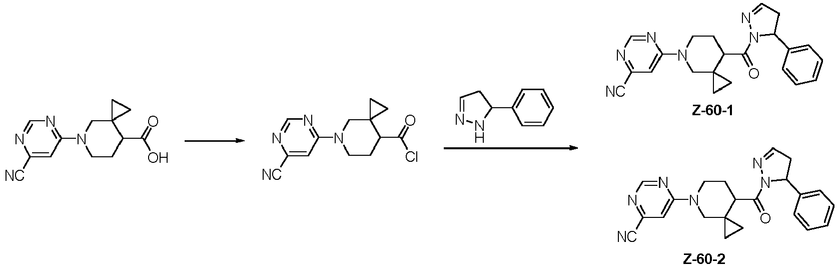

Step 1: 5-(6-cyanopyrimidin-4-yl)-5-aza-spiro[2.5]octane-8-carboxylic acid (500 mg, 1.93mmol) was dissolved in dichloromethane (10 mL). Then, sulfoxide chloride (3 mL) was added. The mixture was stirred at the room temperature for 0.5 h. The resultant reaction mixture was concentrated at a reduced pressure to give 5-(6-cyanopyrimidin-4-yl)-5-aza-spiro[2.5]octane-8-carbonyl chloride (700 mg, crude product), which was directly used in the next step of the reaction. ES-API: [M+H]⁺ =273.1 (quenched with methanol)

Step 2: 5-(6-cyanopyrimidin-4-yl)-5-aza-spiro[2.5]octane-8-carbonyl chloride (400 mg, 1 mmol, crude product, containing salt) was dissolved in dichloromethane (10 mL). The solution was added to a mixture of triethylamine (404 mg, 4 mmol) and 5-(3,5-difluorophenyl)-4,5-dihydro-1H-pyrazole (292 mg, 2.0 mmol). The mixture was stirred at the room temperature for 2h. The resultant reaction mixture was extracted with dichloromethane, dried, and concentrated at a reduced pressure. The crude product was subjected to thin layer chromatography (petroleum ether/ethyl acetate=2/3) to give 2 racemates:
Z-60-1 (the collected fraction having a Rf of 0.4): a racemate of 6-(8-(5-phenyl-4,5-dihydro-1H-pyrazole-1-carbonyl)-5-aza-spiro[2.5]oct-5-yl)pyrimidine-4-carbonitrile (30 mg, yield: 7.8%, LCMS retention time: 1.89 min); ES-API:[M+H]⁺ =387.2. ¹H NMR (400 MHz, CDCl₃) δ 8.52 (d, *J* = 16.0 Hz, 1H), 7.34 (t, *J* = 7.3 Hz, 3H), 7.17 (d, *J* = 7.5 Hz, 2H), 6.95 (s, 1H), 6.81 (d, *J* = 48.5 Hz, 1H), 5.38 (dd, *J =* 12.2, 5.0 Hz, 1H), 3.81 (s, 2H), 3.44 (dd, *J* = 18.9, 12.1 Hz, 1H), 3.18 (s, 1H), 2.84 (d, *J* = 19.3 Hz, 1H), 2.02 (s, 3H), 1.26 (s, 2H), 0.66 - 0.44 (m, 4H).
Z-60-2 (the collected fraction having a Rf of 0.5): another racemate of 6-(8-(5-phenyl-4,5-dihydro-1H-pyrazole-1-carbonyl)-5-aza-spiro[2.5]oct-5-yl)pyrimidine-4-carbonitrile (40 mg, yield: 10.3%, LCMS retention time: 1.81 min); ES-API:[M+H]⁺ =387.2. ¹H NMR (400 MHz, CDCl₃) δ 8.52 (d, *J* = 16.0 Hz, 1H), 7.34 (t, *J* = 7.3 Hz, 3H), 7.17 (d, *J* = 7.5 Hz, 2H), 6.95 (s, 1H), 6.81 (d, *J* = 48.5 Hz, 1H), 5.38 (dd, *J =* 12.2, 5.0 Hz, 1H), 3.81 (s, 2H), 3.44 (dd, *J* = 18.9, 12.1 Hz, 1H), 3.18 (s, 1H), 2.84 (d, *J* = 19.3 Hz, 1H), 2.02 (s, 3H), 1.26 (s, 2H), 0.66 - 0.44 (m, 4H).

### Example 56 Preparation of (5-(3,5-difluorophenyl)-4,5-dihydro-1H-pyrazol-1-yl)(5-(5-methyl-1,3,4-oxadizol-2-yl)-5-aza-spiro[2.5]octane-8-yl) ketone (Z-61)

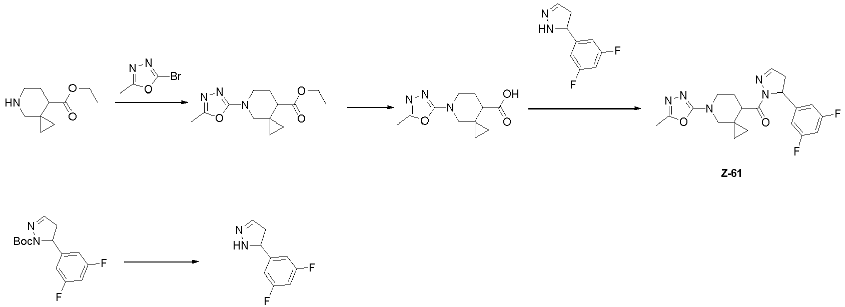

Step 1: ethyl 5-aza-spiro[2.5]octane-8-carboxylate (300 mg, 1.64 mmol), 2-bromo-5-methyl-1,3,4-oxadizole (266 mg, 1.64 mmol) and potassium carbonate (453 mg, 3.28 mmol) were dissolved in N,N-dimethyl formamide (3 mL). The solution was heated to 50°C for reaction for 2 h. The resultant reaction mixture was cooled to the room temperature. Water was added. The resultant mixture was extracted with ethyl acetate. The organic phase was washed with saline, dried over anhydrous sodium sulfate, and concentrated at a reduced pressure to give ethyl 5-(5-methyl-1,3,4-oxadizol-2-yl)-5-aza-spiro[2.5]octane-8-carboxylate (350 mg, 80.5%). ES-API: [M+H]⁺= 266.1.

Step 2: ethyl 5-(5-methyl-1,3,4-oxadizol-2-yl)-5-aza-spiro[2.5]octane-8-carboxylate (350 mg, 1.32 mmol) was dissolved in tetrahydrofuran/water (3 mL/3 mL). Then, lithium hydroxide monohydrate (111 mg, 2.64 mmol) was added. The mixture was heated to 40°C. The reaction was carried out for 0.5 h. The resultant reaction mixture was cooled, adjusted to have a pH of 5-6, and extracted with ethyl acetate. The organic phase was washed with saline, dried over sodium sulfate, and concentrated at a reduced pressure to remove the solvent to give 5-(5-methyl-1,3,4-oxadizol-2-yl)-5-aza-spiro[2.5]octane-8-carboxylic acid (170 mg, 53.6%). ES-API: [M+H]⁺= 238.1.

Step 3: tert-butyl 5-(3,5-difluorophenyl)-4,5-dihydro-1H-pyrazole-1-carboxylate (250 mg, 0.89 mmol) was dissolved in dichloromethane (3 mL). Then, trifluoroacetic acid (3 mL) was added. The reaction was carried out at the room temperature for 0.5 h. The resultant reaction mixture was concentrated at a reduced pressure to remove the solvent to give a crude product of 5-(3,5-difluorophenyl)-4,5-dihydro-1H-pyrazole (250 mg, 100%). ES-API:[M+H]⁺= 183.1.

Step 4: 5-(5-methyl-1,3,4-oxadizol-2-yl)-5-aza-spiro[2.5]octane-8-carboxylic acid (170 mg, 0.72 mmol) was dissolved in dichloromethane (2 mL). Then, thionyl chloride (428 mg,3.6 mmol) was added. The reaction was carried out at 20°C for 1 h. The resultant reaction mixture was spin-dried to remove the solvent. The residue was dissolved in dichloromethane. The resultant solution was added dropwise to a solution of 5-(3,5-difluorophenyl)-4,5-dihydro-1H-pyrazole (250 mg, 1.37 mmol) and N,N-diisopropylethylamine (186 mg,1.44 mmol) in dichloromethane. The reaction was carried out at the room temperature for 1 h. The organic phase was washed with 0.5N diluted hydrochloric acid, washed with saline, dried over anhydrous sodium sulfate, and concentrated at a reduced pressure. The crude product was subjected to preparative HPLC to give (5-(3,5-difluorophenyl)-4,5-dihydro-1H-pyrazol-1-yl)(5-(5-methyl-1,3,4-oxadizo1-2-yl)-5-aza-spiro[2.5]octane-8-yl) ketone (Z-61, 4.4 mg, yield: 1.24%), ES-API:[M+H]⁺= 402.2. ¹H NMR (400 MHz, CDCl₃) δ 6.94 (s, 1H), 6.69 (p, *J* = 9.2 Hz, 3H), 5.40 - 5.26 (m, 1H), 3.84 (d, *J* = 23.9 Hz, 1H), 3.79 - 3.66 (m, 2H), 3.46 (td, *J* = 21.3, 18.8, 10.8 Hz, 1H), 3.19 - 3.00 (m, 2H), 2.83 - 2.73 (m, 1H), 2.36 (s, 3H), 2.03 (d, *J* = 30.7 Hz, 2H), 0.73 - 0.56 (m, 2H), 0.56 - 0.49 (m, 1H), 0.45 (s, 1H).

### Example 57 Preparation of 6-(7-(5-(3,5-difluorophenyl)-4,5-dihydro-1H-pyrazole-1-carbonyl)-4-aza-spiro[2.5]oct-4-yl)pyrimidine-4-carbonitrile (Z-62)

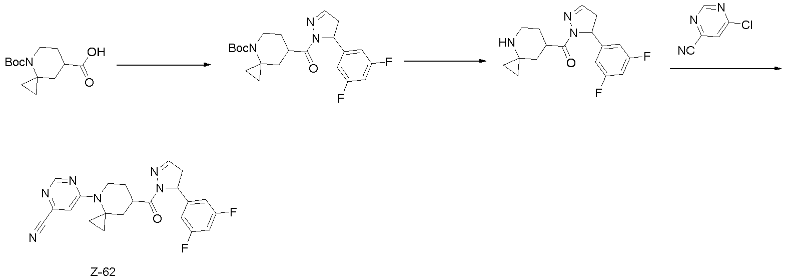

Step 1: 4-(tert-butoxycarbonyl)-4-aza-spiro[2.5]octane-7-carboxylic acid (50 mg, 0.196 mmol), 5-(3,5-difluorophenyl)-4,5-dihydro-1H-pyrazole (36 mg,0.196 mmol), N,N-diisopropylethylamine (63 mg, 0.49 mmol) and 2-(7-azabenzotriazole)-N,N,N',N'-tetramethyluronium hexafluorophosphate (89 mg, 0.235 mmol) was dissolved in N,N-dimethyl formamide (1 mL). The reaction was carried out at 20°C for 16 h. The resultant reaction mixture was extracted with ethyl acetate, washed with 0.5 N diluted hydrochloric acid, washed with a saturated saline solution, dried over anhydrous sodium sulfate, spin-dried to remove the solvent, and subjected to thin layer chromatography (petroleum ether/ethyl acetate=3/1) to give tert-butyl 7-(5-(3,5-difluorophenyl)-4,5-dihydro-1H-pyrazole-1-carbonyl)-4-aza-spiro[2-4]octane-4-carboxylate (30 mg, yield: 36.5%). ES-API:[M+H]⁺= 420.1.

Step 2: tert-butyl 7-(5-(3,5-difluorophenyl)-4,5-dihydro-1H-pyrazole-1-carbonyl)-4-aza-spiro[2-4]octane-4-carboxylate (30 mg, 0.072 mmol) was dissolved in dichloromethane/trifluoroacetic acid (1/1 mL). The reaction was carried out at 25°C for 1 h. The resultant reaction mixture was concentrated at a reduced pressure to give (5-(3,5-difluorophenyl)-4,5-dihydro-1H-pyrazol-1-yl)(4-aza-spiro[2.5]oct-7-yl) ketone trifluoroacetate (30 mg, crude product). ES-API:[M+H]⁺= 320.1.

Step 3: (5-(3,5-difluorophenyl)-4,5-dihydro-1H-pyrazol-1-yl)(4-aza-spiro[2.5]oct-7-yl) ketone trifluoroacetate (30 mg, 0.094 mmol) was dissolved in N,N-dimethyl formamide (1 mL). 6-chloropyrimidine-4-carbonitrile (13 mg, 0.094 mmol) and potassium carbonate (19 mg, 0.141 mmol) were added in an ice-water bath. The reaction was carried out at 0°C for 2 h. Then, the reaction was further carried out at the room temperature overnight. The resultant reaction mixture was quenched with water, and extracted wtih ethyl acetate. The organic was washed with saline, dried over anhydrous sodium sulfate, and concentrated at a reduced pressure. The crued product was subjected to thin layer chromatography (petroleum ether/ethyl acetate=3/1) to give 6-(7-(5-(3,5-difluorophenyl)-4,5-dihydro-1H-pyrazole-1-carbonyl)-4-aza-spiro[2.5]oct-4-yl)pyrimidine-4-carbonitrile (Z-62, 10.6 mg, yield: 26.7%). ES-API:[M+H]⁺= 423.2. ¹H NMR (400 MHz, CDCl₃) δ 8.62 (s, 1H), 7.21 (s, 1H), 6.98 (s, 1H), 6.66 (dd, *J* = 21.6, 6.9 Hz, 3H), 5.27 (dd, *J* = 12.1, 4.8 Hz, 1H), 4.92 (s, 1H), 3.67 (s, 1H), 3.42 (t, *J* = 15.2 Hz, 1H), 3.22 (s, 1H), 2.78 (d, *J* = 19.0 Hz, 1H), 2.21 (d, *J* = 39.8 Hz, 2H), 1.63 (d, *J* = 13.6 Hz, 1H), 1.12 (s, 2H), 0.85 (s, 2H).

### Example 58 Preparation of (5-(3,5-difluorophenyl)-4,5-dihydro-1H-pyrazol-1-yl)(5-(5-methyl-1,3,4-oxadizol-2-yl)-5-aza-spiro[2.5]octane-8-yl) ketone (racemate, Z-63-a), (5-(3,5-difluorophenyl)-4,5-dihydro-1H-pyrazol-1-yl)(5-(5-methyl-1,3,4-oxadizol-2-yl)-5-aza-spiro[2.5]octane-8-yl) ketone (racemate, Z-63-b), (5-(3,5-difluorophenyl)-4,5-dihydro-1H-pyrazol-1-yl)(5-(5-methyl-1,3,4-oxadizol-2-yl)-5-aza-spiro[2.5]octane-8-yl) ketone (an isomer having a single configuration, Z-63-1), (5-(3,5-difluorophenyl)-4,5-dihydro-1H-pyrazol-1-yl)(5-(5-methyl-1,3,4-oxadizol-2-yl)-5-aza-spiro[2.5]octane-8-yl) ketone (an isomer havinga single configuration, Z-63-2)

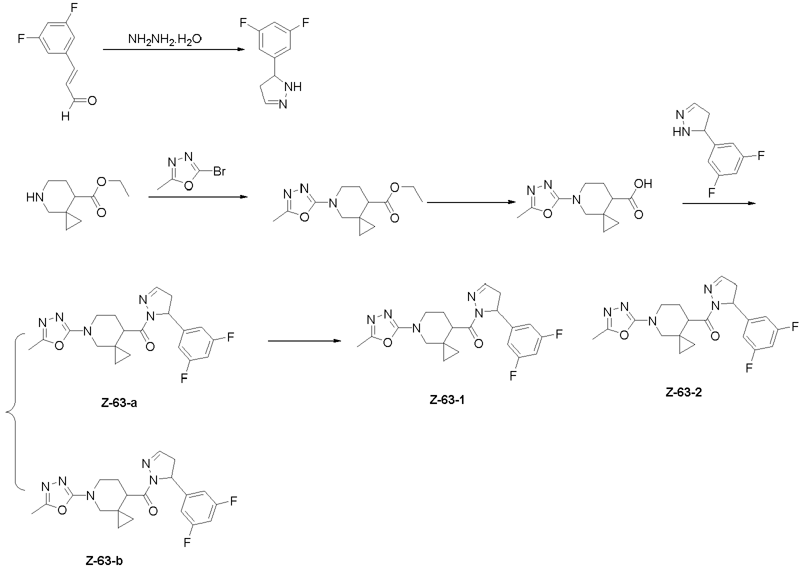

Step 1: ethyl 5-aza-spiro[2.5]octane-8-carboxylate (1000 mg, 5.46 mmol), 2-bromo-5-methyl-1,3,4-oxadizole (900 mg, 5.56 mmol) and potassium carbonate (2000 mg, 14.49 mmol) were dissolved in N,N-dimethyl formamide (10 mL). The mixture was heated to 50°C for reaction for 2 h. The resultant reaction mixture was cooled to the room temperature. Water was added. The resultant mixture was extracted with ethyl acetate. The organic phase was washed with saline, dried over anhydrous sodium sulfate, and concentrated at a reduced pressure to give ethyl 5-(5-methyl-1,3,4-oxadizol-2-yl)-5-aza-spiro[2.5]octane-8-carboxylate (1500 mg, yield: 100.0%). ES-API:[M+H]⁺= 266.1.

Step 2: ethyl 5-(5-methyl-1,3,4-oxadizol-2-yl)-5-aza-spiro[2.5]octane-8-carboxylate (1.5 g, 5.9 mmol) was dissolved in tetrahydrofuran/water/methanol (5 mL/5 mL/5 mL). Lithium hydroxide monohydrate (1.5 g, 35.7 mmol) was added. The mixture was heated to 40°C and reacted for 0.5 h. The resultant reaction mixture was cooled, adjusted to have a pH of 5-6, and extracted with ethyl acetate. The organic phase was washed with saline, dried over sodium sulfate, and concentrated at a reduced pressure to remove the solvent to give 5-(5-methyl-1,3,4-oxadizol-2-yl)-5-aza-spiro[2.5]octane-8-carboxylic acid (900 mg, yield: 64.4%). ES-API:[M+H]⁺= 238.1.

Step 3: 80% hydrazine hydrate (3 g, 47.96 mmol) was added to a reaction flask, and heated to 50°C. (E)-3-(3,5-difluorophenyl)acraldehyde (1.6 g, 9.5 mmol) was dissolved in 350 mL of tert-butanol. The obtained solution was slowly added dropwise to the hydrazine hydrate. Theareafter, the temperature was raised to 80°C. The reaction was carried out for 16 h. As monitored by TLC when the raw materials were substantially depleted, the reaction mixture was cooled, concentrated, and added to ethyl acetate (50mL). The resultant mixture was washed with water for three times (10 mL^{∗}3). The organic phases were combined, dried over sodium sulfate, and filtered. The filtrate was concentrated to remove the solvent to give a crude product of 5-(3,5-difluorophenyl)-4,5-dihydro-1H-pyrazole as a yellow oil (1.6 g) (yield: 100%). ES-API:[M+1]⁺= 183.1

Step 4: 5-(5-methyl-1,3,4-oxadizol-2-yl)-5-aza-spiro[2.5]octane-8-carboxylic acid (900 mg, 3.81 mmol) was dissolved in dichloromethane (14 mL). Then, thionyl chloride (2000 mg, 16.8 mmol) was added. The reaction was carried out at 20°C for 1 h. The resultant reaction mixture was spin-dried to remove the solvent. The residue was dissolved in dichloromethane. The resultant solution was added dropwise to a solution of 5-(3,5-difluorophenyl)-4,5-dihydro-1H-pyrazole (1.6 g, 8.77 mmol) and N,N-diisopropylethylamine (900 mg, 6.97 mmol) in dichloromethane. The reaction was carried out for 1 h. The organic phase was washed with 0.5N diluted hydrochloric acid, washed with saline, dried over anhydrous sodium sulfate, and concentrated at a reduced pressure. The crude product was subjected to preparative HPLC (column: Ultimate XB-C18, 50^{∗}250mm, 10um; mobile phase system: A: pure water; B: pure acetonitrile; flow rate: 80 ml/min, gradient: over 40 minutes, B%=20%-100%; column temperature: room temperature) to give 2 racemates:
Z-63-a (the 1^{st} peak): a racemate of (5-(3,5-difluorophenyl)-4,5-dihydro-1H-pyrazol-1-yl)(5-(5-methyl-1,3,4-oxadizol-2-yl)-5-aza-spiro[2.5]octane-8-yl) ketone; (17.8 mg, yield: 1.16%, LCMS retention time: 1.68 min). ES-API: [M+H]⁺= 402.2. ¹H NMR (400 MHz, CDCl₃) δ 6.95 (s, 1H), 6.75-6.63 (m, 3H), 5.33 (d, *J* = 13.6 Hz, 1H), 3.87 (d, *J* = 13.1 Hz, 1H), 3.78-3.71 (m, 1H), 3.57-3.38 (m, 2H), 3.15-3.06 (m, 2H), 2.77 (d, *J* = 19.0 Hz, 1H), 2.36 (s, 3H), 2.07 (s, 2H), 0.61 (s, 2H), 0.54 (s, 1H), 0.38 (s, 1H).
Z-63-b (the 2^{nd} peak): another racemate of (5-(3,5-difluorophenyl)-4,5-dihydro-1H-pyrazol-1-yl)(5-(5-methyl-1,3,4-oxadizol-2-yl)-5-aza-spiro[2.5]octane-8-yl) ketone; (18.0 mg, yield: 1.18%, LCMS retention time: 1.68 min). ES-API:[M+H]⁺= 402.2. ¹H NMR (400 MHz, CDCl₃) δ 6.94 (s, 1H), 6.69 (s, 3H), 5.33 (s, 1H), 3.82-3.69 (m, 3H), 3.48-3.39 (m, 1H), 3.15 - 3.03 (m, 2H), 2.82-2.73 (m, 1H), 2.36 (s, 3H), 2.11-1.94 (m, 2H), 0.69-0.59 (m, 2H), 0.56-0.43 (m, 2H).

Step 5: the Z-63-a (12mg) as obtained in the above step was subjected to a chiral resolution (mobile phase: Hex:EtOH=60:40); column: IF (250mm^{∗}4.6mm 5um); flow rate: 1.0 ml/min; column temperature: 30°C) to give 2 enantiomers:
Isomer Z-63-1 (retention time: 8.972 min): (4 mg, purity: 100%, ee value: 100%). ES-API:[M+H]⁺= 402.1.
Isomer Z-63-2 (retention time: 10.769 min): (6 mg, purity100%, ee value: 99.9%). ES-API:[M+H]⁺= 402.1.

### Example 59 Preparation of 6-(7-(5-(3,5-difluorophenyl)-4,5-dihydro-1H-pyrazole-1-carbonyl)-4-aza-spiro[2.5]oct-4-yl)pyrimidine-4-carboxamide (racemate, Z-64-a), 6-(7-(5-(3,5-difluorophenyl)-4,5-dihydro-1H-pyrazole-1-carbonyl)-4-aza-spiro [2.5]oct-4-yl)pyrimidine-4-carboxamide (racemate, Z-64-b), 6-(7-(5-(3,5-difluorophenyl)-4,5-dihydro-1H-pyrazole-1-carbonyl)-4-aza-spiro[2.5]oct-4-yl)pyrimidine-4-carboxamide (an isomer having a single configuration, Z-64-1), 6-(7-(5-(3,5-difluorophenyl)-4,5-dihydro-1H-pyrazole-1-carbonyl)-4-aza-spiro[2.5]oct-4-yl)pyrimidine-4-carboxamide (an isomer having a single configuration, Z-64-2)

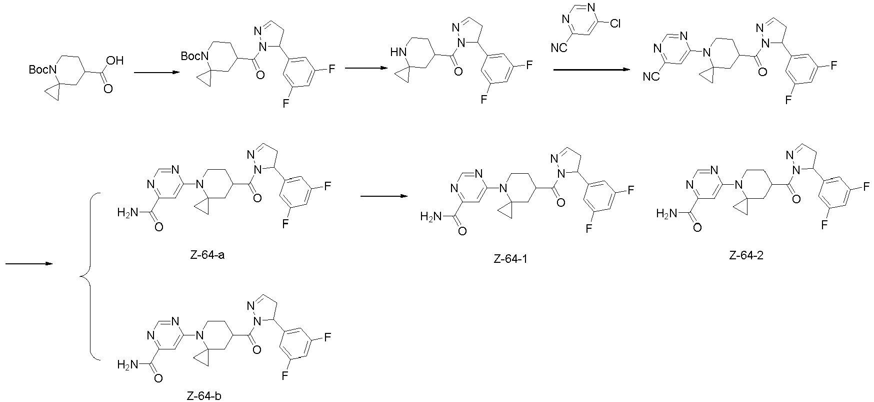

Step 1: 4-(tert-butoxycarbonyl)-4-aza-spiro[2.5]octane-7-carboxylic acid (1223 mg, 4.8 mmol), 5-(3,5-difluorophenyl)-4,5-dihydro-1H-pyrazole (36 mg, 0.196 mmol), N,N-diisopropylethylamine (1.6 g, 12.4 mmol), 2-(7-azabenzotriazole)-N,N,N',N'-tetramethyluronium hexafluorophosphate (2.2 g, 5.8 mmol) were dissolved in N,N-dimethyl formamide (28 mL). The reaction was carried out at 20°C for 16 h. The resultant reaction mixture was extracted with ethyl acetate, washed with 0.5 N diluted hydrochloric acid, washed with saturated saline solution, dried over anhydrous sodium sulfate, spin-dried to remove the solvent, and subjected to thin layer chromatography (petroleum ether/ethyl acetate=3/1) to give tert-butyl 7-(5-(3,5-difluorophenyl)-4,5-dihydro-1H-pyrazole-1-carbonyl)-4-aza-spiro[2-4]octane-4-carboxylate (1.749 g, yield: 87.0 %). ES-API:[M+H]⁺= 420.1.

Step 2: tert-butyl 7-(5-(3,5-difluorophenyl)-4,5-dihydro-1H-pyrazole-1-carbonyl)-4-aza-spiro[2-4]octane-4-carboxylate (1.749 g, 4.2 mmol) was dissolved in dichloromethane/trifluoroacetic acid (5/5 mL). The reaction was carried out at 25°C for 1 h. The resultant reaction mixture was concentrated at a reduced pressure to give (5-(3,5-difluorophenyl)-4,5-dihydro-1H-pyrazol-1-yl)(4-aza-spiro[2.5]oct-7-yl) ketone trifluoroacetate (1.7 g, crude product). ES-API:[M+H]⁺= 320.1.

Step 3: (5-(3,5-difluorophenyl)-4,5-dihydro-1H-pyrazol-1-yl)(4-aza-spiro[2.5]oct-7-yl) ketone trifluoroacetate (1.7 g, 5.3 mmol) was dissolved in N,N-dimethyl formamide (10 mL). 6-chloropyrimidine-4-carbonitrile (0.6 g, 4.3 mmol) and potassium carbonate (3.0 g, 21.7 mmol) were added in an ice-water bath. The reaction was carried out at 0°C for 2 h. Then, the reaction was further carried out at the room temperature overnight. The resultant reaction mixture was quenched with water, and extracted with ethyl acetate. The organic phase was washed with saline, dried over anhydrous sodium sulfate, and concentrated at a reduced pressure. The crude product was subjected to thin layer chromatography (petroleum ether/ethyl acetate=3/1) to give 6-(7-(5-(3,5-difluorophenyl)-4,5-dihydro-1H-pyrazole-1-carbonyl)-4-aza-spiro[2.5]oct-4-yl)pyrimidine-4-carbonitrile (1.357 g, yield: 60.3%). ES-API: [M+H]⁺= 423.2.

Step 4: 6-(7-(5-(3,5-difluorophenyl)-4,5-dihydro-1H-pyrazole-1-carbonyl)-4-aza-spiro[2.5]oct-4-yl)pyrimidine-4-carbonitrile (1.357 g, 3.22 mmol) was dissolved in water/acetonitrile (45/15 mL). Sodium hydroxide (135.7 mg, 3.39 mmol) was added in an ice-water bath. Then, 30% hydrogen peroxide (15 mL) was added. The reaction was carried out at 20°C for 2 h. The organic phase was washed with a solution of sodium sulphite, washed with a saline solution, dried over anhydrous sodium sulfate, and concentrated at a reduced pressure. The crude product was subjected to thin layer chromatography (dichloromethane/methanol=10/1) to give 2 racemates.
Z-64-a (the collected fraction having a Rf of 0.4): a racemate of 6-(7-(5-(3,5-difluorophenyl)-4,5-dihydro-1H-pyrazole-1-carbonyl)-4-aza-spiro[2.5]oct-4-yl)pyrimidine-4-carboxamide (66 mg, yield: 4.67%, LCMS retention time: 1.65 min). ¹H NMR (400 MHz, CDCl₃) δ 8.59 (s, 1H), 7.70 (d, *J* = 6.0 Hz, 1H), 6.97 (s, 1H), 6.76 - 6.52 (m, 3H), 5.65 (s, -1H), 5.27 (d, *J* = 11.4 Hz, 1H), 3.67 (s, -1H), 3.49 - 3.33 (m, -1H), 2.77 (d, *J* = 18.8 Hz, -1H), 2.00 (s, -1H), 1.79 (s, 2H), 0.88 (t, *J* = 6.6 Hz, 4H).
Z-64-b (the collected fraction having a Rf of 0.2): another racemate of 6-(7-(5-(3,5-difluorophenyl)-4,5-dihydro-1H-pyrazole-1-carbonyl)-4-aza-spiro[2.5]oct-4-yl)pyrimidine-4-carboxamide (90 mg, yield: 6.367%, LCMS retention time: 1.68 min). ES-API:[M+H]⁺= 441.2. ¹H NMR (400 MHz, CDCl₃) δ 8.59 (s, 1H),7.71 (s, 1H), 6.97 (s, 1H), 6.63 (s, 3H), 5.59 (s, 1H), 5.27 (d, *J* = 9.4 Hz, 1H), 3.68 (s, 1H), 3.48 - 3.35 (m, 1H), 2.77 (d, *J* = 17.6 Hz, 1H), 2.31 (s, 1H), 1.99 (dd, *J* = 21.4, 12.5 Hz, 2H), 0.81 (s, 4H).

Step 5: the Z-64-a (66mg) as obtained in the above step was subjected to a chiral resolution (mobile phase: Hex:EtOH:DEA=70:30:0.2); column: IB (250mm^{∗}4.6mm 5um); flow rate: 1.0 ml/min; column temperature: 30°C) to give 2 enantiomers:
Isomer Z-64-1 (retention time: 6.542 min) (23 mg, P: 100%, ee value: 100%). ES-API: [M+H]⁺= 441.1.
Isomer Z-64-2 (retention time: 8.146 min) (24 mg, P: 100%, ee value: 99.9%). ES-API: [M+H]⁺= 441.1.

### Example 60 Preparation of 6-((R)-7-((S)-5-(5-fluoropyridin-3-yl)-4,5-dihydro-1H-pyrazole-1-carbonyl)-4-aza-spiro[2.5]oct-4-yl)pyrimidine-4-carbonitrile (Z-65-1), 6-((S)-7-((S)-5-(5-fluoropyridin-3-yl)-4,5-dihydro-1H-pyrazole-1-carbonyl)-4-aza-spiro[2.5]oct-4-yl)pyrimidine-4-carbonitrile (Z-65-2), 6-((S)-7-((R)-5-(5-fluoropyridin-3-yl)-4,5-dihydro-1H-pyrazole-1-carbonyl)-4-aza-spiro[2.5]oct-4-yl)pyrimidine-4-carbonitrile (Z-65-3), 6-((R)-7-((R)-5-(5-fluoropyridin-3-yl)-4,5-dihydro-1H-pyrazole-1-carbonyl)-4-aza-spiro[2.5]oct-4-yl)pyrimidine-4-carbonitrile (Z-65-4)

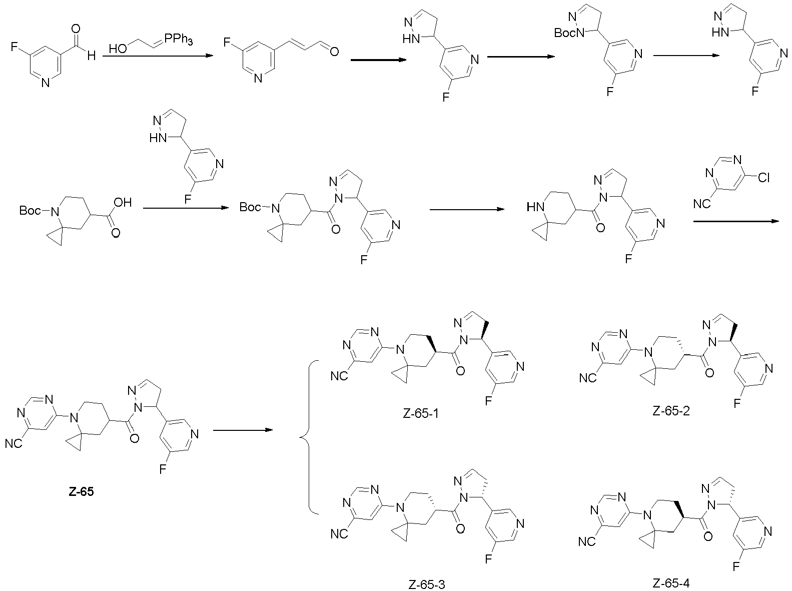

Step 1: 5-fluoropyridine-3-carboxaldehyde (7 g, 56 mmol) and 2-(triphenylphosphoranylene)acetaldehyde (17.9 g, 58.8 mmol) were dissolved in tetrahydrofuran (100 mL). The solution was heated to 50° overnight. The resultant reaction mixture was spin-dried to remove the solvent, and subjected to column chromatography (petroleum ether/ethyl acetate=5/1) to give 3-(5-fluoropyridin-3-yl)acraldehyde (8 g, yield: 94.6%). ES-API:[M+H]⁺= 152.0.

Step 2: hydrazine hydrate (6.62 g, 106 mmol) was dissolved in ethanol (100 mL). Then, acetic acid (7.95 g, 132.5 mmol) was added. The mixture was heated to 45°C. 3-(5-fluoropyridin-3-yl)acraldehyde (8 g, 53 mmol) was added in batches to the reaction mixture over 30 minutes. Thereafter, the temperature was kept at 100°C for reation overnight. The resultant reaction mixture was spin-dried to remove the solvent. Water was added. The resultant solution was adjusted to have a pH of about 7 with sodium bicarbonate, and extracted with dichloromethane. The organic phase was washed with saline, dried over anhydrous sodium sulfate, and concentrated at a reduced pressure to give 3-(4,5-dihydro-1H-pyrazol-5-yl)-5-fluoropyridine (10 g, yield: crude product), which was directly used in the next step of the reaction. ES-API:[M+H]⁺= 166.0.

Step 3: 3-(4,5-dihydro-1H-pyrazol-5-yl)-5-fluoropyridine (10 g, 60.6 mmol) was dissolved in tetrahydrofuran (100 mL). Then, Boc₂O (13.2 g, 60.6 mmol) was added. The reaction was carried out at the room temperature overnight. Thereafter, water and ethyl acetate were added. The resultant mixture was separated to two phases. The organic phase was washed with saturated saline solution, dried over anhydrous sodium sulfate, and concentrated at a reduced pressure. The crude product was purified by column chromatography to give tert-butyl 5-(5-fluoropyridin-3-yl)-4,5-dihydro-1H-pyrazole-1-carboxylate (2.8 g, 16.9%). ES-API:[M+H]⁺= 266.0.

Step 4: tert-butyl 5-(5-fluoropyridin-3-yl)-4,5-dihydro-1H-pyrazole-1-carboxylate (1.2 g, 4.53 mmol) was dissolved in dichloromethane (5 mL). Trifluoro acetic acid (5 mL) was added dropwise in an ice-water bath. The reaction was carried out for 0.5 h. The resultant reaction mixture was spin-dried to remove the solvent to give 3-(4,5-dihydro-1H-pyrazol-5-yl)-5-fluoropyridine trifluoroacetate (2 g, yield: crude product), which was directly used in the next step.

Step 5: 4-(tert-butoxycarbonyl)-4-aza-spiro[2.5]octane-7-carboxylic acid (1 g, 3.92 mmol), 3-(4,5-dihydro-1H-pyrazol-5-yl)-5-fluoropyridine trifluoroacetate (2 g, crude product), N,N-diisopropylethylamine (1.01 g, 7.84 mmol) and 2-(7-azabenzotriazole)-N,N,N',N'-tetramethyluronium hexafluorophosphate (1.79 g, 4.7 mmol) were dissolved in N,N-dimethyl formamide (10 mL). The reaction was carried out at 20°C for 1 h. The resultant reaction mixture was washed with 0.5 N diluted hydrochloric acid, washed with saturated saline solution, dried over anhydrous sodium sulfate, spin-dried to remove the solvent, and subjected to thin layer chromatography (petroleum ether/ethyl acetate=3/1) to give tert-butyl 7-(5-(5-fluoropyridin-3-yl)-4,5-dihydro-1H-pyrazole-1-carbonyl)-4-aza-spiro[2-4]octane-4-carboxylate (640 mg, 40.6%). ES-API:[M+H]⁺= 403.2.

Step 6: tert-butyl 7-(5-(5-fluoropyridin-3-yl)-4,5-dihydro-1H-pyrazole-1-carbonyl)-4-aza-spiro[2,4]octane-4-carboxylate (640 mg, 1.59 mmol) was dissolved in dichloromethane/trifluoro acetic acid (5/5 mL). The reaction was carried out at 25°C for 1 h. The resultant reaction mixture was concentrated at a reduced pressure to give (5-(5-fluoropyridin-3-yl)-4,5-dihydro-1H-pyrazol-1-yl)(4-aza-spiro[2.5]oct-7-yl) ketone (800 mg, crude product). ES-API:[M+H]⁺= 303.2.

Step 7: ((5-(5-fluoropyridin-3-yl)-4,5-dihydro-1H-pyrazol-1-yl)(4-aza-spiro[2.5]oct-7-yl) ketone (800 mg, crude product) was dissolved in N,N-dimethyl formamide (10 mL). 6-chloropyrimidine-4-carbonitrile (221.3 mg, 1.59 mmol) and potassium carbonate (439 mg, 3.18 mmol) were added in an ice-water bath. The reaction was carried out at 0°C for 2 h. Then, the reaction was further carried out at the room temperature overnight. The resultant reaction mixture was quenched with water, and extracted with ethyl acetate. The organic phase was washed with saline, dried over anhydrous sodium sulfate, and concentrated at a reduced pressure. The crude product was subjected to preparative chromatography to give 6-(7-(5-(5-fluoropyridin-3-yl)-4,5-dihydro-1H-pyrazole-1-carbonyl)-4-aza-spiro[2.5]oct-4-yl)pyrimidine-4-carbonitrile (Z-65, 253 mg, 39.2%). ES-API:[M+H]⁺= 406.2. ¹H NMR (400 MHz, CDCl₃) δ 8.61 (s, 1H), 8.37 (s, 1H), 8.27 (s, 1H), 7.17 (q, *J* = 8.6 Hz, 2H), 7.03 (s, 1H), 5.36 (dd, *J* = 12.1, 4.7 Hz, 1H), 4.91 (s, 1H), 3.66 (s, 1H), 3.49 (s, 1H), 3.19 (s, 1H), 2.84 (d*, J* = 19.0 Hz, 1H), 2.13 (s, 1H), 1.82 (s, 2H), 1.64 (dd, *J* = 36.7, 12.8 Hz, 1H), 1.12 (s, 2H), 0.84 (t, *J* = 8.6 Hz, 2H).

Step 8: the Z-65 (50 mg) as obtained in the above step was subjected to a chiral resolution (mobile phase: ACN:IPA:AMMN=90:10:0.2); column: IG (250mm^{∗}4.6mm 5um); flow rate: 1.0 ml/min; column temperature: 30°C) to give 4 isomers having a single configuration (the structures of the 4 isomers having a single configuration are each arbitrarily assigned):
Isomer 1 (retention time: 4.499 min); arbitrarily assigned as the compound of Z-65-1, 6-((R)-7-((S)-5-(5-fluoropyridin-3-yl)-4,5-dihydro-1H-pyrazole-1-carbonyl)-4-aza-spiro[2.5]oct-4-yl)pyrimidine-4-carbonitrile (6.5 mg, purity: 100%, ee value: 99.6%)). ES-API:[M+H]⁺= 406.1.
Isomer 2 (retention time: 4.702 min); arbitrarily assigned as the compound of Z-65-2, 6-((S)-7-((S)-5-(5-fluoropyridin-3-yl)-4,5-dihydro-1H-pyrazole-1-carbonyl)-4-aza-spiro[2.5]oct-4-yl)pyrimidine-4-carbonitrile (6.7 mg, purity: 100%, ee value: 95.5%). ES-API:[M+H]⁺= 406.1.
Isomer 3 (retention time: 8.644 min); arbitrarily assigned as the compound of Z-65-3, 6-((S)-7-((R)-5-(5-fluoropyridin-3-yl)-4,5-dihydro-1H-pyrazole-1-carbonyl)-4-aza-spiro[2.5]oct-4-yl)pyrimidine-4-carbonitrile (8.8 mg, purity: 100%, ee value: 99.7%). ES-API:[M+H]⁺= 406.1.
Isomer 4 (retention time: 10.152 min); arbitrarily assigned as the compound of Z-65-4, 6-((R)-7-((R)-5-(5-fluoropyridin-3-yl)-4,5-dihydro-1H-pyrazole-1-carbonyl)-4-aza-spiro[2.5]oct-4-yl)pyrimidine-4-carbonitrile (10.2 mg, purity: 100%, ee value: 99.0%). ES-API:[M+H]⁺= 406.1.

### Example 61 Preparation of 6-(7-(5-(3,5-difluorophenyl)-4,5-dihydro-1H-pyrazole-1-carbonyl)-4-aza-spiro[2.5]oct-4-yl)pyrimidine-4-carboxamide (Z-66)

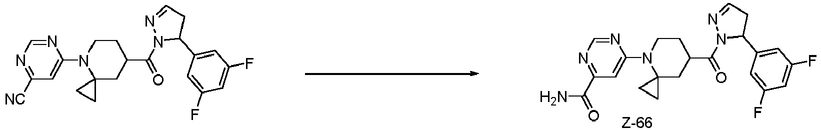

Step 1: 6-(7-(5-(3,5-difluorophenyl)-4,5-dihydro-1H-pyrazole-1-carbonyl)-4-aza-spiro[2.5]oct-4-yl)pyrimidine-4-carbonitrile (Z-62, 5 mg, 0.012 mmol) was dissolved in water/acetonitrile (0.75/0.25 mL). Sodium hydroxide (0.5 mg, 0.013 mmol) was added in an ice-water bath. Then, 30% hydrogen peroxide (0.25 mL) was added. The reaction was carried out at 20°C for 2 h. The organic phase was washed with a solution of sodium sulphite, washed with a saline solution, dried over anhydrous sodium sulfate, and concentrated at a reduced pressure. The crude product was subjected to thin layer chromatography (dichloromethane/methanol=10/1) to give 6-(7-(5-(3,5-difluorophenyl)-4,5-dihydro-1H-pyrazole-1-carbonyl)-4-aza-spiro[2.5]oct-4-yl)pyrimidine-4-carboxamide (Z-66, 1.5 mg, yield: 28.8%), ES-API:[M+H]⁺= 441.2. ¹H NMR (400 MHz, CDCl₃) δ 8.59 (s, 1H), 7.70 (d*, J* = 6.0 Hz, 1H), 6.97 (s, 1H), 6.76 - 6.52 (m, 3H), 5.65 (s,-1H), 5.27 (d, *J* = 11.4 Hz, 1H), 3.67 (s,-1H), 3.49 - 3.33 (m,-1H), 2.77 (d, *J =* 18.8 Hz,-1H), 2.00 (s,-1H), 1.79 (s, 2H), 0.88 (t, *J* = 6.6 Hz, 4H).

### Example 62 Preparation of 6-(7-(5-(5-fluoropyridin-3-yl)-4,5-dihydro-1H-pyrazole-1-carbonyl)-4-aza-spiro[2.5]oct-4-yl)pyrimidine-4-carboxamide (Z-67)

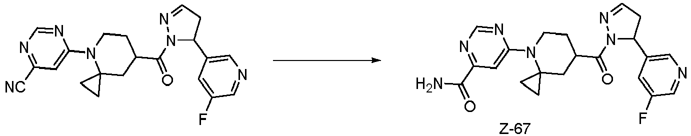

Step 1: 6-(7-(5-(5-fluoropyridin-3-yl)-4,5-dihydro-1H-pyrazole-1-carbonyl)-4-aza-spiro[2.5]oct-4-yl)pyrimidine-4-carbonitrile (Z-65, 200 mg, 0.49 mmol) was dissolved in water/acetonitrile (3/1 mL). Sodium hydroxide (22 mg, 0.54 mmol) was added in an ice-water bath. Then, 30% hydrogen peroxide (1 mL) was added. The reaction was carried out at 20°C for 2 h. The organic phase was washed with a solution of sodium sulphite, washed with a saline solution, dried over anhydrous sodium sulfate, and concentrated at a reduced pressure. The crude product was subjected to thin layer chromatography (dichloromethane/methanol=10/1) to give 6-(7-(5-(5-fluoropyridin-3-yl)-4,5-dihydro-1H-pyrazole-1-carbonyl)-4-aza-spiro[2.5]oct-4-yl)pyrimidine-4-carboxamide (92 mg, yield: 44.4%), ES-API:[M+H]⁺= 424.2. ¹H NMR (400 MHz, CDCl₃) δ 8.58 (s, 1H), 8.36 (s, 1H), 8.26 (s, 1H), 7.82 (s, 1H), 7.67 (d, *J* = 9.3 Hz, 1H), 7.13 (d*, J* = 8.8 Hz, 1H), 7.01 (s, 1H), 5.68 (s, 1H), 5.35 (dd, *J* = 12.1, 5.0 Hz, 1H), 4.91 (s, 1H), 3.66 (s, 1H), 3.47 (dd, *J* = 18.9, 12.4 Hz, 1H), 3.21 (s, 1H), 2.82 (d, *J* = 19.0 Hz, 1H), 2.23 (d, *J* = 44.3 Hz, 1H), 1.91 (d, *J* = 13.5 Hz, 1H), 1.19 (s, 2H), 0.84 - 0.71 (m, 2H).

### Example 63 Preparation of (5-(5-fluoropyridin-3-yl)-4,5-dihydro-1H-pyrazol-1-yl)(5-(5-methyl-1,3,4-oxadizol-2-yl)-5-aza-spiro[2.5]oct-8-yl) methanone (Z-68)

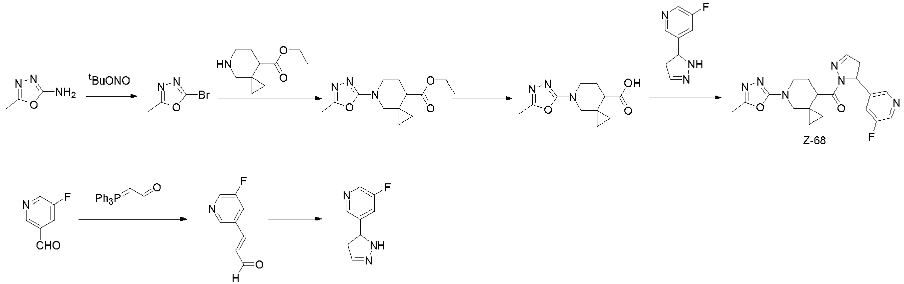

Step 1: 5-methyl-1,3,4-oxadizole-2-amine (2 g, 20.2 mmol) and copper bromide (6.8 g, 30.8 mmol) was dissolved in acetonitrile (20 mL). The solution was cooled to 0°C. Then, tert-butyl nitrite (4.8 g, 46.6 mmol) was added. The mixture was warmed to 65°C and reacted for 4 h. The resultant reaction mixture was cooled. Water was added. The resultant mixture was extracted with ethyl acetate. The organic phase was washed with saline, dried over anhydrous sodium sulfate, and concentrated at a reduced pressure to give 2-bromo-5-methyl-1,3,4-oxadizole (2.1 g, yield: 74.78%). ES-API:[M+H]⁺= 163.1.

Step 2: ethyl 5-aza-spiro[2.5]octane-8-carboxylate (2.0 g, 11 mmol), 2-bromo-5-methyl-1,3,4-oxadizole (2.1 g, 13 mmol) and potassium carbonate (4 g, 29 mmol) were dissolved in dimethylformamide (20 mL). The solution was heated to 50°C and reacted for 2 h. The resultant reaction mixture was cooled. Water was added. The resultant mixture was extracted with ethyl acetate. The organic phase was washed with saline, dried over anhydrous sodium sulfate, and concentrated at a reduced pressure, to give ethyl 5-(5-methyl-1,3,4-oxadizol-2-yl)-5-aza-spiro[2.5]octane-8-carboxylate (3.4 g, 100.0%). ES-API:[M+H]⁺= 266.1.

Step 3: ethyl 5-(5-methyl-1,3,4-oxadizol-2-yl)-5-aza-spiro[2.5]octane-8-carboxylate (3.4 g, 13.3 mmol) was dissolved in tetrahydrofuran/water/methanol (10 mL/10 mL/10 mL). Then, lithium hydroxide monohydrate (3.4 g, 80.9 mmol) was added. The mixture was heated to 40°C and reacted for 0.5 h. The resultant reaction mixture was cooled, adjusted to have a pH of 5-6, and extracted with ethyl acetate. The organic phase was washed with saline, dried over sodium sulfate, and concentrated at a reduced pressure to remove the solvent to give 5-(5-methyl-1,3,4-oxadizol-2-yl)-5-aza-spiro[2.5]octane-8-carboxylic acid (1.4 g, yield: 76.03%). ES-API: [M+H]⁺= 238.1.

Step 4: 5-fluoronicotine (1 g, 8 mmol) was dissolved in tetrahydrofuran (15 mL). Then, 2-(triphenyl-λ5-phosphanylidene) acetaldehyde (2.43 g, 8 mmol) was added. The mixture was warmed to 80°C and reacted for 16 h. The resultant reaction mixture was cooled, and concentrated by spin-drying to remove the solvent. The residue was subjected to thin layer chromatography (petroleum ether/ethyl acetate=5/1) to give (E)-3-(5-fluoropyridin-3-yl)acraldehyde (1 g, 100%). ES-API:[M+H]⁺= 152.1.

Step 5: (E)-3-(5-fluoropyridin-3-yl)acraldehyde (1 g, 6.6 mmol) was dissolved in tert-butanol (30mL). Then, hydrazine hydrate (1.7 g, 34 mmol) was added to the reaction mixture. The reaction mixture was warmed to 80°C and reacted for 16 h. As monitored by TLC when the raw materials were substantially depleted, the reaction mixture was cooled, and concentrated. Ethyl acetate (50 mL) was added. The resultant mixture was washed with water (10 mL^{∗}3). The organic phases were combined, dried over sodium sulfate, and filtered. The filtrate was concentrated to remove the solvent to give a crude product of 3-(4,5-dihydro-1H-pyrazol-5-yl)-5-fluoropyridine (1 g, yield: 35.5%). ES-API:[M+H]⁺= 166.1.

Step 6: 5-(5-methyl-1,3,4-oxadizol-2-yl)-5-aza-spiro[2.5]octane-8-carboxylic acid (900 mg, 3.81 mmol) was added to dichloromethane (14 mL). Then, thionyl chloride (2000 mg, 16.8 mmol) was added. The reaction was carried out at 20°C for 1 hour. The resultant reaction mixture was spin-dried to remove the solvent. The residue was dissolved in dichloromethane. The solution was added dropwise to a solution of 5-(3,5-difluorophenyl)-4,5-dihydro-1H-pyrazole (1.6 g, 8.77 mmol) and N,N-diisopropylethylamine (900 mg, 6.97 mmol) in dichloromethane. The reaction was carried out at the room temperature for 1 hour. The organic phase was washed with 0.5N diluted hydrochloric acid, washed with saline, dried over anhydrous sodium sulfate, and concentrated at a reduced pressure. The crude product was subjected to preparative HPLC to give (5-(5-fluoropyridin-3-yl)-4,5-dihydro-1H-pyrazol-1-yl)(5-(5-methyl-1,3,4-oxadizol-2-yl)-5-aza-spiro[2.5]oct-8-yl) methanone (Z-68, 22.0 mg, yield: 0.91%). ES-API:[M+H]⁺= 384.1. 1H NMR (400 MHz, CDCl₃) δ 8.41 (d, *J* = 2.6 Hz, 1H), 8.31 (d, *J* = 7.2 Hz, 1H), 7.21 - 7.16 (m, 1H), 6.99 (dt, *J =* 3.2, 1.7 Hz, 1H), 5.42 (ddd, *J =* 12.1, 5.2, 1.9 Hz, 1H), 3.81 - 3.64 (m, 2H), 3.55 - 3.41 (m, 1H), 3.15 - 3.01 (m, 2H), 2.87 - 2.80 (m, 1H), 2.35 (s, 3H), 2.03 (ddt, *J* = 18.9, 14.6, 4.6 Hz, 2H), 0.63 - 0.54 (m, 2H), 0.53 - 0.46 (m, 1H), 0.40 (dd, *J* = 20.4, 3.9 Hz, 1H).

### Example 64 Preparation of 6-((S)-7-((S)-5-(3,5-difluorophenyl)-4,5-dihydro-1H-pyrazole-1-carbonyl)-4-aza-spiro[2.5]oct-4-yl)-N,N-dimethylpyrimidine-4-carboxamide (racemate, Z-69) and preparation of 6-((S)-7-((S)-5-(3,5-difluorophenyl)-4,5-dihydro-1H-pyrazole-1-carbonyl)-4-aza-spiro[2.5]oct-4-yl)-N,N-dimethylpyrimidine-4-carboxamide (racemate, Z-71)

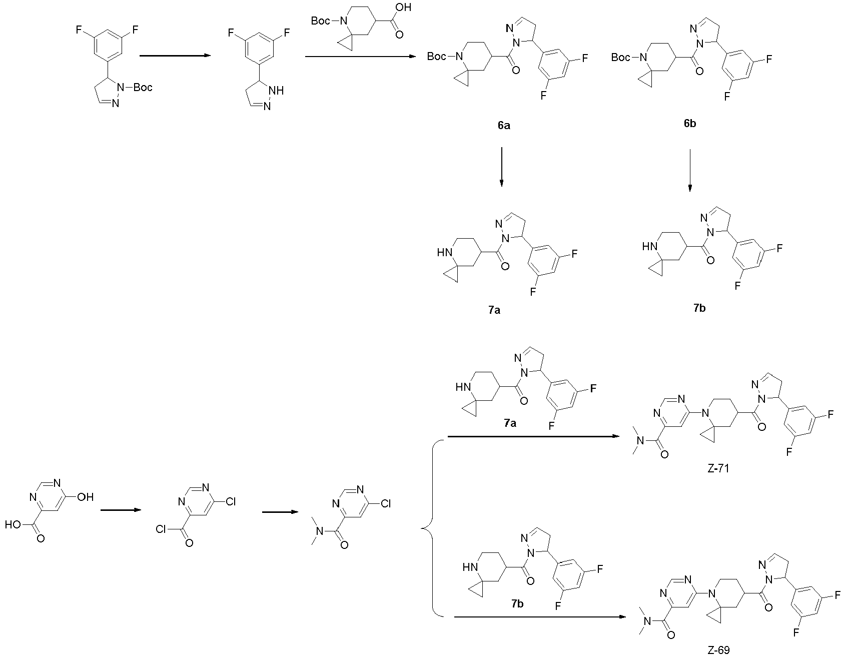

Step 1: tert-butyl 5-(3,5-difluorophenyl)-4,5-dihydro-1H-pyrazole-1-carboxylate (170 mg, 0.6 mmol) was dissolved in dichloromethane (1 mL). Then, trifluoroacetic acid (1 mL) was added dropwise. The reaction was carried out at the room temperature for 1 h. After the reaction was completed, the reaction mixture was concentrated by spin-drying to give 5-(3,5-difluorophenyl)-4,5-dihydro-1H-pyrazole (109 mg, yield: 100.00%). ES-API:[M+H]⁺= 182.1.

Step 2: 5-(3,5-difluorophenyl)-4,5-dihydro-1H-pyrazole (109 mg, 0.6 mmol) and 4-(tert-butoxycarbonyl)-4-aza-spiro[2.5]octane-7-carboxylic acid (100 mg, 0.24 mmol) were dissolved in N,N-dimethylformamide (5 mL). Then, N,N-diisopropylethylamine (1.5 mmol) was added dropwise to adjust the pH to 8 to 9 until the mixture was weakly alkaline. The reaction was carried out at the room temperature for 16 h. The resultant reaction mixture was cooled, and extracted with ethyl acetate. The organic phase was washed with saline, dried over sodium sulfate, concentrated at a reduced pressure to remove the solvent, and subjected to thin layer chromatography (PE: EA/3: 1) to give:
6a (relatively weak polar, Rf=0.4), a racemate of tert-butyl 7-(5-(5-(3,5-difluorophenyl)-4,5-dihydro-1H-pyrazole-1-carbonyl)-4-aza-spiro[2.5]octane-4-carboxylate (50 mg, yield: 43.1%). ES-API:[M+H]⁺= 419.2; and
6b (relative strong polar, Rf=0.3), a racemate of tert-butyl 7-(5-(5-(3,5-difluorophenyl)-4,5-dihydro-1H-pyrazole-1-carbonyl)-4-aza-spiro[2.5]octane-4-carboxylate (70 mg, yield: 43.20%), ES-API: [M+H]⁺= 419.2.

Step 3: 6a (50 mg, 0.12 mmol) was dissolved in dichloromethane (1 mL). Then, trifluoroacetic acid (1 mL) was added. The reaction was carried out at the room temperature for 1 h. The resultant reaction mixture was concentrated at a reduced pressure to remove the solvent to give a crude product of 7a, (5-(3,5-difluorophenyl)-4,5-dihydro-1H-pyrazol-1-yl)(4-aza-spiro[2.5]oct-7-yl) ketone trifluoroacetate (38 mg, yield: 100.00%). ES-API: [M+H]⁺= 319.1.

Step 4: 6-hydroxypyrimidine-4-carboxylic acid (5.0 g, 35.7 mmol) was dissolved in trifluoroacetic acid (20 mL). The mixture was warmed to 115°C and reacted for 16 h. The crude product was subjected to preparative HPLC to give 6-chloropyrimidine-4-carbonyl chloride (5.0 mg, yield: 79.55%), ES-API:[M+H]⁺= 176.0.

Step 5: 6-chloropyrimidine-4-carbonyl chloride (2.5 g, 14.2 mmol) was dissolved in dichloromethane (40 mL). Then, a solution of dimethylamine in tetrahydrofuran (6 mL) was added. After the mixtue was cooled to 0°C, N, N-diisopropylethylamine (6.3 g, 48.8 mmol) was added dropwise. Then, the mixture was purged with nigtrogen gas, and kept at 0°C and reacted for 1 h. Then, the reaction mixture was warmed to the room tempertue, and reacted for 1 h. After the reaction was completed, the reaction mixture was quenched with water, extracted with ethyl acetate, concentrated by spin-drying, and subjected to thin layer chromatography (EA/PE=1/3) to give the product of 6-chloro-N,N-dimethyl pyrimidine-4-carboxamide (300 mg, yield:11.42%). ES-API:[M+H]⁺=185.0.

Step 6: 7a (38 mg, 0.12 mmol) was dissolved in N,N-dimethylformamide (2 mL). Then, N, N-diisopropylethylamine (1 mL) was added dropwise to adjust the pH to 9. The oil bath was pre-heated to 50°C. Then, 6-chloro-N,N-dimethylpyrimidine-4-carboxamide (100 mg, 0.54 mmol) was added. The reaction was carried out at 50°C for 16 h. The crude product was subjected to preparative HPLC (column: Ultimate XB-C18,50^{∗}250mm,10um; mobile phase system: A: pure water; B: pure acetonitrile; flow rate: 80 ml/min; B%=20%-100%; column temperature: room temperature) to give Z-71, a racemate of 6-((S)-7-((S)-5-(3,5-difluorophenyl)-4,5-dihydro-1H-pyrazole-1-carbonyl)-4-aza-spiro[2.5]oct-4-yl)-N,N-dimethylpyrimidine-4-carboxamide (4 mg, yield: 7.17%); LCMS retention time: 1.68 min, ES-API: [M+H]⁺= 468.2. ¹H NMR (400 MHz, CDCl₃) δ 7.10 (s, 1H), 6.97 (s, 1H), 6.72 - 6.54 (m, 3H), 5.26 (dd, *J* = 12.0, 5.1 Hz, 1H), 3.65 (s, 1H), 3.42 (dd, *J* = 19.0, 12.0 Hz, 1H), 3.09 (s, 3H), 3.06 (s, 3H), 2.77 (dd, *J =* 19.0, 5.1 Hz, 1H), 2.25 - 2.17 (m, 1H), 1.26 (s, 4H), 1.16 (s, 2H), 0.86 (d, *J* = 7.6 Hz, 1H), 0.78 (t, *J* = 8.2 Hz, 2H).

Step 7: 6b (70 mg, 0.24 mmol) was dissolved in dichloromethane (1 mL). Then, trifluoroacetic acid (1 mL) was added. The reaction was carried out at the room temperatue for 1 h. The resultant reaction mixture was concentrated at a reduced pressure to remove the solvent to give the crude product of 7b, (5-(3,5-difluorophenyl)-4,5-dihydro-1H-pyrazol-1-yl)(4-aza-spiro[2.5]oct-7-yl) ketone trifluoroacetate (76 mg, yield: 100.00%). ES-API:[M+H]⁺= 319.1.

Step 8: 7b (76 mg, 0.24 mmol) was dissolved in N,N-dimethylformamide (2 mL). Then, N,N-diisopropylethylamine (1 mL) was added dropwise, adjust the pH to 9. The oil bath was pre-heated to 60°C. Then, 6-chloro-N,N-dimethylpyrimidine-4-carboxamide (100 mg, 0.54 mmol) was added. The reaction was carried out at 50°C for 16 h. The crude product was subjected to preparative HPLC (column: Ultimate XB-C18,50^{∗}250mm,10um; mobile phase system: A: pure water; B: pure acetonitrile; flow rate: 80 ml/min; B%=20%-100%; column temperature: room temperature) to give Z-69, a racemate of 6-((S)-7-((S)-5-(3,5-difluorophenyl)-4,5-dihydro-1H-pyrazole-1-carbonyl)-4-aza-spiro[2.5]oct-4-yl)-N,N-dimethylpyrimidine-4-carboxamide (2.0 mg, yield: 1.79%); LCMS retention time: 1.66 min, ES-API: [M+H]⁺= 468.2. ¹H NMR (400 MHz, CDCl₃) δ 8.58 (s, 1H), 7.13 (s, 1H), 6.97 (s, 1H), 6.71 - 6.59 (m, 3H), 5.27 (dd, *J =* 12.1, 4.9 Hz, 1H), 4.92 (s, 1H), 3.64 *(d, J =* 11.7 Hz, 1H), 3.41 (dd, *J* = 18.9, 12.0 Hz, 1H), 3.20 (s, 1H), 3.09 (s, 3H), 3.06 (s, 3H), 2.76 (dd, *J =* 19.0, 5.0 Hz, 1H), 2.29 (s, 1H), 1.95 (d*, J* = 13.4 Hz, 1H), 0.79 (s, 1H), 0.70 (s, 1H).

### Example 65 Preparation of Compounds Z-70-a and Z-70-b

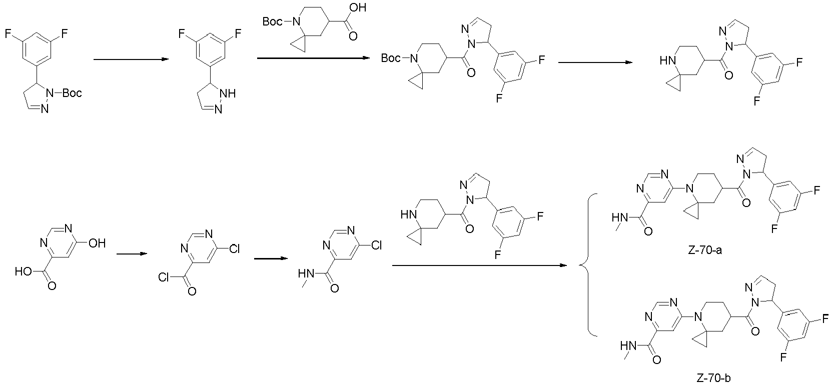

Step 1: tert-butyl 5-(3,5-difluorophenyl)-4,5-dihydro-1H-pyrazole-1-carboxylate (170 mg, 0.6 mmol) was dissolved in dichloromethane (1 mL). Then, trifluoroacetic acid (1 mL) was added dropwise. The reaction was carried out at the room temperature for 1 h. After the reaction was completed, the reaction mixture was concentrated by spin-drying to give 5-(3,5-difluorophenyl)-4,5-dihydro-1H-pyrazole (109 mg, yield: 100.00%). ES-API:[M+H]⁺= 182.1.

Step 2: 5-(3,5-difluorophenyl)-4,5-dihydro-1H-pyrazole (109 mg, 0.6 mmol) and 4-(tert-butoxycarbonyl)-4-aza-spiro[2.5]octane-7-carboxylic acid (100 mg, 0.24 mmol) were dissolved in N,N-dimethylformamide (2 mL). Then, N,N-diisopropylethylamine (0.5 mmol) was added dropwise, adjust the pH to 8 to 9 until the mixture was weakly alkaline. The reaction was carried out at the room temperature for 16 h. The resultant reaction mixture was cooled, and extracted with ethyl acetate. The organic phase was washed with saline, dried over sodium sulfate, concentrated at a reduced pressure to remove the solvent, and subjected to thin layer chromatography (PE: EA/3: 1) to give tert-butyl 7-(5-(5-(3,5-difluorophenyl)-4,5-dihydro-1H-pyrazole-1-carbonyl)-4-aza-spiro[2.5]ocatane-4-carboxylate (108 mg, yield: 65.73%). ES-API:[M+H]⁺= 419.2.

Step 3: tert-butyl 7-(5-(5-(3,5-difluorophenyl)-4,5-dihydro-1H-pyrazole-1-carbonyl)-4-aza-spiro[2.5]octane-4-carboxylate (108 mg, 0.26 mmol) was dissolved in dichloromethane (1 mL). Then, trifluoroacetic acid (1 mL) was added. The reaction was carried out at the room temperature for 1 h. The resultant reaction mixture was concentrated at a reduced pressure to remove the solvent to give a crude product of (5-(3,5-difluorophenyl)-4,5-dihydro-1H-pyrazol-1-yl)(4-aza-spiro[2.5]oct-7-yl) ketone (82 mg, yield: 100.00%). ES-API: [M+H]⁺= 319.1.

Step 4: 6-hydroxypyrimidine-4-carboxylic acid (5.0 g, 35.7 mmol) was dissolved in trifluoroacetic acid (20 mL). The mixture was warmed to 115°C and reacted for 16 h. The crude product was subjected to preparative HPLC to give 6-chloropyrimidine-4-carbonyl chloride (5.0 mg, yield: 79.55%), ES-API:[M+H]⁺= 176.0.

Step 5: 6-chloropyrimidine-4-carbonyl chloride (2.5 g, 14.2 mmol) was dissolved in dichloromethane (40 mL). Then, a solution of methylamine in tetrahydrofuran (6 mL) was added. After the mixtue was cooled to 0°C, N, N-diisopropylethylamine (6.3 g, 48.8 mmol) was added dropwise. Then, the mixture was purged with nigtrogen gas, and kept at 0°C and reacted for 1 h. Then, the reaction mixture was warmed to the room tempertue, and reacted for 1 h. After the reaction was completed, the reaction mixture was quenched with water, extracted with ethyl acetate, concentrated by spin-drying, and subjected to thin layer chromatography (EA/PE=1/3) to give the product of 6-chloro-N-methylpyrimidine-4-carboxamide (300 mg, yield: 12.35%), ES-API:[M+H]⁺=171.0

Step 6: (5-(3,5-difluorophenyl)-4,5-dihydro-1H-pyrazol-1-yl)(4-aza-spiro[2.5]oct-7-yl) ketone (82 mg, 0.26 mmol) was dissolved in N,N-dimethylformamide (2 mL). Then, N,N-diisopropylethylamine (1 mL) was added dropwise , adjust the pH to 9. The oil bath was pre-heated to 60°C. Then, 6-chloro-N-methylpyrimidine-4-carboxamide (300 mg, 1.75 mmol) was added. The reaction was carried out at 60°C for 16 h. The crude product was subjected to preparative HPLC (column: Ultimate XB-C18, 50^{∗}250mm,10 um; mobile phase system: A: pure water; B: pure acetonitrile; flow rate: 80 ml/min; B%=20%-100%; column temperature: room temperature) to give:
Z-70-a (retention time: 1.73 min): a racemate of 6-(7-(5-(3,5-difluorophenyl)-4,5-dihydro-1H-pyrazole-1-carbonyl)-4-aza-spiro[2.5]oct-4-yl)-N-methylpyrimidine-4-carboxamide (15.5 mg,yield: 26.49%); ES-API: [M+H]⁺= 454.2. ¹H NMR (400 MHz, CDCl₃) δ 8.54 (d, *J =* 1.2 Hz, 1H), 7.95 (d, *J =* 5.9 Hz, 1H), 7.67 (s, 1H), 6.96 (s, 1H), 6.73 - 6.57 (m, 3H), 5.26 (dd, *J* = 12.0, 5.1 Hz, 1H), 4.89 (s, 1H), 3.65 (s, 1H), 3.41 (ddd, *J =* 18.9, 12.0, 1.6 Hz, 1H), 3.19 (s, 1H), 2.99 (d, *J* = 5.1 Hz, 3H), 2.76 (ddd, *J* = 18.9, 5.1, 1.8 Hz, 1H), 2.19 (s, 1H), 1.25 (s, 4H), 0.79 (t, *J* = 8.3 Hz, 2H); and
Z-70-b (retention time: 1.75 min): a racemate of 6-(7-(5-(3,5-difluorophenyl)-4,5-dihydro-1H-pyrazole-1-carbonyl)-4-aza-spiro[2.5]oct-4-yl)-N- methylpyrimidine-4-carboxamide (10.0 mg, yield: 17.09%). ES-API: [M+H]⁺= 454.2. ¹H NMR (400 MHz, CDCl₃) δ 8.55 (d, *J =* 1.1 Hz, 1H), 7.95 (d, *J =* 5.7 Hz, 1H), 7.69 (s, 1H), 6.97 (q, *J* = 1.8 Hz, 1H), 6.69 - 6.61 (m, 3H), 5.27 (dd, *J =* 12.0, 5.0 Hz, 1H), 4.92 (s, 1H), 3.66 (*td, J* = 11.5, 5.7 Hz, 1H), 3.45 - 3.36 (m, 1H), 3.22 (s, 1H), 2.99 (d, *J* = 5.1 Hz, 3H), 2.76 (ddd, *J* = 18.9, 5.0, 1.8 Hz, 1H), 2.30 (s, 1H), 1.93 (d, *J* = 13.6 Hz, 1H), 1.30 - 1.15 (m, 4H), 0.81 - 0.70 (m, 2H).

### Example 66 Preparation of 6-(5-(5-phenyl-4,5-dihydro-1H-pyrazole-1-carbonyl)hexahydrocyclopenta[C]pyrrol-2(1H)-yl)pyrimidine-4-carbonitrile (Z-72)

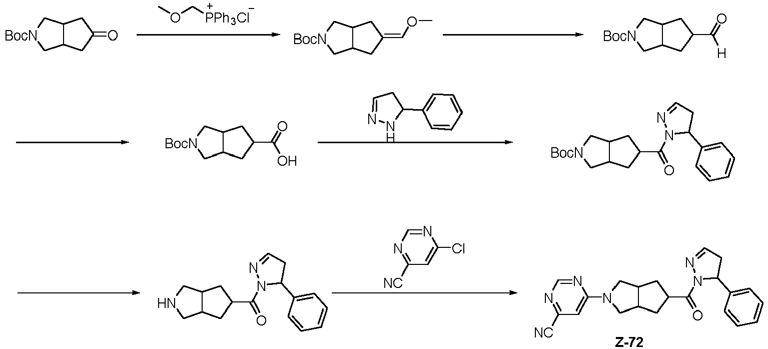

Step 1: tert-butyl 5-oxohexahydrocyclopenta[C]pyrrole-2(1H)-carboxylate (800 mg, 3.556 mmol) and (methoxymethyl)triphenylphosphine chloride (2.189 g, 6.4 mmol) were added to tetrahydrofuran (15 mL). Potassium tert-butoxide (797 mg, 7.11 mmol) was added at 0°C. The reaction mixture was reacted at 25°C for 18 h. Thereafter, the reaction was quenched with water, and extracted with dichloromethane (15 mL x 2). The organic phase was washed with saline, dried over anhydrous magnesium sulfate, rotary evaporated to remove the solvent, and subjected to column chromatography (petroleum ether/ethyl acetate=5/1) to give tert-butyl 5-methoxymethylenehexahydrocyclopenta[C]pyrrole-2(1H)-carboxylate (400 mg, 45%). ES-API: [M+H]⁺= 254.2.

Step 2: tert-butyl 5-methoxymethylenehexahydrocyclopenta[C]pyrrole-2(1H)-carboxylate (400 mg, 1.6 mmol), p-toluenesulfonic acid monohydrate (320 mg, 1.68 mmol) and water (56 mg, 0.32 mmol) was added to acetone (10 mL). The reaction mixture was stirred at 25°C for 18 h. The resultant reaction mixture was directly used in the next step of the reaction. ES-API: [M+H]⁺= 240.1.

Step 3: Potassium monopersulfate complex salt (OXONE, 5.36 g, 8.36 mmol) and water (10 mL) were added to the reaction mixture as obtained in the step 2. The reaction was carried out at 25°C for 2 h. The resultant reaction mixture was extracted with dichloromethane (15 mL x 2). The organic phase was washed with saline, dried over anhydrous magnesium sulfate, and rotary evaporated to remove the solvent, to give 2-(tert-butoxycarbonyl)octahydropenta[c]pyrrole-5-carboxylic acid (370mg, yield: 87%). ES-API:[M+H]⁺= 256.1.

Step 4: 2-(tert-butoxycarbonyl)octahydropenta[c]pyrrole-5-carboxylic acid (370 mg, 1.45 mmol), 5-phenyl-4,5-dihydropyrizole (1.06 g, 7.25 mmol), HATU (661 mg, 1.74 mmol) and triethylamine (293 mg, 2.9 mmol) were added to dichloromethane (10 mL). The reaction was carried out at 25°C for 2 h. The resultant reaction mixture was washed with saline, dried over anhydrous magnesium sulfate, rotary evaporated to remove the solvent, and subjected to column chromatography (petroleum ether/ethyl acetate=3/1) to give tert-butyl 5-(5-phenyl-4,5-dihydro-1H-pyrazole-1-carbonyl)hexahydrocyclopenta[C]pyrrole-2(1H)-carboxylate (200 mg, yield: 36%). ES-API:[M+H]⁺= 384.2.

Step 5: a mixture of tert-butyl 5-(5-phenyl-4,5-dihydro-1H-pyrazole-1-carbonyl)hexahydrocyclopenta[C]pyrrole-2(1H)-carboxylate (200 mg, 0.522 mmol) and hydrogen chloride (a solution in tetrahydrofuran, 3.5 M, 15 mL) was stirred at 25°C for 2 h. The resultant reaction mixture was rotary evaporated to remove the solvent to give (octahydrocyclopenta[c]pyrrol-5-yl)(5-phenyl-4,5-dihydro-1H-pyrazol-1-yl) ketone hydrochloride (210 mg, yield: crude product), which was directly used in the next step of the reaction without purification. ES-API:[M+H]⁺= 284.2.

Step 6: (octahydrocyclopenta[c]pyrrol-5-yl)(5-phenyl-4,5-dihydro-1H-pyrazol-1-yl) ketone hydrochloride (210 mg, 0.657 mmol), potassium carbonate (181 mg, 1.314 mmol) and triethylamine (133 mg, 1.314 mmol) were added to N,N-dimethylformamide (2 mL). The reaction mixtue was reacted at 25°C for 2 h. Thereafter, the reaction mixture was quenched with water, and extracted with ethyl acetate (5 mL x 2). The organic phase was washed with saline, dried over anhydrous magnesium sulfate, rotary evaporated to remove the solvent, and purified by preparative HPLC to give 6-(5-(5-phenyl-4,5-dihydro-1H-pyrazole-1-carbonyl)hexahydrocyclopenta[C]pyrrol-2(1H)-yl)pyrimidine-4-carbonitrile (Z-72, 45 mg, 18%). ES-API: [M+H]⁺= 387.2. ¹H NMR (400 MHz, CDCl₃) δ 8.56 (dd, *J* = 18.6, 1.2 Hz, 1H), 7.36 -6.95 (m, 6H), 6.68 (s, 1H), 6.52 (s, 1H), 5.34 (dt, *J* = 11.1, 5.3 Hz, 1H), 3.79-2.74 (m, 9H), 1.84 (s, 2H).

### Example 67 Preparation of 6-(8-(3-(3,5-difluorophenyl)-4,5-dihydro-1H-pyrazole-1-carbonyl)-5-aza-spiro[2.5]oct-5-yl)pyrimidine-4-carbonitrile (Z-73)

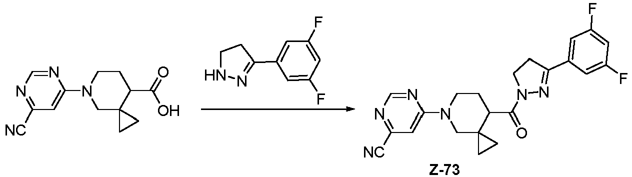

Step 1: 5-(6-cyanopyrimidin-4-yl)-5-aza-spiro[2.5]octane-8-carboxylic acid (50 mg, 0.193bmmol) was dissolved in dichloromethane (5mL). Then, HATU (88 mg, 0.232 mmol), N,N-diisopropylethylamine (47 mg, 0.386 mmol) and 3-(3,5-difluorophenyl)-4,5-dihydro-1H-pyrazole were added. The mixture was stirred at the room temperature for 16 h. Thereafter, dichloromethane (30 mL) was added. The resultant mixture was washed with water (5mL x 3). The organic phases were combined, dried over anhydrous sodium sulfate, and concentrated at a reduced pressure. The crude product was purified by preparative thin layer chromatography on silica to give 6-(8-(3-(3,5-difluorophenyl)-4,5-dihydro-1H-pyrazole-1-carbonyl)-5-aza-spiro[2.5]oct-5-yl)pyrimidine-4-carbonitrile (Z-73, 4.6 m, Y: 5.7%). ES-API:[M+H]⁺ =423.1. ¹H NMR(400 MHz, CDCl₃) δ 8.68 (d, *J* = 107.5 Hz, 1H), 7.53 (d*, J* = 8.5 Hz, 1H), 6.97 (d, *J* = 7.3 Hz, 2H), 6.79 (q*, J* = 15.2, 12.1 Hz, 4H), 3.99 (d, *J* = 13.1 Hz, 1H), 3.77 (d, *J* = 11.5 Hz, 2H), 3.17 (d*, J* = 5.4 Hz, 1H), 2.15 - 1.95 (m, 2H), 1.58 (s, 4H), 1.29 (d, *J* = 21.2 Hz, 1H), 0.94 - 0.39 (m, 4H).

### Biological Assays

The U937 cell strain which was used in the following Testing Examples was derived from ATCC, No.: CRL-1593.2, batch No.: 63479999, culture medium: RPMI-1640 + 10%FBS.

The agents and their suppliers and product Nos are as follows:
RPMI-1640, Gibco, 11875-093;
FBS, Gibco, 10099-141;
Trypsin-EDTA, Gibco, 25200-072;
PS, Gibco, 15140-122;
CellTiter Glo, Progema, G7573;
DMSO, VWR AMRESCO, 0231-500ML;
TNF-α protein (human, recombinant), Peprotech, 300-01A;
Q-VD-Oph, MCE, HY-12305;
V type plate, Corning, 3894;
384-cell low-flange white flat-bottom microplate, Corning, 3570;
RIPK1, Eurofins, 16-022;
MOPS, BDH, 441644J;
EDTA, Sigma, E5134;
Myelin basic protein, Sigma, M1891-25.00 MG;
Magnesium acetate, Merck, DU008026;
ATP (non-radioactive labeled), Sigma, A-7699;
ATP (radioactive labelled), Hartmann Analytic, DU008054;
Phosphoric acid, Metlab, DU003000.

### Testing Exmple 1: Inhibitory activity of the compounds against RIPK1 enzyme

The compound to be tested was dissolved in DMSO, and diluted in 3.16 folds with DMSO to a series of gradient concentrations. Then, the solution was diluted with MOPS buffer pH 7.0 to the final concentration. The resultant working solution was mixed with 36 nM RIPK1 (final concentration) and the substrate MBP homogeneously. Thereafter, 10 mM magnesium ion and P³³ isotopically labeled ATP were added for the reaction. After the reaction was carried out at room temperatue for 2 h, phosphoric acid was added to terminate the reaction. The final reaction system was tested using a liquid scintillation counter after treatment. The IC₅₀ values for the tested result and a positive control group were converted to activity percentage. The activity percentage was plotted against the final concentration of respective compound using a 4-parameter fitting to obtain the inhibitory IC₅₀ of the compound against RIPK1 enzyme. It can be seen from the test results that the exemplary compounds of the present invention have a higher inhibitory activity against RIPK enzyme having IC₅₀ value less than 200 nM, and even less than 100 nM.

**Table 1.**

| Compound No. | RIPK1 enzyme inhibitory activety IC₅₀ (nM) |
|---|---|
| Z-9-1 | 29 |
| Z-10 | 95 |
| Z-11 | 89 |
| Z-12 | 123 |
| Z-13 | 63 |
| Z-25-2 | 42 |
| Z-48-d | 50 |
| Z-72 | 168 |

### Testing Example 2: Inhibitory activity of the compounds against programmed necroptosis of cells

The compound to be tested was dissolved in DMSO, and diluted with DMSO to a series of gradient concentrations. The U937 cells were inoculated in a 384-well plate at 5000/well. The compound at respective concentration was added to each of the wells, and was mixed with the cells homogeneously. Human TNF-α and Q-VD-Oph were added simultaneously to induce programmed necroptosis of the cells. The cells were placed in the incubator at 37°C and 5% CO₂ for further culture of 48 h. Celltiter-Glo reagent was used for detection. After adequate pyrolysis reaction, the chemiluminescence readout value was recorded by a microplate reader. The survival rate was calculated from the detection results using the equation as follows: SR (%) = (RLU compound - RLU blank) / (RLU high control - RLU blank) ×100%. The survival rate was plotted against the final concentration of respective compound using a 4-parameter fitting to calculate the inhibitory IC₅₀ of the compound against programmed necroptosis of cells induced by TNF-a. It can be seen from the test results that the exemplary compounds of the present invention have a higher inhibitory activity against U937 cells having an IC₅₀ value less than 500 nM, and even less than 100 nM. The testing results of certain compounds are shown in Table 2.

**Table 2. Inhibitory activity of the compounds against U937 cells**

| Compound No. | U937 IC₅₀ (µM) | Compound No. | U937 IC₅₀ (µM) |
|---|---|---|---|
| Z-25 | 0.004 | Z-70-a | 0.021 |
| Z-26 | 0.244 | Z-2 | 0.156 |
| Z-46-1 | 0.004 | Z-3 | 0.322 |
| Z-40-1 | 0.009 | Z-72 | 0.030 |
| Z-25-2 | 0.001 | Z-9-1 | 0.157 |
| Z-48-1 | 0.030 | Z-10 | 0.153 |
| Z-48-2 | 0.361 | Z-11 | 0.138 |
| Z-49-1 | 0.279 | Z-13 | 0.146 |
| Z-41-1 | 0.005 | Z-14 | 0.165 |
| Z-44-2 | 0.022 | Z-17 | 0.248 |
| Z-60-2 | 0.007 | Z-18 | 0.094 |
| Z-43-2 | 0.045 | Z-20 | 0.272 |
| Z-62 | 0.007 | Z-22 | 0.070 |
| Z-52-1 | 0.004 | Z-37-2 | 0.022 |
| Z-52-2 | 0.041 | Z-23 | 0.052 |
| Z-53-1 | 0.026 | Z-24 | 0.181 |
| Z-53-2 | 0.331 | Z-30 | 0.449 |
| Z-66 | 0.020 | Z-31 | 0.408 |
| Z-61 | 0.053 | Z-32 | 0.060 |
| Z-65 | 0.052 | Z-34 | 0.380 |
| Z-67 | 0.115 | Z-38-2 | 0.027 |
| Z-48-d | 0.007 | Z-38-4 | 0.082 |
| Z-63-a | 0.032 | Z-39-4 | 0.088 |
| Z-64-a | 0.010 | Z-23-4 | 0.041 |
| Z-63-2 | 0.021 | Z-32-4 | 0.061 |
| Z-64-1 | 0.006 | Z-22-2 | 0.048 |
| Z-65-4 | 0.014 | Z-18-4 | 0.062 |
| Z-68 | 0.364 | Z-69 | 0.127 |

All references as mentioned in the present disclosure are cited for reference in this application, as if each of the references is individualy cited for reference. It is also understood that after reading the above teachings of the present invention, a person skilled in the field can make any variations or modification to the present the invention. These equivalent forms also fall within the scope as defined in the attached claims in the present application.

## Claims

1. A bisheterocyclic carbonyl-substituted dihydropyrazole compound, or a pharmaceutically acceptable salt, stereoisomer, solvate or prodrug thereof, the compound has a structure as represented by formula (I): 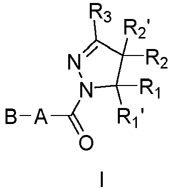 wherein,
R₁ is hydrogen, substituted or unsubstituted C₆₋₁₀ aryl, substituted or unsubstituted C₅₋₁₀ heteroaryl, substituted or unsubstituted C₃₋₆ monocyclic cycloalkyl, or substituted or unsubstituted C₃₋₆ monocyclic heterocyclyl; the "substituted" means that 1, 2 or 3 hydrogen atom(s) in the group are replaced by substituent(s) independently selected from the group S1, and the substituent(s) of the group S1 are selected from the group consisting of: cyano, acetyl, hydroxy, carboxyl, nitro, halo, C₁₋₁₀ alkyl, C₁₋₁₀ alkoxy and halo C₁₋₁₀ alkoxy, wherein the C₁₋₁₀ alkyl, the C₁₋₁₀ alkoxy are unsubstituted, or substituted by 1, 2 or 3 substituent(s) each independently selected from the group S11, the substituent(s) of the group S11 are selected from the group consisting of: acetyl, hydroxy, cyano, carboxyl, halo, C₁₋₆ alkyl, halo C₁₋₆alkyl, C₁₋₆alkoxy, halo C₁₋₆alkoxy, C₃₋₆ monocyclic cycloalkyl, C₃₋₆ monocyclic heterocyclyl, phenyl, 5- or 6-membered monoheteroaryl, NRⁱR^{j}, - C(O)NRⁱR^{j}, -SO₂NRⁱR^{j}; Rⁱ and R^{j} are each independently hydrogen or C₁₋₃ alkyl;
R₁' is hydrogen, cyano, hydroxy, cyanomethyl, cyanoethyl, hydroxymethyl, hydroxyethyl, carboxyl, halo, C₁₋₁₀ alkyl, halo C₁₋₁₀ alkyl, C₁₋₁₀ alkoxy or halo C₁₋₁₀ alkoxy;
R₂ and R₂' are each independently hydrogen, cyano, hydroxy, cyanomethyl, cyanoethyl, hydroxymethyl, hydroxyethyl, carboxyl, halo, C₁₋₁₀ alkyl, halo C₁₋₁₀ alkyl, C₁₋₁₀ alkoxy, halo C₁₋₁₀ alkoxy or C₃₋₆ monocyclic cycloalkyl;
or R₂ and R₂' together with the carbon atom attached thereto form a 3- to 6-membered saturated or partially unsaturated monocycle or a 3- to 6-membered saturated or partially unsaturated monoheterocycle;
R₃ is hydrogen, cyano, hydroxy, cyanomethyl, cyanoethyl, hydroxymethyl, hydroxyethyl, carboxyl, halo, C₁₋₁₀ alkyl, halo C₁₋₁₀ alkyl, C₁₋₁₀ alkoxy, halo C₁₋₁₀ alkoxy or C₃₋₆ monocyclic cycloalkyl;
A has a structure as represented by formula (A), formula (B), formula (C) or formula (D): 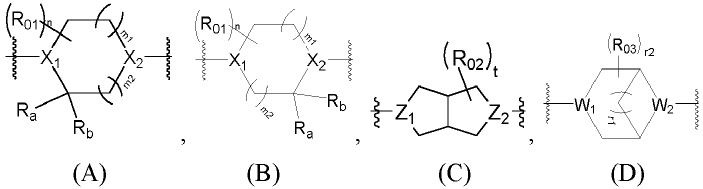
wherein X₁ is N or CR₄; X₂ is N or CR₅;
R₄ and R₅ are each independently hydrogen, hydroxy, cyano, hydroxymethyl, cyanomethyl or C₁₋₁₀ alkyl;
m1 and m2 are each independently 0, 1 or 2;
(R₀₁)ₙ represents that the hydrogen(s) on the ring are replaced by n of R₀₁, n is 0, 1, 2, 3, 4, 5 or 6, each of R₀₁ is the same or different, and is independently cyano, acetyl, hydroxy, carboxyl, nitro, halo, C₁₋₁₀ alkyl, C₁₋₁₀ alkoxy, halo C₁₋₁₀ alkyl or halo C₁₋₁₀ alkoxy;
Rₐ and R_{b} together with the carbon atom attached thereto form a 3- to 6-membered saturated or partially unsaturated monocycle or a 3- to 6-membered saturated or partially unsaturated monoheterocycle; the 3- to 6-membered saturated or partially unsaturated monocycle, the 3- to 6-membered saturated or partially unsaturated monoheterocycle are unsubstituted, or substituted by 1, 2 or 3 substituent(s) independently selected from the group S2, the substituent(s) of the group S2 are selected from the group consisting of: cyano, acetyl, hydroxy, carboxyl, nitro, halo, C₁₋₁₀ alkyl, C₁₋₁₀ alkoxy, halo C₁₋₁₀ alkoxy, C₃₋₆ monocyclic cycloalkyl, C₃₋₆ monocyclic heterocyclyl, phenyl, 5- or 6-membered monoheteroaryl, NR^{e}R^{f}, - C(O)NR^{e}R^{f}, -SO₂NR^{e}R^{f}, wherein the C₃₋₆ monocyclic cycloalkyl, the C₃₋₆ monocyclic heterocyclyl, the phenyl, the 5- or 6-membered monoheteroaryl, the C₁₋₁₀ alkyl, the C₁₋₁₀ alkoxy are unsubstituted, or substituted by 1, 2 or 3 substituent(s) each independently selected from the group S21, the substituent(s) of the group S21 are selected from the group consisting of: acetyl, hydroxy, cyano, carboxyl, halo, C₁₋₆ alkyl, halo C₁₋₆alkyl, C₁₋₆alkoxy, halo C₁₋₆alkoxy, C₃₋₆ monocyclic cycloalkyl, C₃₋₆ monocyclic heterocyclyl, phenyl, 5- or 6-membered monoheteroaryl, NR^{g}R^{h}, - C(O)NR^{g}R^{h}, -SO₂NR^{g}R^{h}; R^{e}, R^{f}, R^{g} and R^{h} are each independently hydrogen, hydroxyethyl, hydroxymethyl or C₁₋₃ alkyl;
Z₁ is N or CR₆; Z₂ is N or CR₇;
R₆ and R₇ are each independently hydrogen, hydroxy, cyano, hydroxymethyl, cyanomethyl or C₁₋₁₀ alkyl;
(R₀₂)ₜ represents that the hydrogen(s) on the ring are replaced by t of R₀₂, t is 0, 1, 2, 3, 4, 5 or 6, each of R₀₂ is the same or different, and is independently cyano, acetyl, hydroxy, carboxyl, nitro, halo, C₁₋₁₀ alkyl, C₁₋₁₀ alkoxy, halo C₁₋₁₀ alkyl or halo C₁₋₁₀ alkoxy;
W₁ is N or CR₈; W₂ is N or CR₉;
R₈ and R₉ are each independently hydrogen, hydroxy, cyano, hydroxymethyl, cyanomethyl or C₁₋₁₀ alkyl;
(R₀₃)ᵣ₂ represents that the hydrogen(s) on the ring are replaced by r2 of R₀₃, r2 is 0, 1, 2, 3, 4, 5 or 6, each of R₀₃ is the same or different, and is independently cyano, acetyl, hydroxyl, carboxyl, nitro, halo, C₁₋₁₀alkyl, C₁₋₁₀ alkoxy, halo C₁₋₁₀ alkyl or halo C₁₋₁₀ alkoxy;
r1 is 0, 1, 2 or 3;
B is a substituted or unsubstituted C₆₋₁₀ aryl, a substituted or unsubstituted C₅₋₁₀ heteroaryl, a substituted or unsubstituted C₃₋₆ monocyclic heterocyclyl; the "substituted" means that 1, 2 or 3 hydrogen atom(s) in the group are replaced by substituent(s) independently selected from the group S3, the substituent(s) of the group S3 are selected from the group consisting of: cyano, acetyl, hydroxy, carboxyl, nitro, halo, C₁₋₁₀ alkyl, C₁₋₁₀ alkoxy, C₁₋₁₀ alkylthio, halo C₁₋₁₀ alkoxy, NR^{a}R^{b}, - CONR^{a}R^{b}, -CONR^{a}NR^{a}R^{b}, -NR^{a}COC₁₋₁₀ alkyl, -CO₂C₁₋₁₀ alkyl, -SO₂NR^{a}R^{b}, -SO₂C₁₋₁₀ alkyl, -CO-C₃₋₆ monocyclic heterocyclyl, -(CH₂)ᵤ-C₃₋₆ monocyclic cycloalkyl, - (CH₂)ᵤ-C₆₋₁₀ aryl, -(CH₂)ᵤ-5- or 6-membered monoheteroaryl, -(CH₂)ᵤ-C₃₋₆ monocyclic heterocyclyl;
wherein, among the substituents of the group S3, the C₁₋₁₀ alkyl, the C₁₋₁₀ alkoxy, the C₃₋₆ monocyclic cycloalkyl, the C₃₋₆ monocyclic heterocyclyl, the C₆₋₁₀ aryl, the 5- or 6-membered monoheteroaryl are unsubstituted, or substituted by 1, 2 or 3 substituent(s) each independently selected from the group S31, the substituent(s) of the group S31 are selected from the group consisting of: acetyl, hydroxy, cyano, carboxyl, nitro, halo, C₁₋₃ alkyl, halo C₁₋₃ alkyl, C₁₋₃ alkoxy, halo C₁₋₃ alkoxy, C₃₋₆ monocyclic cycloalkyl, C₃₋₆ monocyclic heterocyclyl, phenyl, 5- or 6-membered monoheteroaryl, NR^{c}R^{d}, -C(O)NR^{c}R^{d} and -SO₂NR^{c}R^{d};
R^{a}, R^{b}, R^{c}, R^{d} are each independently hydrogen, hydroxymethyl, hydroxyethyl, C₁₋₃ alkyl, C₃₋₆ monocyclic cycloalkyl or C₃₋₆ monocyclic heterocyclyl; wherein the C₃₋₆ monocyclic cycloalkyl is selected from the group consisting of: cyclopropyl, cyclobutyl, cyclopentyl and cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran; and the C₃₋₆ monocyclic heterocyclyl is optionally substituted by 1, 2 or 3 C₁₋₃ alkyl or acetyl;
u is 0, 1, 2, 3 or 4.

2. The compound or a pharmaceutically acceptable salt, stereoisomer, solvate or prodrug thereof according to claim 1, **characterized in that**, the substituent of the group S3 is cyano, acetyl, hydroxy, carboxyl, halo, C₁₋₃ alkyl, C₁₋₃ alkoxy, C₁₋₃ alkylthio, halo C₁₋₃ alkoxy, NR^{a}R^{b}, -CONR^{a}R^{b}, -CONHNR^{a}R^{b}, -NHCOC₁₋₃ alkyl, - CO₂C₁₋₃ alkyl, -SO₂NR^{a}R^{b}, -SO₂C₁₋₃ alkyl, -CO-C₃₋₆ monocyclic heterocyclyl, - (CH₂)ᵤ-C₃₋₆ monocyclic cycloalkyl, -(CH₂)ᵤ-phenyl, -(CH₂)ᵤ-5- or 6-membered monoheteroaryl, -(CH₂)ᵤ-C₃₋₆ monocyclic heterocyclyl;
wherein, among the substituents of the group S3, the C₁₋₃ alkyl, the C₁₋₃ alkoxy, the C₃₋₆ monocyclic cycloalkyl, the C₃₋₆ monocyclic heterocyclyl, the phenyl, the 5- or 6-membered monoheteroaryl are unsubstituted, or substituted by 1, 2 or 3 substituent(s) each independently selected from the group S31, the substituent(s) of the group S31 is selected from the group consisitng of: acetyl, hydroxy, cyano, carboxyl, halo, C₁₋₃ alkyl, halo C₁₋₃ alkyl, C₁₋₃ alkoxy, halo C₁₋₃ alkoxy, C₃₋₆ monocyclic cycloalkyl, C₃₋₆ monocyclic heterocyclyl, phenyl, 5- or 6-membered monoheteroaryl, NR^{c}R^{d}, -C(O)NR^{c}R^{d} and -SO₂NR^{c}R^{d};
R^{a}, R^{b}, R^{c}, R^{d} are each independently hydrogen, hydroxymethyl, hydroxyethyl, C₁₋₃ alkyl, C₃₋₆ monocyclic cycloalkyl or C₃₋₆ monocyclic heterocyclyl; wherein the C₃₋₆ monocyclic cycloalkyl is selected from the group consisting of: cyclopropyl, cyclobutyl, cyclopentyl and cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran; and the C₃₋₆ monocyclic heterocyclyl is optionally substituted by 1, 2 or 3 C₁₋₃ alkyl or acetyl;
u is 0, 1, 2 or 3.

3. The compound or a pharmaceutically acceptable salt, stereoisomer, solvate or prodrug thereof according to claim 1, **characterized in that**, the C₆₋₁₀ aryl in R₁ is a phenyl, a 9- or 10-membered aromatic fused bicyclic ring formed by fusing a phenyl to one 5- or 6-membered monocyclic heterocyclyl ring, or a 9- or 10-membered aromatic fused bicyclic ring formed by fusing a phenyl to one 5- or 6-membered monocyclic cycloalkyl ring.

4. The compound or a pharmaceutically acceptable salt, stereoisomer, solvate or prodrug thereof according to claim 1, **characterized in that**, the C₅₋₁₀ heteroaryl in R₁ is a 5- or 6-membered monoheteroaryl, a 9- or 10-membered biheteroaryl formed by fusing a phenyl to a 5- or 6-membered monoheteroaryl, a 8- to 10-membered biheteroaryl formed by fusing a 5- or 6-membered monoheteroaryl to a 5- or 6-membered monoheteroaryl, a 8- to 10-membered biheteroaryl formed by fusing a 5- or 6-membered monoheteroaryl to one 5- or 6-membered monocyclic heterocyclyl ring, or a 8- to 10-membered biheteroaryl formed by fusing a 5- or 6-membered monoheteroaryl to one 5- or 6-membered monocyclic cycloalkyl ring.

5. The compound or a pharmaceutically acceptable salt, stereoisomer, solvate or prodrug thereof according to claim 1, **characterized in that**, R₁ is a substituted or unsubstituted phenyl, or a substituted or unsubstituted 5- or 6-membered monoheteroaryl, the "substituted" means that 1, 2 or 3 hydrogen atom(s) in the group are each replaced by substituent(s) independently selected from the group consisting of: cyano, halo, C₁₋₁₀ alkyl, halo C₁₋₁₀ alkyl, C₁₋₁₀ alkoxy and halo C₁₋₁₀ alkoxy.

6. The compound or a pharmaceutically acceptable salt, stereoisomer, solvate or prodrug thereof according to claim 1, **characterized in that**, the C₆₋₁₀ aryl in B is a phenyl, a 9- or 10-membered aromatic fused bicyclic ring formed by fusing a phenyl to one 5- or 6-membered monocyclic heterocyclyl ring, or a 9- or 10-membered aromatic fused bicyclic ring formed by fusing a phenyl to one 5- or 6-membered monocyclic cycloalkyl ring.

7. The compound or a pharmaceutically acceptable salt, stereoisomer, solvate or prodrug thereof according to claim 1, **characterized in that**, the C₅₋₁₀ heteroaryl in B is a 5- or 6-membered monoheteroaryl, a 9- or 10-membered biheteroaryl formed by fusing a phenyl to a 5- or 6-membered monoheteroaryl, a 8- to 10-membered biheteroaryl formed by fusing a 5- or 6-membered monoheteroaryl to a 5- or 6-membered monoheteroaryl, a 8- to 10-membered biheteroaryl formed by fusing a 5- or 6-membered monoheteroaryl to one 5- or 6-membered monocyclic heterocyclyl ring, or a 8- to 10-membered biheteroaryl formed by fusing a 5- or 6-membered monoheteroaryl to one 5- or 6-membered monocyclic cycloalkyl ring.

8. The compound or a pharmaceutically acceptable salt, stereoisomer, solvate or prodrug thereof according to claim 4 or 7, **characterized in that**, if the C₅₋₁₀ heteroaryl in R₁, B is a 5- or 6-membered monoheteroaryl, the 5- or 6-membered monoheteroaryl is each independently selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

9. The compound or a pharmaceutically acceptable salt, stereoisomer, solvate or prodrug thereof according to claim 1, **characterized in that**, formula (A) and formula (B) have the structures as represented by formula (A-1) and formula (B-1), respectively, 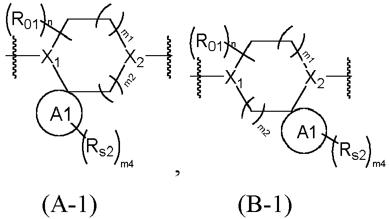
wherein R₀₁, n, m1, m2, X₁, X₂ are as defined in claim 1;
the A1 ring is a 3- to 6-membered saturated monocyclic ring or a 3- to 6-membered saturated monoheterocyclyl ring;
(Rₛ₂)ₘ₄ represents that the hydrogen(s) on the A1 ring are replaced by m4 of Rₛ₂, m4 is 0, 1, 2 or 3, each of Rₛ₂ is the same or different, and is independently a substituent selected from the group S2.

10. The compound or a pharmaceutically acceptable salt, stereoisomer, solvate or prodrug thereof according to claim 1, **characterized in that**, formula (A) and formula (B) have the structures as represented by formula (A-2) and (B-2), respectively, 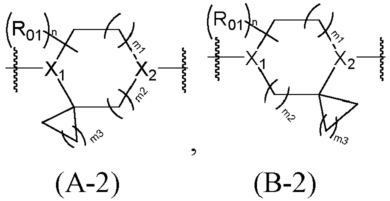 wherein R₀₁, n, m1, m2, X₁ and X₂ are as defined in claim 1; m3 is 1, 2, 3 or 4.

11. A bisheterocyclic carbonyl-substituted dihydropyrazole compound, or a pharmaceutically acceptable salt, stereoisomer, solvate or prodrug thereof, the compound has a structure as represented by formula (I-1-1-4): 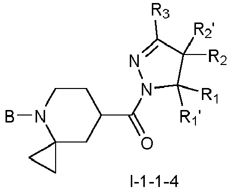 wherein,
R₁ is hydrogen, substituted or unsubstituted C₆₋₁₀ aryl, substituted or unsubstituted C₅₋₁₀ heteroaryl, substituted or unsubstituted C₃₋₆ monocyclic cycloalkyl, or substituted or unsubstituted C₃₋₆ monocyclic heterocyclyl; the "substituted" means that 1, 2 or 3 hydrogen atom(s) in the group are replaced by substituent(s) independently selected from the group S1, the substituent(s) of the group S1 are selected from the group consisting of: cyano, acetyl, hydroxy, carboxyl, nitro, halo, C₁₋₁₀ alkyl, C₁₋₁₀ alkoxy, halo C₁₋₁₀ alkoxy, wherein the C₁₋₁₀ alkyl, the C₁₋₁₀ alkoxy are unsubstituted, or substituted by 1, 2 or 3 substituent(s) each independently selected from the group S11, the substituent(s) of the group S11 are selected from the group consisting of: acetyl, hydroxy, cyano, carboxyl, halo, C₁₋₆ alkyl, halo C₁₋₆alkyl, C₁₋₆alkoxy, halo C₁₋₆alkoxy, C₃₋₆ monocyclic cycloalkyl, C₃₋₆ monocyclic heterocyclyl, phenyl, 5- or 6-membered monoheteroaryl, NRⁱR^{j}, - C(O)NRⁱR^{j}, -SO₂NRⁱR^{j}; Rⁱ and R^{j} are each independently hydrogen or C₁₋₃ alkyl;
R₁' is hydrogen, cyano, hydroxy, cyanomethyl, cyanoethyl, hydroxymethyl, hydroxyethyl, carboxyl, halo, C₁₋₁₀ alkyl, halo C₁₋₁₀ alkyl, C₁₋₁₀ alkoxy or halo C₁₋₁₀ alkoxy;
R₂ and R₂' are each independently hydrogen, cyano, hydroxy, cyanomethyl, cyanoethyl, hydroxymethyl, hydroxyethyl, carboxyl, halo, C₁₋₁₀ alkyl, halo C₁₋₁₀ alkyl, C₁₋₁₀ alkoxy, halo C₁₋₁₀ alkoxy or C₃₋₆ monocyclic cycloalkyl;
or R₂ and R₂' together with the carbon atom attached thereto form a 3- to 6-membered saturated or partially unsaturated monocycle or a 3- to 6-membered saturated or partially unsaturated monoheterocycle;
R₃ is hydrogen, cyano, hydroxy, cyanomethyl, cyanoethyl, hydroxymethyl, hydroxyethyl, carboxyl, halo, C₁₋₁₀ alkyl, halo C₁₋₁₀ alkyl, C₁₋₁₀ alkoxy, halo C₁₋₁₀ alkoxy or C₃₋₆ monocyclic cycloalkyl;
B is a substituted or unsubstituted C₆₋₁₀ aryl, a substituted or unsubstituted C₅₋₁₀ heteroaryl, a substituted or unsubstituted C₃₋₆ monocyclic heterocyclyl; the "substituted" means that 1, 2 or 3 hydrogen atom(s) in the group are replaced by substituent(s) independently selected from the group S3, the substituent(s) of the group S3 are selected from the group consisting of: cyano, acetyl, hydroxy, carboxyl, nitro, halo, C₁₋₁₀alkyl, C₁₋₁₀alkoxy, C₁₋₁₀alkylthio, halo C₁₋₁₀alkoxy, NR^{a}R^{b}, - CONR^{a}R^{b}, -CONR^{a}NR^{a}R^{b}, -NR^{a}COC₁₋₁₀ alkyl, -CO₂C₁₋₁₀ alkyl, -SO₂NR^{a}R^{b}, -SO₂C₁₋₁₀ alkyl, -CO-C₃₋₆ monocyclic heterocyclyl, -(CH₂)ᵤ-C₃₋₆ monocyclic cycloalkyl, - (CH₂)ᵤ-C₆₋₁₀ aryl, -(CH₂)ᵤ-5- or 6-membered monoheteroaryl and -(CH₂)ᵤ-C₃₋₆ monocyclic heterocyclyl;
wherein, among the substituents of the group S3, the C₁₋₁₀ alkyl, the C₁₋₁₀ alkoxy, the C₃₋₆ monocyclic cycloalkyl, the C₃₋₆ monocyclic heterocyclyl, the C₆₋₁₀ aryl, the 5- or 6-membered monoheteroaryl are unsubstituted, or substituted by 1, 2 or 3 substituent(s) each independently selected from the group S31, the substituent(s) of the group S31 are selected from the group consisting of: acetyl, hydroxy, cyano, carboxyl, nitro, halo, C₁₋₃ alkyl, halo C₁₋₃ alkyl, C₁₋₃ alkoxy, halo C₁₋₃ alkoxy, C₃₋₆ monocyclic cycloalkyl, C₃₋₆ monocyclic heterocyclyl, phenyl, 5- or 6-membered monoheteroaryl, NR^{c}R^{d}, -C(O)NR^{c}R^{d} and -SO₂NR^{c}R^{d};
R^{a}, R^{b}, R^{c}, R^{d} are each independently hydrogen, hydroxymethyl, hydroxyethyl, C₁₋₃ alkyl, C₃₋₆ monocyclic cycloalkyl or C₃₋₆ monocyclic heterocyclyl; wherein the C₃₋₆ monocyclic cycloalkyl is selected from the group consisting of: cyclopropyl, cyclobutyl, cyclopentyl and cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran; and the C₃₋₆ monocyclic heterocyclyl is optionally substituted by 1, 2 or 3 C₁₋₃ alkyl, acetyl;
u is 0, 1, 2, 3 or 4.

12. The compound or a pharmaceutically acceptable salt, stereoisomer, solvate or prodrug thereof according to claim 11, the compound has a structure as represented by formula (I-a-1-4): 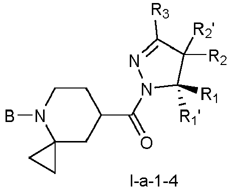 wherein, the R₁, R₁', R₂, R₂', R₃ and B are as defined in claim 11.

13. A bisheterocyclic carbonyl-substituted dihydropyrazole compound, or a pharmaceutically acceptable salt, stereoisomer, solvate or prodrug thereof, the compound has a structure as represented by formula (I-2-1-4): 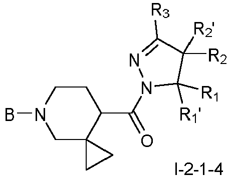 wherein,
R₁ is hydrogen, substituted or unsubstituted C₆₋₁₀ aryl, substituted or unsubstituted C₅₋₁₀ heteroaryl, substituted or unsubstituted C₃₋₆ monocyclic cycloalkyl, or substituted or unsubstituted C₃₋₆ monocyclic heterocyclyl; the "substituted" means that 1, 2 or 3 hydrogen atom(s) in the group are replaced by substituent(s) independently selected from the group S1, the substituent(s) of the group S1 are selected from the group consisting of: cyano, acetyl, hydroxy, carboxyl, nitro, halo, C₁₋₁₀ alkyl, C₁₋₁₀ alkoxy, halo C₁₋₁₀ alkoxy, wherein the C₁₋₁₀ alkyl, the C₁₋₁₀ alkoxy are unsubstituted, or substituted by 1, 2 or 3 substituent(s) each independently selected from the group S11, the substituent(s) of the group S11 are selected from the group consisting of: acetyl, hydroxy, cyano, carboxyl, halo, C₁₋₆ alkyl, halo C₁₋₆alkyl, C₁₋₆alkoxy, halo C₁₋₆alkoxy, C₃₋₆ monocyclic cycloalkyl, C₃₋₆ monocyclic heterocyclyl, phenyl, 5- or 6-membered monoheteroaryl, NRⁱR^{j}, - C(O)NRⁱR^{j}, -SO₂NRⁱR^{j}; Rⁱ and R^{j} are each independently hydrogen or C₁₋₃ alkyl;
R₁' is hydrogen, cyano, hydroxy, cyanomethyl, cyanoethyl, hydroxymethyl, hydroxyethyl, carboxyl, halo, C₁₋₁₀ alkyl, halo C₁₋₁₀ alkyl, C₁₋₁₀ alkoxy or halo C₁₋₁₀ alkoxy;
R₂ and R₂' are each independently hydrogen, cyano, hydroxy, cyanomethyl, cyanoethyl, hydroxymethyl, hydroxyethyl, carboxyl, halo, C₁₋₁₀ alkyl, halo C₁₋₁₀ alkyl, C₁₋₁₀ alkoxy, halo C₁₋₁₀ alkoxy or C₃₋₆ monocyclic cycloalkyl;
or R₂ and R₂' together with the carbon atom attached thereto form a 3- to 6-membered saturated or partially unsaturated monocycle or a 3- to 6-membered saturated or partially unsaturated monoheterocycle;
R₃ is hydrogen, cyano, hydroxy, cyanomethyl, cyanoethyl, hydroxymethyl, hydroxyethyl, carboxyl, halo, C₁₋₁₀ alkyl, halo C₁₋₁₀ alkyl, C₁₋₁₀ alkoxy, halo C₁₋₁₀ alkoxy or C₃₋₆ monocyclic cycloalkyl;
B is a substituted or unsubstituted C₆₋₁₀ aryl, a substituted or unsubstituted C₅₋₁₀ heteroaryl, a substituted or unsubstituted C₃₋₆ monocyclic heterocyclyl; the "substituted" means that 1, 2 or 3 hydrogen atom(s) in the group are replaced by substituent(s) independently selected from the group S3, the substituent(s) of the group S3 are selected from the group consisting of: cyano, acetyl, hydroxy, carboxyl, nitro, halo, C₁₋₁₀alkyl, C₁₋₁₀alkoxy, C₁₋₁₀alkylthio, halo C₁₋₁₀alkoxy, NR^{a}R^{b}, - CONR^{a}R^{b}, -CONR^{a}NR^{a}R^{b}, -NR^{a}COC₁₋₁₀ alkyl, -CO₂C₁₋₁₀ alkyl, -SO₂NR^{a}R^{b}, -SO₂C₁₋₁₀ alkyl, -CO-C₃₋₆ monocyclic heterocyclyl, -(CH₂)ᵤ-C₃₋₆ monocyclic cycloalkyl, - (CH₂)ᵤ-C₆₋₁₀ aryl, -(CH₂)ᵤ-5- or 6-membered monoheteroaryl and -(CH₂)ᵤ-C₃₋₆ monocyclic heterocyclyl;
wherein, among the substituents of the group S3, the C₁₋₁₀ alkyl, the C₁₋₁₀ alkoxy, the C₃₋₆ monocyclic cycloalkyl, the C₃₋₆ monocyclic heterocyclyl, the C₆₋₁₀ aryl, the 5- or 6-membered monoheteroaryl are unsubstituted, or substituted by 1, 2 or 3 substituent(s) each independently selected from the group S31, the substituent(s) of the group S31 are selected from the group consisting of: acetyl, hydroxy, cyano, carboxyl, nitro, halo, C₁₋₃ alkyl, halo C₁₋₃ alkyl, C₁₋₃ alkoxy, halo C₁₋₃ alkoxy, C₃₋₆ monocyclic cycloalkyl, C₃₋₆ monocyclic heterocyclyl, phenyl, 5- or 6-membered monoheteroaryl, NR^{c}R^{d}, -C(O)NR^{c}R^{d} and -SO₂NR^{c}R^{d};
R^{a}, R^{b}, R^{c}, R^{d} are each independently hydrogen, hydroxymethyl, hydroxyethyl, C₁₋₃ alkyl, C₃₋₆ monocyclic cycloalkyl or C₃₋₆ monocyclic heterocyclyl; wherein the C₃₋₆ monocyclic cycloalkyl is selected from the group consisting of: cyclopropyl, cyclobutyl, cyclopentyl and cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran; and the C₃₋₆ monocyclic heterocyclyl is optionally substituted by 1, 2 or 3 C₁₋₃ alkyl, acetyl;
u is 0, 1, 2, 3 or 4.

14. The compound or a pharmaceutically acceptable salt, stereoisomer, solvate or prodrug thereof according to claim 13, the compound has a structure as represented by formula (I-b-1-4): 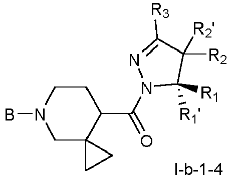 wherein, the R₁, R₁', R₂, R₂', R₃ and B are as defined in claim 13.

15. A bisheterocyclic carbonyl-substituted dihydropyrazole compound, or a pharmaceutically acceptable salt, stereoisomer, solvate or prodrug thereof, the compound has a structure as represented by formula (I-3-1-1) or formula (I-3-1-2): 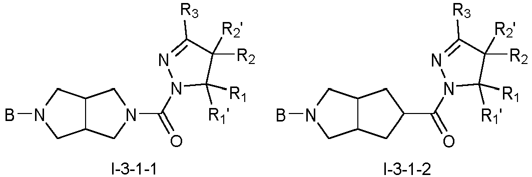 wherein,
R₁ is each independently hydrogen, substituted or unsubstituted C₆₋₁₀ aryl, substituted or unsubstituted C₅₋₁₀ heteroaryl, substituted or unsubstituted C₃₋₆ monocyclic cycloalkyl, or substituted or unsubstituted C₃₋₆ monocyclic heterocyclyl; the "substituted" means that 1, 2 or 3 hydrogen atom(s) in the group are replaced by substituent(s) independently selected from the group S1, the substituent(s) of the group S1 are selected from the group consisting of: cyano, acetyl, hydroxy, carboxyl, nitro, halo, C₁₋₁₀ alkyl, C₁₋₁₀ alkoxy, halo C₁₋₁₀ alkoxy, wherein the C₁₋₁₀ alkyl and C₁₋₁₀ alkoxy are unsubstituted, or substituted by 1, 2 or 3 substituent(s) each independently selected from the group S11, the substituent(s) of the group S11 are selected from the group consisting of: acetyl, hydroxy, cyano, carboxyl, halo, C₁₋₆ alkyl, halo C₁₋₆alkyl, C₁₋₆alkoxy, halo C₁₋₆alkoxy, C₃₋₆ monocyclic cycloalkyl, C₃₋₆ monocyclic heterocyclyl, phenyl, 5- or 6-membered monoheteroaryl, NRⁱR^{j}, - C(O)NRⁱR^{j}, -SO₂NRⁱR^{j}; Rⁱ and R^{j} are each independently hydrogen or C₁₋₃ alkyl;
R₁' is each independently hydrogen, cyano, hydroxy, cyanomethyl, cyanoethyl, hydroxymethyl, hydroxyethyl, carboxyl, halo, C₁₋₁₀ alkyl, halo C₁₋₁₀ alkyl, C₁₋₁₀ alkoxy or halo C₁₋₁₀ alkoxy;
R₂ and R₂' are each independently hydrogen, cyano, hydroxy, cyanomethyl, cyanoethyl, hydroxymethyl, hydroxyethyl, carboxyl, halo, C₁₋₁₀ alkyl, halo C₁₋₁₀ alkyl, C₁₋₁₀ alkoxy, halo C₁₋₁₀ alkoxy or C₃₋₆ monocyclic cycloalkyl;
or R₂ and R₂' together with the carbon atom attached thereto form a 3- to 6-membered saturated or partially unsaturated monocycle or a 3- to 6-membered saturated or partially unsaturated monoheterocycle;
R₃ is each independently hydrogen, cyano, hydroxy, cyanomethyl, cyanoethyl, hydroxymethyl, hydroxyethyl, carboxyl, halo, C₁₋₁₀ alkyl, halo C₁₋₁₀ alkyl, C₁₋₁₀ alkoxy, halo C₁₋₁₀ alkoxy or C₃₋₆ monocyclic cycloalkyl;
B is each independently a substituted or unsubstituted C₆₋₁₀ aryl, a substituted or unsubstituted C₅₋₁₀ heteroaryl, a substituted or unsubstituted C₃₋₆ monocyclic heterocyclyl; the "substituted" means that 1, 2 or 3 hydrogen atom(s) in the group are replaced by substituent(s) independently selected from the group S3, the substituent(s) of the group S3 are selected from the group consisting of: cyano, acetyl, hydroxy, carboxyl, nitro, halo, C₁₋₁₀alkyl, C₁₋₁₀alkoxy, C₁₋₁₀alkylthio, halo C₁₋₁₀alkoxy, NR^{a}R^{b}, -CONR^{a}R^{b}, -CONR^{a}NR^{a}R^{b}, -NR^{a}COC₁₋₁₀ alkyl, -CO₂C₁₋₁₀ alkyl, -SO₂NR^{a}R^{b}, -SO₂C₁₋₁₀ alkyl, -CO-C₃₋₆ monocyclic heterocyclyl, -(CH₂)ᵤ-C₃₋₆ monocyclic cycloalkyl, -(CH₂)ᵤ-C₆₋₁₀ aryl, -(CH₂)ᵤ-5- or 6-membered monoheteroaryl and -(CH₂)ᵤ-C₃₋₆ monocyclic heterocyclyl;
wherein, among the substituents of the group S3, the C₁₋₁₀ alkyl, the C₁₋₁₀ alkoxy, the C₃₋₆ monocyclic cycloalkyl, the C₃₋₆ monocyclic heterocyclyl, the C₆₋₁₀ aryl, the 5- or 6-membered monoheteroaryl are unsubstituted, or substituted by 1, 2 or 3 substituent(s) each independently selected from the group S31, the substituent(s) of the group S31 are selected from the group consisting of: acetyl, hydroxy, cyano, carboxyl, nitro, halo, C₁₋₃ alkyl, halo C₁₋₃ alkyl, C₁₋₃ alkoxy, halo C₁₋₃ alkoxy, C₃₋₆ monocyclic cycloalkyl, C₃₋₆ monocyclic heterocyclyl, phenyl, 5- or 6-membered monoheteroaryl, NR^{c}R^{d}, -C(O)NR^{c}R^{d} and -SO₂NR^{c}R^{d};
R^{a}, R^{b}, R^{c}, R^{d} are each independently hydrogen, hydroxymethyl, hydroxyethyl, C₁₋₃ alkyl, C₃₋₆ monocyclic cycloalkyl or C₃₋₆ monocyclic heterocyclyl; wherein the C₃₋₆ monocyclic cycloalkyl is selected from the group consisting of: cyclopropyl, cyclobutyl, cyclopentyl and cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran; and the C₃₋₆ monocyclic heterocyclyl is optionally substituted by 1, 2 or 3 C₁₋₃ alkyl, acetyl;
u is 0, 1, 2, 3 or 4.

16. The compound or a pharmaceutically acceptable salt, stereoisomer, solvate or prodrug thereof according to any one of claims 1 and 11-15, **characterized in that**, B has a structure as represented by: 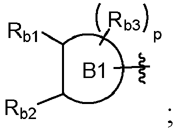 where the B1 ring is a phenyl ring, a 5- or 6-membered monoheteroaryl ring;
wherein the 5- or 6-membered monoheteroaryl ring is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine;
(R_{b3})ₚ represents that the hydrogen(s) on the ring are replaced by p of R_{b3}, p is 0, 1, 2 or 3, each of R_{b3} is the same or different, and is independently a substituent selected from the group S3;
R_{b1} and R_{b2} represent the substituents on adjacent ring atoms, and are each independently hydrogen or a substituent selected from the group S3;
or R_{b1} and R_{b2} together with the ring atoms attached thereto form a fused phenyl, a fused 5- or 6-membered monoheteroaryl ring, a fused 5- or 6-membered monocyclic heterocyclyl ring, or a fused 5- or 6-membered monocyclic cycloalkyl ring; wherein the fused 5- or 6-membered monoheteroaryl ring and the fused 5- or 6-membered monocyclic heterocyclyl ring each have 1, 2 or 3 of heteroatom(s) selected from the group consisting of N, O and S as the ring atom(s); the fused phenyl, the fused 5- or 6-membered monoheteroaryl ring, the fused 5- or 6-membered monocyclic heterocyclyl ring and the fused 5- or 6-membered monocyclic cycloalkyl ring are optionally substituted by 1, 2 or 3 substituent(s) independently selected from the group S3.

17. The compound or a pharmaceutically acceptable salt, stereoisomer, solvate or prodrug thereof according to claim 16, **characterized in that**, the B1 ring is a phenyl ring, R_{b1} and R_{b2} represent the substituents on adjacent ring atoms, and are each independently hydrogen or a substituent selected from the group S3; or R_{b1} and R_{b2} together with the ring atoms attached thereto form a fused 5- or 6-membered monoheteroaryl ring, a fused 5- or 6-membered monocyclic heterocyclyl ring, or a fused 5- or 6-membered monocyclic cycloalkyl ring; wherein the fused 5- or 6-membered monoheteroaryl ring and the fused 5- or 6-membered monocyclic heterocyclyl ring each have 1, 2 or 3 heteroatom(s) selected from the group consisting of N, O and S as the ring atom(s); the fused 5- or 6-membered monoheteroaryl ring, the fused 5- or 6-membered monocyclic heterocyclyl ring and the fused 5- or 6-membered monocyclic cycloalkyl ring are optionally substituted by 1, 2 or 3 substituent(s) independently selected from the group S3.

18. The compound or a pharmaceutically acceptable salt, stereoisomer, solvate or prodrug thereof according to claim 16, **characterized in that**, the B1 ring is a 5- or 6-membered monoheteroaryl ring, wherein the 5- or 6-membered monoheteroaryl ring is selected from the group consisiting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine;
R_{b1} and R_{b2} represent the substituents on adjacent ring atoms, and are each independently hydrogen or a substituent selected from the group S3; or R_{b1} and R_{b2} together with the ring atoms attached thereto form a fused phenyl ring, a fused 5- or 6-membered monoheteroaryl ring, a fused 5- or 6-membered monocyclic heterocyclyl ring, or a fused 5- or 6-membered monocyclic cycloalkyl ring; wherein the fused 5- or 6-membered monoheteroaryl ring and the fused 5- or 6-membered monocyclic heterocyclyl ring each have 1, 2 or 3 heteroatom(s) selected from the group consisting of N, O and S as the ring atom(s); the fused phenyl ring, the fused 5- or 6-membered monoheteroaryl ring, the fused 5- or 6-membered monocyclic heterocyclyl ring and the fused 5- or 6-membered monocyclic cycloalkyl ring are optionally substituted by 1, 2 or 3 substituent(s) independently selected from the group S3.

19. The compound or a pharmaceutically acceptable salt, stereoisomer, solvate or prodrug thereof according to any one of claims 1 and 11-15, **characterized in that**, B has a structure as represented by: 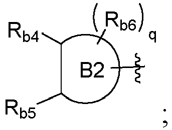
wherein the B2 ring is a 5- or 6-membered monocyclic heterocyclyl ring;
wherein the 5- or 6-membered monocyclic heterocyclyl ring is selected from the group consisting of: oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrol-2,5-dione, furan-2(3H)-one, furan-2(5H)-one, 1,3-dioxol-2-one, oxazol-2(3H)-one, 1,3-dihydro-2H-imidazol-2-one, furan-2,5-dione, 3,6-dihydropyridin-2(1H)-one, pyridin-2,6-(1H,3H)-dione, 5,6-dihydro-2H-pyran-2-one, 3,6-dihydro-2H-pyran-2-one, 3,4-dihydro-2H-1,3-oxazine, 3,6-dihydro-2H-1,3-oxazine and 1,2,3,4-tetrahydropyrimidine;
(R_{b6})_{q} represents that the hydrogen(s) on the ring are replaced by q of R_{b6}, q is 0, 1, 2 or 3, each of R_{b6} is the same or different, and is independently a substituent selected from the group S3;
R_{b4} and R_{b5} represent the substituents on adjacent ring atoms, and are each independently hydrogen or a substituent selected from the group S3;
or R_{b4} and R_{b5} together with the ring atoms attached thereto form a fused phenyl, or a fused 5- or 6-membered monoheteroaryl ring; wherein the fused 5- or 6-membered monoheteroaryl ring has 1, 2 or 3 of heteroatom(s) selected from the group consisting of N, O and S as the ring atom(s); the fused phenyl and the fused 5- or 6-membered monoheteroaryl ring are optionally substituted by 1, 2 or 3 substituent(s) independently selected from the group S3.

20. The compound or a pharmaceutically acceptable salt, stereoisomer, solvate or prodrug thereof according to any one of claims 1 and 11-15, **characterized in that**, B is substituted or unsubstituted phenyl, or substituted or unsubstituted 5- or 6-membered monoheteroaryl, the "substituted" means that 1, 2 or 3 hydrogen atom(s) in the group are replaced by substituent(s) independently selected from the group S3.

21. The compound or a pharmaceutically acceptable salt, stereoisomer, solvate or prodrug thereof according to any one of claims 1 and 11-15, **characterized in that**, B is substituted or unsubstituted pyrimidine, substituted or unsubstituted pyridine, substituted or unsubstituted pyrazine, substituted or unsubstituted pyridazine, substituted or unsubstituted oxazole, substituted or unsubstituted thiazole, substituted or unsubstituted oxadiazole, substituted or unsubstituted pyrimidoimidazole, substituted or unsubstituted pyrimidopyrazole, substituted or unsubstituted pyrazo[1,5-a]pyrimidine, substituted or unsubstituted imidazo[1,2-b]pyridazine, substituted or unsubstituted quinoxaline, substituted or unsubstituted 5,7-dihydro-6H-pyrrolo[2,3-d]pyrimidin-6-one, substituted or unsubstituted 1,7-dihydro-4H-pyrazo[3,4-d]pyrimidin-4-one, substituted or unsubstituted pyrimidin-4(3H)-one; the "substituted" means that 1, 2 or 3 hydrogen atom(s) in the group are replaced by substituent(s) independently selected from the group S3.

22. The compound or a pharmaceutically acceptable salt, stereoisomer, solvate or prodrug thereof according to any one of claims 1 and 11-15, **characterized in that**, B has a structure selected from the group consisting of: 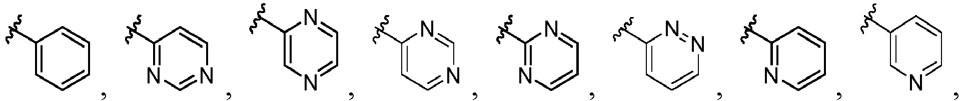 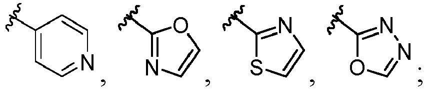 the structure is unsubstituted, or is substituted by 1, 2 or 3 substituent(s) independently selected from the group S3.

23. The compound or a pharmaceutically acceptable salt, stereoisomer, solvate or prodrug thereof according to any one of claims 1 and 11-15, **characterized in that**, B has a structure selected from the group consisting of: 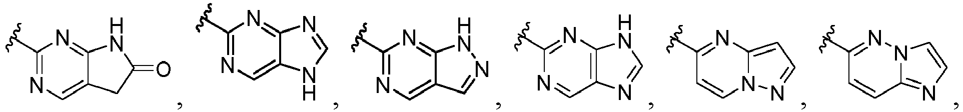 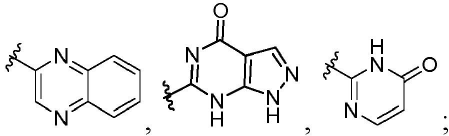 the structure is unsubstituted, or is substituted by 1, 2 or 3 substituent(s) independently selected from the group consisting of halo and C₁₋₃ alkyl.

24. The compound or a pharmaceutically acceptable salt, stereoisomer, solvate or prodrug thereof according to any one of claims 1 and 11-15, **characterized in that**, B has a structure selected from the group consisting of:

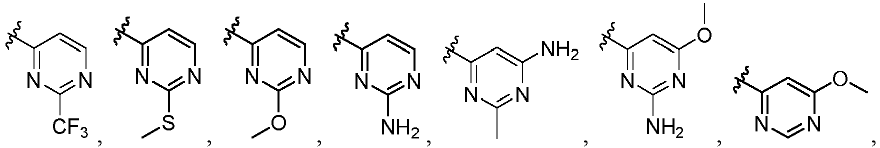

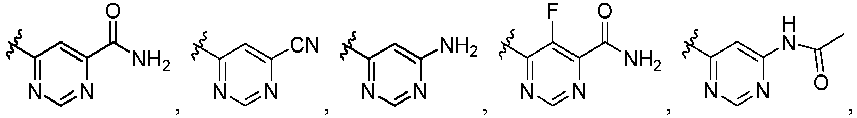

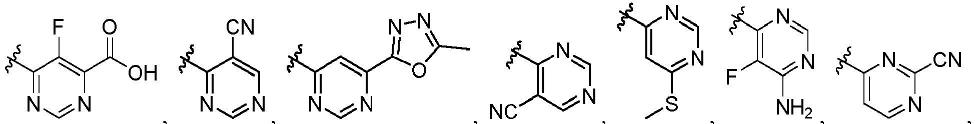

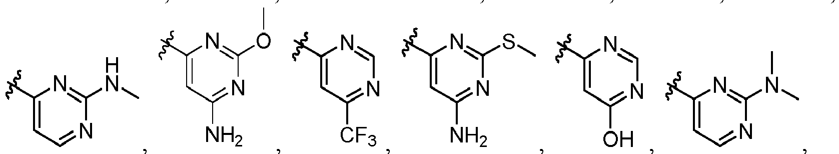

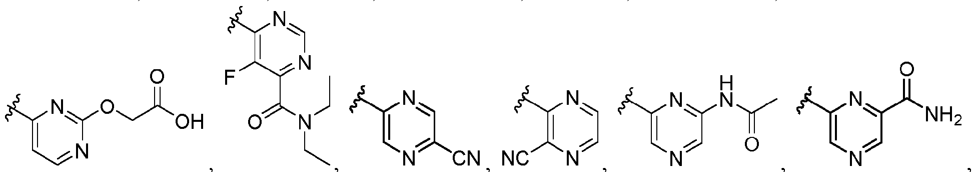

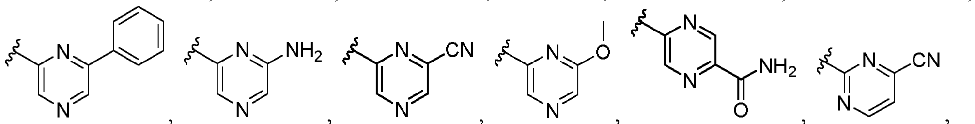

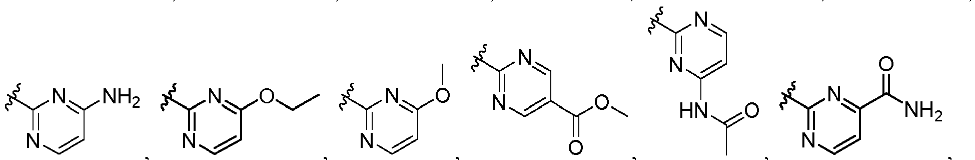

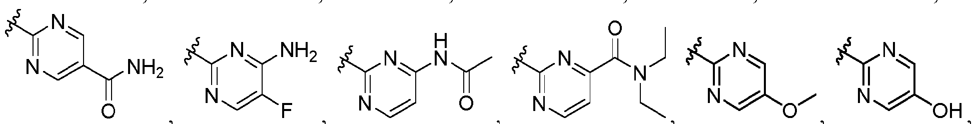

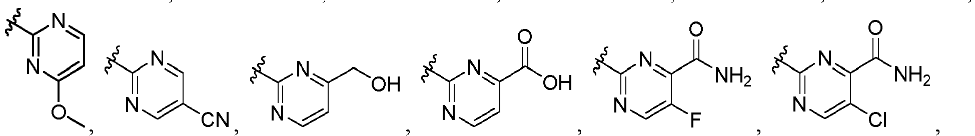

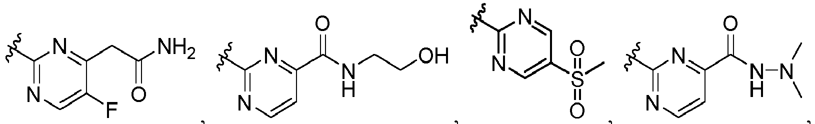

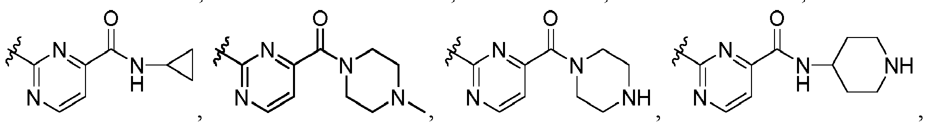

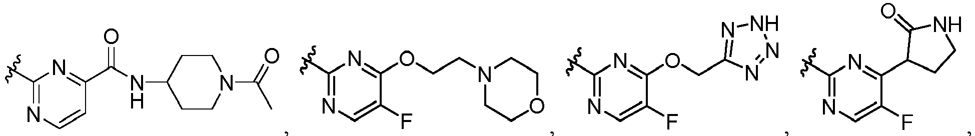

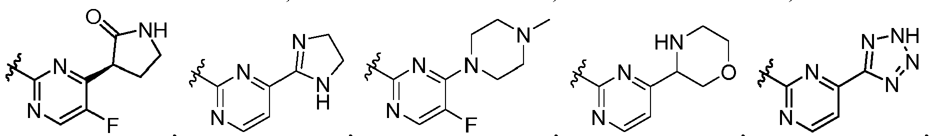

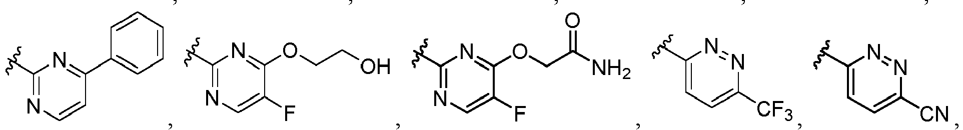

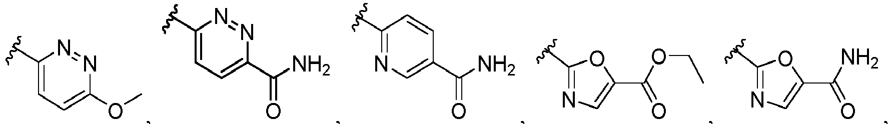

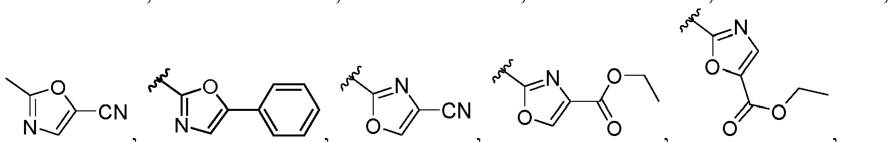

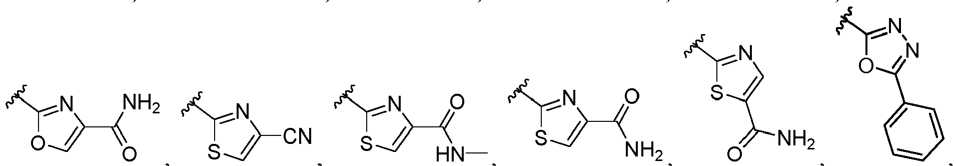

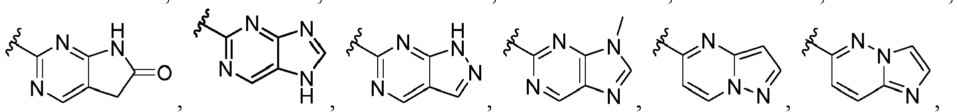

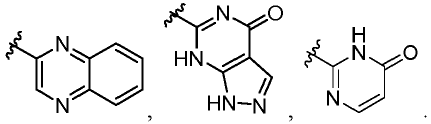

25. The compound or a pharmaceutically acceptable salt, stereoisomer, solvate or prodrug thereof according to claim 1, 9 or 10, **characterized in that**, m1 and m2 is 1.

26. The compound or a pharmaceutically acceptable salt, stereoisomer, solvate or prodrug thereof according to claim 1, 9 or 10, **characterized in that**, X₁ is N; X₂ is N or CH.

27. The compound or a pharmaceutically acceptable salt, stereoisomer, solvate or prodrug thereof according to claim 1, **characterized in that**, Rₐ and R_{b} together with the carbon atom attached thereto form a 3- to 6-membered saturated or partially unsaturated monocyclic ring selected from a group consisting of: cyclopropyl ring, cyclobutyl ring, cyclopentyl ring, cyclopentenyl ring, cyclohexyl ring, cyclohexenyl ring, cyclohexdienyl ring, cyclobutanone, cyclobutan-1,2-dione, cyclopentanone, cyclopentan-1,3-dione, cyclohexanone and cyclohexan-1,3-dione.

28. The compound or a pharmaceutically acceptable salt, stereoisomer, solvate or prodrug thereof according to claim 1, **characterized in that**, Rₐ and R_{b} together with the carbon atom attached thereto form a cyclopropyl ring.

29. The compound or a pharmaceutically acceptable salt, stereoisomer, solvate or prodrug thereof according to claim 1, **characterized in that**, Rₐ and R_{b} together with the carbon atom attached thereto form a 3- to 6-membered saturated or partially unsaturated monocyclic heterocyclyl ring selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran.

30. The compound or a pharmaceutically acceptable salt, stereoisomer, solvate or prodrug thereof according to any one of claims 1 and 11-15, **characterized in that**, the compound of formula (I) is selected from the group consisting of the compounds as prepared in the Examples of the present application.

31. A pharmaceutical composition, comprising the compound or a pharmaceutically acceptable salt, stereoisomer, solvate or prodrug thereof according to any one of claims 1-30; and a pharmaceutically acceptable carrier.

32. Use of the compound or a pharmaceutically acceptable salt, stereoisomer, solvate or prodrug thereof according to any one of claims 1-30 or the pharmaceutical composition according to claim 31 in the preparation of a medicament for preventing and/or treating a disease, the disease is selected from the group consisting of: inflammatory bowel disease, ulcerative colitis, Crohn's disease, psoriasis, rheumatoid arthritis, NASH and heart failure.

33. Use of the compound or a pharmaceutically acceptable salt, stereoisomer, solvate or prodrug thereof according to any one of claims 1-30 or the pharmaceutical composition according to claim 31 in the preparation of a selective RIPK1 inhibitor, the selective RIPK1 inhibitor is useful in treating a RIPK1-related disease or disorder.

34. Use of the compound or a pharmaceutically acceptable salt, stereoisomer, solvate or prodrug thereof according to any one of claims 1-30 or the pharmaceutical composition according to claim 31 in the preparation of a medicament for preventing and/or treating tumor or cancer, the tumor or cancer is selected from the group consisting of: colorectal cancer, multiple myeloma, lung cancer, bone cancer, head or neck cancer, pancreatic cancer, bile duct cancer, prostate cancer, skin cancer, skin or intraocular malignant melanoma, uterine cancer, ovarian cancer, rectal cancer, anal region cancer, stomach cancer, testicular cancer, breast cancer, uterine cancer, fallopian tube cancer, endometrial cancer, cervical cancer, vaginal cancer, vulva cancer, Hodgkin's disease, non-Hodgkin's lymphoma, esophageal cancer, small intestinal carcinoma, endocrine system cancer, thyroid cancer, parathyroid carcinoma, adrenal cancer, soft tissue sarcoma, urethra cancer, penile cancer, chronic or acute leukemia, including acute myeloid leukemia, chronic myeloid leukemia, acute lymphocytic leukemia, chronic lymphocytic leukemia, pediatric solid tumor, lymphocytic lymphoma, bladder cancer, renal or ureteral cancer, renal pelvis cancer, central nervous system (CNS) tumor, primary CNS lymphoma, tumor angiogenesis, spinal tumor, brainstem glioma, pituitary adenoma, Kaposi's sarcoma, epidermoid carcinoma, squamous cell carcinoma, T-cell lymphoma, environmentally induced cancers, including asbestos-induced cancers, and a combination of the cancers.
